# EUROPEAN PATENT SPECIFICATION

(11) **EP 4 118 200 B1**
(45) Date of publication and mention of the grant of the patent: **08.10.2025**
(21) Application number: 21752123.6
(22) Date of filing: 29.07.2021
(51) Int. Cl.: C12N 9/24, A61K 38/47, A61K 38/00

(54) **MUTATED BETA-GLUCOCEREBROSIDASE WITH IMPROVED STABILITY**
MUTIERTE BETA-GLUKOZEREBROSIDASE MIT VERBESSERTER STABILITÄT
BÊTA-GLUCOCÉRÉBROSIDASE MUTÉE À STABILITÉ AMÉLIORÉE

(30) Priority: 29.07.2020 GB 202011813; 18.01.2021 GB 202100648; 26.04.2021 GB 202105924
(43) Date of publication of application: 18.01.2023
(60) Divisional application: 25199900.9
(73) Proprietor: Spur Therapeutics Limited, Stevenage, Hertfordshire SG1 2BP (GB)
(72) Inventor: COMPER, Fabrizio, Stevenage, Hertfordshire SG1 2BP (GB); NATHWANI, Amit, Stevenage, Hertfordshire SG1 2BP (GB); MCINTOSH, Jenny, Stevenage, Hertfordshire SG1 2BP (GB); CORBAU, Romuald, Stevenage, Hertfordshire SG1 2BP (GB); KIA, Azadeh, Stevenage, Hertfordshire SG1 2BP (GB); MIRANDA, Carlos, Stevenage, Hertfordshire SG1 2BP (GB)
(74) Representative: J A Kemp LLP
(86) International application number: PCT/GB2021/051969
(87) International publication number: WO 2022/023761

(56) References cited:
- WO-A1-2020/157248
- WO-A2-01/49830
- WO-A2-2012/064709
- WO-A2-2012/064709
- WO-A2-2012/064709
- US-A1- 2016 237 414
- DOUGLAS B CRAIG ET AL: "Disulfide by Design 2.0: a web-based tool for disulfide engineering in proteins", BMC BIOINFORMATICS, BIOMED CENTRAL , LONDON, GB, vol. 14, no. 1, 1 December 2013 (2013-12-01), pages 346, XP021171914, ISSN: 1471-2105, DOI: 10.1186/1471-2105-14-346
- ALAN A DOMBKOWSKI ET AL: "Protein disulfide engineering", FEBS LETTERS, ELSEVIER, AMSTERDAM, NL, vol. 588, no. 2, 26 November 2013 (2013-11-26), pages 206 - 212, XP071253980, ISSN: 0014-5793, DOI: 10.1016/J.FEBSLET.2013.11.024
- SALAM NOERIS K. ET AL: "Structure-based approach to the prediction of disulfide bonds in proteins", PROTEIN ENGINEERING, DESIGN AND SELECTION, vol. 27, no. 10, 8 May 2014 (2014-05-08), GB, pages 365 - 374, XP093072375, ISSN: 1741-0126, DOI: 10.1093/protein/gzu017
- COMPER FABRIZIO ET AL: "Generation of [beta]-Glucocerebrosidase variants with increased half-life in human plasma for liver directed AAV gene therapy aimed at the treatment of Gaucher disease type 1", MOLECULAR GENETICS AND METABOLISM, ACADEMIC PRESS, AMSTERDAM, NL, vol. 132, no. 2, 1 February 2021 (2021-02-01), XP086480780, ISSN: 1096-7192, [retrieved on 20210201], DOI: 10.1016/J.YMGME.2020.12.047
- SMITH LAURA ET AL: "Insights into the structural biology of Gaucher disease", EXPERIMENTAL NEUROLOGY, vol. 298, 18 September 2017 (2017-09-18), pages 180 - 190, XP085261722, ISSN: 0014-4886, DOI: 10.1016/J.EXPNEUROL.2017.09.010
- DOUGLAS B CRAIG ET AL: "Disulfide by Design 2.0: a web-based tool for disulfide engineering in proteins", BMC BIOINFORMATICS, BIOMED CENTRAL , LONDON, GB, vol. 14, no. 1, 1 December 2013 (2013-12-01), pages 346, XP021171914, ISSN: 1471-2105, DOI: 10.1186/1471-2105-14-346
- ALAN A DOMBKOWSKI ET AL: "Protein disulfide engineering", FEBS LETTERS, ELSEVIER, AMSTERDAM, NL, vol. 588, no. 2, 26 November 2013 (2013-11-26), pages 206 - 212, XP071253980, ISSN: 0014-5793, DOI: 10.1016/J.FEBSLET.2013.11.024
- SALAM NOERIS K. ET AL: "Structure-based approach to the prediction of disulfide bonds in proteins", PROTEIN ENGINEERING, DESIGN AND SELECTION, vol. 27, no. 10, 8 May 2014 (2014-05-08), GB, pages 365 - 374, XP93072375, ISSN: 1741-0126, DOI: 10.1093/protein/gzu017
- COMPER FABRIZIO ET AL: "Generation of [beta]-Glucocerebrosidase variants with increased half-life in human plasma for liver directed AAV gene therapy aimed at the treatment of Gaucher disease type 1", MOLECULAR GENETICS AND METABOLISM, ACADEMIC PRESS, AMSTERDAM, NL, vol. 132, no. 2, 1 February 2021 (2021-02-01), XP086480780, ISSN: 1096-7192, [retrieved on 20210201], DOI: 10.1016/J.YMGME.2020.12.047
- SMITH LAURA ET AL: "Insights into the structural biology of Gaucher disease", EXPERIMENTAL NEUROLOGY, vol. 298, 18 September 2017 (2017-09-18), pages 180 - 190, XP085261722, ISSN: 0014-4886, DOI: 10.1016/J.EXPNEUROL.2017.09.010
- SALAM NOERIS K. ET AL: "Structure-based approach to the prediction of disulfide bonds in proteins", PROTEIN ENGINEERING, DESIGN AND SELECTION, vol. 27, no. 10, 8 May 2014 (2014-05-08), pages 365 - 374, XP093072375, ISSN: 1741-0126, DOI: 10.1093/protein/gzu017
- DVIR HAY ET AL: "X-ray structure of human acid-beta-glucosidase, the defective enzyme in Gaucher disease", EMBO REPORTS, NATURE PUBLISHING GROUP, LONDON, GB, vol. 4, no. 7, 1 July 2003 (2003-07-01), pages 704 - 709, XP002436115, ISSN: 1469-221X, DOI: 10.1038/SJ.EMBOR.EMBOR873
- LIEBERMAN RAQUEL L.: "A Guided Tour of the Structural Biology of Gaucher Disease: Acid- [beta] -Glucosidase and Saposin C", ENZYME RESEARCH, vol. 2011, 22 November 2011 (2011-11-22), pages 1 - 15, XP055812098

## Description

### Field of the invention

The present invention relates to a modified β-Glucocerebrosidase (GCase) polypeptide and a polynucleotide comprising a modified glucocerebrosidase (GBA) nucleotide sequence. The invention further relates to a viral particle comprising a recombinant genome comprising the polynucleotide of the invention, and a composition comprising the modified GCase polypeptide, polynucleotide, or viral particle of the invention. The invention also relates to methods of using, and uses of, the modified GCase polypeptide, polynucleotide, viral particle and/or composition of the invention. The invention further relates to the modified GCase polypeptide, polynucleotide, viral particle, or composition of the invention for use in a method of treatment.

### Background of the invention

Gaucher disease (GD) is an autosomal recessive lipid storage disease characterised by the deposition of glucocerebroside in cells of the macrophage-monocyte system. GD is caused by mutations in the housekeeping GBA gene that impairs activity and/or production of the enzyme β-Glucocerebrosidase (GCase). GCase is an enzyme with glucosylceramidase activity (EC 3.2.1.45) that hydrolyses the beta-glucosidic linkage of the chemical glucocerebroside, an intermediate in glycolipid metabolism that is abundant in cell membranes. The mutations result in the production of a misfolded GCase having reduced activity and can lead to an accumulation of glucocerebrosides in macrophages that infiltrate many vital organs, which manifests as GD.

There are three major types of GD which are characterised by the specific mutations which have been identified, and each type can display differing clinical symptoms. Type 1 GD has little or no involvement with the central nervous system but is mainly characterised by visceral manifestations such as enlarged spleen and liver, low blood cell counts, bleeding problems and bone disease. For the past 20 years, enzyme replacement therapy has emerged as the standard of care for type 1 GD. In addition to its high cost (~$200,000 or ~£150,000/patient/year), enzyme replacement therapy treatment in GD generally requires one or more injections every other week for life. This leads to a high proportion of GD patients displaying high levels of treatment burden.

Accordingly, there is a need to provide improved and effective therapy for the treatment of GD, *i.e.* one that increases the availability of functioning GCase to affected target organs, which would avoid the need for frequent and lifelong intravenous injections of GCase.

D1 (WO 2012/064709 A2) relates to variant, recombinant beta-gluococerebrosidase proteins with increased stability and increased retained catalytic activity having amino acid variations at specific positions.

### Summary of the invention

The invention is defined by the claims. Further aspects and preferred embodiments are defined in the dependent claims. Any aspects, embodiments and examples of the present disclosure which do not fall under the scope of the appended claims do not form part of the claimed invention and are merely provided for illustrative purposes.

The present invention provides a modified β-Glucocerebrosidase (GCase) polypeptide which comprises at least one mutation, wherein the at least one mutation comprises:
(i) a mutation at a position corresponding to position 351 of SEQ ID NO: 1 that is a substitution with cysteine; and
(ii) a mutation at a position corresponding to position 380 of SEQ ID NO: 1 that is a substitution with cysteine,

wherein the modified GCase polypeptide comprises an amino acid sequence that is at least 90% identical to SEQ ID NO: 2, and
wherein the modified GCase polypeptide has increased stability and has higher effective activity relative to a reference GCase polypeptide, wherein the reference GCase polypeptide is the polypeptide of SEQ ID NO: 3, 4, or 5.

The present invention also provides a polynucleotide comprising a modified glucocerebrosidase (GBA) nucleotide sequence, wherein the modified GBA nucleotide sequence encodes the modified GCase polypeptide of the invention.

The present invention further provides a viral particle comprising a recombinant genome comprising the polynucleotide of the invention.

The present invention also provides a composition comprising the modified GCase polypeptide, polynucleotide, or viral particle of the invention and a pharmaceutically acceptable excipient.

The present invention further provides the modified GCase polypeptide, polynucleotide, viral particle, or composition of the invention for use in a method of treatment, wherein:
(i) the method of treatment is a method of treating a synucleinopathy; or
(ii) the method of treatment is a method of treating Parkinson's disease; or
(iii) the method of treatment is a method of treating Gaucher disease, optionally wherein the Gaucher disease is Gaucher disease type I, II or III; or
(iv) the method of treatment is a method of treating Gaucher disease and wherein the patient has antibodies or inhibitors to a recombinant GCase with which the patient has previously been treated as part of an enzyme replacement therapy, optionally wherein the Gaucher disease is Gaucher disease type I, II or III.

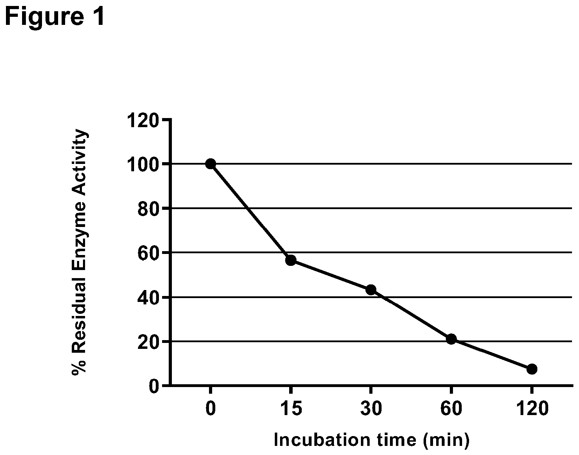

GCase is a lysosomal protein which normally functions and is stable at an acidic pH. It is usually unstable at physiological pH (*e.g.* pH 7.4) with a half-life of only a few minutes. For example, see Example 2 and Figure 1 showing that the activity of VPRIV (velaglucerase alfa, a commercially available ERT which is a recombinant GCase having an identical amino acid sequence as a naturally-occurring human GCase polypeptide) dramatically reduces following incubation at pH 7.4. SEQ ID NO: 3 comprises the sequence of velaglucerase alfa. Existing enzyme replacement treatment (ERT) is the standard treatment approach for those with GD. In ERT, the GCase enzymes are usually provided intravenously, which means that such treatments also encounter issues with effective delivery of the GCase enzymes to the target organs due to the distance that they need to travel and their instability in blood. The present inventors have developed modified GCase polypeptides with increased stability. The present inventors have identified amino acid substitutions in the GCase polypeptide that are able to increase the stability of the GCase polypeptide.

Without wishing to be bound by theory, it is believed that the instant amino acid mutations may serve to enhance the stability at physiological pH (*e.g.* pH 7.4), thereby extending the time for which the GCase is stable, and thus provide higher effective activity. The modified GCase polypeptide according to the invention may have a higher residual activity than a wild-type GCase polypeptide under various pHs (for example see Examples 3 and 4). The modified GCase polypeptide according to the invention may have a longer half-life under various pHs than a wild-type GCase polypeptide (for example see Example 5). The modified GCase polypeptide according to the invention may show a higher GCase effective activity than a wild-type GCase polypeptide (for example see Examples 8 and 9). The modified GCase polypeptide of the invention may have improved thermostability (for example see Example 12). The modified GCase polypeptide according to the invention may be provided as a protein for enzyme replacement therapy. Also, the modified GCase polypeptide of the invention may be provided by expression in the liver (such as in the form of gene therapy) and allowing the protein to be transported through blood to reach the target organs. Provision of the modified GCase polypeptide according to the invention (such as in the form of gene therapy) may result in a higher uptake in relevant tissues than provision of a wild-type GCase polypeptide (for example see Example 10). The present inventors have also found that the modified GCase polypeptide according to the invention may have a reduced potential immunogenicity risk compared to a wild type GCase polypeptide. The modified GCase polypeptide according to the invention may have a lower predicted number of "strong" binders to HLA-I and a lower predicted number of strong binders to HLA-II (for example see Example 7).

By extending the time for which the GCase is stable, and thus providing a higher effective activity, the modified GCase polypeptide of the invention may achieve a greater therapeutic effect when administered at a dose which is the same or similar to the dose of a GCase of lower stability (*e.g.* wild-type GCase). Furthermore, a lower dose of the modified GCase polypeptide of the invention may be able to achieve the same therapeutic effect as a GCase of lower stability (*e.g.* wild-type GCase) when the modified GCase polypeptide of the invention is administered at a lower dose than the GCase of lower stability. Thus, the costs associated with therapy may be reduced and the safety profile may be improved.

### Description of the figures

Figure 1 shows the enzymatic activity of VPRIV in PBS (pH 7.4). The residual enzymatic activity (residual GCase activity) was measured at 0, 15, 30, 60 and 120 minutes of incubation with PBS. The residual enzymatic activity at each time point was calculated as a percentage of the initial activity.

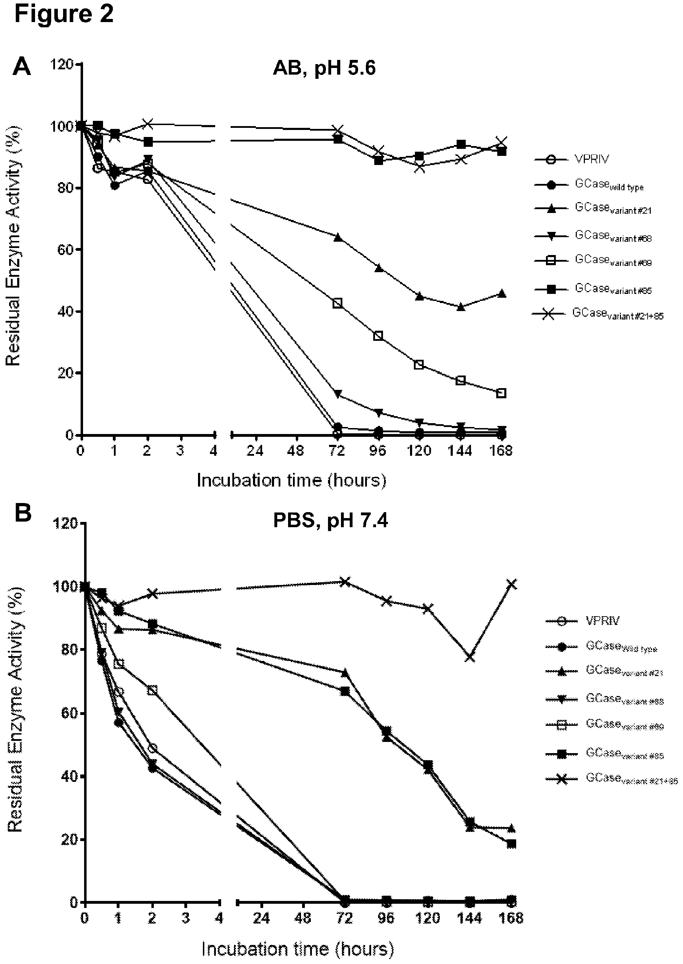

Figure 2 A and B provide a comparison of the residual enzymatic activity of various GCase variants (#21, #68, #69, #85, and #21+85, see Table 1), wild type GCase and VPRIV (velaglucerase alfa, a recombinant GCase having an identical amino acid sequence as naturally-occurring human GCase; VPRIV is a commercially available ERT) incubated under various conditions, *i.e.* in AB buffer (pH 5.6) (Figure 2A) and PBS buffer (pH 7.4) (Figure 2B) measured over 7 days at 37°C. The residual enzymatic activity (residual GCase activity) was measured after 0 min, 10 mins, 30 mins, 60 mins, 120 mins, 3 days, 4 days, 5 days, 6 day, and 7 days of incubation. The residual enzymatic activity at each time point was calculated as a percentage of the initial activity.

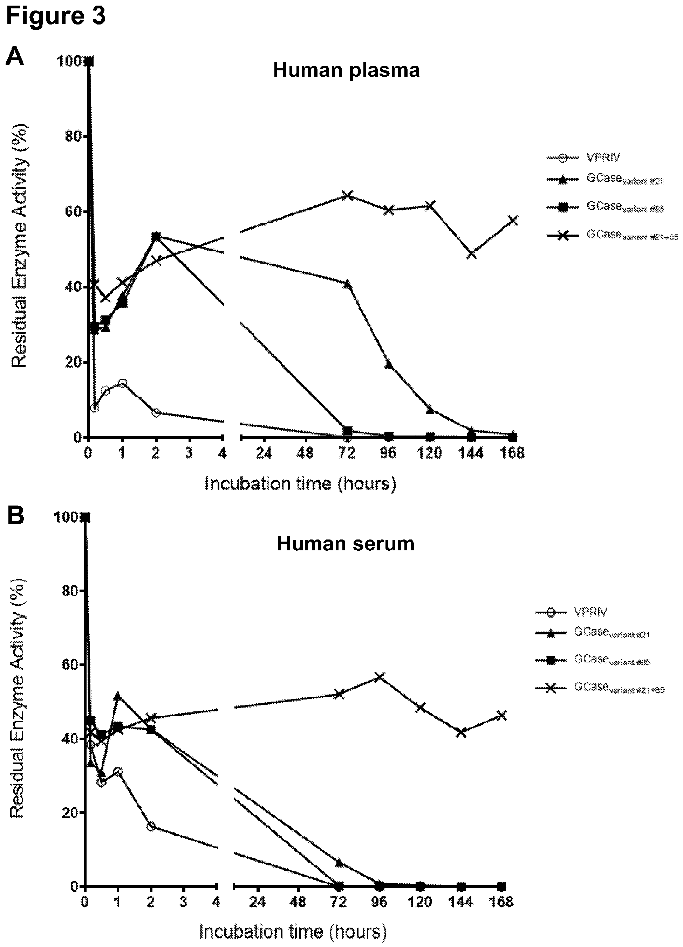

Figures 3A and B provide a comparison of the residual enzymatic activity of various GCase variants (#21, #85, and #21+85, see Table 1), wild type GCase and VPRIV (velaglucerase alfa, a recombinant GCase having an identical amino acid sequence as naturally-occurring human GCase; VPRIV is a commercially available ERT) incubated under various conditions, *i.e.* in human plasma (Figure 3A) and human serum (Figure 3B) measured over 7 days at 37°C. The residual enzymatic activity (residual GCase activity) was measured after 0 min, 10 mins, 30 mins, 60 mins, 120 mins, 3 days, 4 days, 5 days, 6 day, and 7 days of incubation. The residual enzymatic activity at each time point was calculated as a percentage of the initial activity.

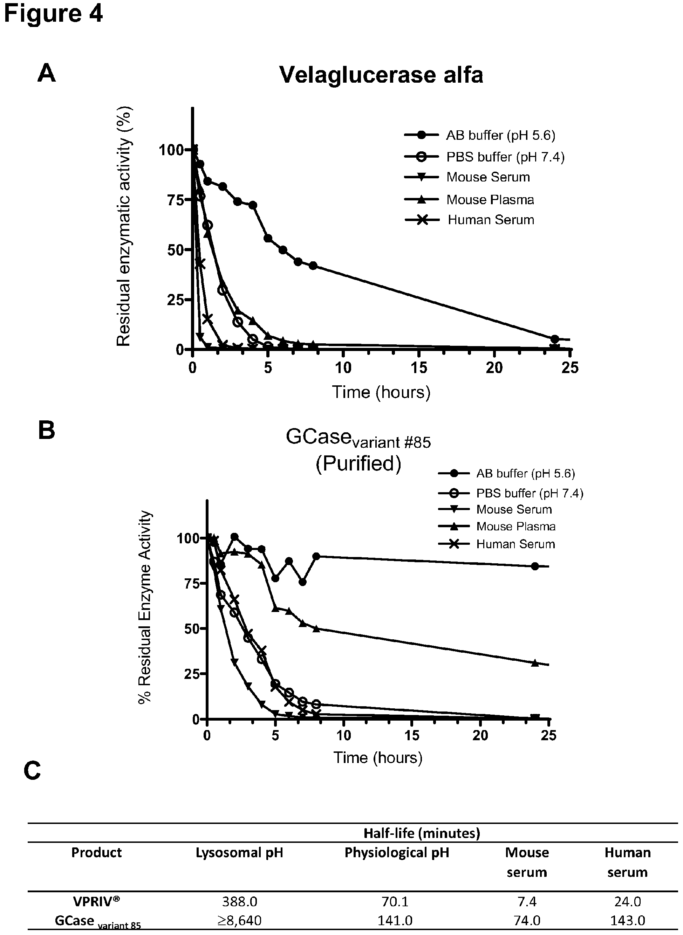

Figures 4A-C provides a comparison of the half-life of purified GCase variant #85 (Figure 4B) and velaglucerase alfa (VPRIV, a recombinant GCase having an identical amino acid sequence as naturally-occurring human GCase) (Figure 4A) in various physiological matrices, *i.e.* AB buffer (pH 5.6), PBS buffer (pH 7.4), mouse serum, mouse plasma and human serum. The residual enzymatic activity (residual GCase activity) was measured after 0, 0.5, 1, 2, 3, 4, 5, 6, 7, 8, 24, 48, 120, and 144 hours of incubation with the physiological matrix (not all time points shown). The residual enzymatic activity at each time point was calculated as a percentage of the initial activity. The half-life in minutes of the purified GCase variant #85 and the wild-type GCase (velaglucerase alfa) were determined (Figure 4C). The time calculated for the "lysosomal pH" corresponds to the results using AB buffer (pH 5.6) and the time calculated for "physiological pH" corresponds to the results using the PBS buffer (pH 7.4).

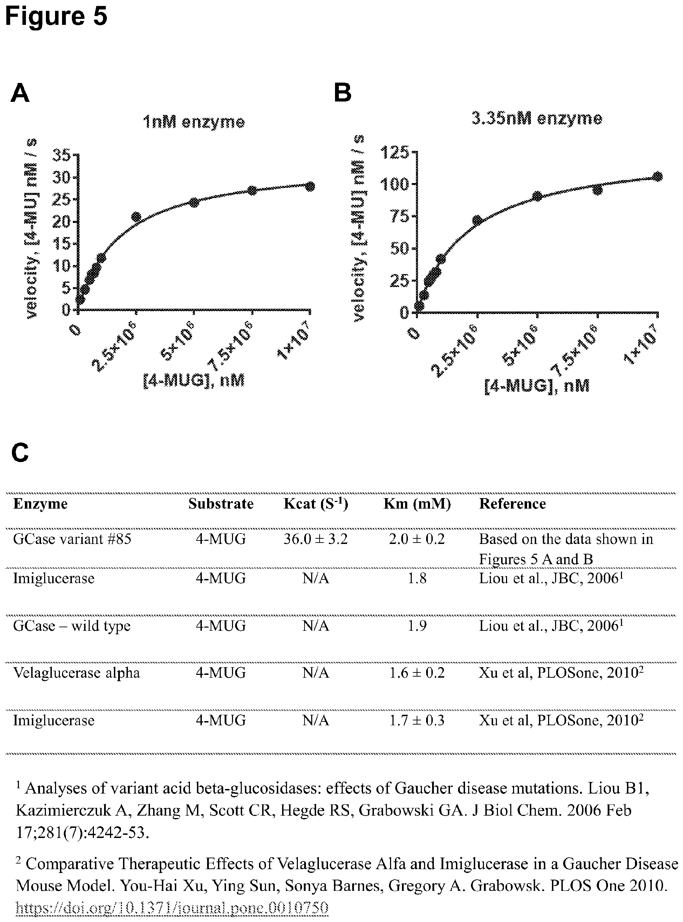

Figures 5A-C provide a comparison of the enzymatic efficiency (*i.e.* ability of the GCase to process a substrate) of the GCase variant #85, with the enzymatic efficiency reported by others for wild type GCase and the enzyme replacement therapies (ERTs) Imiglucerase and Velaglucerase alpha. The enzymatic efficiency was measured by processing 4-Methylumbelliferyl-β-D-glucopyranosiduronic acid (4-MUG). In Figures 5A and 5B, 1 nM and 3.35 nM, respectively, of the GCase variant #85 was incubated with 2.5 x 10⁶, 5 x 10⁶, 7.5 x 10⁶ or 1 x 10⁷ nM of 4-MUG. The 4-MU formation velocity for each 4-MUG concentration is plotted against 4-MUG concentration and fitted with the Michaelis-Menten model. The velocity is measured in nM per second. Figure 5C provides the Kcat (s⁻¹) and Km (mM) of the GCase variant #85 in addition to the Km (mM) values reported by others for wild type GCase and the enzyme replacement therapies (ERTs) Imiglucerase and Velaglucerase alpha.

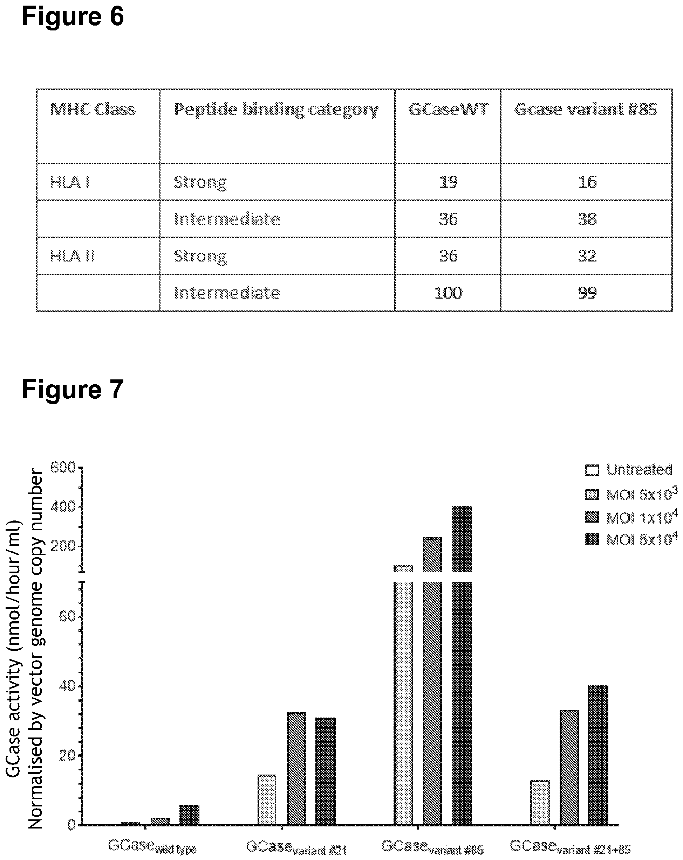

Figure 6 provides the results of an *in silico* assessment of the potential immunogenicity risk profile for the GCase variant #85. Fragments from the GCase variant #85 and wild type GCase (GCase_{WT}) were assessed for their predicted ability to bind HLA class I and HLA class II. MHC: major histocompatibility complex; HLA: human leukocyte antigen.
Figure 7 shows the effective GCase activity for a number of GCase variants and wild type GCase upon AAV2/'37' transduction of Huh-7 cells (human liver cell line). The effective GCase activity of each of the GCase variant #21, GCase variant # 85, and GCase variant #21+85 was tested. The effective GCase activity is presented in nmol/hour/ml based on a 4-MU standard curve. The MOI used for transduction were 5x10³, 1x10⁴ and 5x10⁴vg/cell, MOI: multiplicity of infection. An untreated control was included. The results were normalised by vector genome copy number (as determined according to the section entitled "*Vector genome copy number*" in Example 1). The normalisation was across the MOI groups to account for transduction efficiency. Where reference is made to *"GCase activity"* in the figure, *"effective GCase activity"* is meant.

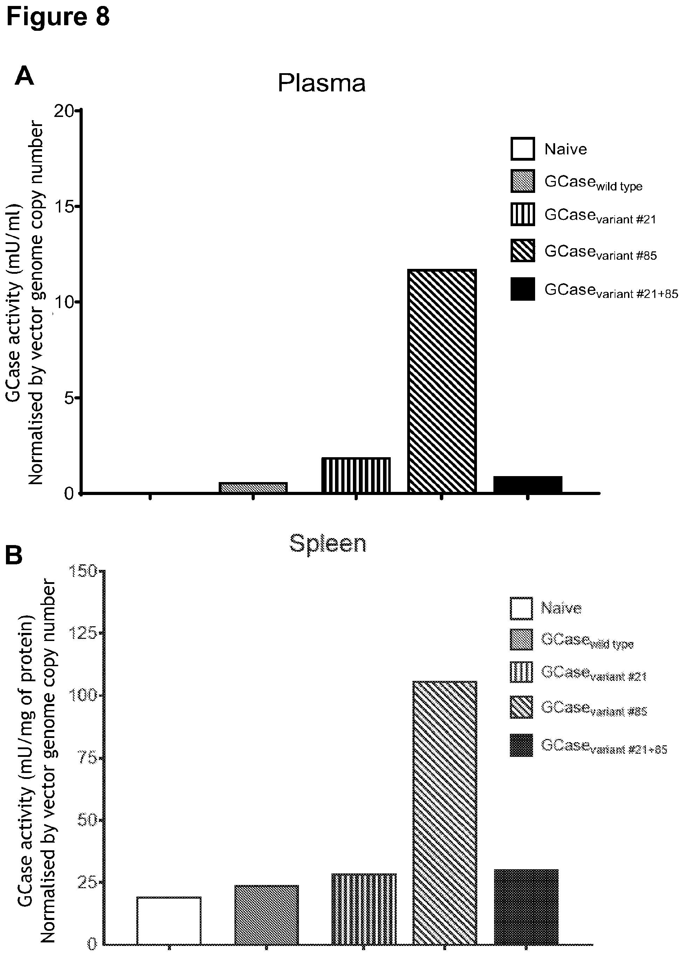

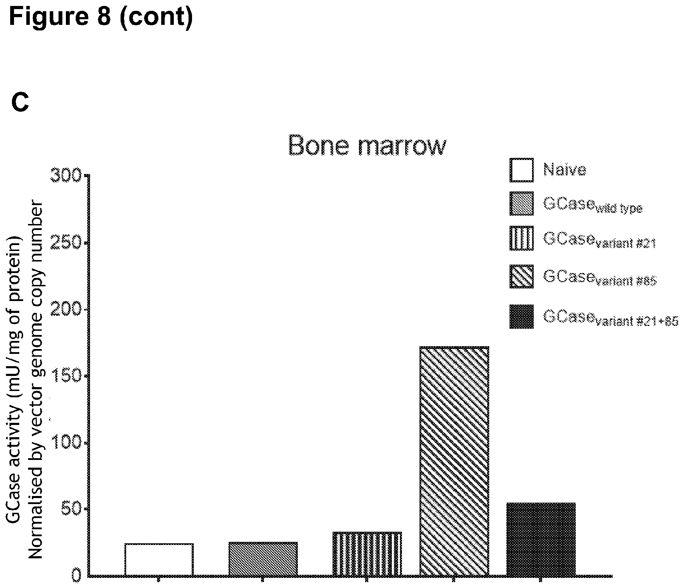

Figure 8A-C shows the effective GCase activity levels in plasma (Figure 8A), spleen (Figure 8B), and bone marrow (Figure 8C) following injection into wild type (C57BL/6) male mice of AAV2/8 viral particles expressing sequences encoding one of the GCase variants GCase variant #21, GCase variant #85 and GCase variant #21+85 or wild type GCase. Control (naive) mice were left untreated. The AAV2/8 viral particles were injected into mice at a dose of 6x10¹⁰ vg/kg. Animals were culled 4 weeks post-treatment. The level of effective GCase activity is presented in mU/ml of plasma or mU/mg of protein (spleen and bone marrow) according to a VPRIV standard curve. The results were normalised by vector genome copy number (as determined according to the section entitled "*Vector genome copy number*" in Example 1). Where reference is made to *"GCase activity"* in the figure, *"effective GCase activity"* is meant.

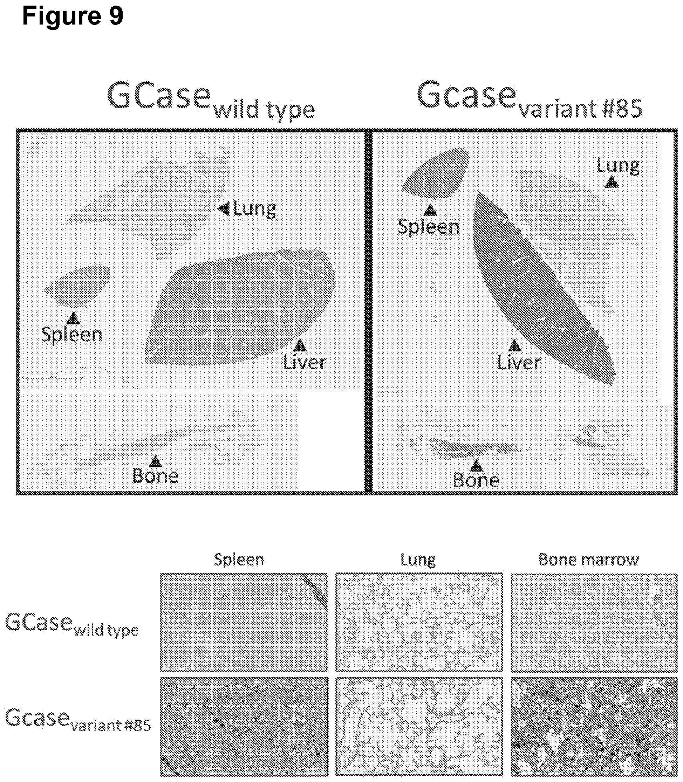

Figure 9 shows the levels of GCase in liver, spleen, lung and bone marrow following injection into wild type (C57BL/6) male mice of AAV2/8 viral particles expressing sequences encoding GCase variant #85 or wild type GCase as determined by immunohistochemical staining. Animals were culled 4 weeks post-treatment.

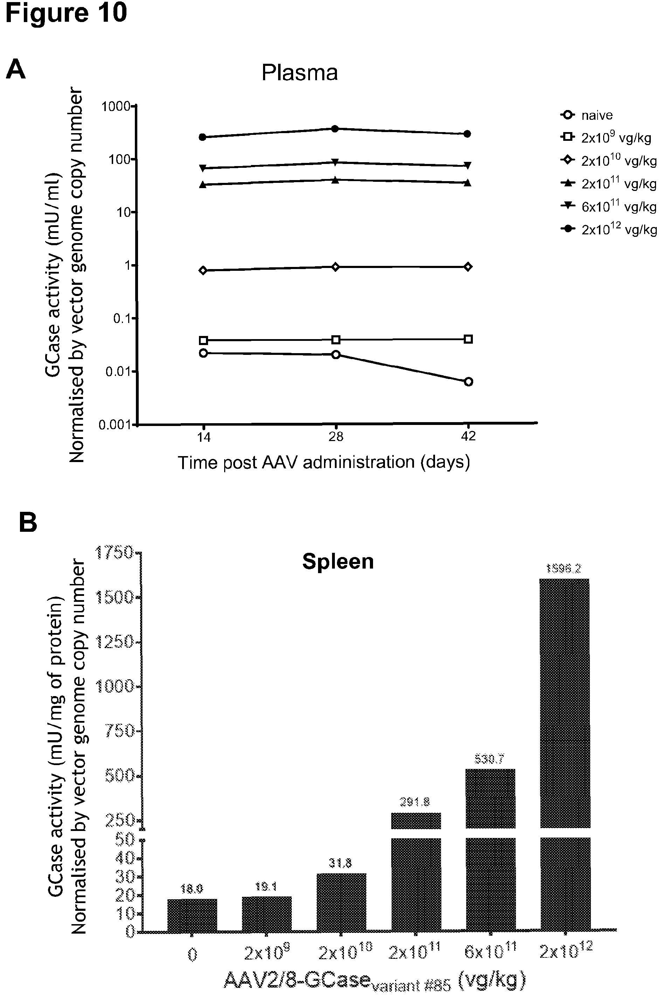

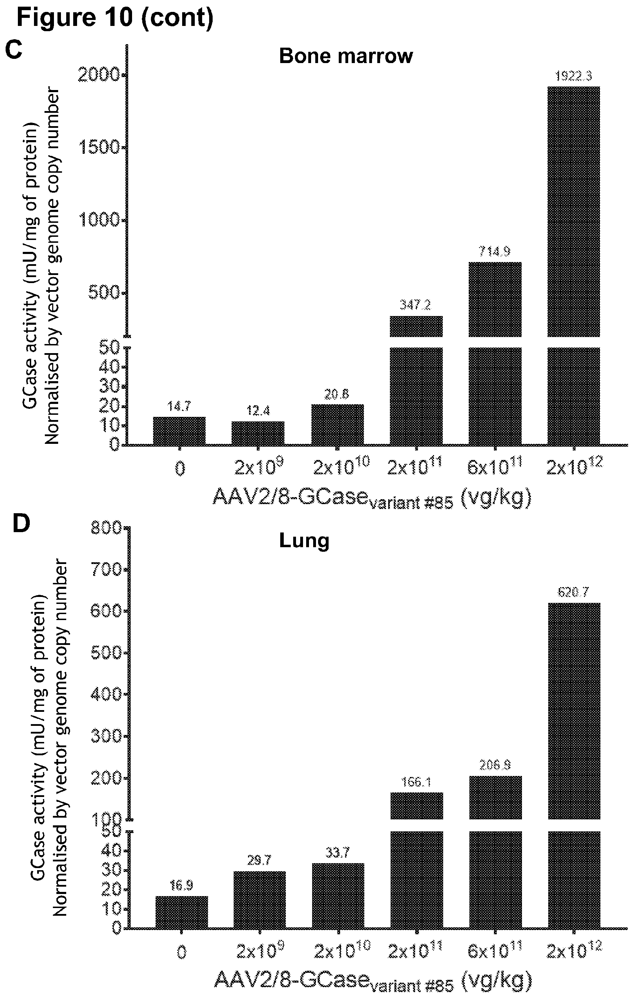

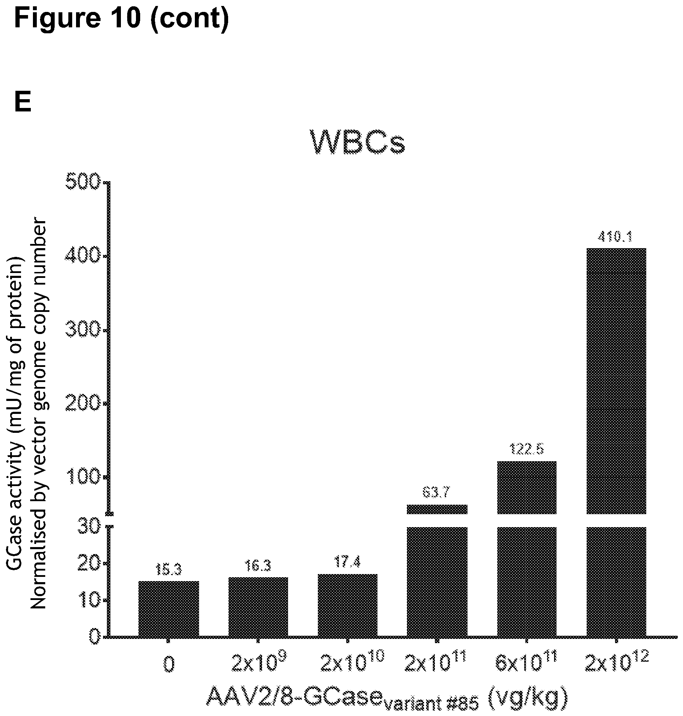

Figures 10 A-E show the GCase effective activity levels in plasma (Figure 10A), spleen (Figure 10B), bone marrow (Figure 10C), lung (Figure 10D) and white blood cells (WBCs) (Figure 10E) following injection into wild type (C57BL/6) male mice of AAV2/8 viral particles expressing sequences encoding GCase variant #85. Control (naive) mice were left untreated. The AAV2/8 viral particles were injected into mice at the following doses: 2x10⁹, 2x10¹⁰ , 2x10¹¹, 6x10¹¹ and 2x10¹² vg/kg. For Figure 10A, plasma was obtained 14, 28 and 42 days post-treatment. For Figures 10B to 10E, the animals were culled 6 weeks post-treatment. The level of effective GCase activity in plasma is presented in mU/ml of plasma, and the level of effective GCase activity in tissues and WBCs is presented in mU/mg of protein, which were all determined using a VPRIV standard curve. The results were normalised by vector genome copy number (as determined according to the the section entitled "*Vector genome copy number*" in Example 1). Where reference is made to *"GCase activity"* in the figure, *"effective GCase activity"* is meant.

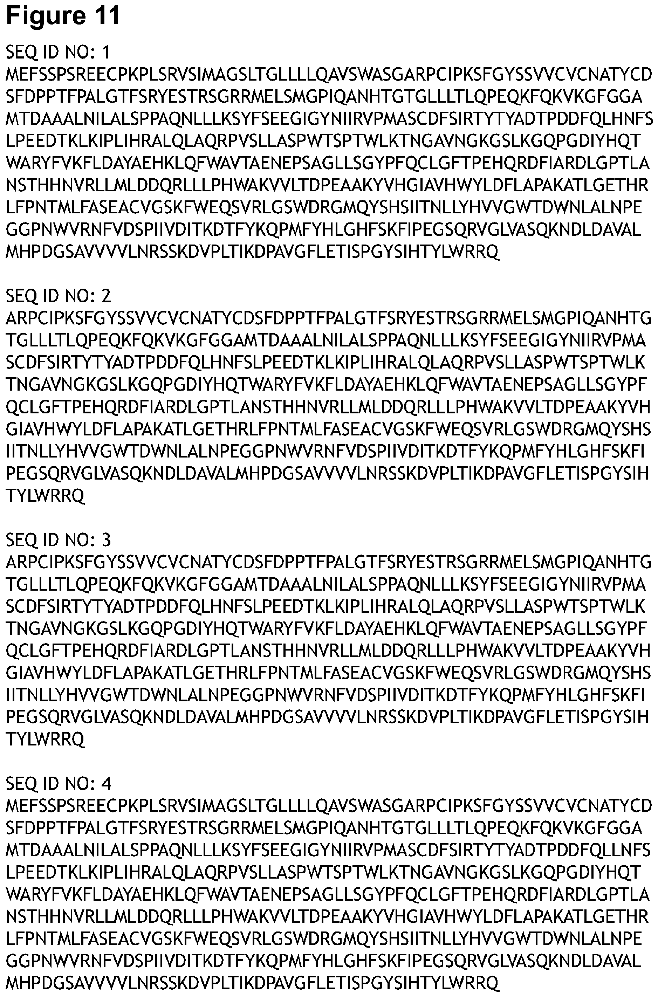

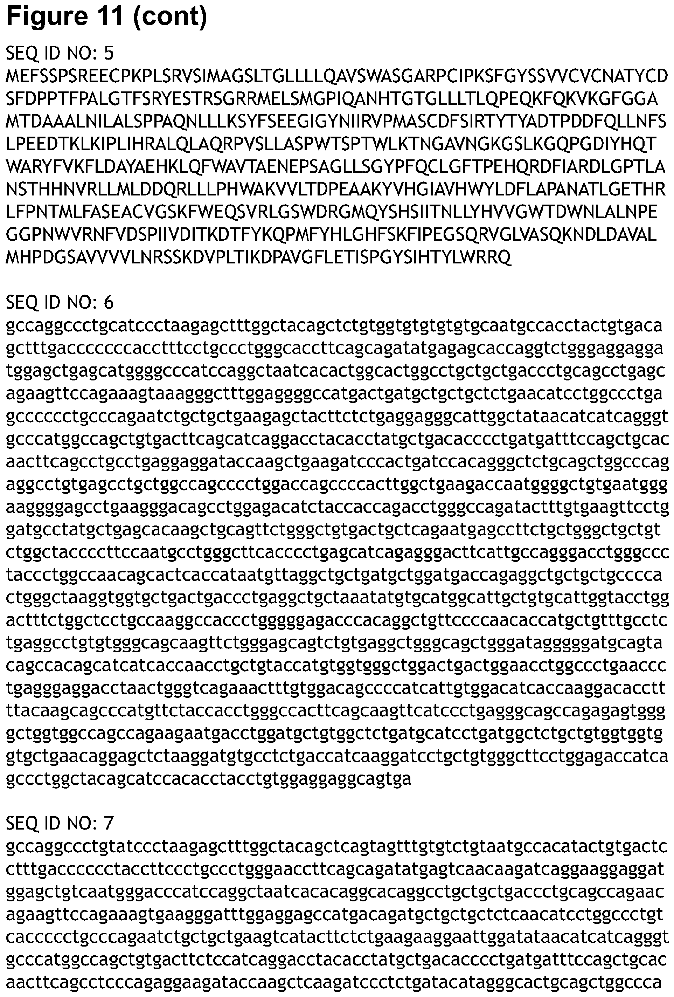

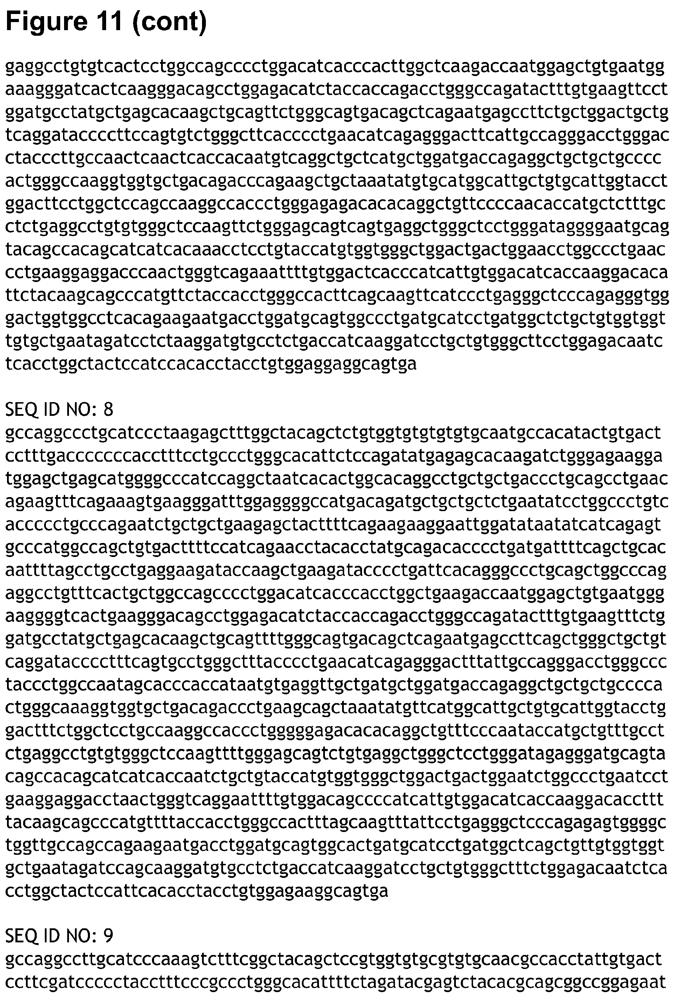

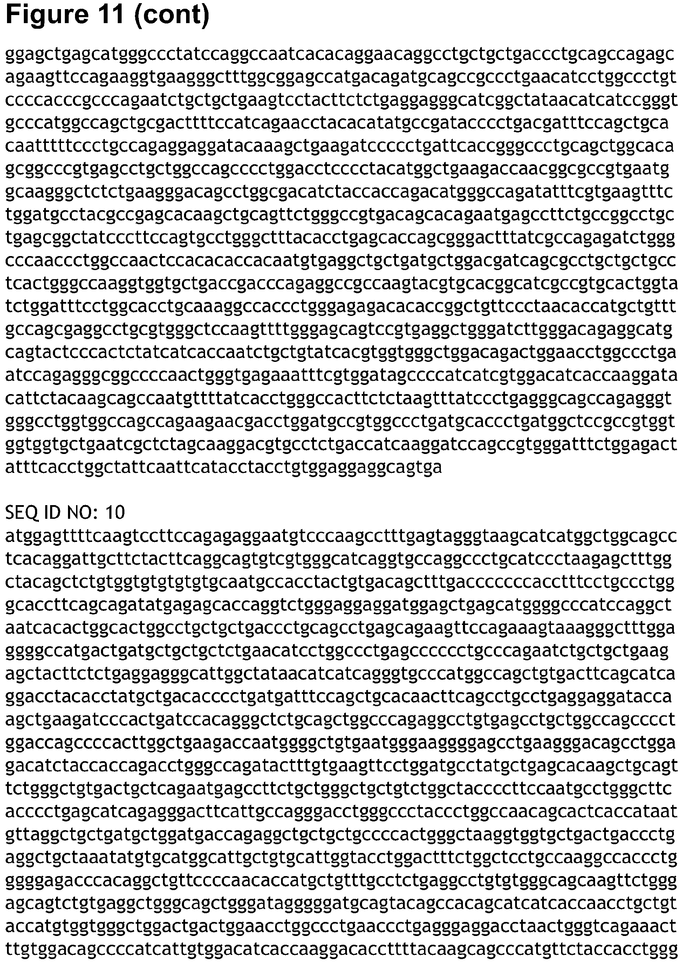

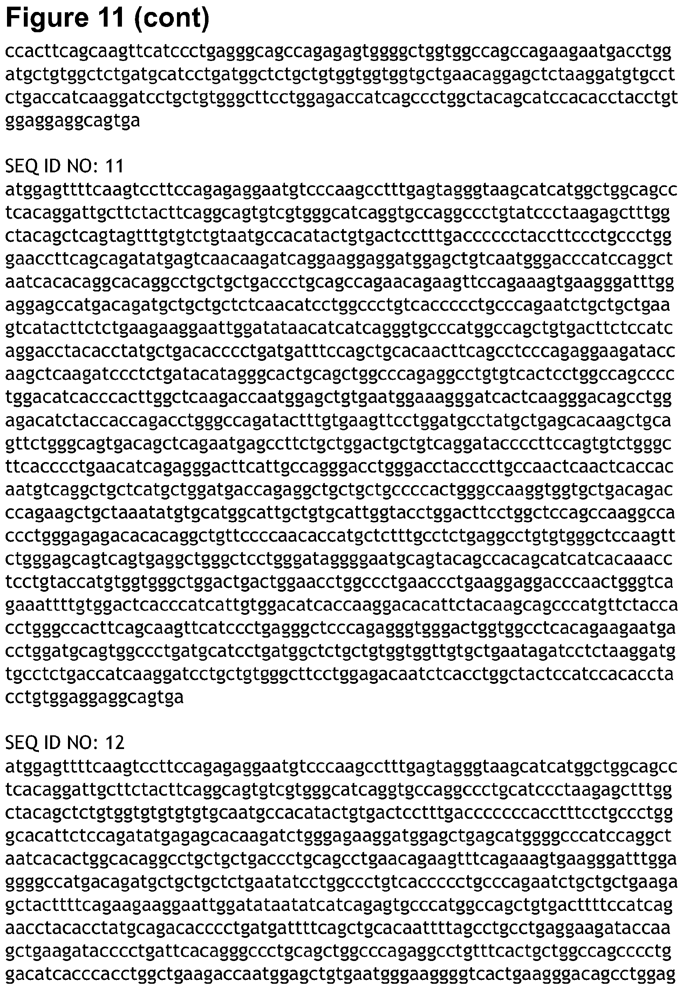

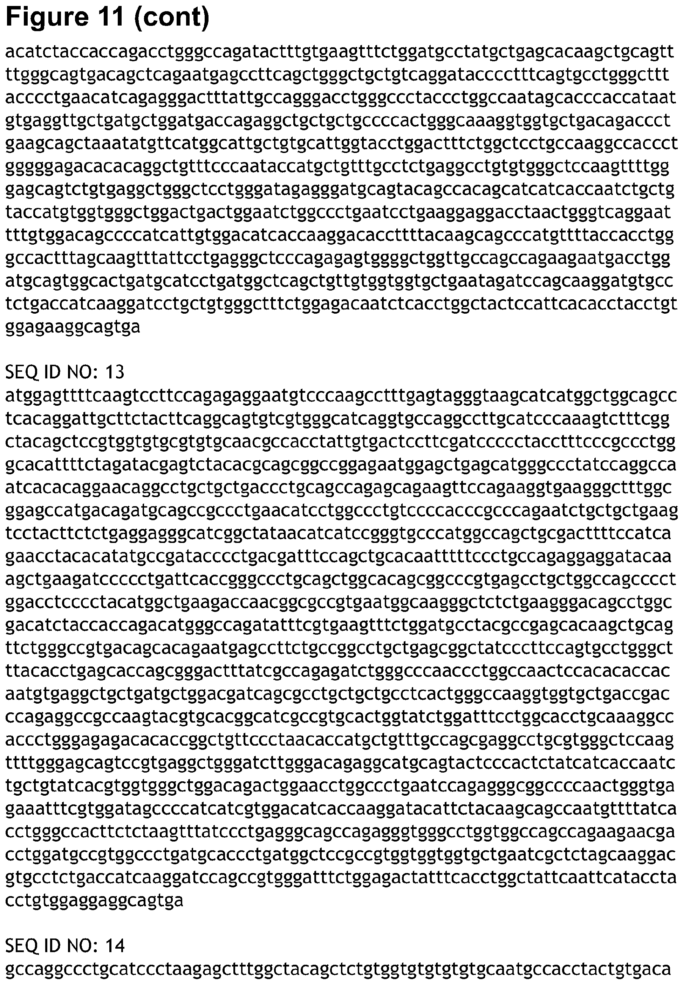

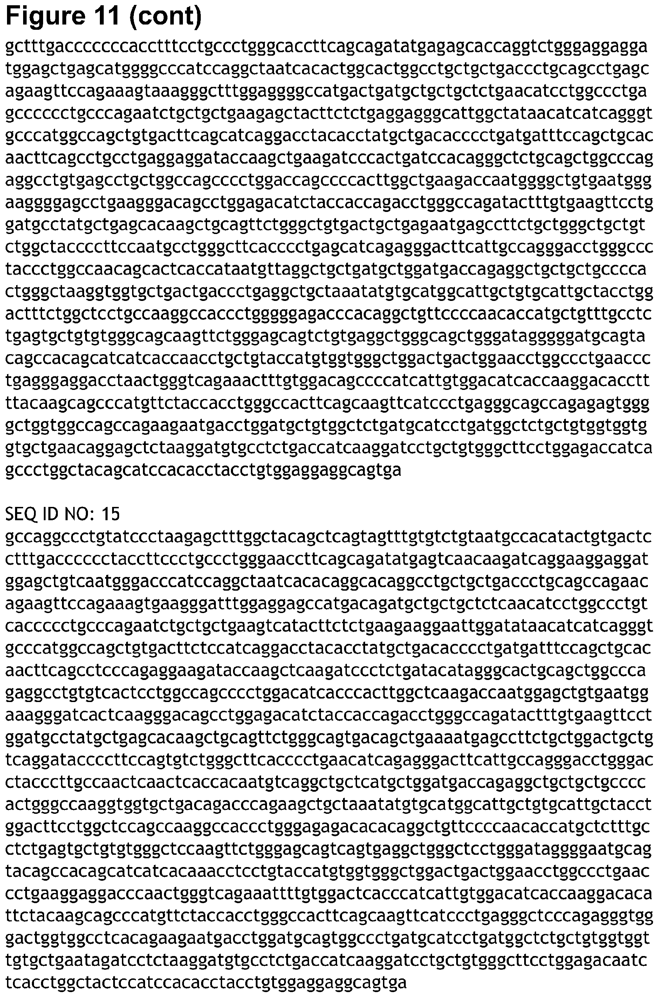

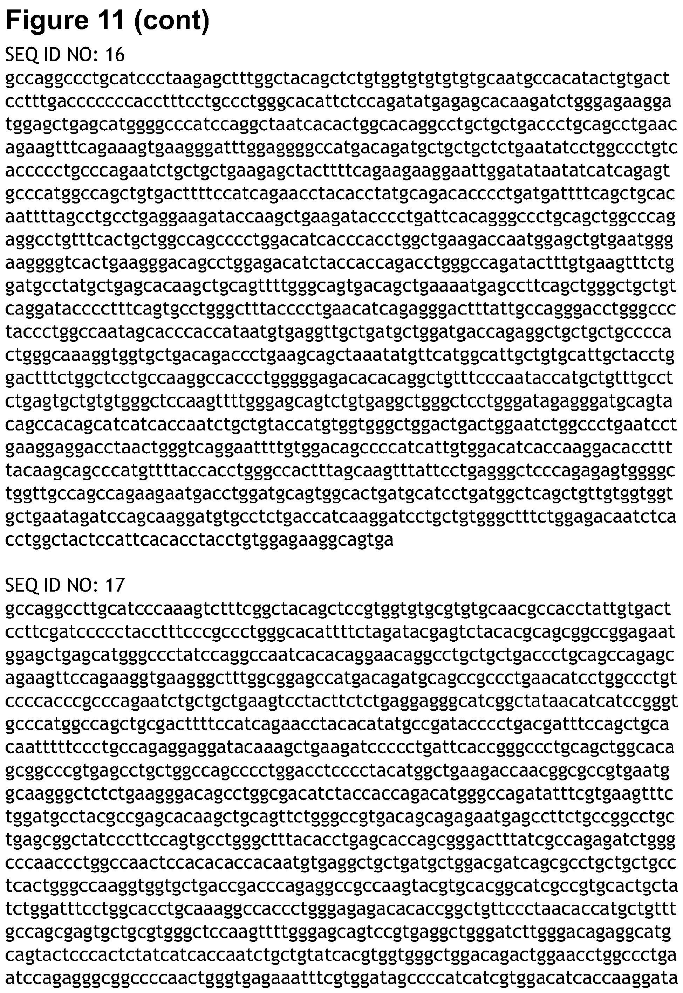

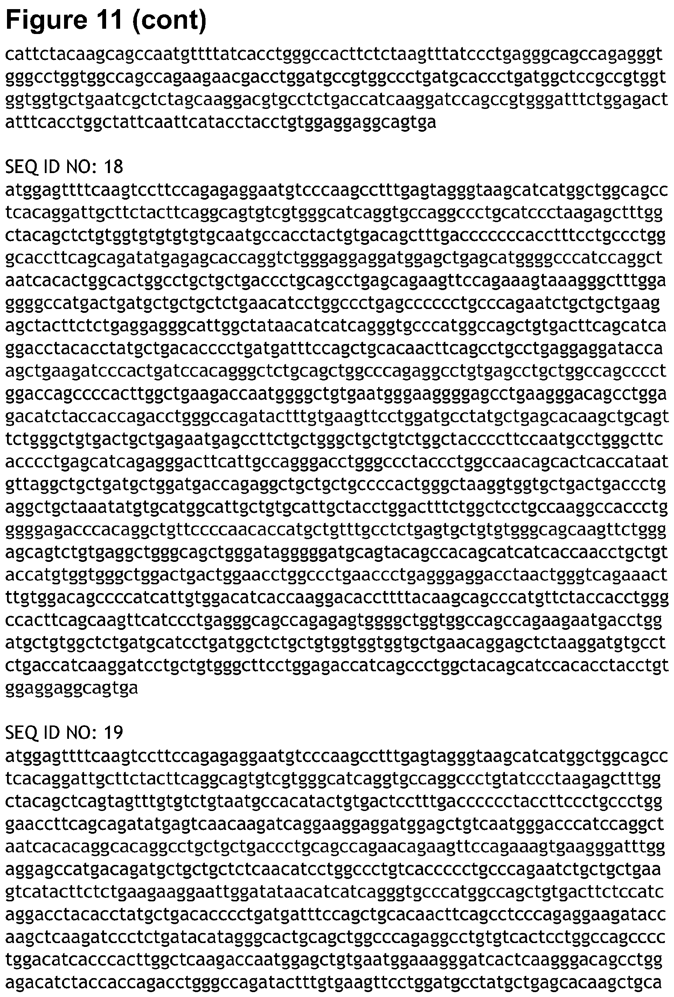

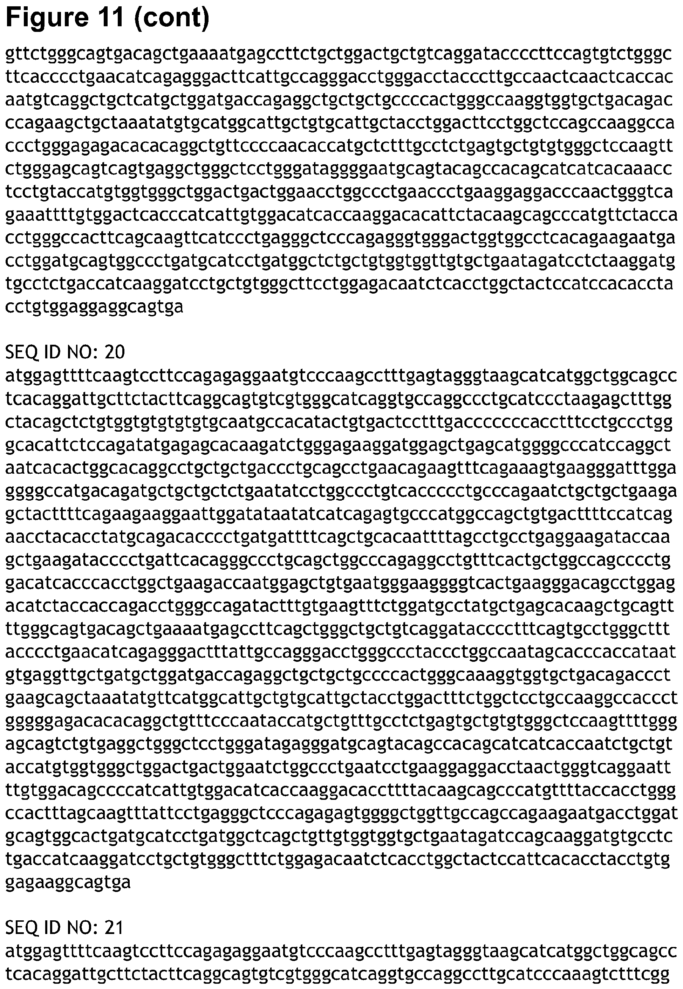

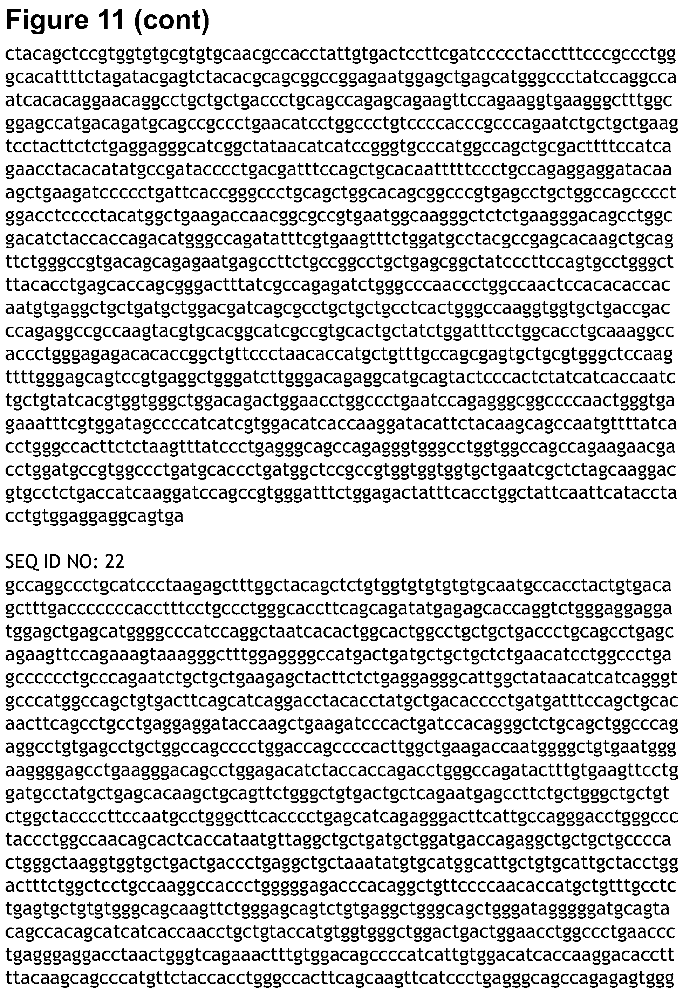

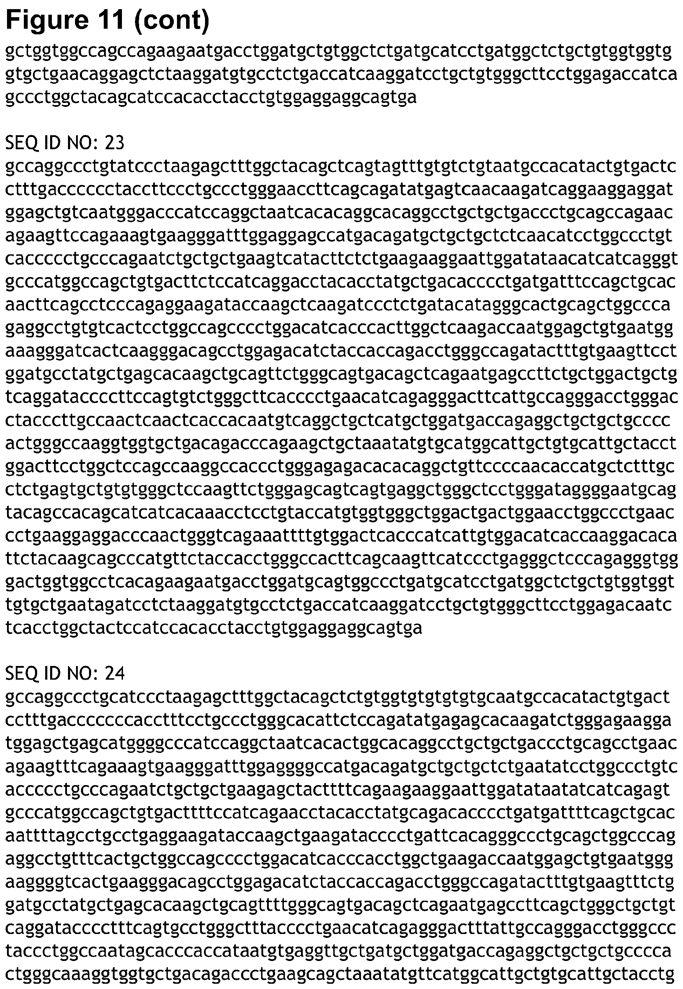

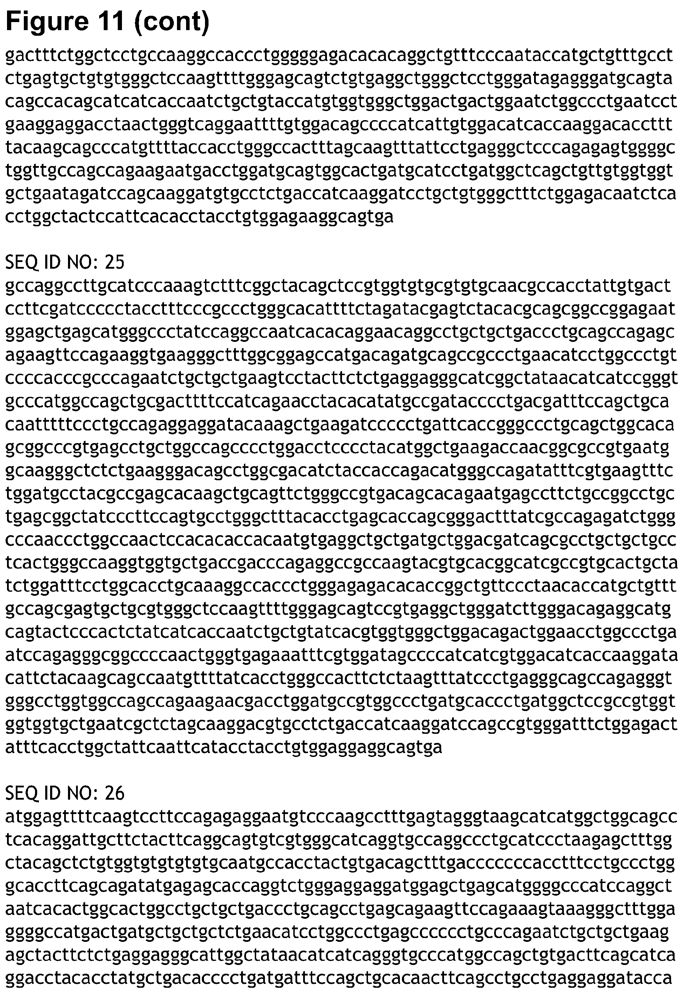

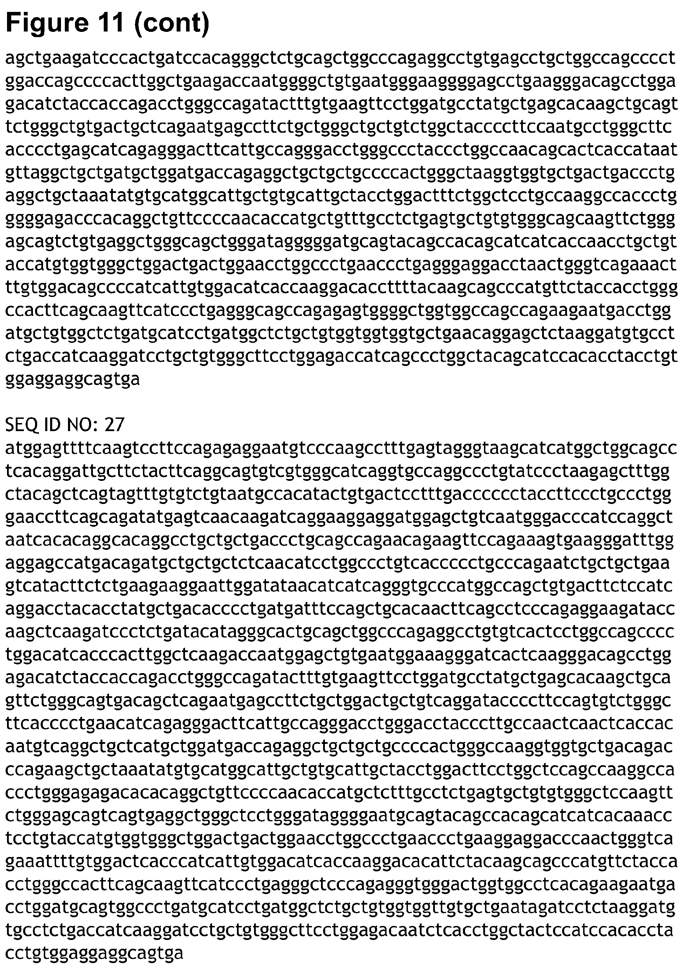

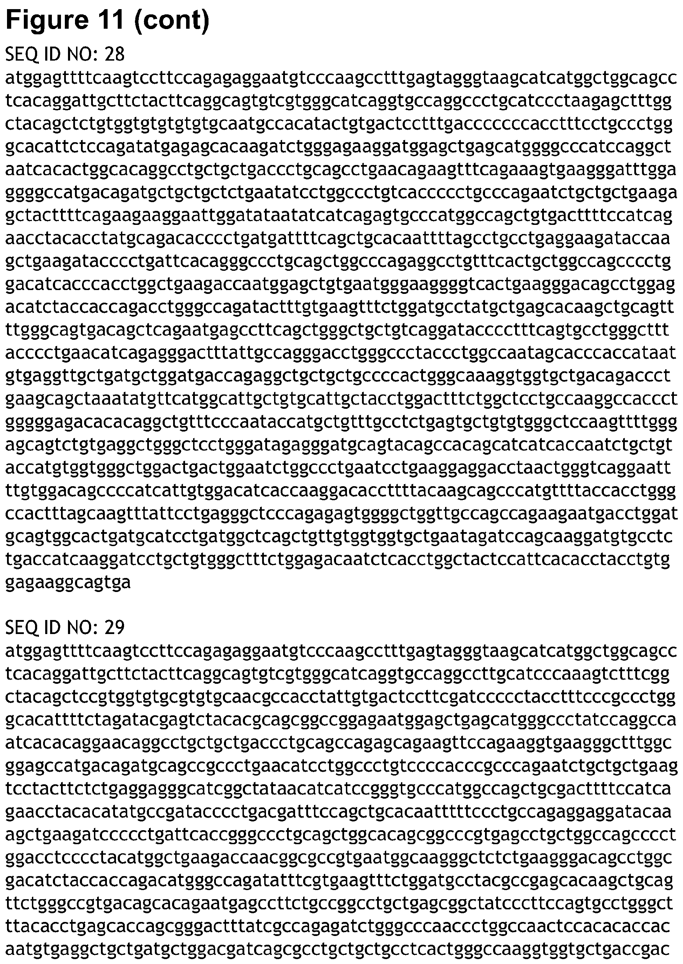

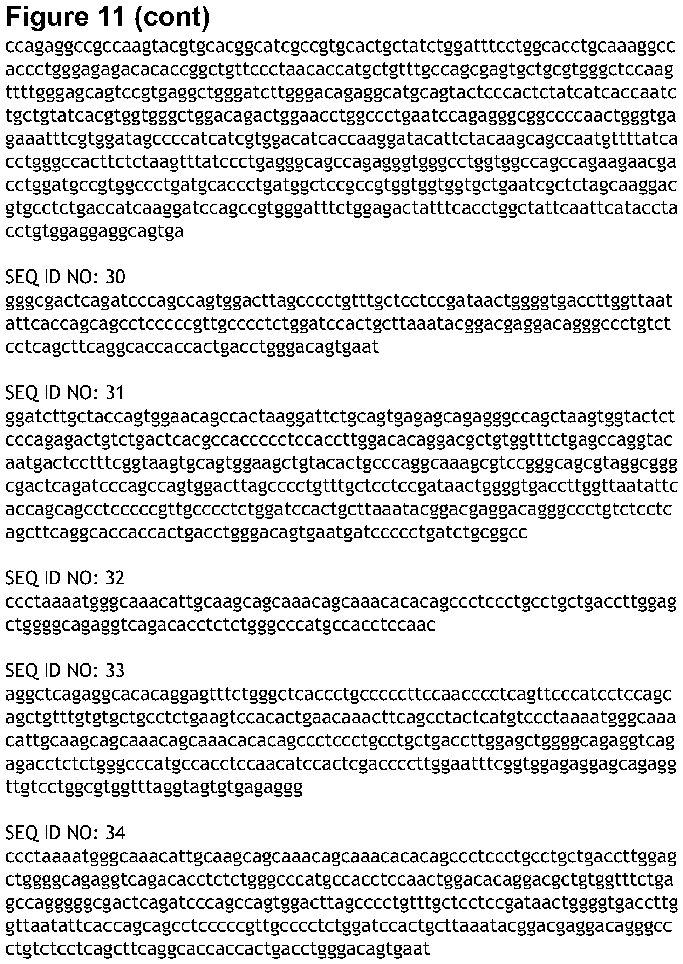

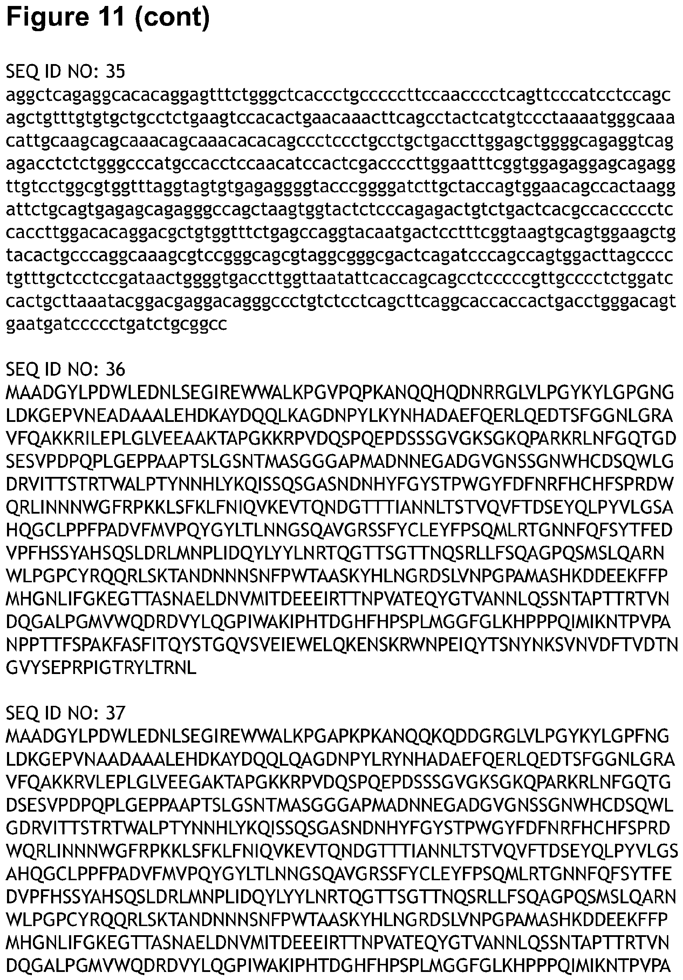

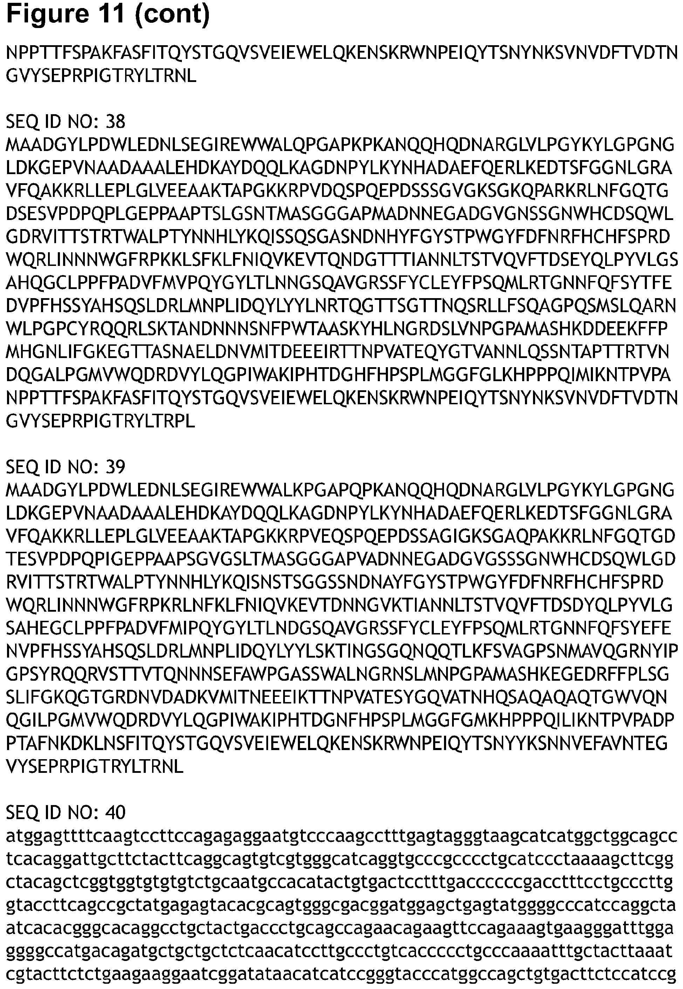

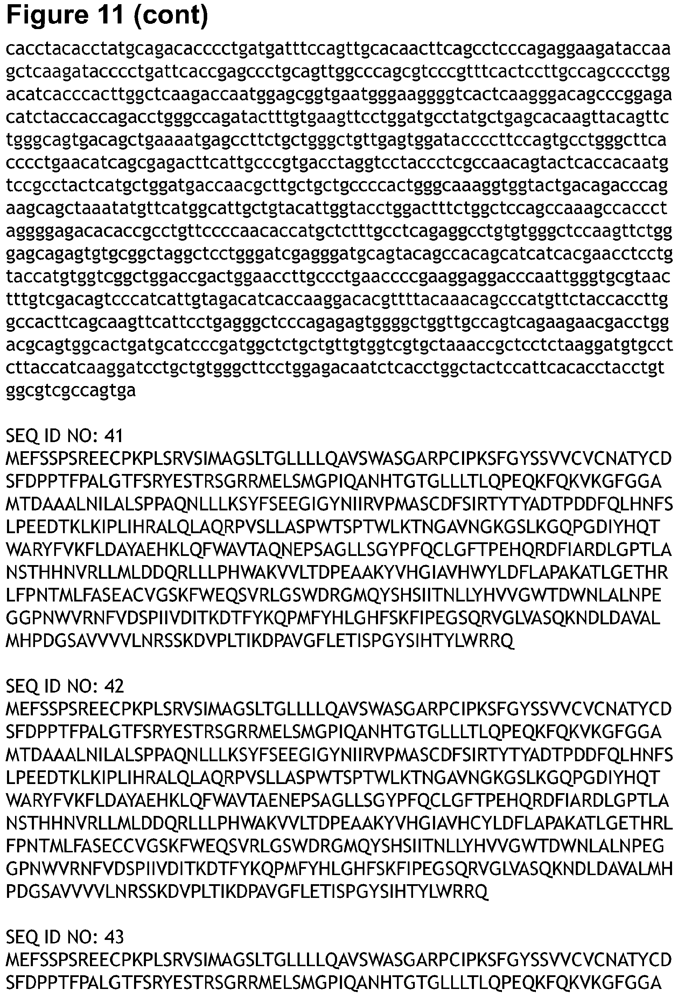

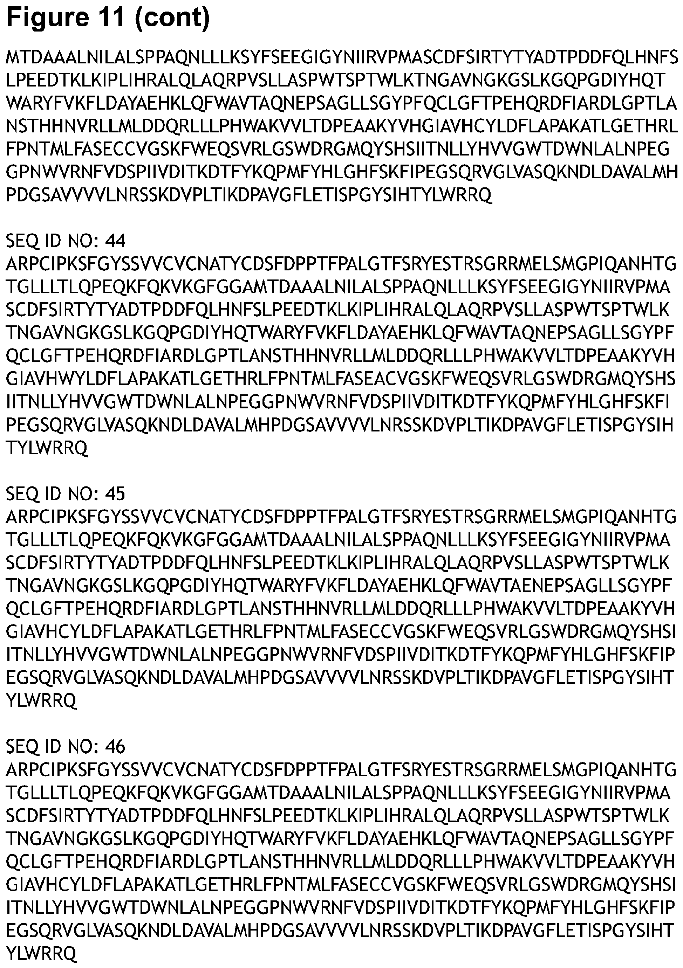

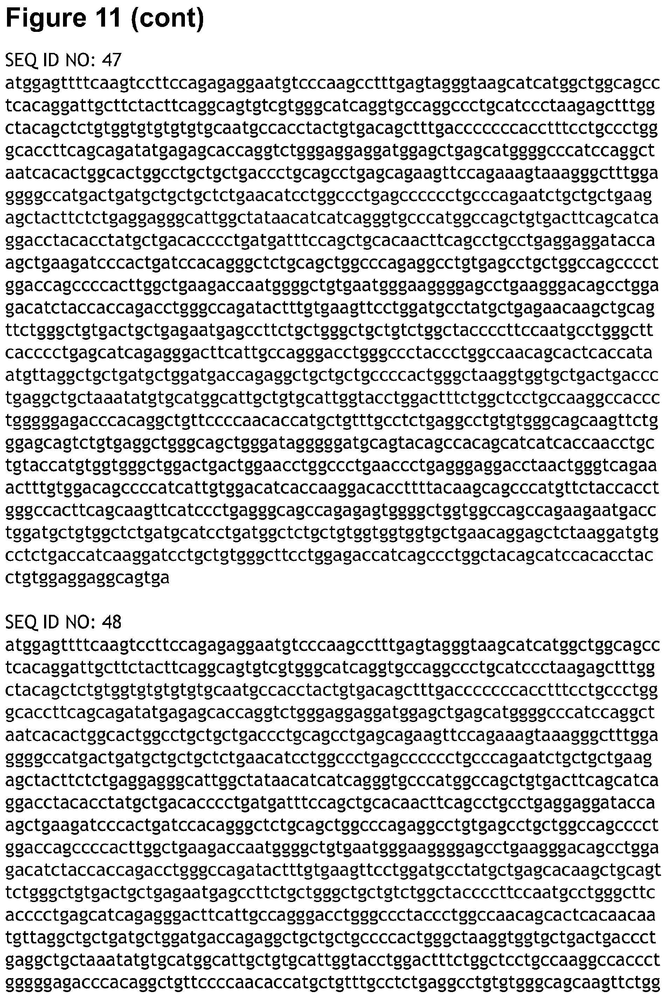

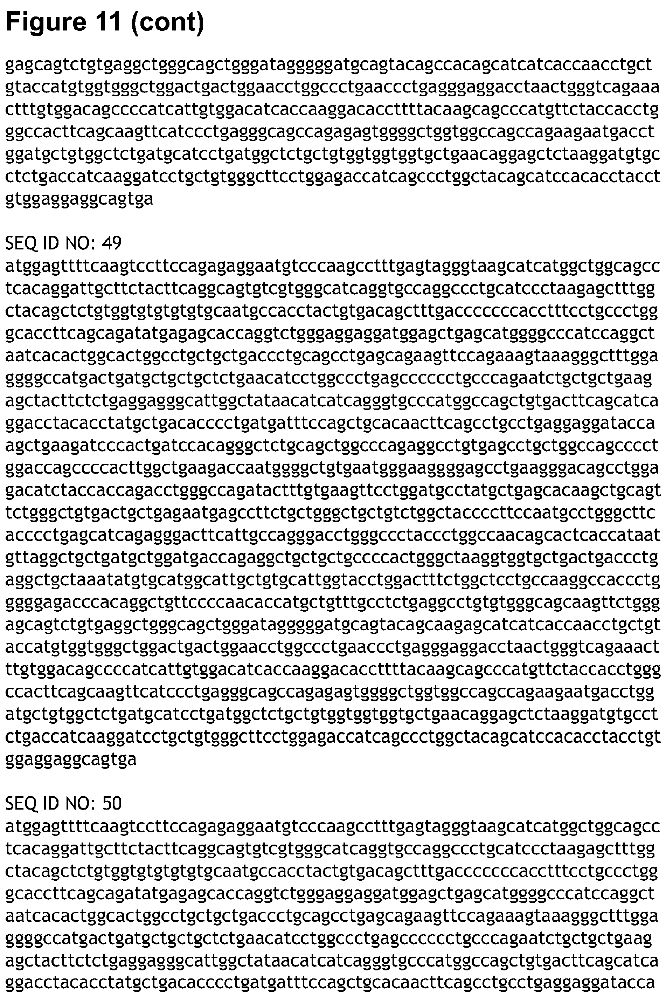

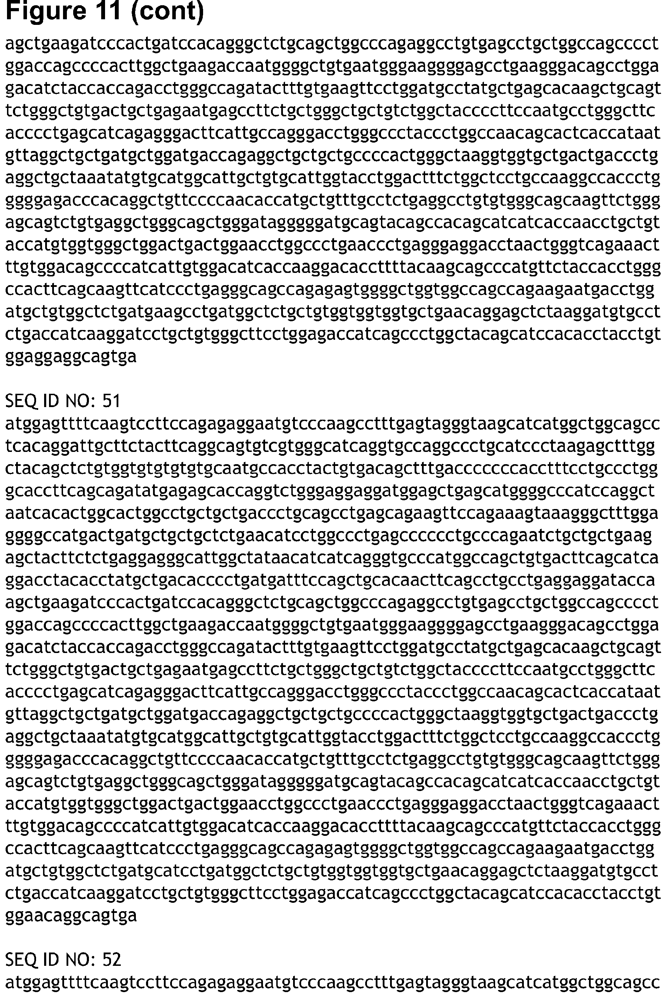

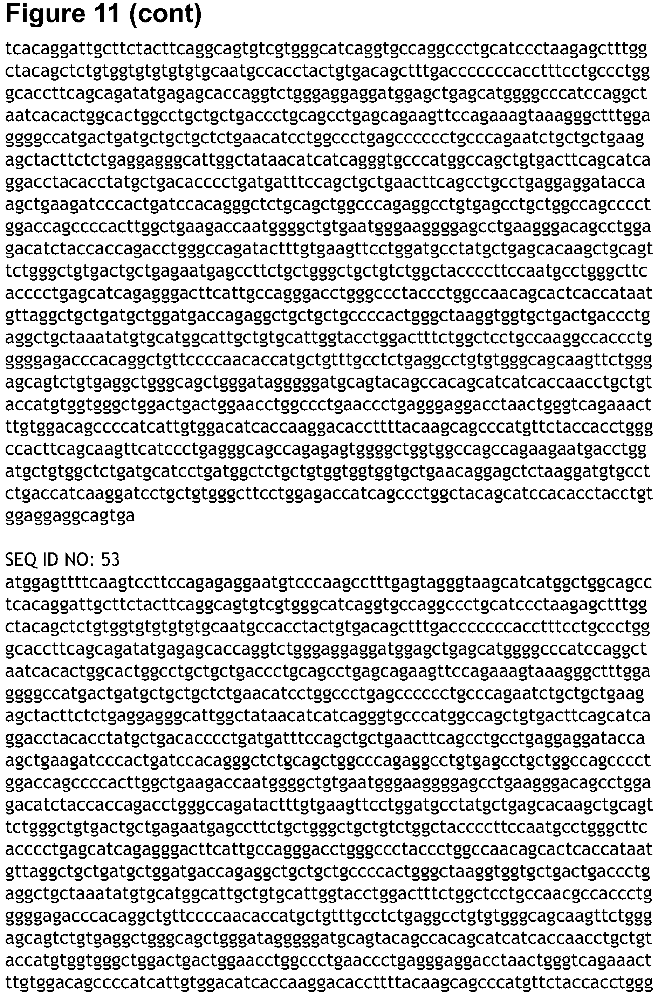

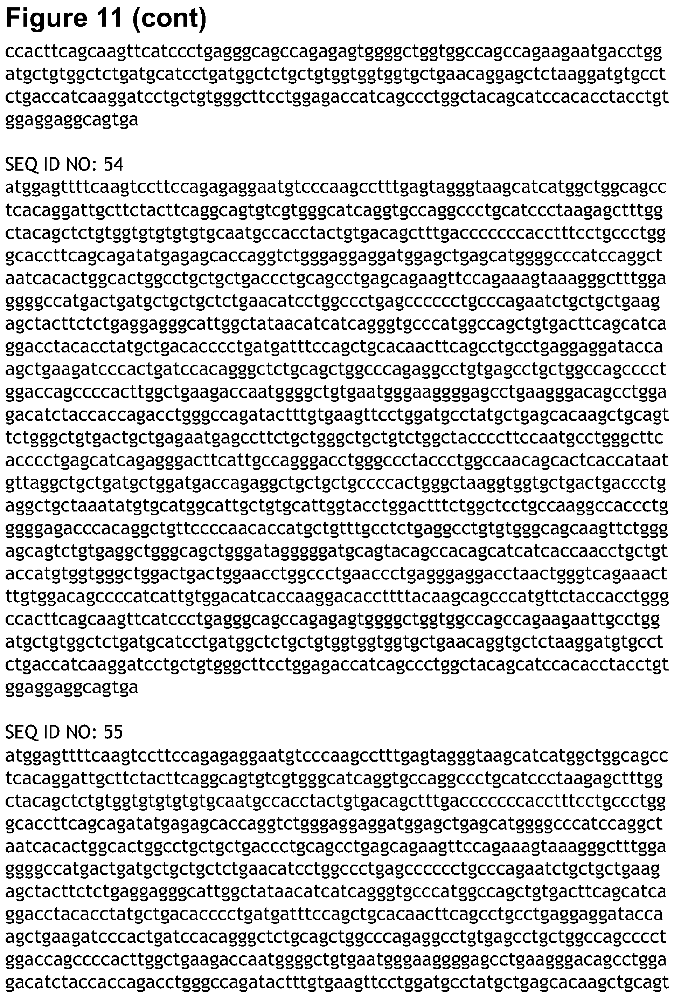

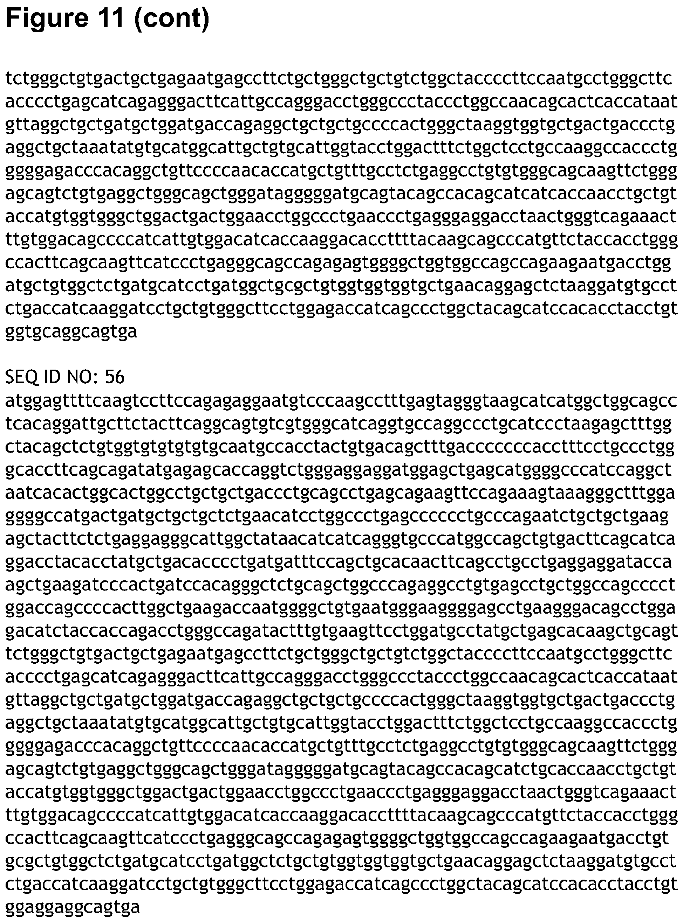

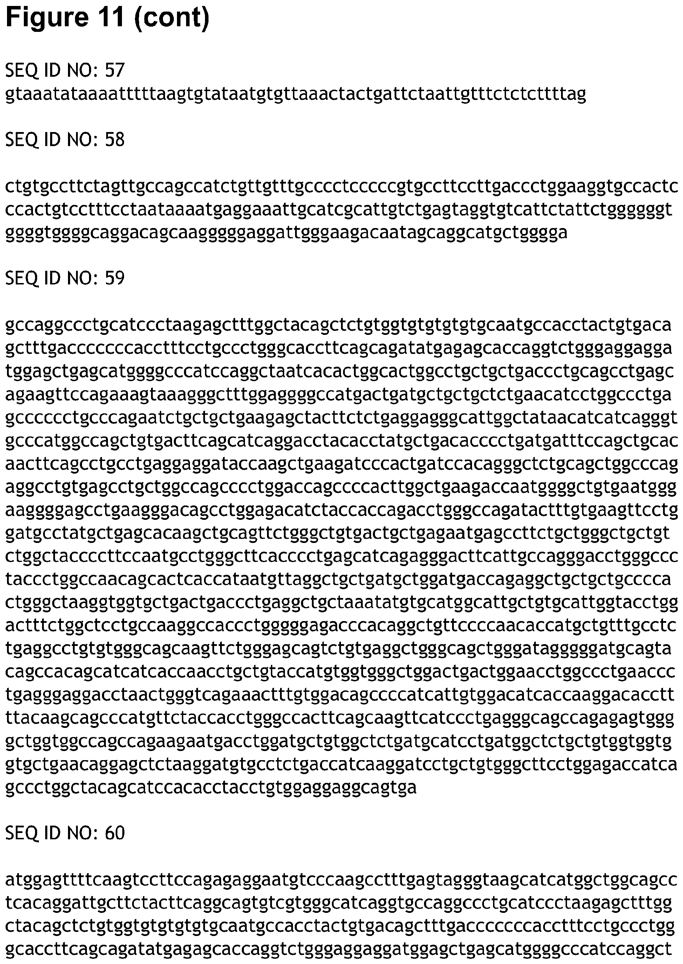

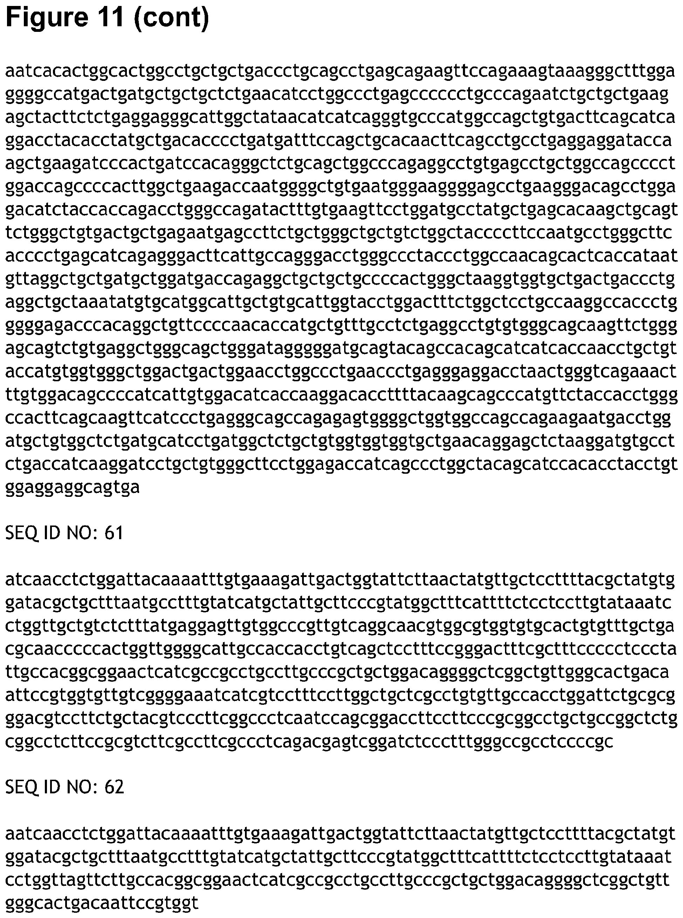

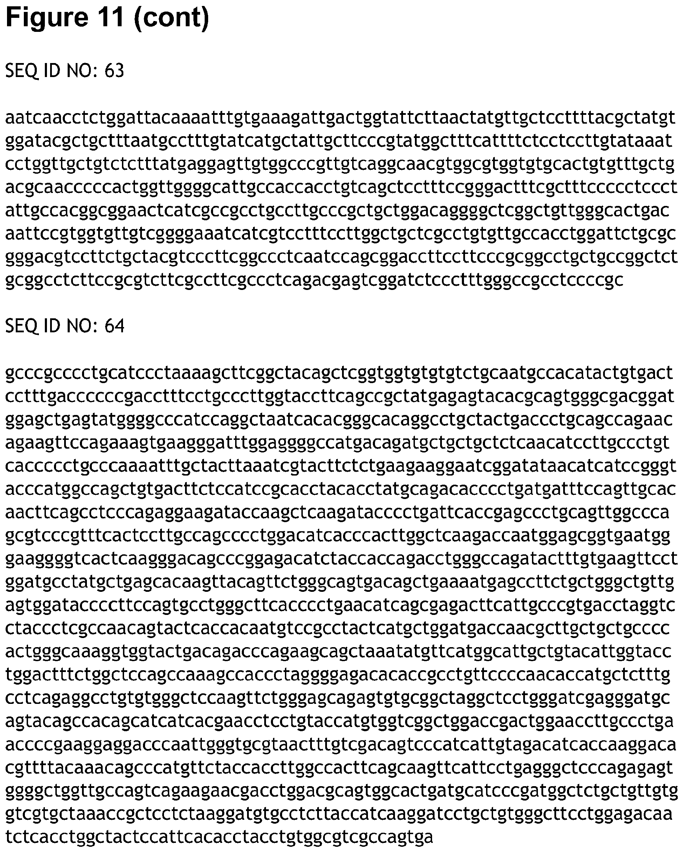

Figure 11 - Sequence listing

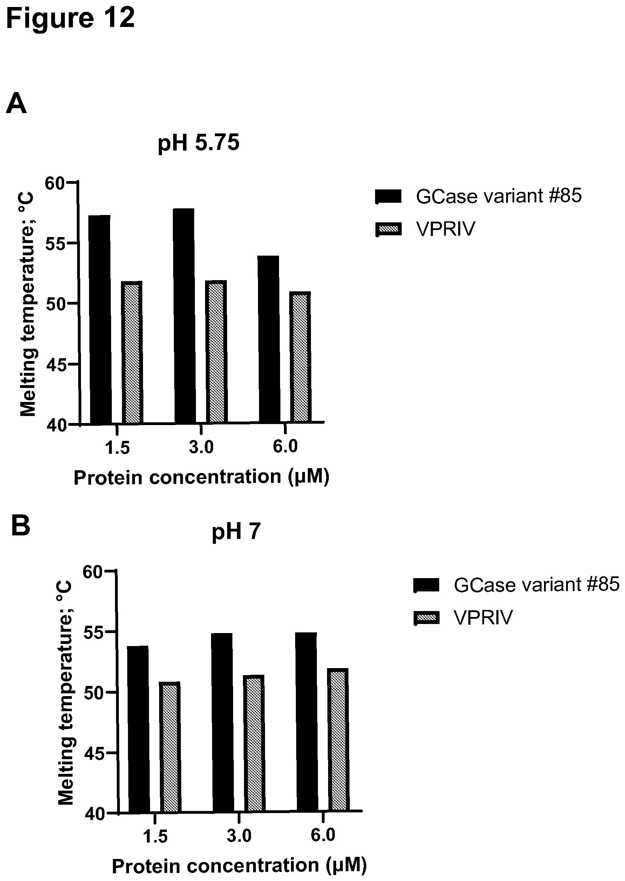

Figure 12 shows a comparison of thermostability between GCase variant #85 and velaglucerase alfa (VPRIV) at concentrations of 1.5, 3 and 6 µM in a solution at pH 5.75 (Figure 12A) and at pH 7 (Figure 12B).

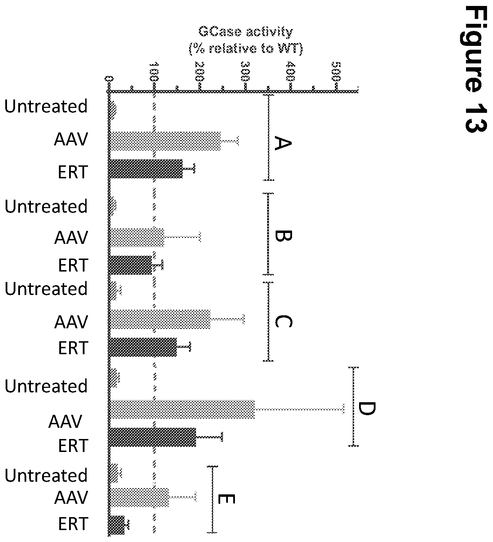

Figure 13 shows a comparison in levels of GCase activity in liver (Figure 13A), white blood cells (Figure 13B), bone marrow (Figure 13C), spleen (Figure 13D) and lung (Figure 13E) tissues following a single injection of AAV encoding the GCase variant #85 (at a dose of 2x10¹² vg/kg, "AAV"), or a course of velaglucerase alfa (VPRIV delivered at 60 U/kg every other week, "ERT") in *Gba*-deficient mice. Measurements were taken 12 weeks post-injection of AAV, and within 2 hours of ERT delivery. The average levels of GCase activity are shown relative to the average levels of GCase activity found in non-deficient (wild type) mice, with the result being calculated as a percentage (y-axis, "% relative to WT"). The dotted line represents the average level of GCase activity in wild type mice for comparison. "Untreated" = control. Data represented as mean ± SD.

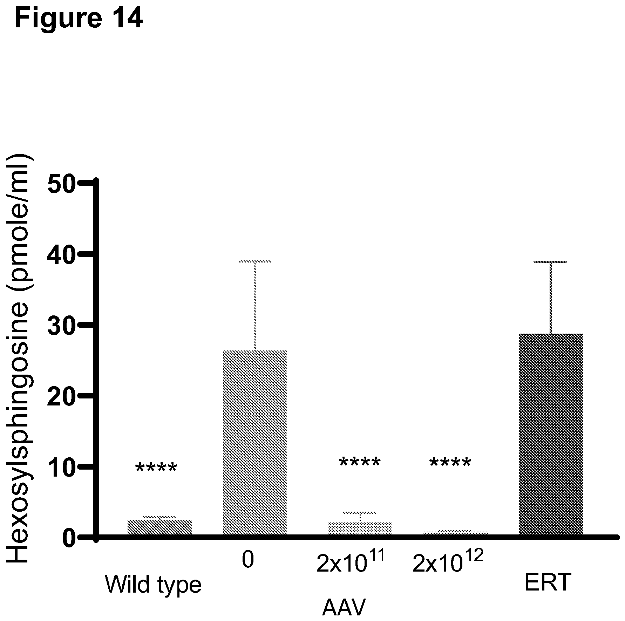

Figure 14 shows a comparison in levels of hexosylsphingosine in plasma following a single injection of AAV at the doses indicated, or a course of velaglucerase alfa (VPRIV, "ERT" delivered at 60 U/kg every other week) in *Gba*-deficient mice. Measurements were taken 12 weeks post-injection of AAV, and within 2 hours of ERT delivery. Levels of hexosylsphingosine in non-deficient mice ("Wild type") are shown for comparison. 0 = untreated control. Data represented as mean ± SD. **** = statistically-significant difference (p<0.0001) relative to the untreated control "0". Analysis performed = one-way ANOVA.

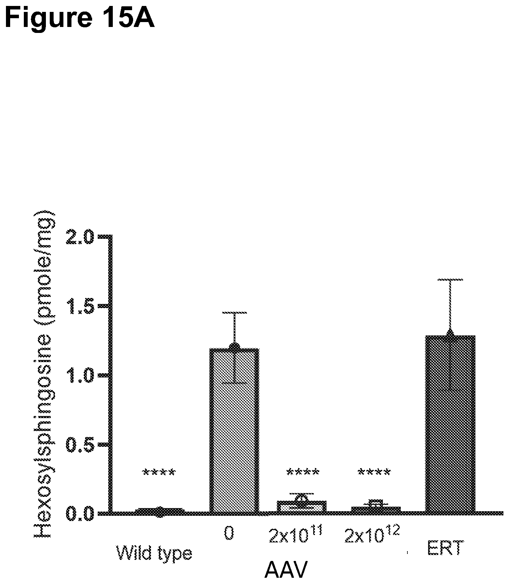

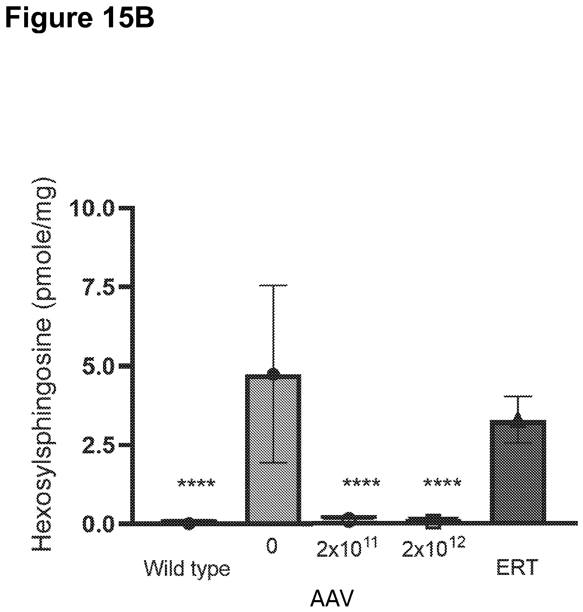

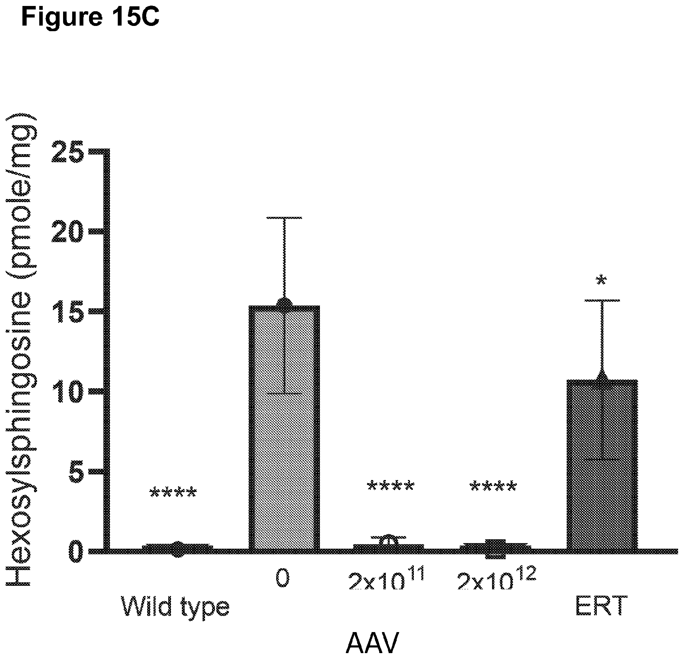

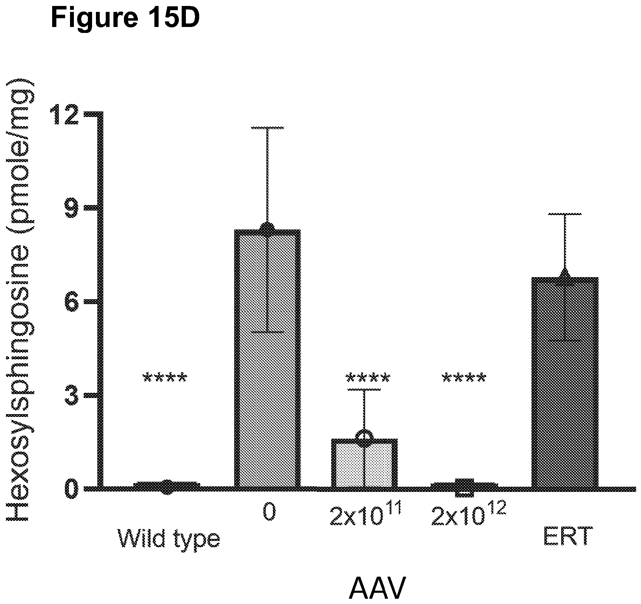

Figure 15 shows a comparison in levels of hexosylsphingosine in liver (Figure 15A), spleen (Figure 15B), bone marrow (Figure 15C) and lung (Figure 15D) following a single injection of AAV at the doses indicated (2x10¹¹ and 2x10¹² vg/kg), or a course of velaglucerase alfa (VPRIV, "ERT" delivered at 60 U/kg every other week) in *Gba-*deficient mice. Measurements were taken 12 weeks post-injection of AAV, and within 2 hours of ERT delivery. Levels of hexosylsphingosine in non-deficient mice ("Wild type") are shown for comparison. 0 = untreated control. Data represented as mean ± SD. * = difference (p<0.1) relative to the untreated control "0". **** = statistically-significant difference (p<0.0001) relative to the untreated control "0". Analysis performed = one-way ANOVA.

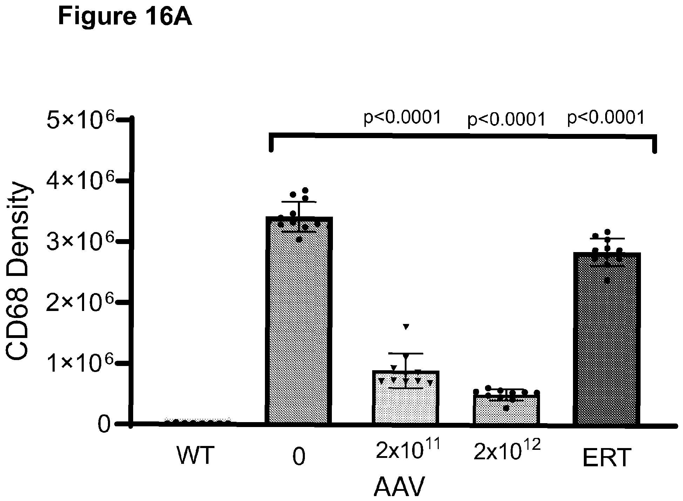

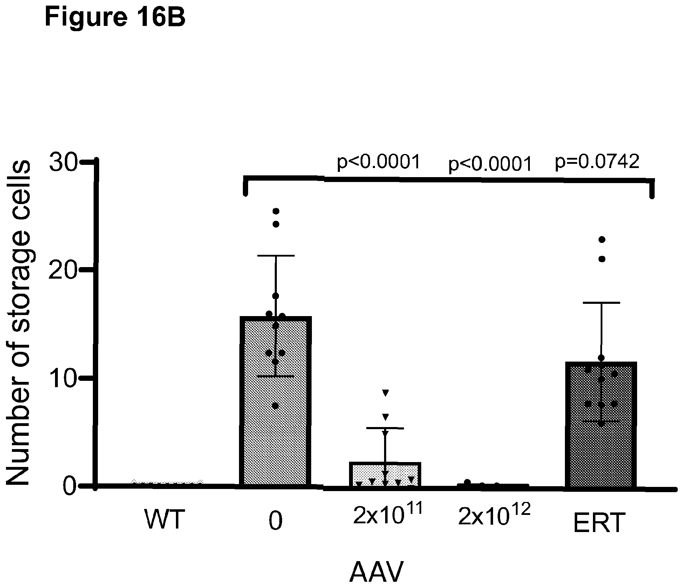

Figure 16 shows a comparison in levels of CD68 density (Figure 16A) or the number of storage cells (Figure 16B) in the lung following a single injection of AAV at the doses indicated, or a course of velaglucerase alfa (VPRIV, "ERT" delivered at 60 U/kg every other week) in *Gba*-deficient mice. Measurements were taken 12 weeks post-injection of AAV, and within 2 hours of ERT delivery. Levels of CD68 density and the number of storage cells in non-deficient mice ("Wild type") are shown for comparison. 0 = untreated control. Indicated p-values are relative to the untreated control "0". Data represented as mean ± SD.

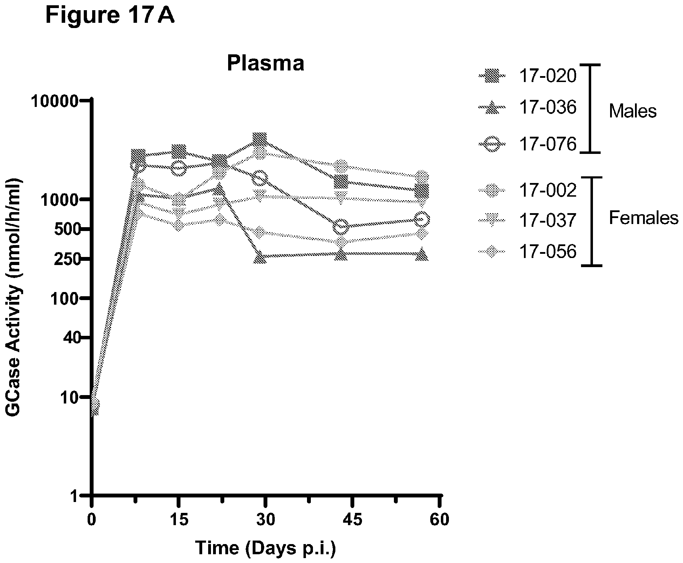

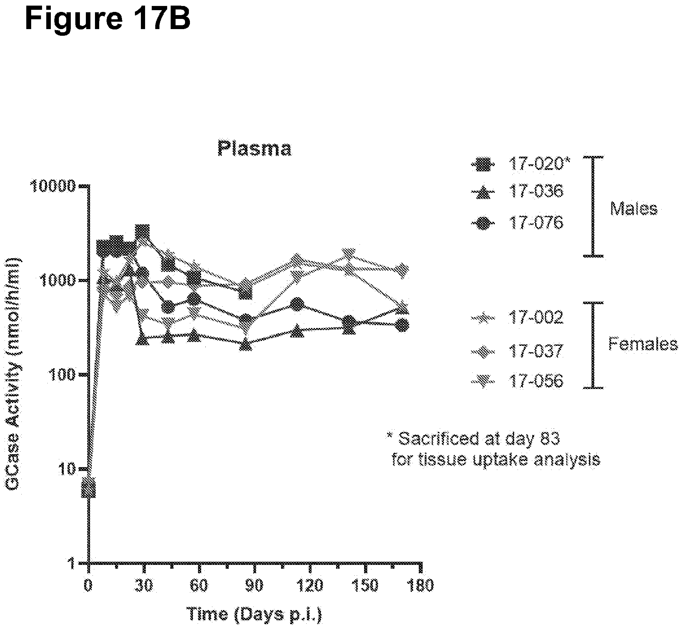

Figure 17 shows a rapid and sustained increase in plasma GCase activity levels following administration of AAV encoding GCase variant #85 (a single injection of 2x10¹² vg/kg) to rhesus macaques observed up to (Figure 17A) day 57 or (Figure 17B) day 170 post-AAV administration. The AAV shows a good safety profile and was found to be well tolerated. In (Figure 17B), animal 17-020 was sacrificed at day 83 for tissue uptake studies.

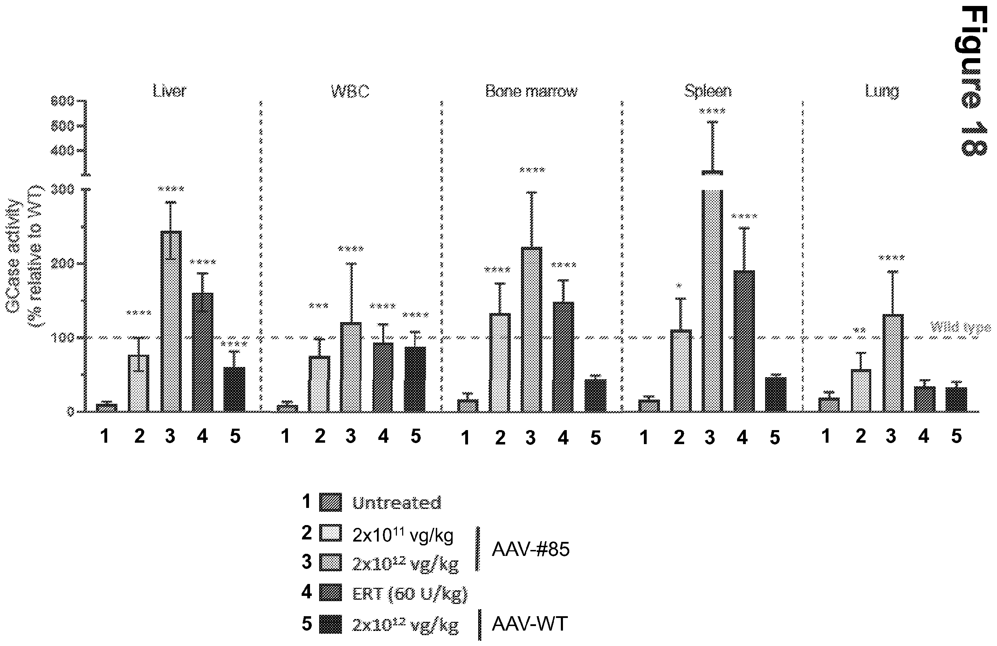

Figure 18 shows a comparison in levels of GCase activity in liver, white blood cells, bone marrow, spleen and lung tissues following a single injection of AAV encoding the GCase variant #85 ("AAV-#85", at a dose of 2 x 10¹¹ vg/kg and 2x10¹² vg/kg), a course of velaglucerase alfa (VPRIV delivered at 60 U/kg every other week, "ERT"), or following a single injection of AAV encoding wild-type GCase ("AAV-WT", at a dose of 2x10¹² vg/kg) in *Gba*-deficient mice. Measurements were taken 12 weeks post-injection of AAV, and within 2 hours of the last ERT delivery. The average levels of GCase activity are shown relative to the average levels of GCase activity found in non-deficient (wild type) mice, with the result being calculated as a percentage (y-axis, "% relative to WT"). The dotted line represents the average level of GCase activity in wild type mice for comparison. "Untreated" = control. Data represented as mean ± SD. n= 9 to 16 per treatment group. * P≤0.05, ** P≤0.01, ***P≤0.001, **** P≤0.0001, one-way ANOVA. Indicated p-values are relative to the untreated control "0". For x-axis: 1 = untreated control; 2 = 2 x 10¹¹ vg/kg of "AAV-#85"; 3 = 2 x 10¹² vg/kg of "AAV-#85"; 4 = "ERT"; 5 = 2 x 10¹² vg/kg of "AAV-WT".

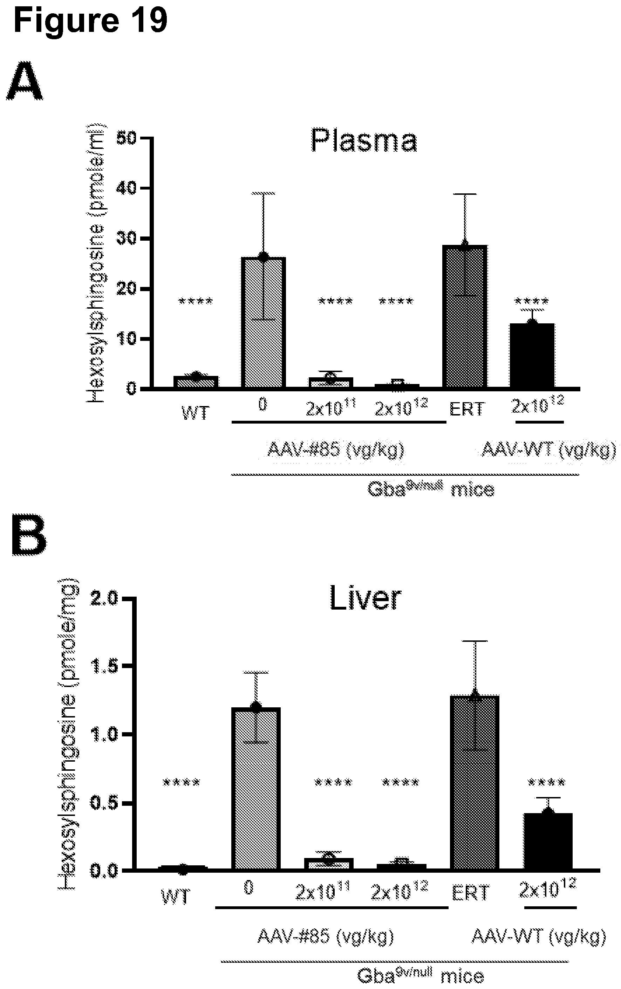

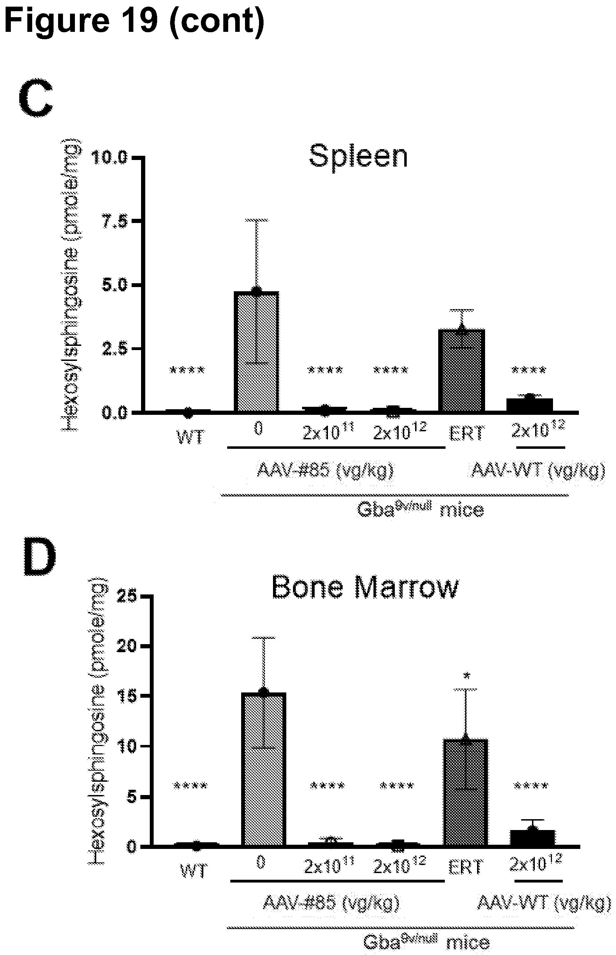

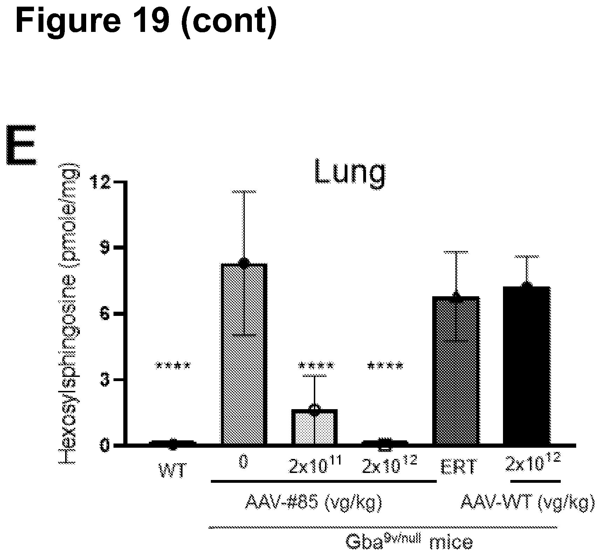

Figure 19 shows a comparison in levels of hexosylsphingosine in plasma (Figure 19A), liver (Figure 19B), spleen (Figure 19C), bone marrow (Figure 19D) and lung (Figure 19E) following a single injection of AAV encoding the GCase variant #85 ("AAV-#85", at a dose of 2 x 10¹¹ vg/kg or 2x10¹² vg/kg), a course of velaglucerase alfa (VPRIV, "ERT" delivered at 60 U/kg every other week), or following a single injection of AAV encoding wild-type GCase ("AAV-WT", at a dose of 2x10¹² vg/kg) in *Gba*-deficient mice. Measurements were taken 12 weeks post-injection of AAV, and within 2 hours of last ERT delivery. Levels of hexosylsphingosine in non-deficient mice ("Wild type") are shown for comparison. 0 = untreated control. Data represented as mean ± SD. n= 9 to 16 per group. * P≤ 0.05; **** P ≤ 0.0001, one-way ANOVA. Indicated p-values are relative to the untreated control "0".

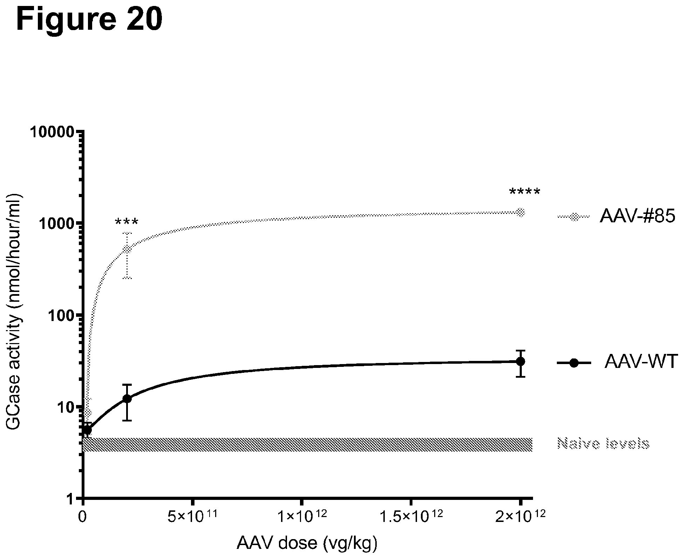

Figure 20 shows a comparison of the level of GCase activity in plasma following injection of differing doses (2 x 10¹⁰ vg/kg, 2 x 10¹¹ vg/kg or 2x10¹² vg/kg) of AAV encoding the GCase variant #85 ("AAV-#85") or AAV encoding wild-type GCase ("AAV-WT") in Gba-deficient mice. Measurements were taken 12 weeks post-injection of AAV. Data represented as mean ± SD. n= 9 to 16 per treatment group. *** P≤0.001, **** P≤0.0001, Student T-test.

### Description of the Sequence Listing

| SEQ ID NO | Sequence description |
|---|---|
| 1 | Polypeptide sequence of wild type human GCase with signal peptide (immature) |
| 2 | Polypeptide sequence of wild type human GCase without signal peptide (mature) |
| 3 | VPRIV |
| 4 | Polypeptide sequence of GCase_{H184L} with signal peptide |
| 5 | Polypeptide sequence of GCase_{H184L/K360N} with signal peptide |
| 6 | Codon-optimised modified GBA nucleotide sequence, without signal peptide portion, containing mutation corresponding to E272Q in the encoded amino acid sequence |
| 7 | Codon-optimised modified GBA nucleotide sequence, without signal peptide portion, containing mutation corresponding to E272Q in the encoded amino acid sequence |
| 8 | Codon-optimised modified GBA nucleotide sequence, without signal peptide portion, containing mutation corresponding to E272Q in the encoded amino acid sequence |
| 9 | Codon-optimised modified GBA nucleotide sequence, without signal peptide portion, containing mutation corresponding to E272Q in the encoded amino acid sequence |
| 10 | Codon-optimised modified GBA nucleotide sequence, with signal peptide portion, containing mutation corresponding to E272Q in the encoded amino acid sequence |
| 11 | Codon-optimised modified GBA nucleotide sequence, with signal peptide portion, containing mutation corresponding to E272Q in the encoded amino acid sequence |
| 12 | Codon-optimised modified GBA nucleotide sequence, with signal peptide portion, containing mutation corresponding to E272Q in the encoded amino acid sequence |
| 13 | Codon-optimised modified GBA nucleotide sequence, with signal peptide portion, containing mutation corresponding to E272Q in the encoded amino acid sequence |
| 14 | Codon-optimised modified GBA nucleotide sequence, without signal peptide portion, containing mutation corresponding to W351C/A380C in the encoded amino acid sequence |
| 15 | Codon-optimised modified GBA nucleotide sequence, without signal peptide portion, containing mutation corresponding to W351C/A380C in the encoded amino acid sequence |
| 16 | Codon-optimised modified GBA nucleotide sequence, without signal peptide portion, containing mutation corresponding to W351C/A380C in the encoded amino acid sequence |
| 17 | Codon-optimised modified GBA nucleotide sequence, without signal peptide portion, containing mutation corresponding to W351C/A380C in the encoded amino acid sequence |
| 18 | Codon-optimised modified GBA nucleotide sequence, with signal peptide portion, containing mutation corresponding to W351C/A380C in the encoded amino acid sequence |
| 19 | Codon-optimised modified GBA nucleotide sequence, with signal peptide portion, containing mutation corresponding to W351C/A380C in the encoded amino acid sequence |
| 20 | Codon-optimised modified GBA nucleotide sequence, with signal peptide portion, containing mutation corresponding to W351C/A380C in the encoded amino acid sequence |
| 21 | Codon-optimised modified GBA nucleotide sequence, with signal peptide portion, containing mutation corresponding to W351C/A380C in the encoded amino acid sequence |
| 22 | Codon-optimised modified GBA nucleotide sequence, without signal peptide portion, containing mutation corresponding to E272Q and W351C/A380C in the encoded amino acid sequence |
| 23 | Codon-optimised modified GBA nucleotide sequence, without signal peptide portion, containing mutation corresponding to E272Q and W351C/A380C in the encoded amino acid sequence |
| 24 | Codon-optimised modified GBA nucleotide sequence, without signal peptide portion, containing mutation corresponding to E272Q and W351C/A380C in the encoded amino acid sequence |
| 25 | Codon-optimised modified GBA nucleotide sequence, without signal peptide portion, containing mutation corresponding to E272Q and W351C/A380C in the encoded amino acid sequence |
| 26 | Codon-optimised modified GBA nucleotide sequence, with signal peptide portion, containing mutation corresponding to E272Q and W351C/A380C in the encoded amino acid sequence |
| 27 | Codon-optimised modified GBA nucleotide sequence, with signal peptide portion, containing mutation corresponding to E272Q and W351C/A380C in the encoded amino acid sequence |
| 28 | Codon-optimised modified GBA nucleotide sequence, with signal peptide portion, containing mutation corresponding to E272Q and W351C/A380C in the encoded amino acid sequence |
| 29 | Codon-optimised modified GBA nucleotide sequence, with signal peptide portion, containing mutation corresponding to E272Q and W351C/A380C in the encoded amino acid sequence |
| 30 | A1AT promoter portion of LSP-S |
| 31 | A1AT promoter portion of LSP-L |
| 32 | HCR enhancer portion of LSP-S |
| 33 | HCR enhancer portion of LSP-L |
| 34 | LSP-S transcription regulatory element |
| 35 | LSP-L transcription regulatory element |
| 36 | Polypeptide sequence of liver-tropic capsid |
| 37 | Polypeptide sequence of liver-tropic capsid |
| 38 | Polypeptide sequence of liver-tropic capsid |
| 39 | Polypeptide sequence of CNS-tropic capsid |
| 40 | Wild type human GBA nucleotide sequence with signal peptide (from GenBank NM 000157.3) |
| 41 | Polypeptide sequence of GCase_{E272Q} with signal peptide |
| 42 | Polypeptide sequence of GCase_{W351C/A380C} with signal peptide |
| 43 | Polypeptide sequence of GCase_{E272Q/W351C/A380C} with signal peptide |
| 44 | Polypeptide sequence of GCase_{E2720} without signal peptide |
| 45 | Polypeptide sequence of GCase_{W351C/A380C} without signal peptide |
| 46 | Polypeptide sequence of GCase_{E2720/W351C/A380C} without signal peptide |
| 47 | Codon-optimised modified GBA nucleotide sequence, with signal peptide portion, containing mutation corresponding to H262N in the encoded amino acid sequence |
| 48 | Codon-optimised modified GBA nucleotide sequence, with signal peptide portion, containing mutation corresponding to H313N in the encoded amino acid sequence |
| 49 | Codon-optimised modified GBA nucleotide sequence, with signal peptide portion, containing mutation corresponding to H404K in the encoded amino acid sequence |
| 50 | Codon-optimised modified GBA nucleotide sequence, with signal peptide portion, containing mutation corresponding to H490K in the encoded amino acid sequence |
| 51 | Codon-optimised modified GBA nucleotide sequence, with signal peptide portion, containing mutation corresponding to R534N in the encoded amino acid sequence |
| 52 | Codon-optimised modified GBA nucleotide sequence, with signal peptide portion, containing mutation corresponding to H184L in the encoded amino acid sequence |
| 53 | Codon-optimised modified GBA nucleotide sequence, with signal peptide portion, containing mutation corresponding to H184L/K360N in the encoded amino acid sequence |
| 54 | Codon-optimised modified GBA nucleotide sequence, with signal peptide portion, containing mutation corresponding to D482C/S503C in the encoded amino acid sequence |
| 55 | Codon-optimised modified GBA nucleotide sequence, with signal peptide portion, containing mutation corresponding to S494C/R534C in the encoded amino acid sequence |
| 56 | Codon-optimised modified GBA nucleotide sequence, with signal peptide portion, containing mutation corresponding to I407C/D484C in the encoded amino acid sequence |
| 57 | Nucleotide sequence of SV40 intron |
| 58 | Nucleotide sequence of bovine growth hormone poly A sequence |
| 59 | Codon-optimised GBA nucleotide sequence encoding wild type GCase, without signal peptide portion |
| 60 | Codon-optimised GBA nucleotide sequence encoding wild type GCase, with signal peptide portion |
| 61 | Woodchuck hepatitis post-transcriptional regulatory element |
| 62 | Woodchuck hepatitis post-transcriptional regulatory element |
| 63 | Woodchuck hepatitis post-transcriptional regulatory element |
| 64 | Wild type human GBA nucleotide sequence without signal peptide |

### Detailed description

### General definitions

Unless defined otherwise, technical and scientific terms used herein have the same meaning as commonly understood by a person skilled in the art to which this invention belongs.

In general, the term *"comprising"* is intended to mean including but not limited to. For example, the phrase *"a modified GCase polypeptide comprising two mutations"* should be interpreted to mean that the modified GCase polypeptide has at least two mutations, but may contain further mutations. Similarly, the phrase *"a polynucleotide comprising a modified glucocerebrosidase (GBA) nucleotide sequence"* refers to a polynucleotide that has a modified GBA nucleotide sequence, but the polynucleotide may contain additional nucleotides.

In some embodiments of the invention, the word *"comprising"* is replaced with the phrase *"consisting essentially of".* The term *"consisting essentially of"* means that specific further components can be present, namely those not materially affecting the essential characteristics of the subject matter.

In some embodiments of the invention, the word *"comprising"* is replaced with the phrase *"consisting of".* The term *"consisting of"* is intended to be limiting. For example, the phrase *"a modified GCase polypeptide consisting of two mutations"* should be interpreted to mean that the modified GCase polypeptide has two mutations only and no additional mutations. Similarly, the phrase *"a polynucleotide consisting of a modified glucocerebrosidase (GBA) nucleotide sequence "* should be understood to mean that the polynucleotide has a modified GBA nucleotide sequence and no additional nucleotides.

In some embodiments of the invention, the word *"have"* can be replaced with the word *"comprise"* or the phrase *"consist of".* In some embodiments of the invention, the word *"has"* can be replaced with the word *"comprises"* or the phrase *"consists of".*

As used herein, *"between"* when referring to two endpoints to define a range of values should be taken to mean "*between and including*". Thus, a range defined as "*between 5 and 10"* includes all values greater than 5 and less than 10, as well as the discrete values 5 and 10 themselves.

The terms *"protein"* and *"polypeptide"* are used interchangeably herein, and are intended to refer to a polymeric chain of amino acids of any length.

The terms "*mutation*", "*substitution mutation*" and "*amino acid substitution*" are used interchangeably herein, and are intended to mean the substitution of one amino acid in an amino acid sequence with a different amino acid. For example, a modified GCase polypeptide comprising a mutation at a position corresponding to position 272 of SEQ ID NO: 1 may correspond to a GCase polypeptide that has the sequence of SEQ ID NO: 1 except that the amino acid at position 272 of the GCase polypeptide is different to the amino acid at position 272 in SEQ ID NO: 1. Alternatively, a modified GCase polypeptide comprising a mutation at a position corresponding to position 272 of SEQ ID NO: 1 may correspond to a GCase polypeptide that has the sequence of SEQ ID NO: 1, except that a few amino acids including the amino acid at position 272 are different to the corresponding amino acids in SEQ ID NO:1 (for example a contiguous portion of the GCase polypeptide comprising position 272 may contain amino acids which are different from the corresponding amino acids in SEQ ID NO: 1). In the phrases "*the mutation at a position corresponding to position X of SEQ ID NO: Z is a substitution with amino acid Y*" and "*the at least one mutation comprises a mutation at a position corresponding to position X of SEQ ID NO: Z*", the substituted residue is the amino acid at the position corresponding to position X of SEQ ID NO: Z. Amino acid Y is the different amino acid which replaces the original or native amino acid in an amino acid sequence at the position corresponding to position X of SEQ ID NO: Z. In the phrases "*substitution of*" amino acid X or "*amino acid X that is (to be) substituted*", amino acid X is the original or native amino acid that is present within an amino acid sequence and that is to be replaced. For example, substitution of glutamic acid means that an original or native glutamic acid amino acid is replaced by another amino acid. In the phrase "*substitution with*" amino acid Y or *"mutation to"* amino acid Y, amino acid Y is the different amino acid which replaces the original or native amino acid in an amino acid sequence. For example, substitution with glutamine refers to replacement of an original or native (non-glutamine) amino acid with glutamine. The standard shorthand nomenclature used to define a substitution mutation lists the original or native amino acid at a position within an amino acid sequence that is to be substituted, and the amino acid which replaces the original or native amino acid. For example, a modified GCase polypeptide comprising the substitution mutation E272Q refers to a modified GCase amino acid sequence which comprises a substitution of the glutamic acid residue at a position corresponding to position 272 with a glutamine residue *(i.e.* which comprises a glutamine residue at a position corresponding to position 272).

Amino acids *"corresponding to"* specified positions of a specified SEQ ID NO may be amino acids at the specified positions of the particular SEQ ID NO recited. For example, the amino acid "*corresponding to position 272 of SEQ ID NO: 1"* may be the amino acid at position 272 of SEQ ID NO: 1. Alternatively, amino acids *"corresponding to"* specified positions of a specified SEQ ID NO may be amino acids from an alternative amino acid sequence which correspond to the specified positions of the specified SEQ ID NO. For example, the amino acid "*corresponding to position 272 of SEQ ID NO: 1"* may be the amino acid from an alternative amino acid sequence which corresponds to position 272 of SEQ ID NO: 1. It is within the capabilities of the person skilled in the art to determine which amino acids in an alternative amino acid sequence *"correspond to"* the specified positions in the specified SEQ ID NO. For example, the person skilled in the art merely needs to perform a sequence alignment of the alternative amino acid sequence with the specified SEQ ID NO using a suitable alignment algorithm such as that of Needleman and Wunsch described herein, and determine which region of the alternative amino acid sequence aligns to the specified positions in the specified SEQ ID NO. For example, the skilled person is able to align the alternative amino acid sequence with SEQ ID NO: 1 and determine which amino acid aligns, and therefore corresponds to, *e.g.* position 272 of SEQ ID NO. 1.

The term *"conservative substitution"* refers to a substitution mutation in which an amino acid is substituted with another amino acid which has similar biochemical properties, such as size, charge or hydrophobicity. Amino acids may be categorised into groups on the basis of the structure of their side chains: aliphatic (glycine, alanine, valine, leucine, isoleucine); hydroxyl/sulphur-containing (serine, threonine, cysteine, methionine); cyclic (proline); aromatic (phenylalanine, tyrosine, tryptophan); basic (histidine, lysine, arginine); acidic (aspartic acid, glutamic acid); and acid amine (asparagine, glutamine). A *"conservative substitution"* thus refers to a substitution mutation in which an amino acid is substituted with another amino acid in the same group. Conversely, the term *"non-conservative substitution"* refers to a substitution in which an amino acid is substituted with another amino acid which has different biochemical properties, *i.e.* a substitution mutation in which an amino acid is substituted with an amino acid in another group. For example, substitution of aspartic acid with glutamic acid may be considered to be a *"conservative substitution",* whilst substitution of glutamic acid with glutamine may be considered to be a "*non-conservative"* substitution.

The terms *"wild-type"* and *"native "* are used interchangeably herein, and are intended to describe something which is naturally occurring. For example, a *"wild-type GCase amino acid sequence"* is a GCase amino acid sequence which occurs in nature.

The term "*physiological pH*" refers to the pH that normally prevails in the human body, which is typically between pH 7.35 and 7.45. For the purposes of the present invention, the term "*physiological pH*" refers in particular to the pH found in the blood, which is about pH 7.4, typically between pH 7.35 and 7.45. Thus, where the at least one mutation provides structural stabilisation at physiological pH, this is to be understood as providing structural stabilisation at a pH which can be found in the blood, *e.g.* pH 7.4. As a further example, if the stability of a polypeptide is being measured at physiological pH, then the stability is being measured at a pH level which can be found in the blood, *e.g.* pH 7.4. Preferably, the physiological pH is pH 7.4.

The term "*lysosomal pH*" refers to a pH level of the lysosome. Put another way, the lysosomal pH is a pH level which can be found in the lysosome, *e.g.* pH 5.6. For example, if the stability of a polypeptide is being measured at a lysosomal pH, then the stability is being measured at a pH level which can be found in the lysosome, *e.g.* pH 5.6. The lysosomal pH may be between pH 5.4 to pH 5.8. The lysosomal pH may be between pH 5.5 and 5.7. The lysosomal pH may be between pH 5.55 and 5.65. Preferably, the lysosomal pH is pH 5.6.

The terms *"AAV viral particle"* and *"AAV vector"* are used interchangeably herein.

The term *"around"* used in the context of describing the length of nucleotide or amino acid sequences indicates that a sequence may comprise or consist of a defined number of nucleotides or amino acids, plus or minus 10%, more particularly plus or minus 5%, more particularly plus or minus 1%, or more particularly plus or minus a single integer. For example, reference to a nucleotide sequence of *"around"* 1494 nucleotides in length may refer to a nucleotide sequence of 1345-1643 nucleotides, more particularly 1420-1568 nucleotides, more particularly 1480-1508 nucleotides, and more particularly 1493-1495 nucleotides in length.

The term *"around"* used in the context of a length of time (*e.g.* 16 hours) indicates that the length of time includes the specified length of time plus or minus 10%, more particularly plus or minus 5%, or more particularly plus or minus 1%.

For the purpose of this invention, in order to determine the percent identity of two sequences (such as two polynucleotide or two polypeptide sequences), the sequences are aligned for optimal comparison purposes (*e.g*., gaps can be introduced in a first sequence for optimal alignment with a second sequence). The nucleotides or amino acid residues at each position are then compared. When a position in the first sequence is occupied by the same nucleotide or amino acid at the corresponding position in the second sequence, then the nucleotides or amino acids are identical at that position. The percent identity between the two sequences is a function of the number of identical positions shared by the sequences (*i.e.,* % identity = number of identical positions /total number of positions in the reference sequence x 100).

Typically the sequence comparison is carried out over the length of the reference sequence. For example, if the user wished to determine whether a given ("test") sequence is 95% identical to SEQ ID NO: 1, SEQ ID NO: 1 would be the reference sequence. To assess whether a sequence is at least 95% identical to SEQ ID NO: 1 (an example of a reference sequence), the skilled person would carry out an alignment over the length of SEQ ID NO: 1, and identify how many positions in the test sequence were identical to those of SEQ ID NO: 1. If at least 95% of the positions are identical, the test sequence is at least 95% identical to SEQ ID NO: 1. If the test sequence is shorter than SEQ ID NO: 1, the gaps or missing positions should be considered to be non-identical positions.

The skilled person is aware of different computer programs that are available to determine the homology or identity between two sequences. For instance, a comparison of sequences and determination of percent identity between two sequences can be accomplished using a mathematical algorithm. In an embodiment, the percent identity between two amino acid or nucleic acid sequences is determined using the Needleman and Wunsch (1970) algorithm which has been incorporated into the GAP program in the Accelrys GCG software package (available at http://www.accelrys.com/products/gcg/), using either a Blosum 62 matrix or a PAM250 matrix, and a gap weight of 16, 14, 12, 10, 8, 6, or 4 and a length weight of 1, 2, 3, 4, 5, or 6.

The singular forms "*a*", "*an*"*,* and *"the"* include plural referents unless the content clearly dictates otherwise. Thus, for example, reference to *"an amino acid"* includes two or more instances or versions of such amino acids.

For the purposes of the present invention, the term *"fragment"* refers to a contiguous portion of a sequence. For example, a fragment of SEQ ID NO: 1 of 50 amino acids refers to 50 contiguous amino acids of SEQ ID NO: 1.

### A modified GCase polypeptide

A modified GCase polypeptide (also referred to as a GCase variant) comprising at least one mutation is described herein. The term "*modified*" means that the polypeptide has at least one difference compared to a wild-type GCase polypeptide, *e.g.* a mutation has been introduced. It is believed that when moving from an acidic pH to a physiological pH, a residue previously positively charged can become neutral, and a neutral one can become negatively charged, resulting in the abolition of attractive or formation of repulsive forces which may destabilise the polypeptide. Without wishing to be bound by theory, it is believed that the instant amino acid mutations may serve to enhance the stability at physiological pH (and therefore in the blood), thereby extending the time for which the GCase is stable, and thus active. For example, substituting an amino acid which is a proton donor at an acidic pH but not a proton donor at physiological pH *(e.g.* glutamic acid) with an amino acid which is a proton donor at an acidic pH and also a proton donor at physiological pH (e.g. glutamine) may serve to stabilise the GCase polypeptide.

Optionally, the at least one mutation comprises (or consists of) 20 or fewer, 10 or fewer, 5 or fewer, 4 or fewer, 3 or fewer, or 2 or fewer mutations. Optionally, the at least one mutation comprises (or consists of) 10 or fewer, 5 or fewer, 4 or fewer, 3 or fewer, or 2 or fewer mutations. Optionally, the at least one mutation comprises (or consists of) 5 or fewer, 4 or fewer, 3 or fewer, or 2 or fewer mutations. Optionally, the at least one mutation comprises (or consists of) 3 or fewer mutations. Optionally, the at least one mutation comprises (or consists of) 2 or fewer mutations. Optionally, the at least one mutation comprises (or consists of) one mutation. Optionally, the at least one mutation comprises (or consists of) two mutations.

The modified GCase polypeptide of the invention is functional. A functional GCase polypeptide is one which carries out hydrolysis of glucocerebroside. It is within the abilities of the person skilled in the art to determine whether a GCase polypeptide is functional. The skilled person merely needs to test whether the GCase polypeptide is active. For example, the skilled person could determine the specific activity of the GCase polypeptide as discussed herein. If the modified GCase polypeptide has at least 20% (optionally, at least 30%, at least 40%, at least 50%, or at least 75%) of the specific activity of a wild-type GCase polypeptide (determined using the same method), then it is functional.

Preferably, the modified GCase polypeptide of the invention is a modified human GCase polypeptide.

Reference to the "*activity*" (also referred to as "*GCase activity"* or *"enzymatic activity")* of a GCase polypeptide herein refers to the observed activity in a functional assay for determining the activity of the GCase polypeptide. The activity of the GCase polypeptide can be analysed using a chromogenic assay or fluorometric assay such as those described herein. For example, a suitable fluorometric assay is as follows. The GCase polypeptide is incubated with 4-Methylumbelliferyl-β-D-glucopyranoside (4-MUG) which is a fluorogenic substrate of GCase. Hydrolysis of 4-MUG by GCase releases a fluorescent product, 4-Methylumbelliferone (4-MU). The level of fluorescent product generated can be measured using emission and excitation wavelengths of 365nm and 445nm, respectively. Optionally, the fluorescence levels are converted to nanomoles/hour/mL based on a 4-MU (e.g. Sigma-Aldrich) standard curve. Optionally, fluorescence levels are converted to mU/ml *(e.g.* of plasma) or mU/mg *(e.g.* of protein) based on a VPRIV standard curve. Optionally, the GCase polypeptide is incubated with 4-MUG for 30 minutes. Optionally, the GCase polypeptide is incubated with 4-MUG at 37°C. Optionally, the GCase polypeptide is incubated with 4-MUG at 37°C for 30 minutes at pH 5.75. Optionally, the GCase polypeptide is incubated with 4-MUG for 1 hour at 37°C in the presence and absence of an irreversible GCase inhibitor, e.g. Conduritol B epoxide. For example, a portion of the sample is incubated in the presence of, and a portion of the sample is incubated in the absence of, an irreversible GCase inhibitor, e.g. Conduritol B epoxide, and the results are compared to obtain the level of activity of the GCase. Optionally, the GCase polypeptide is incubated at pH 5.6 or pH 5.75. The activity of the GCase polypeptide may be measured according to the protocol described in the section entitled *"GCase activity determination"* in Example 1 or described in the paragraph beginning *"GCase activity assay"* in the section entitled "*Methods*"in Example 13.

In some embodiments, the GCase polypeptide is purified prior to measuring the activity. Protein may be purified using known techniques in the art, such as column chromatography (e.g. affinity chromatography, ion exchange chromatography, hydrophobic interaction chromatography, size exclusion chromatography), HPLC or separation based on charge or hydrophobicity. For example, protein may be purified using a sepharose column (affinity chromatography resin). For example, protein may be purified using a sepharose column (affinity chromatography resin) as described in Example 1. The GCase polypeptide activity may be measured by a fluorometric assay (such as those described herein) carried out on the purified GCase polypeptide.

Reference to the *"effective activity"* (also referred to as *"effective GCase activity"*) herein refers to the observed activity of a GCase polypeptide following the expression of a GBA nucleotide sequence encoding the GCase polypeptide in a biological system. The "*effective activity"* is a function of the enzymatic efficiency of the GCase polypeptide and the stability of the GCase polypeptide. *"Effective activity"* may also be referred to as *"total resultant activity ".* In some embodiments, the increase in effective activity is as a result of an increase in stability of the modified GCase polypeptide. In some embodiments, the increase in effective activity is as a result of an increase in enzymatic efficiency of the modified GCase polypeptide. In some embodiments, the increase in effective activity is as a result of an increase in enzymatic efficiency and stability of the modified GCase polypeptide. In some embodiments, the effective activity of the GCase polypeptide is determined by generating an AAV particle comprising a polynucleotide comprising a GBA nucleotide sequence encoding the GCase polypeptide operably linked to a promoter, transducing cells (such as human cells, optionally Huh-7 cells) with the AAV particle, harvesting GCase polypeptide from the cells and/or culture medium, and measuring the activity of the GCase polypeptide, optionally, using a fluorometric assay. Optionally, the AAV particle comprising a polynucleotide comprising a GBA nucleotide sequence encoding the GCase polypeptide is incubated with Huh-7 cells for around 16 hours to allow transduction, and the GCase polypeptide is harvested from the culture medium. Optionally, the AAV particle comprises the capsid of SEQ ID NO: 37. Optionally, the transduction occurs at a multiplicity of infection of 5x10³, 1x10⁴ or 5x10⁴ vg/cell. The activity of the GCase polypeptide harvested may be measured according to a fluorometric assay protocol as described herein. For example, the GCase polypeptide may be incubated with 4-MUG for 30 minutes at 37°C. Optionally, the GCase polypeptide is incubated with 4-MUG at 37°C for 30 minutes at pH 5.75. Optionally, the GCase polypeptide is incubated with 4-MUG for 1 hour at 37°C in the presence and absence of an irreversible GCase inhibitor, *e.g.* Conduritol B epoxide. For example, a portion of the sample is incubated in the presence of, and a portion of the sample is incubated in the absence of, an irreversible GCase inhibitor, *e.g.* Conduritol B epoxide, and the results are compared to obtain the level of activity of the GCase. Optionally, the GCase polypeptide is incubated at pH 5.6 or pH 5.75. The level of fluorescent product generated can be measured using emission and excitation wavelengths of 365nm and 445nm, respectively. Fluorescence levels may be converted to nanomoles/hour/mL based on a 4-Methylumbelliferone (4-MU, e.g. Sigma-Aldrich) standard curve. Optionally, the activity of the GCase polypeptide harvested is measured according to the fluorometric assay protocol described in the section entitled "*GCase activity determination*" in Example 1 or described in the paragraph beginning "*GCase activity assay*" in the section entitled "*Methods*" in Example 13. Optionally, the activity is normalized by vector genome copy number. The number of vector genomes present in the cells post-transduction may be determined (e.g. by qPCR) and used to adjust the activity of the GCase polypeptide measured. Optionally, the number of vector genomes present in the cells post-transduction is determined by qPCR. Optionally, the number of vector genomes present in the cells post-transduction is determined by qPCR using primer sets that bind to the promoter (e.g. than bind to an LSP-L promoter). Optionally, the vector genome copy number is determined according to the first paragraph of the section entitled "*Vector genome copy number*" in Example 1. Optionally, the effective activity of the GCase polypeptide is determined using the methodology in Example 8.

In some embodiments, the effective activity of the GCase polypeptide is determined by generating an AAV particle comprising a polynucleotide comprising a GBA nucleotide sequence encoding the GCase polypeptide operably linked to a promoter, injecting mice with the AAV particle, and measuring the activity level in plasma, white blood cells and/or tissues (such as liver, spleen, bone marrow and/or lung) from the mice, optionally, using a fluorometric assay. Optionally, the AAV particle comprising a polynucleotide comprising a GBA nucleotide sequence encoding the GCase polypeptide may be injected into mice at a dose of *e.g.* from 2x10⁹ vg/kg to 2x10¹² vg/kg via a single tail vein injection. Optionally, the dose is selected from 2x10⁹ vg/kg, 2x10¹⁰ vg/kg, 2x10¹¹ vg/kg, 6x10¹¹ vg/kg, and 2x10¹² vg/kg. Optionally, the dose is 6x10¹⁰ vg/kg. Optionally, the AAV particle comprises an AAV8 capsid. Plasma samples may be obtained 14 days post-treatment (by retro-orbital bleeding) or 28 days post-treatment (by retro-orbital bleeding). The animal may be culled 4 or 6 weeks post-treatment and the plasma, white blood cells, and/or other tissues (e.g. liver, spleen, bone marrow, and/or lung) harvested. Optionally, the animal is culled 4 weeks post-treatment. The activity of the GCase polypeptide in the harvested plasma, white blood cells, and/or other tissues may be measured according to a fluorometric assay protocol as described herein. For example, the GCase polypeptide may be incubated with 4-MUG for 30 minutes at 37°C. Optionally, the GCase polypeptide is incubated with 4-MUG at 37°C for 30 minutes at pH 5.75. Optionally, the GCase polypeptide is incubated with 4-MUG for 1 hour at 37°C in the presence and absence of an irreversible GCase inhibitor, *e.g.* Conduritol B epoxide. For example, a portion of the sample is incubated in the presence of, and a portion of the sample is incubated in the absence of, an irreversible GCase inhibitor, *e.g.* Conduritol B epoxide, and the results are compared to obtain the level of activity of the GCase. Optionally, the GCase polypeptide is incubated at pH 5.6 or pH 5.75. The level of fluorescent product generated can be measured using emission and excitation wavelengths of 365nm and 445nm, respectively. The activity of the GCase polypeptide in plasma, tissue and WBCs can be presented in mU/ml of plasma (for plasma) or mU/mg of protein (for tissue and WBCs), using a VPRIV standard curve. Optionally, GCase activity can be calculated as nmole/h/ml or nmole/h/mg, for plasma or tissues, respectively, using a 4-MU standard curve. Optionally, the activity of the GCase polypeptide in the harvested plasma, white blood cells, and/or other tissues may be measured according to the fluorometric assay protocol described in the section entitled *"GCase activity determination"* in Example 1 or described in the paragraph beginning *"GCase activity assay"* in the section entitled "*Methods*"in Example 13. Optionally, the activity is normalized by vector genome copy number. The number of vector genomes present in the liver post-AAV particle injection may be determined (*e.g.* by qPCR) and used to adjust the activity of the GCase polypeptide measured. Optionally, the number of vector genomes present in the liver post-AAV particle injection is determined by qPCR. Optionally, the number of vector genomes present in the liver post-AAV particle injection is determined by qPCR using primer sets that bind to the promoter (e.g. that bind to an LSP-L promoter). Optionally, the vector genome copy number is determined according to the second paragraph of the section entitled *"Vector genome copy number"* in Example 1. Optionally, the effective activity of the GCase polypeptide is determined using the methodology in Example 9.

A modified GCase polypeptide which comprises at least one mutation is described. The at least one mutation may:
(i) provide higher effective activity; and/or
(ii) provide increased stability; and/or
(iii) provide structural stabilisation at physiological pH; and/or
(iv) provide a longer half-life; and/or
(v) provide increased thermostability.

The at least one mutation may provide higher effective activity. Exemplary mutations which provide higher effective activity are disclosed herein. By specifying that the *"at least one mutation provides higher effective activity",* the modified GCase polypeptide comprising the at least one mutation has higher effective activity than a reference GCase polypeptide that does not comprise the at least one mutation but is otherwise identical to the modified GCase polypeptide comprising the at least one mutation. For example, if the at least one mutation provides higher effective activity and the modified GCase polypeptide comprising the at least one mutation is otherwise identical to a wild type GCase polypeptide except that it comprises the at least one mutation (*e.g.* mutations W351C and A380C), the modified GCase polypeptide comprising the at least one mutation has higher effective activity than the wild type GCase polypeptide. For example, the at least one mutation *"provides higher effective activity"* if a *"test"* polypeptide that is identical to SEQ ID NO: 1 except for the at least one mutation has higher effective activity compared to the polypeptide of SEQ ID NO: 1, optionally where the *"test"* polypeptide and SEQ ID NO: 1 are produced by expression in the same cell line.

The modified GCase polypeptide comprising the at least one mutation may not necessarily have higher effective activity than a wild type GCase polypeptide. For example, it is envisaged that the modified GCase polypeptide comprising the at least one mutation may comprise one or more further modifications (such as a deleted portion) which reduce the effective activity of the polypeptide. In such a case, the at least one mutation disclosed herein may serve to provide higher effective activity for the modified GCase polypeptide such that the modified GCase polypeptide may not have the same effective activity as a wild type GCase polypeptide but nevertheless has higher effective activity than a reference GCase polypeptide that is otherwise identical to the modified GCase polypeptide but lacks the at least one mutation. Alternatively, the at least one mutation may provide increased effective activity which restores the effective activity of the modified GCase polypeptide to the level of effective activity of a wild type GCase polypeptide.

The modified GCase polypeptide of the invention comprising at least one mutation has higher effective activity relative to a reference GCase polypeptide. The reference GCase polypeptide may be the polypeptide of SEQ ID NO: 3. The reference GCase polypeptide may be the polypeptide of SEQ ID NOs: 4 or 5. The effective activity of a modified GCase may be compared to the effective activity of a reference GCase polypeptide. Exemplary mutations which increase the effective activity of a modified GCase polypeptide are disclosed herein.

The *"reference GCase polypeptide"* is a GCase polypeptide that does not comprise the at least one mutation of the modified GCase polypeptide of the invention. Optionally, the reference GCase polypeptide does not comprise the at least one mutation but is otherwise identical to the modified GCase polypeptide of the invention. The reference GCase polypeptide may be the polypeptide of any one of SEQ ID NOs: 1-5. The reference GCase polypeptide may be a wild-type GCase polypeptide. The reference GCase polypeptide may be any wild-type GCase polypeptide. The reference GCase polypeptide may be the polypeptide of any one of SEQ ID NOs: 1-3. The reference GCase polypeptide may be the polypeptide of SEQ ID NO: 1. The reference GCase polypeptide may be the polypeptide of SEQ ID NO: 2 or SEQ ID NO: 3. The reference GCase polypeptide may be the polypeptide of SEQ ID NO: 3. Optionally, the reference GCase polypeptide is not a wild-type GCase polypeptide and does not comprise the at least one mutation. Optionally, the reference GCase polypeptide comprises a mutation to leucine at a position corresponding to position 184 of SEQ ID NO: 1, optionally H184L. Optionally, the reference GCase polypeptide comprises a mutation to leucine at a position corresponding to position 184 of SEQ ID NO: 1, optionally H184L, and the reference GCase polypeptide also comprises a mutation to asparagine at a position corresponding to position 360 of SEQ ID NO: 1, optionally K360N. Optionally, the reference GCase polypeptide comprises H184L and K360N. The reference GCase polypeptide may be the polypeptide of SEQ ID NO: 4 or 5. In some embodiments, the reference GCase polypeptide comprises the polypeptide of SEQ ID NO: 41 or 44. In some embodiments, the reference GCase polypeptide comprises the polypeptide of SEQ ID NO: 43 or 46. In some embodiments, the reference GCase polypeptide comprises the polypeptide of SEQ ID NO: 42 or 45.

In some embodiments, the reference GCase polypeptide may be a wild-type GCase polypeptide such as VPRIV. Optionally, VPRIV is reconstituted in sterile or nuclease-free water. In some embodiments, the reference GCase polypeptide may be imiglucerase. In some embodiments, the reference GCase polypeptide comprises a mutation to histidine at a position corresponding to position 534 of SEQ ID NO: 1, optionally R534H. In some embodiments, the reference GCase polypeptide comprises or is the sequence of SEQ ID NO: 1 or 2 except for a mutation to histidine at a position corresponding to position 534 of SEQ ID NO: 1.

For the purposes of determining whether a modified GCase polypeptide has higher effective activity relative to a reference GCase polypeptide (as described herein, such as a wild-type GCase polypeptide), the effective activity of the modified GCase polypeptide and reference GCase polypeptide are determined using the same method and the results compared. The method may be any of the methods described herein for determining effective activity.

The modified GCase polypeptide of the invention may have an effective activity which is at least 1.2 fold, at least 1.5 fold, at least 2 fold, at least 2.5 fold, at least 3 fold, at least 3.5 fold, at least 4 fold, at least 4.5 fold, at least 5 fold, at least 5.5 fold, at least 6 fold, at least 6.5 fold, at least 7 fold, at least 7.5 fold, at least 8 fold, at least 9 fold, at least 10 fold, at least 15 fold, at least 20 fold, at least 25 fold, at least 30 fold, at least 35 fold, at least 40 fold, at least 45 fold, at least 50 fold higher than the effective activity of the reference GCase polypeptide (such as a wild-type GCase polypeptide). The modified GCase polypeptide may have an effective activity which is at least 1.2 fold, at least 1.5 fold, at least 2 fold, at least 2.5 fold, at least 3 fold, at least 3.5 fold, at least 4 fold, at least 4.5 fold, at least 5 fold, at least 5.5 fold, at least 6 fold, at least 6.5 fold, at least 7 fold, at least 7.5 fold, at least 8 fold, at least 10 fold, at least 15 fold, at least 20 fold, at least 35 fold, at least 40 fold, at least 45 fold, or at least 50 fold higher than the effective activity of the reference GCase polypeptide (such as wild-type GCase polypeptide). The modified GCase polypeptide may have an effective activity which is at least 5 fold, at least 10 fold, at least 25 fold, or at least 50 fold higher than the effective activity of the reference GCase polypeptide (such as wild-type GCase polypeptide). The modified GCase polypeptide may have an effective activity which is between 1.2 and 3 fold, between 1.2 and 5 fold, between 3 fold and 6 fold, between 5 fold and 10 fold, between 1.5 fold and 8 fold, between 2 fold and 10 fold, between 3 fold and 15 fold, between 6 fold and 15 fold, between 10 fold and 15 fold, between 15 fold and 20 fold, between 10 fold and 30 fold, between 20 fold and 30 fold, between 25 fold and 30 fold, between 25 fold and 40 fold, or between 30 fold and 50 fold higher than the effective activity of the reference GCase polypeptide (such as a wild-type GCase polypeptide). The modified GCase polypeptide may have an effective activity which is between 2 and 100 fold, between 10 and 100 fold, between 10 and 80 fold, between 25 fold and 100 fold, or between 25 fold and 80 fold higher than the effective activity of the reference GCase polypeptide (such as a wild-type GCase polypeptide). When referring to fold changes of activity, the term *"between"* includes the specified values. Thus, for example, *"between 1.5 fold and 8 fold"* includes the values 1.5 fold and 8 fold.

The at least one mutation may provide increased stability (also referred to as higher stability). Exemplary mutations which provide increased stability are disclosed herein. By specifying that the *"at least one mutation provides increased stability ",* the modified GCase polypeptide comprising the at least one mutation has higher stability than a reference GCase polypeptide that does not comprise the at least one mutation but is otherwise identical to the modified GCase polypeptide comprising the at least one mutation. For example, if the at least one mutation provides increased stability and the modified GCase polypeptide comprising the at least one mutation is otherwise identical to a wild type GCase polypeptide except that it comprises the at least one mutation (*e.g.* mutations W351C and A380C), the modified GCase polypeptide comprising the at least one mutation has higher stability than the wild type GCase polypeptide. For example, the at least one mutation *"provides increased stability"* if a *"test"* polypeptide that is identical to SEQ ID NO: 1 except for the at least one mutation has higher stability compared to the polypeptide of SEQ ID NO: 1, optionally where the *"test"* polypeptide and SEQ ID NO: 1 are produced by expression in the same cell line. The modified GCase polypeptide comprising the at least one mutation may not necessarily have higher stability than a wild type GCase polypeptide. For example, it is envisaged that the modified GCase polypeptide comprising the at least one mutation may comprise one or more further modifications (such as a deleted portion) which reduce the stability of the polypeptide. In such a case, the at least one mutation disclosed herein may serve to provide higher stability for the modified GCase polypeptide such that the modified GCase polypeptide may not have the same stability as a wild type GCase polypeptide but nevertheless has higher stability than a reference GCase polypeptide that is otherwise identical to the modified GCase polypeptide but lacks the at least one mutation. Alternatively, the at least one mutation may provide increased stability which restores the stability of the modified GCase polypeptide to the level of stability of a wild type GCase polypeptide.

The modified GCase polypeptide of the invention comprising at least one mutation has increased stability relative to a reference GCase polypeptide. In some embodiments, the increased stability provided by the at least one mutation or the increased stability of the modified GCase polypeptide comprising at least one mutation is increased stability at pH 7.4. In some embodiments, the increased stability is measured after 120 minutes of incubation at pH 7.4 and 37 degrees Celsius. In some embodiments, the increased stability provided by the at least one mutation or the increased stability of the modified GCase polypeptide comprising at least one mutation is increased stability at pH 5.6. In some embodiments, the increased stability is measured after 72 hours of incubation at pH 5.6 and 37 degrees Celsius. The reference GCase polypeptide may be the polypeptide of SEQ ID NO: 3. The reference GCase polypeptide may be the polypeptide of SEQ ID NO: 4 or 5. Optionally, the modified GCase polypeptide comprising at least one mutation has increased stability relative to the reference GCase polypeptide. Optionally, the modified GCase polypeptide comprising at least one mutation has increased stability relative to the reference GCase polypeptide and the increased stability is increased stability at pH 7.4. The stability of a modified GCase may be compared to the stability of a reference GCase polypeptide. Exemplary mutations which increase stability of a modified GCase polypeptide are disclosed herein. For example, mutations which enable the formation of a disulphide bridge.

A modified GCase polypeptide which has increased stability relative to a reference GCase polypeptide retains a higher proportion of GCase activity over time than the reference GCase polypeptide. In some embodiments, the stability of a GCase polypeptide is determined by measuring the residual enzymatic activity of a GCase polypeptide. Reference to the *"residual enzymatic activity"* (also referred to as *"residual GCase activity"* or *"residual activity"*) herein refers to the activity of a GCase polypeptide which is retained after a specific time period has lapsed. The residual enzymatic activity of GCase may be measured in a sample after one or more specific time periods have lapsed. Optionally, the sample is obtained by transfecting a host cell (*e.g.* an Expi293F cell) with an expression vector comprising a nucleotide sequence encoding the GCase polypeptide, and harvesting GCase polypeptide from the culture medium. Optionally, the GCase polypeptide is harvested from the culture medium four days post transfection. Optionally the sample is obtained according to the methods described in the sections entitled "*Expansion of Expi293F cells*"*,* "*The day before transfection of Expi293F cells*", "*Transfection of Expi293F cells*" and "*Harvest of transfected Expi293F cells*" in Example 1. A sample containing the GCase polypeptide may be transferred into a matrix (such as AB buffer at pH 5.6, PBS at pH 7.4, serum, or plasma) and incubated. The pH of the matrix may be selected in order to determine the residual activity (or stability) at that particular pH. The temperature of the incubation may be selected in order to determine the residual activity (or stability) at that particular temperature. Aliquots of the incubated sample containing the GCase polypeptide may be taken at a series of time points, *e.*g. two or more, three or more, four or more, five or more, or six or more time points, in addition to taking an aliquot of the sample at an initial time point, and GCase activity in each aliquot may be determined. The initial time point (*i.e.* 0 mins or 0 hours) is the time at which the sample is transferred into the matrix for incubation. Comparing GCase activity at a given time point with GCase activity at the initial time point allows residual GCase activity to be determined at the given time point. Aliquots of the sample containing the GCase polypeptide may be taken at 0 min, 30 mins, 60 mins, 120 mins, 3 days, 4 days, 5 days, 6 days, and/or 7 days. Optionally, the sample is incubated in PBS at pH 7.4 for 120 minutes. Optionally, the sample is incubated in PBS at pH 7.4 for 72 hrs. Optionally, the sample is incubated in PBS at pH 7.4 for 7 days. Optionally, the sample is incubated in AB buffer at pH 5.6 for 72 hours. Optionally, the residual enzymatic activity is measured in a sample according to the section entitled "*Stability assessment*" in Example 1. The activity of the GCase polypeptide in the aliquots may be measured according to a fluorometric assay protocol as described herein. For example, the GCase polypeptide may be incubated with 4-MUG for 30 minutes at 37°C. Optionally, the GCase polypeptide is incubated with 4-MUG at 37°C for 30 minutes at pH 5.75. Optionally, the GCase polypeptide is incubated with 4-MUG for 1 hour at 37°C in the presence and absence of, an irreversible GCase inhibitor, *e.g.* Conduritol B epoxide. For example, a portion of the sample is incubated in the presence of, and a portion of the sample is incubated in the absence of, an irreversible GCase inhibitor, *e.g.* Conduritol B epoxide, and the results are compared to obtain the level of activity of the GCase. Optionally, the GCase polypeptide is incubated at pH 5.6 or pH 5.75. The level of fluorescent product generated can be measured using emission and excitation wavelengths of 365nm and 445nm, respectively. Optionally, the activity of the GCase polypeptide in the aliquots may be measured according to the fluorometric assay protocol described in the section entitled *"GCase activity determination"* in Example 1 or described in the paragraph beginning *"GCase activity assay"* in the section entitled "*Methods*" in Example 13. The GCase activity at a given time point may be compared to the initial GCase activity of the sample (*i.e.* by also determining GCase activity at the initial time point), and the residual GCase activity may be calculated as a percentage of the initial GCase activity. Thus, the modified GCase polypeptide having increased stability relative to the reference GCase polypeptide may have a higher residual activity (*i.e.* as a percentage of the initial GCase polypeptide activity) than the reference GCase polypeptide. Optionally, the reference GCase polypeptide may be a wild-type GCase polypeptide such as VPRIV. Optionally, VPRIV is reconstituted in nuclease-free or sterile water. Optionally, the residual enzymatic activity of the GCase polypeptide is determined using the methodology of Example 3. Optionally, the residual enzymatic activity of the GCase polypeptide is determined using the methodology of Example 4.

Optionally, specific activity may be determined at each time point. "*Specific activity"* refers to the activity per unit of GCase polypeptide such that the activity is 'normalised' to take account of the amount or concentration of GCase polypeptide in the sample. This can be done by measuring the concentration of the GCase polypeptide in the sample, for example by using a standard ELISA assay, and dividing the activity by the GCase concentration. A fluorometric assay may be used to measure *"specific activity".* The fluorometric assay may be any one of the fluorometric assays described herein.

In an example of an ELISA assay, an antibody that binds to the GCase polypeptide could be bound to a plate. The sample, comprising the GCase polypeptide at unknown concentration, could be passed over the plate. A second detection antibody that binds to the GCase polypeptide could be applied to the plate, and any excess washed off. The detection antibody that remains (*i.e.* is not washed off) will be bound to the GCase polypeptide. The detection antibody could be linked to an enzyme such as horse radish peroxidase. The level of detection antibody that binds to the GCase polypeptide on the plate could be measured by measuring the amount of the detection antibody. For example, if the detection antibody is linked to horse radish peroxidase, the horse radish peroxidase can catalyse the production of a blue reaction product from a substrate such as TMB (3,3',5,5'-tetramethylbenzidine), and the level of the blue product can be detected by absorbance at 450 nm. The level of the blue product is proportional to the amount of detection antibody that remained after the washing step, which is proportional to the amount of the GCase polypeptide in the sample. Alternatively, for example when using purified protein, the amount or concentration of GCase polypeptide may be determined spectrophotometrically.

In some embodiments, the stability of a GCase polypeptide is determined by determining the half-life of a GCase polypeptide. The half-life may be determined as described herein.

For the purposes of determining whether a modified GCase polypeptide has increased stability relative to a reference GCase polypeptide (as described herein, such as a wild-type GCase polypeptide), the stability of the modified GCase polypeptide and reference GCase polypeptide are determined using the same method and the results compared. The method may be any of the methods described herein for determining stability.

The stability or residual activity of the modified GCase polypeptide may be determined after incubation of at least 5 minutes, at least 10 minutes, at least 15 minutes, at least 20 minutes, at least 25 minutes, at least 30 minutes, at least 45 minutes, at least 1 hour, at least 2 hours, at least 3 hours, at least 4 hours, at least 5 hours, at least 6 hours, at least 7 hours, at least 8 hours, at least 9 hours, at least 10 hours, at least 11 hours, at least 12 hours, at least 18 hours, at least 24 hours, at least 2 days, at least 3 days, at least 4 days, at least 5 days, at least 6 days, or at least 7 days. Optionally, increased stability or increased residual activity of the modified GCase polypeptide in a sample may be observed after incubation of at least 5 minutes, at least 10 minutes, at least 15 minutes, at least 20 minutes, at least 25 minutes, at least 30 minutes, at least 45 minutes, at least 1 hour, at least 2 hours, at least 3 hours, at least 4 hours, at least 5 hours, at least 6 hours, at least 7 hours, at least 8 hours, at least 9 hours, at least 10 hours, at least 11 hours, at least 12 hours, at least 18 hours, at least 24 hours, at least 2 days, at least 3 days, at least 4 days, at least 5 days, at least 6 days, or at least 7 days.

The residual activity of the modified GCase polypeptide may be expressed as a percentage of the initial GCase activity. Herein, where it is specified that the modified GCase polypeptide *"retains"* at least X% activity (*e.g.* X may be 30) at a particular time point, this should be interpreted to mean that the modified GCase polypeptide has at least X% residual activity (*e.g*. has at least 30% residual activity) at the particular time point. The modified GCase polypeptide may retain at least 30%, at least 35%, at least 40%, at least 45%, at least 50%, at least 55%, at least 60%, at least 65%, at least 70%, at least 75%, at least 80%, at least 85%, at least 90%, or at least 95% activity when measured as described herein (e.g. when the activity is measured over time and the activity at a given time point is compared to the initial GCase activity of the sample). The activity may be measured after incubation at pH 5.6. The modified GCase polypeptide may retain at least 70%, at least 75%, at least 80%, or at least 85% activity when measured after 120 mins of incubation at pH 5.6 and 37 degrees Celsius. The modified GCase polypeptide may retain at least 80%, or at least 85% activity when measured after 120 mins of incubation at pH 5.6 and 37 degrees Celsius. The modified GCase polypeptide may retain at least 90%, or at least 95% activity when measured after 120 mins of incubation at pH 5.6 and 37 degrees Celsius. The modified GCase polypeptide may retain at least 60% activity when measured after 72 hrs of incubation at pH 5.6 and 37 degrees Celsius. The modified GCase polypeptide may retain at least 80% activity when measured after 72 hrs of incubation at pH 5.6 and 37 degrees Celsius. The modified GCase polypeptide may retain at least 85% activity when measured after 72 hrs of incubation at pH 5.6 and 37 degrees Celsius. The modified GCase polypeptide may retain at least 40% activity when measured after 7 days of incubation at pH 5.6 and 37 degrees Celsius. The modified GCase polypeptide may retain at least 60% activity when measured after 7 days of incubation at pH 5.6 and 37 degrees Celsius. The modified GCase polypeptide may retain at least 80% activity when measured after 7 days of incubation at pH 5.6 and 37 degrees Celsius. The activity may be measured after incubation at pH 7.4. The activity may be measured after incubation at 37 degrees Celsius. The activity may be measured after incubation at pH 7.4 and 37 degrees Celsius. The incubation may be in PBS. The incubation may be in serum or plasma. The modified GCase polypeptide may retain at least 30%, at least 35%, at least 40%, at least 45%, at least 50%, at least 55%, at least 60%, at least 65%, at least 70%, at least 75%, at least 80%, or at least 85% activity when measured after at least 10 minutes, at least 30 minutes, at least 60 minutes, at least 120 minutes, at least 1 day, at least 2 days, at least 3 days, at least 4 days, at least 5 days, at least 6 days, or at least 7 days of incubation at pH 7.4 and 37 degrees Celsius. In some embodiments, the modified GCase polypeptide retains activity that is at least 1.1 fold, at least 1.2 fold, at least 1.3 fold, at least 1.4 fold, at least 1.5 fold, at least 1.6 fold, at least 1.7 fold, at least 1.8 fold, at least 1.9 fold, at least 2 fold, at least 2.1 fold, at least 2.2 fold, at least 2.3 fold, at least 2.4 fold, at least 2.5 fold, at least 2.6 fold, at least 2.7 fold, at least 2.8 fold, at least 2.9 fold, or at least 3 fold higher than the activity of the reference GCase polypeptide (such as wild-type GCase polypeptide) as measured under the same conditions (*e.g.* activity of the modified GCase polypeptide and the reference (*e.g.* wild type) GCase each being measured after 120 minutes of incubation at pH 7.4 and 37 degrees Celsius). The reference to *"same conditions"* indicates that any variations which are acceptable in view of experimental tolerances are encompassed. The modified GCase polypeptide may retain activity which is at least 50 fold, at least 60 fold, at least 70 fold, at least 80 fold, or at least 90 fold higher than the activity of the reference GCase polypeptide as measured under the same conditions. The modified GCase polypeptide may retain activity which is at least 1.1 fold, at least 1.2 fold, at least 1.3 fold, at least 1.4 fold, at least 1.5 fold, at least 1.8 fold, at least 2 fold, at least 2.5 fold, at least 2.8 fold, or at least 3 fold higher than the activity of the reference GCase polypeptide when measured after 120 minutes of incubation at pH 7.4 and 37 degrees Celsius. The modified GCase polypeptide may retain activity which is between 1.1 fold and 10 fold, between 2 fold and 8 fold, or between 2 fold and 5 fold higher than the activity of the reference GCase polypeptide when measured after 120 minutes of incubation at pH 7.4 and 37 degrees Celsius. The modified GCase polypeptide may retain at least 50%, at least 55%, at least 60%, at least 65%, at least 70%, at least 75%, at least 80%, or at least 85% activity when measured after 120 minutes of incubation at pH 7.4 and 37 degrees Celsius. The modified GCase polypeptide may retain at least 70%, at least 75%, at least 80%, or at least 85% activity when measured after 120 mins of incubation at pH 7.4 and 37 degrees Celsius. The modified GCase polypeptide may retain activity which is at least 50 fold, at least 60 fold, at least 70 fold, at least 80 fold, or at least 90 fold higher than the activity of the reference GCase polypeptide when measured after 72 hrs of incubation at pH 7.4 and 37 degrees Celsius. The modified GCase polypeptide may retain activity which is between 1.1 fold and 150 fold, between 2 fold and 120 fold, or between 2 fold and 100 fold higher than the activity of the reference GCase polypeptide when measured after 72 hrs of incubation at pH 7.4 and 37 degrees Celsius. The modified GCase polypeptide may retain at least 50%, at least 55%, at least 60%, at least 65%, at least 70%, at least 75%, at least 80%, or at least 85% activity when measured after 72 hrs of incubation at pH 7.4 and 37 degrees Celsius. The modified GCase polypeptide may retain at least 60%, at least 70%, at least 75%, at least 80%, or at least 85% activity when measured after 72 hrs of incubation at pH 7.4 and 37 degrees Celsius. The modified GCase polypeptide may retain activity which is at least 1.1 fold, at least 1.2 fold, at least 1.5 fold, at least 2 fold, at least 5 fold, at least 10 fold, at least 20 fold, at least 50 fold, at least 60 fold, at least 70 fold, at least 80 fold, or at least 90 fold higher than the activity of the reference GCase polypeptide when measured after 7 days of incubation at pH 7.4 and 37 degrees Celsius. The modified GCase polypeptide may retain activity which is between 1.1 fold and 150 fold, between 2 fold and 120 fold, or between 2 fold and 100 fold higher than the activity of the reference GCase polypeptide when measured after 7 days of incubation at pH 7.4 and 37 degrees Celsius. The modified GCase polypeptide may retain at least 15% activity when measured after 7 days of incubation at pH 7.4 and 37 degrees Celsius. The modified GCase polypeptide may retain at least 20% activity when measured after 7 days of incubation at pH 7.4 and 37 degrees Celsius. The modified GCase polypeptide may retain at least 40% activity when measured after 7 days of incubation at pH 7.4 and 37 degrees Celsius. The incubation may be in serum or plasma. The incubation may be in PBS. The modified GCase polypeptide may retain at least 60% activity when measured after 7 days of incubation at pH 7.4 and 37 degrees Celsius. The modified GCase polypeptide may retain at least 80% activity when measured after 7 days of incubation at pH 7.4 and 37 degrees Celsius. The activity may be measured after incubation in human plasma. The modified GCase polypeptide may retain activity which is at least 1.1 fold, at least 1.2 fold, at least 1.5 fold, at least 1.8 fold, at least 2 fold, at least 2.5 fold, at least 3 fold, at least 4 fold, or at least 5 fold higher than the activity of the reference GCase polypeptide when measured after 120 minutes of incubation in human plasma and at 37 degrees Celsius. The modified GCase polypeptide may retain activity which is between 1.1 fold and 10 fold, between 2 fold and 8 fold, or between 4 fold and 8 fold higher than the activity of the reference GCase polypeptide when measured after 120 minutes of incubation in human plasma and at 37 degrees Celsius. The modified GCase polypeptide may retain at least 20%, at least 25%, at least 30%, at least 35%, or at least 40% activity when measured after 120 minutes of incubation in human plasma and at 37 degrees Celsius. The modified GCase polypeptide may retain activity which is at least 10 fold, at least 20 fold, at least 30 fold, at least 40 fold, or at least 50 fold higher than the activity of the reference GCase polypeptide when measured after 72 hrs of incubation in human plasma and at 37 degrees Celsius. The modified GCase polypeptide may retain activity which is between 10 fold and 100 fold, between 20 fold and 100 fold, or between 20 fold and 80 fold higher than the activity of the reference GCase polypeptide when measured after 72 hrs of incubation in human plasma and at 37 degrees Celsius. The modified GCase polypeptide may retain at least 20%, at least 30%, at least 40%, or at least 50% activity when measured after 72 hrs of incubation in human plasma and at 37 degrees Celsius. The modified GCase polypeptide may retain activity which is at least 10 fold, at least 20 fold, at least 30 fold, at least 40 fold, or at least 50 fold higher higher than the activity of the reference GCase polypeptide when measured after 7 days of incubation in human plasma and at 37 degrees Celsius. The modified GCase polypeptide may retain activity which is between 10 fold and 100 fold, between 20 fold and 100 fold, or between 20 fold and 80 fold higher than the activity of the reference GCase polypeptide when measured after 7 days of incubation in human plasma and at 37 degrees Celsius.

The modified GCase polypeptide may retain at least 20% activity when measured after 7 days of incubation in human plasma and at 37 degrees Celsius. The modified GCase polypeptide may retain at least 30% activity when measured after 7 days of incubation in human plasma and at 37 degrees Celsius. The modified GCase polypeptide may retain at least 40% activity when measured after 7 days of incubation in human plasma and at 37 degrees Celsius. The activity may be measured after incubation in human serum. The modified GCase polypeptide may retain activity which is at least 1.1 fold, at least 1.2 fold, at least 1.5 fold, at least 1.8 fold, or at least 2 fold higher than the activity of the reference GCase polypeptide when measured after 120 minutes of incubation in human serum and at 37 degrees Celsius. The modified GCase polypeptide may retain activity which is between 1.1 fold and 10 fold, between 1.1 fold and 8 fold, or between 1.1 fold and 5 fold higher than the activity of the reference GCase polypeptide when measured after 120 minutes of incubation in human serum and at 37 degrees Celsius. The modified GCase polypeptide may retain at least 20%, at least 25%, at least 30%, at least 35%, or at least 40% activity when measured after 120 minutes of incubation in human serum and at 37 degrees Celsius. The modified GCase polypeptide may retain activity which is at least 1.1 fold, at least 1.2 fold, at least 1.5 fold, at least 1.8 fold, at least 2 fold, at least 2.5 fold, at least 3 fold, at least 4 fold, at least 5 fold higher, at least 10 fold, at least 20 fold, at least 30 fold, or at least 40 fold higher than the activity of the reference GCase polypeptide when measured after 72 hrs of incubation in human serum and at 37 degrees Celsius. The modified GCase polypeptide may retain activity which is between 1.1 fold and 100 fold, between 2 fold and 80 fold, or between 5 fold and 80 fold higher than the activity of the reference GCase polypeptide when measured after 72 hrs of incubation in human serum and at 37 degrees Celsius. The modified GCase polypeptide may retain at least 2%, at least 5%, at least 20%, at least 30%, or at least 40% activity when measured after 72 hrs of incubation in human serum and at 37 degrees Celsius. The modified GCase polypeptide may retain activity which is at least 10 fold, at least 20 fold, at least 30 fold, or at least 40 fold higher higher than the activity of the reference GCase polypeptide when measured after 7 days of incubation in human serum and at 37 degrees Celsius. The modified GCase polypeptide may retain activity which is between 10 fold and 100 fold, between 20 fold and 100 fold, or between 20 fold and 80 fold higher than the activity of the reference GCase polypeptide when measured after 7 days of incubation in human serum and at 37 degrees Celsius. The modified GCase polypeptide may retain at least 20% activity when measured after 7 days of incubation in human serum and at 37 degrees Celsius. The modified GCase polypeptide may retain at least 30% activity when measured after 7 days of incubation in human serum and at 37 degrees Celsius. The modified GCase polypeptide may retain at least 40% activity when measured after 7 days of incubation in human serum and at 37 degrees Celsius.

The modified GCase polypeptide may retain activity which is at least 1.5 fold, at least 1.7 fold or at least 1.8 fold higher than the activity of the reference GCase polypeptide when measured after 120 minutes of incubation at pH 7.4 (optionally in PBS) and at 37 degrees Celsius, wherein the reference GCase polypeptide comprises a histidine to leucine substitution at a position corresponding to position 184 of SEQ ID NO: 1. Optionally, the reference GCase polypeptide is SEQ ID NO: 4. The modified GCase polypeptide may retain activity which is at least 1.1 fold, or at least 1.2 fold higher than the activity of the reference GCase polypeptide when measured after 120 minutes of incubation at pH 7.4 (optionally in PBS) and at 37 degrees Celsius, wherein the reference GCase polypeptide comprises a histidine to leucine substitution at a position corresponding to position 184 of SEQ ID NO: 1 and a lysine to asparagine substitution at a position corresponding to position 360 of SEQ ID NO: 1. Optionally, the reference GCase polypeptide is SEQ ID NO: 5. The modified GCase polypeptide may retain activity which is at least 1.5, or at least 1.7 fold higher than the activity of the reference GCase polypeptide of SEQ ID NO: 3 when measured after 120 minutes of incubation at pH 7.4 (optionally in PBS) and at 37 degrees Celsius. The modified GCase polypeptide may retain activity which is at least 50 fold, at least 60 fold, at least 70 fold, at least 80 fold, or at least 90 fold higher than the activity of the reference GCase polypeptide when measured after 72 hours of incubation at pH 7.4 (optionally in PBS) and at 37 degrees Celsius, wherein reference GCase polypeptide comprises a histidine to leucine substitution at a position corresponding to position 184 of SEQ ID NO: 1. Optionally, the reference GCase polypeptide is SEQ ID NO: 4. The modified GCase polypeptide may retain activity which is at least 50 fold, at least 60 fold, at least 70 fold, at least 80 fold, or at least 90 fold higher than the activity of the reference GCase polypeptide when measured after 72 hours of incubation at pH 7.4 (optionally in PBS) and at 37 degrees Celsius, wherein the reference GCase polypeptide comprises a histidine to leucine substitution at a position corresponding to position 184 of SEQ ID NO: 1 and a lysine to asparagine substitution at a position corresponding to position 360 of SEQ ID NO: 1. Optionally, the reference GCase polypeptide is SEQ ID NO: 5. The modified GCase polypeptide may retain activity which is at least 50 fold, at least 60 fold, at least 70 fold, at least 80 fold, or at least 90 fold higher than the activity of the reference GCase polypeptide of SEQ ID NO: 3 when measured after 72 hours of incubation at pH 7.4 (optionally in PBS) and at 37 degrees Celsius. The modified GCase polypeptide may retain activity which is at least 1.1 fold, at least 1.5 fold, at least 2 fold, at least 5 fold, or at least 10 fold higher than the activity of the reference GCase polypeptide when measured after 7 days of incubation at pH 7.4 (optionally in PBS) and at 37 degrees Celsius, wherein reference GCase polypeptide comprises a histidine to leucine substitution at a position corresponding to position 184 of SEQ ID NO: 1. Optionally, the reference GCase polypeptide is SEQ ID NO: 4. The modified GCase polypeptide may retain activity which is at least 1.1 fold, at least 1.5 fold, at least 2 fold, at least 5 fold, or at least 10 fold higher than the activity of the reference GCase polypeptide when measured after 7 days of incubation at pH 7.4 (optionally in PBS) and at 37 degrees Celsius, wherein the reference GCase polypeptide comprises a histidine to leucine substitution at a position corresponding to position 184 of SEQ ID NO: 1 and a lysine to asparagine substitution at a position corresponding to position 360 of SEQ ID NO: 1. Optionally, the reference GCase polypeptide is SEQ ID NO: 5. The modified GCase polypeptide may retain activity which is at least 1.1 fold, at least 1.5 fold, at least 2 fold, at least 5 fold, or at least 10 fold higher than the activity of the reference GCase polypeptide of SEQ ID NO: 3 when measured after 7 days of incubation at pH 7.4 (optionally in PBS) and at 37 degrees Celsius. The modified GCase polypeptide may retain activity which is at least 3 fold, at least 4 fold, or at least 5 fold higher than the activity of the reference GCase polypeptide of SEQ ID NO: 3 when measured after 120 minutes of incubation in human plasma and at 37 degrees Celsius. The modified GCase polypeptide may retain activity which is at least 1.2 fold, at least 1.5 fold, at least 1.8 fold, or at least 2 fold higher than the activity of the reference GCase polypeptide of SEQ ID NO: 3 when measured after 120 minutes of incubation in human serum and at 37 degrees Celsius.

The modified GCase polypeptide may retain at least 30%, at least 35%, at least 40%, at least 45%, at least 50%, at least 55%, at least 60%, at least 65%, at least 70%, at least 75%, at least 80%, or at least 85% activity when measured after at least 3 days, at least 4 days, at least 5 days, at least 6 days, or at least 7 days of incubation at pH 5.6 and 37 degrees Celsius. Optionally the modified GCase polypeptide retains at least 40%, at least 45%, at least 50%, at least 55%, or at least 60% activity when measured after 72 hours of incubation in PBS at pH 7.4 and 37 degrees Celsius. Optionally, the modified GCase polypeptide retains at least 50% activity when measured after 72 hours of incubation in PBS at pH 7.4 and 37 degrees Celsius. The modified GCase polypeptide may retain activity which is at least 10 fold, at least 15 fold, at least 20 fold, at least 30 fold, or at least 50 fold higher than the activity of a reference GCase polypeptide when measured after 72 hours of incubation at pH 5.6 and 37 degrees Celsius. For example, the modified GCase polypeptide may retain activity which is at least 30 fold higher than the activity of a reference GCase polypeptide when measured after 72 hours of incubation at pH 5.6 and 37 degrees Celsius. The modified GCase polypeptide may retain activity which is at least 10 fold, at least 15 fold, at least 20 fold, at least 30 fold, or at least 50 fold higher than the activity of a reference GCase polypeptide when measured after 72 hours of incubation in PBS at pH 7.4 and 37 degrees Celsius. For example, the modified GCase polypeptide may retain activity which is at least 30 fold higher than the activity of a reference GCase polypeptide when measured after 72 hours of incubation in PBS at pH 7.4 and 37 degrees Celsius. Optionally, the modified GCase polypeptide retains at least 80% activity when measured after 72 hours of incubation at pH 5.6 and 37 degrees Celsius.

In some embodiments, the at least one mutation provides structural stabilisation at physiological pH *(e.g.* pH 7.4). Exemplary mutations which provide structural stabilisation are disclosed herein. By specifying that the *"at least one mutation provides structural stabilisation at physiological pH",* the modified GCase polypeptide comprising the at least one mutation is more structurally stable at physiological pH than a reference GCase polypeptide that does not comprise the at least one mutation but is otherwise identical to the modified GCase polypeptide comprising the at least one mutation. For example, if the at least one mutation provides structural stabilisation at physiological pH and the modified GCase polypeptide comprising the at least one mutation is otherwise identical to a wild type GCase polypeptide except that it comprises the at least one mutation (e.g. mutations W351C and A380C), the modified GCase polypeptide comprising the at least one mutation is more structurally stable at physiological pH than the wild type GCase polypeptide. In some embodiments, the at least one mutation *"provides structural stabilisation at physiological pH"* if a *"test"* polypeptide that is identical to SEQ ID NO: 1 except for the at least one mutation is more structurally stable at physiological pH compared to the polypeptide of SEQ ID NO: 1, optionally where the *"test"* polypeptide and SEQ ID NO: 1 are produced by expression in the same cell line. The modified GCase polypeptide comprising the at least one mutation may not necessarily be more structurally stable at physiological pH than a wild type GCase polypeptide. For example, it is envisaged that the modified GCase polypeptide comprising the at least one mutation may comprise one or more further modifications (such as a deleted portion) which reduce the structural stability at physiological pH of the polypeptide. In such a case, the at least one mutation disclosed herein may serve to provide more structural stability at physiological pH for the modified GCase polypeptide such that the modified GCase polypeptide may not have the same structural stability at physiological pH as a wild type GCase polypeptide but nevertheless has more structural stability at physiological pH than a reference GCase polypeptide that is otherwise identical to the modified GCase polypeptide but lacks the at least one mutation. Alternatively, the at least one mutation may provide structural stabilisation at physiological pH which restores the structural stability at physiological pH of the modified GCase polypeptide to the level of structural stability of a wild type GCase polypeptide.

In some embodiments, the modified GCase polypeptide comprising at least one mutation is more structurally stable at physiological pH (*e.g.* pH 7.4).

In some embodiments, the at least one mutation provides structural stabilisation at pH 7.4. In some embodiments, the modified GCase polypeptide comprising at least one mutation is more structurally stable at pH 7.4. In some embodiments, the modified GCase polypeptide comprising at least one mutation is more structurally stable at physiological pH (*e.g.* pH 7.4) relative to a reference GCase polypeptide as described herein. For example, the reference GCase polypeptide may be a wild-type GCase polypeptide. The reference GCase polypeptide may be the polypeptide of SEQ ID NO: 3. The reference GCase polypeptide may be the polypeptide of SEQ ID NOs: 4 or 5. Optionally, the modified GCase polypeptide comprising at least one mutation is more structurally stable at physiological pH relative to the reference GCase polypeptide. Optionally, the modified GCase polypeptide comprising at least one mutation is more structurally stable at pH 7.4 relative to the reference GCase polypeptide. In some embodiments, the structural stability at physiological pH of a modified GCase is compared to the structural stability at physiological pH of a reference GCase polypeptide. Exemplary mutations which provide structural stabilisation at physiological pH of the modified GCase polypeptide comprising at least one mutation are disclosed herein. For example, mutations which enable the formation of a disulphide bridge.

The structural stability of a GCase polypeptide at pH 7.4 can be determined using any one of the methods described herein for determining the stability or half-life of a GCase polypeptide. In such methods, any incubation of a sample containing the GCase polypeptide would be at physiological pH (*e.g.* pH 7.4).

For the purposes of determining whether a modified GCase polypeptide is more structurally stable at physiological pH relative to a reference GCase polypeptide (as described herein, such as a wild-type GCase polypeptide), the structural stability at physiological pH of the modified GCase polypeptide and reference GCase polypeptide are determined using the same method and the results compared. The method may be any of the methods described herein for determining stability or half-life.

The stability or structural stability of a polypeptide is typically mediated by interactions between amino acid side chains and/or by interactions between an amino acid side chain(s) and the protein backbone. The interactions may be non-covalent interactions. Alternatively, covalent bond(s) (*e.g.* disulphide bond(s)) may be formed. The GCase polypeptide may therefore be stabilised (*e.g.* relative to a reference GCase polypeptide) by amino acid substitution(s) (substitution mutations) which stabilise the interactions between amino acid side chains and/or between an amino acid side chain(s) and the protein backbone of the GCase polypeptide. For example, a disulphide bond formed between cysteines at positions corresponding to positions 351 and 380 of SEQ ID NO: 1 in the modified GCase polypeptide may provide increased stability or structural stabilisation. The modified GCase polypeptide in which a disulphide bond is formed between cysteines at positions corresponding to positions 351 and 380 of SEQ ID NO: 1 may have increased stability or structural stability relative to the reference GCase polypeptide.

In some embodiments, the at least one mutation provides a longer half-life. Exemplary mutations which provide a longer half-life are disclosed herein. By specifying that the *"at least one mutation provides a longer half-life ",* the modified GCase polypeptide comprising the at least one mutation has a longer half-life than a reference GCase polypeptide that does not comprise the at least one mutation but is otherwise identical to the modified GCase polypeptide comprising the at least one mutation. For example, if the at least one mutation provides a longer half-life and the modified GCase polypeptide comprising the at least one mutation is otherwise identical to a wild type GCase polypeptide except that it comprises the at least one mutation (*e.g.* mutations W351C and A380C), the modified GCase polypeptide comprising the at least one mutation has a longer half-life than the wild type GCase polypeptide. In some embodiments, the at least one mutation *"provides a longer half-life"* if a "*test"* polypeptide that is identical to SEQ ID NO: 1 except for the at least one mutation has a longer half-life compared to the polypeptide of SEQ ID NO: 1, optionally where the *"test"* polypeptide and SEQ ID NO: 1 are produced by expression in the same cell line. The modified GCase polypeptide comprising the at least one mutation may not necessarily have a longer half-life than a wild type GCase polypeptide. For example, it is envisaged that the modified GCase polypeptide comprising the at least one mutation may comprise one or more further modifications (such as a deleted portion) which reduce the half-life of the polypeptide. In such a case, the at least one mutation disclosed herein may serve to provide a longer half-life for the modified GCase polypeptide such that the modified GCase polypeptide may not have the same half-life as a wild type GCase polypeptide but nevertheless has a longer half-life than a reference GCase polypeptide that is otherwise identical to the modified GCase polypeptide but lacks the at least one mutation. Alternatively, the at least one mutation may provide a longer half-life which restores the half-life of the modified GCase polypeptide to the typical half-life of a wild type GCase polypeptide.

In some embodiments, the modified GCase polypeptide comprising at least one mutation has a longer half-life. In some embodiments, the modified GCase polypeptide has a longer half-life relative to a reference GCase polypeptide as described herein. For example, the reference GCase polypeptide may be a wild-type GCase polypeptide. The reference GCase polypeptide may be the polypeptide of SEQ ID NO: 3. The reference GCase polypeptide may be the polypeptide of SEQ ID NOs: 4 or 5. In some embodiments, the half-life of the modified GCase polypeptide comprising at least one mutation is compared to the half-life of the reference GCase polypeptide. Exemplary mutations which increase the half-life of the modified GCase polypeptide comprising at least one mutation are disclosed herein. In some embodiments, the stability or structural stability of the modified GCase polypeptide comprising at least one mutation may be determined by determining the half-life.

Optionally, the modified GCase polypeptide may have a half-life at least 1.2 fold, at least 1.5 fold, at least 2 fold, at least 3 fold, at least 4 fold, at least 5 fold, or at least 10 fold longer than the half-life of the reference GCase polypeptide. In other words, the modified GCase polypeptide may have a longer half-life which is at least 1.2 fold, at least 1.5 fold, at least 2 fold, at least 3 fold, at least 4 fold, at least 5 fold, or at least 10 fold longer than the half-life of the reference GCase polypeptide. Optionally, the modified GCase polypeptide may have a half-life between 1.2 fold and 30 fold, or between 1.2 fold and 25 fold longer than the half-life of the reference GCase polypeptide. In other words, the modified GCase polypeptide may have a longer half-life which is between 1.2 fold and 30 fold, or between 1.2 fold and 25 fold longer than the half-life of the reference GCase polypeptide. Optionally, the modified GCase polypeptide may have a half-life between 1.2 fold and 20 fold, between 1.2 fold and 15 fold, or between 2 fold and 15 fold longer than the half-life of the reference GCase polypeptide. In other words, the modified GCase polypeptide may have a longer half-life which is between 1.2 fold and 20 fold, between 1.2 fold and 15 fold, or between 2 fold and 15 fold longer than the half-life of the reference GCase polypeptide. The longer half-life may be longer half-life at physiological pH, such as pH 7.4. Optionally, the half-life may be measured at pH 5.6 or pH 7.4. The longer half-life may be longer half-life at pH 7.4. For example, the modified GCase polypeptide may have a half-life at pH 7.4 of at least 1.8 fold longer than the half-life of the reference GCase polypeptide. The longer half-life may be longer half-life at lysosomal pH. The longer half-life may be longer half-life at pH 5.6. For example, the modified GCase polypeptide may have a half-life at pH 5.6 of at least 20 fold longer than the half-life of the reference GCase polypeptide. Optionally, the half-life may be measured in a suitable matrix, for example, serum (*e.g.* human or mouse) or plasma (*e.g.* human or mouse). The longer half-life may be longer half-life in serum or plasma. Preferably, the half-life is at least 5 fold longer than the half-life of the reference GCase polypeptide when the half-life is measured in serum (*e.g.* human). Half-life may be measured using a fluorometric assay. Half-life may be determined by any known methods in the art. Half-life may be determined by measuring the residual enzymatic activity in a sample after a series of specific time periods have lapsed. Optionally, the sample is obtained by transfecting a host cell (*e.g.* an Expi293F cell) with an expression vector comprising a nucleotide sequence encoding the GCase polypeptide, and harvesting GCase polypeptide from the culture medium. Optionally, the GCase polypeptide is harvested from the culture medium four days post transfection. Optionally the sample is obtained according to the methods described in the sections entitled "*Expansion of Expi293F cells*"*,* "*The day before transfection of Expi293F cells*", *"Transfection of Expi293F cells"* and "*Harvest of transfected Expi293F cells*" in Example 1. A sample containing the GCase polypeptide may be transferred into a matrix (such as AB buffer at pH 5.6, PBS at pH 7.4, serum or plasma) and incubated. The pH of the matrix may be selected in order to determine the half-life at that particular pH. The temperature of the incubation may be selected in order to determine the half-life at that particular temperature. The incubation may be at 37°C. Aliquots of the incubated sample containing the GCase polypeptide may be removed at suitable time points (such as 0, 0.5, 1, 2, 3, 4, 5, 6, 7, 8, 24, 48, 120, and 144 hours). The initial time point (*i.e.* 0 mins or 0 hours) is the time at which the sample is transferred into the matrix for incubation. Comparing GCase activity at a given time point with GCase activity at the initial time point allows residual GCase activity to be determined at the given time point. Optionally, the residual enzymatic activity is measured in a sample according to the section entitled "*Stability assessment*" in Example 1 at time points 0, 0.5, 1, 2, 3, 4, 5, 6, 7, 8, 24, 48, 120, and 144 hours. The activity of the GCase polypeptide in the aliquots may be measured according to a fluorometric assay protocol as described herein. For example, the GCase polypeptide may be incubated with 4-MUG for 30 minutes at 37°C. Optionally, the GCase polypeptide is incubated with 4-MUG for 1 hour at 37°C in the presence and absence of an irreversible GCase inhibitor, *e.g.* Conduritol B epoxide. For example, a portion of the sample is incubated in the presence of, and a portion of the sample is incubated in the absence of, an irreversible GCase inhibitor, *e.g.* Conduritol B epoxide, and the results are compared to obtain the level of activity of the GCase. Optionally, the GCase polypeptide is incubated at pH 5.6 or pH 5.75. The level of fluorescent product generated can be measured using emission and excitation wavelengths of 365nm and 445nm, respectively. Optionally, the activity of the GCase polypeptide in the aliquots may be measured according to the fluorometric assay protocol described in the section entitled *"GCase activity determination"* in Example 1 or described in the paragraph beginning *"GCase activity assay"* in the section entitled "*Methods*" in Example 13. The values may be applied in a one-phase decay model, such as that outlined in Example 5. Optionally, the GCase polypeptide may be purified prior to determining the half-life. The GCase polypeptide may be purified as described herein, for example in accordance with the section entitled "*GCase protein purification*" in Example 1. Optionally, the half-life of the GCase polypeptide is determined using the methodology of Example 5.

For the purposes of determining whether a modified GCase polypeptide has a longer half-life relative to a reference GCase polypeptide (as described herein, such as a wild-type GCase polypeptide), the half-life of the modified GCase polypeptide and reference GCase polypeptide are determined using the same method and the results compared. The method may be any of the methods described herein for determining half-life.

The effective activity, stability, residual activity or half-life of the modified GCase polypeptide may be determined at pH 5, pH 5.1, pH 5.2, pH 5.3, pH 5.4, pH 5.5, pH 5.6, pH 5.7, pH 5.8, pH 5.9, pH 6.0, pH 6.1, pH 6.2, pH 6.3, pH 6.4, pH 6.5, pH 6.6, pH 6.7, pH 6.8, pH 6.9, pH 7.0, pH 7.1, pH 7.2, pH 7.3, pH 7.4, pH 7.5, pH 7.6, pH 7.7, pH 7.8, pH 7.9 or pH 8, or between pH 5 and pH 6, between pH 5.3 and pH 5.7, between pH 5.6 and pH 5.8, between pH 6.8 and pH 7.8, between pH 7 and pH 7.6, between pH 7.2 and pH 7.5, or between pH 7.3 and pH 7.8. Optionally, the pH is a lysosomal pH (such as pH 5.6) or physiological pH (such as pH 7.4). Optionally, the pH is between pH 7.35 and pH 7.45. Preferably, the pH is physiological pH, such as pH 7.4. Optionally, higher effective activity, increased stability, higher residual activity or longer half-life of the modified GCase polypeptide refers to higher effective activity, increased stability, higher residual activity or longer half-life, respectively, at pH 5, pH 5.1, pH 5.2, pH 5.3, pH 5.4, pH 5.5, pH 5.6, pH 5.7, pH 5.8, pH 5.9, pH 6.0, pH 6.1, pH 6.2, pH 6.3, pH 6.4, pH 6.5, pH 6.6, pH 6.7, pH 6.8, pH 6.9, pH 7.0, pH 7.1, pH 7.2, pH 7.3, pH 7.4, pH 7.5, pH 7.6, pH 7.7, pH 7.8, pH 7.9 or pH 8, or between pH 5 and pH 6, between pH 5.3 and pH 5.7, between pH 5.6 and pH 5.8, between pH 6.8 and pH 7.8, between pH 7 and pH 7.6, between pH 7.2 and pH 7.5, or between pH 7.3 and pH 7.8. Optionally, the pH is a lysosomal pH (such as pH 5.6) or physiological pH (such as pH 7.4). Optionally, the pH is between pH 7.35 and pH 7.45. Preferably, the pH is physiological pH, such as pH 7.4.

The effective activity, stability, residual activity or half-life of the modified GCase polypeptide may be determined at 18°C (degrees Celsius), 20°C, 25°C, 30°C, 35°C, or 37°C. Preferably, effective activity, stability, residual activity or half-life of the modified GCase polypeptide is determined at 37°C. Optionally, higher effective activity, increased stability, increased residual activity or longer half-life of the modified GCase polypeptide refers to higher effective activity, increased stability, increased residual activity or longer half-life, respectively, at 18°C (degrees Celsius), 20°C, 25°C, 30°C, 35°C, or 37°C. Preferably, the higher effective activity, increased stability, increased residual activity or longer half-life is higher effective activity, increased stability, increased residual activity or longer half-life, respectively, at 37°C.

The modified GCase polypeptide may have increased stability in a liquid *(i.e.* when in solution). Optionally, the GCase polypeptide has a longer half-life in a liquid. Optionally, the liquid is conditioned medium, such as conditioned medium in which a host cell expressing a GCase polypeptide is cultured. Optionally, the liquid is a biological sample. A biological sample may be blood, serum or plasma. Optionally, the modified GCase polypeptide may have higher stability in plasma. Optionally, the modified GCase polypeptide may have a longer half-life in plasma.

In some embodiments, the at least one mutation provides increased thermostability. Exemplary mutations which provide increased thermostability are disclosed herein. By specifying that the "*at least one mutation provides increased thermostability*", the modified GCase polypeptide comprising the at least one mutation has increased thermostability compared to a reference GCase polypeptide that does not comprise the at least one mutation but is otherwise identical to the modified GCase polypeptide comprising the at least one mutation. For example, if the at least one mutation provides increased thermostability and the modified GCase polypeptide comprising the at least one mutation is otherwise identical to a wild type GCase polypeptide except that it comprises the at least one mutation (e.g. mutations W351C and A380C), the modified GCase polypeptide comprising the at least one mutation has increased thermostability compared to the wild type GCase polypeptide. In some embodiments, the at least one mutation *"provides increased thermostability"* if a "*test"* polypeptide that is identical to SEQ ID NO: 1 except for the at least one mutation has increased thermostability compared to the polypeptide of SEQ ID NO: 1, optionally where the *"test"* polypeptide and SEQ ID NO: 1 are produced by expression in the same cell line. The modified GCase polypeptide comprising the at least one mutation may not necessarily have increased thermostability compared to a wild type GCase polypeptide. For example, it is envisaged that the modified GCase polypeptide comprising the at least one mutation may comprise one or more further modifications (such as a deleted portion) which reduce the thermostability of the polypeptide. In such a case, the at least one mutation disclosed herein may serve to provide increased thermostability for the modified GCase polypeptide such that the modified GCase polypeptide may not have the same thermostability as a wild type GCase polypeptide but nevertheless has higher thermostability than a reference GCase polypeptide that is otherwise identical to the modified GCase polypeptide but lacks the at least one mutation. Alternatively, the at least one mutation may provide increased thermostability which restores the thermostability of the modified GCase polypeptide to the typical thermostability of a wild type GCase polypeptide.

Thermostability may be measured by determining the melting temperature (also known as the mid-denaturation temperature or the temperature at which half the polypeptide is denatured). A polypeptide with a higher melting temperature is more thermally stable than a polypeptide with a lower melting temperature, and so in some embodiments, the at least one mutation "*provides increased thermostability*" if a *"test"* polypeptide that is identical to SEQ ID NO: 1 except for the at least one mutation has a higher melting temperature compared to the polypeptide of SEQ ID NO: 1, optionally where the *"test"* polypeptide and SEQ ID NO: 1 are produced by expression in the same cell line. In some embodiments, the modified GCase polypeptide has a melting temperature of at least 55, at least 56, or at least 57 degrees Celsius at pH 5.75. Optionally, the modified GCase polypeptide has a melting temperature of at least 53, or at least 54 degrees Celsius at pH 5.75. Optionally, the modified GCase polypeptide has a melting temperature of at least 52, at least 53, or at least 53.5 degrees Celsius at pH 7.

In some embodiments, the modified GCase polypeptide has increased thermostability relative to a reference GCase polypeptide as described herein. The reference GCase polypeptide may be the polypeptide of SEQ ID NO: 3. The reference GCase polypeptide may be the polypeptide of SEQ ID NO: 4 or 5. In some embodiments, the thermostability of the modified GCase polypeptide comprising at least one mutation is compared to the thermostability of the reference GCase polypeptide. Exemplary mutations which increase the thermostability of the modified GCase polypeptide comprising at least one mutation are disclosed herein.

Optionally, the modified GCase polypeptide melting temperature is at least 1, at least 2, at least 3, at least 4, at least 5 or at least 5.4 degrees Celsius higher relative to the reference GCase polypeptide at pH 5.75. Optionally, the modified GCase polypeptide melting temperature is at least 1, at least 2, or at least 2.5 degrees Celsius higher relative to the reference GCase polypeptide at pH 7.

Optionally, the melting temperature is the melting temperature calculated at a modified GCase polypeptide concentration of 1.5 µM, 3 µM, or 6 µM. Optionally, the melting temperature is the melting temperature calculated at a modified GCase polypeptide concentration of 1.5 µM or 3 µM. Optionally, the melting temperature is the melting temperature calculated at a modified GCase polypeptide concentration of 3 µM.

The thermostability may be measured using a thermal shift assay. Measuring thermostability of a polypeptide (such as a modified GCase polypeptide or a reference GCase polypeptide as described herein) using a thermal shift assay essentially involves incubating the polypeptide at increasing temperatures and monitoring unfolding of the polypeptide as the temperature increases. For example, the polypeptide (such as the modified GCase polypeptide or a reference GCase polypeptide) may be held at 25°C for a period of time (such as two minutes), and then the temperature increased gradually over time. Optionally, the temperature is increased by 1 degree Celsius per minute. Optionally, the temperature is increased by 1 degree Celsius per minute until a temperature at which all of the modified GCase polypeptide has denatured, such as a temperature of 95 degrees Celsius. Optionally, the thermal shift assay comprises incubating the modified GCase polypeptide at a temperature increasing by 1 degree Celsius per minute. Optionally, the thermal shift assay comprises measuring unfolding of the modified GCase polypeptide or reference GCase polypeptide as the modified GCase polypeptide or reference GCase polypeptide is incubated at a temperature increasing by 1 degree Celsius per minute, *i.e.* the melting temperature is calculated by measuring unfolding of the modified GCase polypeptide or reference GCase polypeptide as the modified GCase polypeptide or reference GCase polypeptide is incubated at a temperature increasing by 1 degree Celsius per minute.

Unfolding of the polypeptide may be monitored using a fluorescent dye that is sensitive to changes in protein structure. For example, dyes such as SYPRO^{™} Orange Protein gel Stain bind to hydrophobic protein regions, and emit fluorescence as a protein unfolds. When a fluorescent dye is used to monitor unfolding of the polypeptide, the fluorescent dye is added to the medium in which the polypeptide is incubated during the thermal shift assay, and fluorescence is monitored.

A melt curve may be generated, plotting level of fluorescence (in relative fluorescence units or RFU) on the Y axis and temperature on the X axis. The melting temperature corresponds to the minimum derivative of the negative first derivative of the melt curve. Optionally, the melting temperature can be measured using the assay set out in Example 1 under the heading *"Thermostability testing".*

Optionally, the modified GCase polypeptide and/or the reference GCase polypeptide are stored in pH 5.75 buffer (such as sodium citrate buffer) prior to performing a thermostability assay such as a thermal shift assay. Optionally, the reference GCase polypeptide may be a wild-type GCase polypeptide such as VPRIV. Optionally, VPRIV is reconstituted in nuclease-free water prior to performing a thermostability assay such as a thermal shift assay.

In some embodiments, the at least one mutation reduces the number of Human leukocyte antigen (HLA)-I and/or HLA-II binders. Exemplary mutations which reduce the number of HLA-I and/or HLA-II binders are disclosed herein. Reference to a reduction in HLA-I and/or HLA-II binders, for example that the at least one mutation *"reduces the number of HLA-I and*/*or HLA-II binders"* or that the modified GCase polypeptide *"has a reduced number of HLA-I and*/*or HLA-II binders",* should be interpreted to mean that the number of HLA-I and/or HLA-II binders is predicted to be reduced. The number of HLA-I and/or HLA-II binders can be predicted as described herein. By specifying that the *"at least one mutation reduces the number of HLA-I and*/*or HLA-II binders ",* the modified GCase polypeptide comprising the at least one mutation has a reduced number of HLA-I and/or HLA-II binders than a reference GCase polypeptide that does not comprise the at least one mutation but is otherwise identical to the modified GCase polypeptide comprising the at least one mutation. For example, if the at least one mutation reduces the number of HLA-I and/or HLA-II binders and the modified GCase polypeptide comprising the at least one mutation is otherwise identical to a wild type GCase polypeptide except that it comprises the at least one mutation (*e.g.* mutations W351C and A380C), the modified GCase polypeptide comprising the at least one mutation has a reduced number of HLA-I and/or HLA-II binders in comparison to the wild type GCase polypeptide. In some embodiments, the at least one mutation *"reduces the number of HLA-I and*/*or HLA-II binders"* if a *"test"* polypeptide that is identical to SEQ ID NO: 1 except for the at least one mutation has a reduced number of HLA-I and/or HLA-II binders compared to the polypeptide of SEQ ID NO: 1, optionally where the *"test"* polypeptide and SEQ ID NO: 1 are produced by expression in the same cell line.

In some embodiments, the at least one mutation reduces the number of HLA-I and/or HLA-II binders. In some embodiments, the modified GCase polypeptide comprising at least one mutation has a reduced number of HLA-I and/or HLA-II binders. Optionally, the HLA-I and/or HLA-II binders are strong binders. Optionally, the HLA-I binders are strong binders. Optionally, the HLA-II binders are strong binders. *"HLA-I binders"* are fragments from the polypeptide which can bind to HLA-I. *"HLA-II binders"* are fragments from the polypeptide which can bind to HLA-II. The risk of a polypeptide inducing an immune response is potentially lower if the number of HLA-I and/or HLA-II binders (in particular "strong" binders) from a polypeptide is lower. The number of HLA-I and HLA-II binders (in particular "strong" binders) can be predicted as discussed further below. As discussed further below, a "strong" binder can be defined as having an IC50 cut-off range of 0 to ≤50nM. In some embodiments, the number of HLA-I and/or HLA-II binders are predicted using the IC50 binding affinity. Preferably, the reduced number of HLA-I and/or HLA-II binders are strong binders. The reduced number of HLA-I binders may be strong binders. The reduced number of HLA-II binders may be strong binders. In some embodiments, the reduced number of HLA-I and/or HLA-II binders (*e.g.* strong binders) is relative to the number of HLA-I and/or HLA-II binders (*e.g.* strong binders), respectively, for a reference GCase polypeptide as described herein. For example, the reference GCase polypeptide may be a wild-type GCase polypeptide. The reference GCase polypeptide may be the polypeptide of SEQ ID NO: 3. The reference GCase polypeptide may be the polypeptide of SEQ ID NOs: 4 or 5. The at least one mutation may reduce the number of HLA-I binders (*e.g.* strong binders). The modified GCase polypeptide comprising at least one mutation may have a reduced number of HLA-I binders (*e.g.* strong binders) relative to the number of HLA-I binders (*e.g.* strong binders) for the reference GCase polypeptide. The at least one mutation may reduce the number of HLA-II binders (*e.g.* strong binders). The modified GCase polypeptide comprising at least one mutation may have a reduced number of HLA-II binders (*e.g.* strong binders) relative to the number of HLA-II binders (*e.g.* strong binders) for the reference GCase polypeptide. Exemplary mutations which reduce the number of HLA-I and/or HLA-II binders in the modified GCase polypeptide are disclosed herein.

The immunological risk profile of a GCase polypeptide may be assessed using known *in silico* analysis or prediction. For instance, data may be input for calculations using the Immune Epitope Database and Analysis Resource database. The prediction methods used may be NetMHCpan EL 4.0 (for HLA-I) and NetMHCIIpan 3.2 (for HLA-II). For example, the immunogenicity risk profile of the GCase polypeptide may be assessed in accordance with the methodology of Example 7. For instance, peptide fragments of nine and fifteen amino acids in length (for calculating binding affinities to HLA-I and HLA-II receptor molecules respectively) from the region spanning 50 amino acids upstream and 50 amino acids downstream of the relevant amino acid substitutions (*e.g.* from a region spanning 50 amino acids upstream of W351C to 50 amino acids downstream of A380C) in the modified GCase polypeptide may be used as inputs for calculations using the Immune Epitope Database and Analysis Resource database. Prediction methods that may be used may include NetMHCpan EL 4.0 (for HLA-I) and NetMHCIIpan 3.2 (for HLA-II). The IC50 values may be used as a measure of peptide binding affinity. The IC50 values may be used to categorise the binders as "strong" binders or "intermediate" binders. The lower the IC50 value, the better the binding is predicted to be. For "strong" binders an IC50 cut-off range of 0 to ≤50nM may be used, and a IC50 cut-off of >50 to≤500nM may be used for "intermediate" binders.

For the purposes of determining whether a modified GCase polypeptide has a reduced number of HLA-I and/or HLA-II binders relative to a reference GCase polypeptide (as described herein, such as a wild-type GCase polypeptide), the number of HLA-I and/or HLA-II binders of the modified GCase polypeptide and reference GCase polypeptide are predicted using the same method and the results compared. The method may be any of the methods described herein for predicting the number of HLA-I and/or HLA-II binders.

In some embodiments, the at least one mutation may provide reduced immunogenicity. In some embodiments, the modified GCase polypeptide comprising at least one mutation has reduced immunogenicity. In some embodiments, the level of immunogenicity of the modified GCase polypeptide comprising at least one mutation is compared to the level for a reference GCase polypeptide as described herein. In some embodiments, the modified GCase polypeptide comprising at least one mutation has reduced immunogenicity relative to the immunogenicity of a reference GCase polypeptide as described herein. Exemplary mutations which may reduce the immunogenicity of the modified GCase polypeptide are disclosed herein.

By specifying that the *"at least one mutation provides reduced immunogenicity ",* the modified GCase polypeptide comprising the at least one mutation has a reduced immunogenicity compared to a reference GCase polypeptide that does not comprise the at least one mutation but is otherwise identical to the modified GCase polypeptide comprising the at least one mutation. For example, if the at least one mutation reduces the immunogenicity and the modified GCase polypeptide comprising the at least one mutation is otherwise identical to a wild type GCase polypeptide except that it comprises the at least one mutation (*e.g.* mutations W351C and A380C), the modified GCase polypeptide comprising the at least one mutation has a reduced immunogenicity in comparison to the wild type GCase polypeptide. In some embodiments, the at least one mutation *"provides reduced immunogenicity"* if a *"test"* polypeptide that is identical to SEQ ID NO: 1 except for the at least one mutation has reduced immunogenicity compared to the polypeptide of SEQ ID NO: 1, optionally where the *"test"* polypeptide and SEQ ID NO: 1 are produced by expression in the same cell line.

In some embodiments, the immunogenicity of a GCase polypeptide is assessed by predicting the number of HLA-I and/or HLA-II binders as described herein.

In some embodiments, the immunogenicity of a GCase polypeptide is assessed by measuring the immune response generated against the GCase polypeptide. For example, the GCase polypeptide may be administered to a test animal, such as a mouse, and the immune response in the test animal can be measured and compared to the immune response in a comparable test animal which has been administered with a reference GCase polypeptide. The immune response may be measured by testing for inflammatory markers in the blood, such as cytokines or acute-phase proteins. Examples of cytokines which may be measured are TNF-α, IL-1β and/or INF-γ. Examples of acute-phase proteins which may be measured are C-reactive protein, mannan-binding lectin and/or complement factors. A reduction in inflammatory markers may be used to indicate lower immunogenicity.

In some embodiments, the immunogenicity of a GCase polypeptide is assessed using a T-cell proliferation assay which tests the level of activation of T-cells in human peripheral blood mononuclear cells (PMBCs) upon incubation with whole protein (*e.g.* a wild type GCase polypeptide or a modified GCase polypeptide of the invention). For example, the ProMap^{®} naive CFSE T cell proliferation assays could be used (https://www.proimmune.com/promap-t-cell-proliferation-assays/).

For the purposes of determining whether a modified GCase polypeptide has reduced immunogenicity relative to a reference GCase polypeptide (as described herein, such as a wild-type GCase polypeptide), the immunogenicity of the modified GCase polypeptide and reference GCase polypeptide are assessed using the same method and the results compared. The method may be any of the methods described herein for assessing immunogenicity (*e.g.* predicting the number of HLA-I and/or HLA-II binders).

A no observation of adverse event level (NOAEL) may be calculated for a modified GCase polypeptide of the invention. If the NOAEL is high, this suggests that the modified GCase polypeptide of the invention has low immunogenicity. For example, the GCase polypeptide may be administered to test animals, such as mice, at increasing doses and the mice monitored to see whether they have an adverse event. The lowest dose which is associated with an adverse event is the NOAEL. As discussed in more detail below, the modified GCase polypeptide of the invention may be administered as part of a viral particle. For example, the mice may be administered a viral particle encoding the GCase polypeptide at increasing doses, such as doses of 1x10⁸, 1x10⁹, 5x10⁹, 1x10¹⁰, 5x10¹⁰ *etc.* Optionally, the modified GCase polypeptide is encoded by a modified GBA nucleotide sequence, and the GBA nucleotide sequence is administered in a viral particle comprising a recombinant genome which comprises a polynucleotide comprising the GBA nucleotide sequence and the NOAEL is at least 1x10¹² vg/kg, at least 5x10¹²vg/kg, at least 1x10¹³ vg/kg, or at least 2x10¹³ vg/kg.

The enzymatic efficiency of the modified GCase polypeptide of the invention may be the same as the enzymatic efficiency of a wild-type GCase polypeptide. Describing the enzymatic efficiency (*i.e.* ability to process a substrate) of a first GCase polypeptide (*e.g.* modified GCase polypeptide) as being the *"same "* as the enzymatic efficiency of a second GCase polypeptide (*e.g.* wild-type GCase polypeptide) indicates that the enzymatic efficiency of the first GCase polypeptide may be identical to the enzymatic efficiency of the second GCase polypeptide plus or minus 10%, more particularly plus or minus 5%, or more particularly plus or minus 1%. For the purposes of the present invention, *"enzymatic efficiency"* refers to the ability of a GCase polypeptide to process a substrate, such as Methylumbelliferyl-β-D-glucopyranosiduronic acid (4-MUG) or its natural substrate glucocerebroside. The enzymatic efficiency of the modified GCase polypeptide of the invention may be the same enzymatic efficiency as that of a wild-type GCase polypeptide, but the effective activity or residual activity of the modified GCase polypeptide of the invention may be higher than a wild-type GCase polypeptide. This observation may be as a result of the modified GCase polypeptide of the invention being more stable and therefore being active for longer. Thus, the modified GCase polypeptide of the invention may have the same ability to process a substrate as a wild-type GCase polypeptide, but remain active for longer, resulting in higher effective activity and/or higher residual activity.

The enzymatic efficiency of a GCase polypeptide may be measured by determining the Km and/or Kcat values. The Km and/or Kcat values may be determined by standard methods in the art. The enzymatic efficiency may be measured by processing 4-Methylumbelliferyl-β-D-glucopyranosiduronic acid (4-MUG), such as by the method described in Example 6. For instance, the GCase polypeptide may be purified as described herein, such as in Example 1. Purified protein (*e.g.* 1 or 3.5 nM) can be incubated with increasing amounts (*e.g.* 2.5 x 10⁶, 5 x 10⁶, 7.5 x 10⁶ or 1 x 10⁷ nM) of 4-MUG in AB buffer. Optionally, the purified protein is incubated with 4-MUG in a composition at pH 5.75. 4-methylumbelliferone (4-MU) formation can be monitored over, *e.g.,* 15 minutes by measuring the resulting fluorescence (excitation wavelength: 365nm, emission wavelength: 445nm). 4-MU formation velocity at each 4-MUG concentration can be plotted against 4-MUG concentration and fitted with the Michaelis-Menten model. The Kcat value may be between 32 s⁻¹ and 40 s⁻¹, between 32.8 s⁻¹ and 39.2 s⁻¹, between 33 s⁻¹ and 39 s⁻¹, or between 35 s⁻¹ and 37 s⁻¹. The Kcat value may be around 36.0 s⁻¹. The Km value for the modified GCase polypeptide of the invention may be between 1.4mM and 2.5mM, between 1.6mM and 2.2mM, or between 1.8mM and 2.2mM. Optionally, the Km value for the modified GCase polypeptide may be around 2.0mM.

The modified GCase polypeptide of the invention may have higher effective activity and/or increased stability and/or structural stabilisation at physiological pH and/or a longer half-life. The modified GCase polypeptide of the invention may have a reduced number of HLA-I and/or HLA-II binders. The modified GCase polypeptide of the invention may have reduced immunogenicity. The modified GCase polypeptide of the invention may have increased thermostability. The modified GCase polypeptide of the invention may be expressed at a higher level in a host cell than a reference GCase polypeptide as described herein.

The modified GCase polypeptide described herein may comprise at least one mutation. The at least one mutation (*i.e.* one or more substitution mutations) may be at a position corresponding to a position selected from the group consisting of 351, 380, 272, 262, 313, 404, 407, 482, 484, 490, 494, 503 and 534 of SEQ ID NO: 1. The at least one mutation (such as a mutation at a position corresponding to a position listed above) may provide one or more of the properties as discussed herein (*e.g.* higher effective activity, increased stability, structural stabilisation at physiological pH, longer half-life, higher residual activity, reduced number of HLA-I and/or HLA-II binders, reduced immunogenicity or increased thermostability). The modified GCase polypeptide comprising at least one mutation (such as a mutation at a position corresponding to a position listed above) may have one or more of the properties as discussed herein (*e.g.* higher effective activity, increased stability, structural stabilisation at physiological pH, longer half-life, higher residual activity, reduced number of HLA-I and/or HLA-II binders, reduced immunogenicity, increased thermostability, etc.).

In some embodiments, the at least one mutation may comprise a substitution with an amino acid that acts as a proton donor at pH 7.4.

Optionally, the at least one mutation comprises a mutation at a position corresponding to position 272 of SEQ ID NO: 1. Preferably, the mutation is a substitution with a glutamine. For example, the mutation may be E272Q.

Optionally, the at least one mutation comprises a mutation at a position corresponding to position 262 of SEQ ID NO: 1. The mutation may be a substitution with an asparagine or tyrosine. Preferably, the mutation is a substitution with an asparagine. For example, the mutation may be H262N.

Optionally, the at least one mutation comprises a mutation to tyrosine at a position corresponding to position 262 of SEQ ID NO: 1, for example H262Y.

Optionally, the at least one mutation comprises a mutation at a position corresponding to position 313 of SEQ ID NO: 1. Preferably, the mutation is a substitution with a asparagine. For example, the mutation may be H313N.

Optionally, the at least one mutation comprises a mutation at a position corresponding to position 404 of SEQ ID NO: 1. Preferably, the mutation is a substitution with a lysine. For example, the mutation may be H404K.

Optionally, the at least one mutation comprises a mutation at a position corresponding to position 490 of SEQ ID NO: 1. Preferably, the mutation is a substitution with a lysine. For example, the mutation may be H490K.

Optionally, the at least one mutation comprises a mutation at a position corresponding to position 534 of SEQ ID NO: 1. Preferably, the mutation is a substitution with an asparagine. For example, the mutation may be R534N.

Optionally, the at least one mutation of the modified GCase polypeptide comprises two mutations, wherein each of the two mutations is a substitution with cysteine which enables the formation of a disulphide bridge. The formation of a disulphide bridge may stabilise the structure of the modified GCase, for example at physiological pH. The formation of a disulphide bond may be determined by mass spectroscopy. For example the formation of a disulphide bond may be determined by analysing the fragmentation pattern of a polypeptide suspected of containing a disulphide bond, optionally following limited proteolysis, for example as outlined in Gorman et al. 2002, Mass Spectrometry Reviews 21, 183-216. Alternatively, the formation of a disulphide bond may be determined by performing limited proteolysis on a polypeptide and analysing the resulting protein fragments by SDS-PAGE under both reducing and non-reducing conditions, optionally in combination with N-terminal sequencing.

Optionally, the at least one mutation (*e.g.* comprising two mutations) comprises (i) a mutation at a position corresponding to position 482 of SEQ ID NO: 1, and (ii) a mutation at a position corresponding to position 503 of SEQ ID NO: 1. Optionally, (i) the mutation at a position corresponding to position 482 of SEQ ID NO: 1 is a substitution with cysteine, and (ii) the mutation at a position corresponding to position 503 of SEQ ID NO: 1 is a substitution with cysteine. Optionally, (i) the mutation at a position corresponding to position 482 of SEQ ID NO: 1 is an aspartic acid to cysteine mutation, and (ii) the mutation at a position corresponding to position 503 of SEQ ID NO: 1 is a serine to cysteine mutation. For example, the mutations may be D482C/S503C.

Optionally, the at least one mutation (*e.g.* comprising two mutations) comprises (i) a mutation at a position corresponding to position 494 of SEQ ID NO: 1, and (ii) a mutation at a position corresponding to position 534 of SEQ ID NO: 1. Optionally, (i) the mutation at a position corresponding to position 494 of SEQ ID NO: 1 is a substitution with cysteine, and (ii) the mutation at a position corresponding to position 534 of SEQ ID NO: 1 is a substitution with cysteine. Optionally, (i) the mutation at a position corresponding to position 494 of SEQ ID NO: 1 is a serine to cysteine mutation, and (ii) the mutation at a position corresponding to position 534 of SEQ ID NO: 1 is an arginine to cysteine mutation. For example, the mutations may be S494C/R534C.

Optionally, the at least one mutation (*e.g.* comprising two mutations) comprises (i) a mutation at a position corresponding to position 351 of SEQ ID NO: 1, and (ii) a mutation at a position corresponding to position 380 of SEQ ID NO: 1. Optionally, (i) the mutation at a position corresponding to position 351 of SEQ ID NO: 1 is a substitution with cysteine, and (ii) the mutation at a position corresponding to position 380 of SEQ ID NO: 1 is a substitution with cysteine. Optionally, (i) the mutation at a position corresponding to position 351 of SEQ ID NO: 1 is a tryptophan to cysteine mutation, and (ii) the mutation at a position corresponding to position 380 of SEQ ID NO: 1 is an alanine to cysteine mutation. For example, the mutations may be W351C/A380C.

Optionally, the at least one mutation (*e.g.* comprising two mutations) comprises (i) a mutation at a position corresponding to position 407 of SEQ ID NO: 1, and (ii) a mutation at a position corresponding to position 484 of SEQ ID NO: 1. Optionally, (i) the mutation at a position corresponding to position 407 of SEQ ID NO: 1 is a substitution with cysteine, and (ii) the mutation at a position corresponding to position 484 of SEQ ID NO: 1 is a substitution with cysteine. Optionally, (i) the mutation at a position corresponding to position 407 of SEQ ID NO: 1 is an isoleucine to cysteine mutation; and (ii) the mutation at a position corresponding to position 484 of SEQ ID NO: 1 is an aspartic acid to cysteine mutation. For example, the mutations may be I407C/D484C.

Optionally, the at least one mutation (*e.g.* comprising three mutations) comprises (i) a mutation at a position corresponding to position 272 of SEQ ID NO: 1, (ii) a mutation at a position corresponding to position 351 of SEQ ID NO: 1, and (iii) a mutation at a position corresponding to position 380 of SEQ ID NO: 1. Optionally, (i) the mutation at a position corresponding to position 272 of SEQ ID NO: 1 is a substitution with a glutamine, (ii) the mutation at a position corresponding to position 351 of SEQ ID NO: 1 is a substitution with cysteine, and (iii) the mutation at a position corresponding to position 380 of SEQ ID NO: 1 is a substitution with cysteine. Optionally, (i) the mutation at a position corresponding to position 272 of SEQ ID NO: 1 is a glutamic acid to glutamine mutation, (ii) the mutation at a position corresponding to position 351 of SEQ ID NO: 1 is a tryptophan to cysteine mutation, and (iii) the mutation at a position corresponding to position 380 of SEQ ID NO: 1 is an alanine to cysteine mutation. For example, the mutations may be E272Q/W351C/A380C.

Optionally, the at least one mutation comprises a substitution with glutamine at a position corresponding to position 272 of SEQ ID NO: 1, and the modified GCase polypeptide has increased stability compared to a reference GCase polypeptide as described herein (such as a wildtype GCase polypeptide), retaining at least 85% activity when measured after 120 mins of incubation at pH 7.4 and 37 degrees Celsius. Optionally, the at least one mutation comprises (i) a substitution with cysteine (*e.g.* a tryptophan to cysteine mutation) at a position corresponding to position 351 of SEQ ID NO: 1; and (ii) a substitution with cysteine (e.g an alanine to cysteine mutation) at a position corresponding to position 380 of SEQ ID NO: 1, and the modified GCase polypeptide has increased stability compared to a reference GCase polypeptide as described herein (such as a wildtype GCase polypeptide), retaining at least 85% activity when measured after 120 mins of incubation at pH 7.4 and 37 degrees Celsius. In one embodiment, the at least one mutation comprises a substitution with glutamine at a position corresponding to position 272 of SEQ ID NO: 1, and wherein the modified GCase polypeptide has increased stability compared to a reference GCase polypeptide, retaining at least 60% activity when measured after 72 hours of incubation in PBS at pH 7.4 and 37 degrees Celsius, optionally wherein the reference GCase polypeptide is a wild type GCase polypeptide. In one embodiment, the at least one mutation comprises a substitution with glutamine at a position corresponding to position 272 of SEQ ID NO: 1, and wherein the modified GCase polypeptide has increased stability compared to a reference GCase polypeptide, retaining at least 50% or at least 55% activity when measured after 72 hours of incubation at pH 5.6 and 37 degrees Celsius, optionally wherein the reference GCase polypeptide is a wild type GCase polypeptide.

Optionally, the at least one mutation comprises (i) a substitution with glutamine at a position corresponding to position 272 of SEQ ID NO: 1 (*e.g.* E272Q); and (ii) a substitution with cysteine (*e.g.* a tryptophan to cysteine mutation) at a position corresponding to position 351 of SEQ ID NO: 1; and (iii) a substitution with cysteine (*e.g.* an alanine to cysteine mutation) at a position corresponding to position 380 of SEQ ID NO: 1. Optionally, the modified GCase polypeptide has increased stability compared to a reference GCase polypeptide as described herein (such as a wildtype GCase polypeptide), retaining at least 85% activity when measured after 120 mins of incubation at pH 7.4 and 37 degrees Celsius. In one embodiment, the at least one mutation comprises:
(i) a tryptophan to cysteine mutation at a position corresponding to position 351 of SEQ ID NO: 1; and
(ii) an alanine to cysteine mutation at a position corresponding to position 380 of SEQ ID NO: 1;
and the modified GCase polypeptide has increased stability compared to a reference GCase polypeptide, retaining at least 60% activity when measured after 72 hours of incubation in PBS at pH 7.4 and 37 degrees Celsius, optionally wherein the reference GCase polypeptide is a wild type GCase polypeptide. In one embodiment, the at least one mutation comprises:
(i) a tryptophan to cysteine mutation at a position corresponding to position 351 of SEQ ID NO: 1; and
(ii) an alanine to cysteine mutation at a position corresponding to position 380 of SEQ ID NO: 1;
and the modified GCase polypeptide has increased stability compared to a reference GCase polypeptide, retaining at least 50%, or at least 55% activity when measured after 72 hours of incubation at pH 5.6 and 37 degrees Celsius, optionally wherein the reference GCase polypeptide is a wild type GCase polypeptide. In one embodiment, the at least one mutation comprises:
(i) a substitution with glutamine at a position corresponding to position 272 of SEQ ID NO: 1, optionally E272Q; and
(ii) a tryptophan to cysteine mutation at a position corresponding to position 351 of SEQ ID NO: 1; and
(iii) an alanine to cysteine mutation at a position corresponding to position 380 of SEQ ID NO: 1;
and the modified GCase polypeptide has increased stability compared to a reference GCase polypeptide, retaining at least 60% activity when measured after 72 hours of incubation in PBS at pH 7.4 and 37 degrees Celsius, optionally wherein the reference GCase polypeptide is a wild type GCase polypeptide. In one embodiment, the at least one mutation comprises:
(i) a substitution with glutamine at a position corresponding to position 272 of SEQ ID NO: 1, optionally E272Q; and
(ii) a tryptophan to cysteine mutation at a position corresponding to position 351 of SEQ ID NO: 1; and
(iii) an alanine to cysteine mutation at a position corresponding to position 380 of SEQ ID NO: 1;
and the modified GCase polypeptide has increased stability compared to a reference GCase polypeptide, retaining at least 50%, or at least 55% activity when measured after 72 hours of incubation at pH 5.6 and 37 degrees Celsius, optionally wherein the reference GCase polypeptide is a wild type GCase polypeptide.

Optionally, the at least one mutation comprises (i) a substitution with cysteine (*e.g.* a tryptophan to cysteine mutation) at a position corresponding to position 351 of SEQ ID NO: 1; and (ii) a substitution with cysteine (e.g an alanine to cysteine mutation) at a position corresponding to position 380 of SEQ ID NO: 1, and the modified GCase polypeptide has increased stability compared to a reference GCase polypeptide as described herein (such as a wildtype GCase polypeptide), retaining at least 15%, or at least 20% activity when measured after 7 days of incubation at pH 7.4 and 37 degrees Celsius. In one embodiment, the at least one mutation comprises a substitution with glutamine at a position corresponding to position 272 of SEQ ID NO: 1, and wherein the modified GCase polypeptide has increased stability compared to a reference GCase polypeptide, retaining at least 15%, or at least 20% activity when measured after 7 days of incubation in PBS at pH 7.4 and 37 degrees Celsius, optionally wherein the reference GCase polypeptide is a wild type GCase polypeptide. In one embodiment, the at least one mutation comprises:
(i) a substitution with glutamine at a position corresponding to position 272 of SEQ ID NO: 1, optionally E272Q; and
(ii) a tryptophan to cysteine mutation at a position corresponding to position 351 of SEQ ID NO: 1; and
(iii) an alanine to cysteine mutation at a position corresponding to position 380 of SEQ ID NO: 1;
and the modified GCase polypeptide has increased stability compared to a reference GCase polypeptide, retaining at least 60%, or at least 80% activity when measured after 7 days of incubation in PBS at pH 7.4 and 37 degrees Celsius, optionally wherein the reference GCase polypeptide is a wild type GCase polypeptide.

Optionally, the modified GCase polypeptide has higher effective activity and/or increased stability compared to a reference GCase polypeptide as described herein. Optionally, the reference GCase polypeptide does not comprise the at least one mutation but is otherwise identical to the modified GCase polypeptide. Optionally, the reference GCase polypeptide is selected from any one of SEQ ID NOs: 1 to 5.

Optionally, the at least one mutation does not comprise one or more mutations selected from H184L, E272L, W351C, K360N and A380T which are positions corresponding to positions 184, 272, 351, 360 and 380, respectively, in SEQ ID NO: 1. Optionally, the at least one mutation does not comprise a H184L mutation (which is a position corresponding to position 184 in SEQ ID NO: 1). Optionally, the at least one mutation does not comprise a K360N mutation (which is a position corresponding to position 360 in SEQ ID NO: 1). Optionally, the at least one mutation does not comprise a H184L mutation and a K360N mutation (which are positions corresponding to positions 184 and 360 respectively in SEQ ID NO: 1). Optionally, the modified GCase polypeptide does not comprise or have an amino acid sequence of SEQ ID NO: 4 or 5.

Optionally, the at least one mutation does not comprise a A380T mutation (which is a position corresponding to position 380 in SEQ ID NO: 1). Optionally, the at least one mutation does not comprise a R534H mutation (which is a position corresponding to position 534 in SEQ ID NO: 1).

Optionally, the at least one mutation does not comprise one or more mutations selected from H184L, H184F, F355A, L356F, K360N, and K360A which are positions corresponding to positions 184, 355, 356, and 360, respectively, in SEQ ID NO: 1. Optionally, the at least one mutation does not comprise one or more mutations selected from H184F, F355A, L356F, and K360A which are positions corresponding to positions 184, 355, 356, and 360, respectively, in SEQ ID NO: 1. Optionally, the at least one mutation does not comprise a F355A mutation and a L356F mutation (which are positions corresponding to positions 355 and 356 respectively in SEQ ID NO: 1). Optionally, the at least one mutation does not comprise a F355A mutation, a L356F mutation and a K360N mutation (which are positions corresponding to positions 355, 356 and 360, respectively, in SEQ ID NO: 1).

Optionally, the modified GCase polypeptide comprises an amino acid sequence as set forth in any one of SEQ ID NOs: 41 to 46, or an amino acid sequence which is at least 90% at least 95%, at least 96%, at least 97%, at least 98% or at least 99% identical to an amino acid sequence as set forth in any one of SEQ ID NOs 41 to 46. Optionally, the modified GCase polypeptide comprises an amino acid sequence as set forth in SEQ ID NO: 42 or 45.

Typically, GCase polypeptide is initially expressed as a precursor *"immature"* form (*e.g.* the GCase polypeptide of SEQ ID NO: 1), comprising a signal peptide (*e.g.* amino acid residues 1 to 39 of SEQ ID NO: 1) and a mature GCase polypeptide region. After processing, the *"mature"* form of GCase polypeptide lacks the signal peptide. The term *"mature GCase "* or *"mature GCase polypeptide"* refers to a GCase polypeptide that does not comprise the signal peptide, such as the GCase polypeptide of SEQ ID NO: 2.

The sequence of a wild-type human GCase polypeptide is set forth in SEQ ID NO: 1 (immature) and SEQ ID NO: 2 (mature). The modified GCase polypeptide may comprise an amino acid sequence that is at least 80%, at least 90%, at least 95%, at least 96%, at least 97%, at least 98%, at least 99%, at least 99.5%, at least 99.6%, at least 99.7%, or least 99.8% identical to a fragment of SEQ ID NO: 1. The modified GCase polypeptide may comprise an amino acid sequence that is at least 80%, at least 90%, at least 95%, at least 96%, at least 97%, at least 98%, at least 99%, at least 99.5%, at least 99.6%, or at least 99.7% identical to a fragment of SEQ ID NO: 2. Optionally the fragment is at least 200, at least 250, at least 300, at least 350, at least 400, or at least 450 amino acids, or between 300 and 497, between 400 and 497, or between 450 and 497 amino acids. Optionally, the modified GCase polypeptide comprises an amino acid sequence at least 80%, at least 90%, at least 95%, at least 98%, or at least 99% identical to a fragment of SEQ ID NO: 1 or SEQ ID NO: 2 of at least 200, at least 250, at least 300, at least 400, between 300 and 497, between 400 and 497, or between 450 and 497 amino acids. Optionally, the modified GCase polypeptide comprises an amino acid sequence at least 80%, at least 90%, at least 95%, at least 98%, or at least 99% identical to SEQ ID NO: 1 or SEQ ID NO: 2.

Optionally, the modified GCase polypeptide comprises an amino acid sequence at least 98% identical to a fragment of between 400 and 536 amino acids of SEQ ID NO: 1.

Optionally, the modified GCase polypeptide comprises an amino acid sequence at least 98% identical to a fragment of between 400 and 497 amino acids of SEQ ID NO: 2.

Optionally, the modified GCase polypeptide comprises an amino acid sequence that is at least 80%, at least 90%, at least 95%, at least 96%, at least 97%, at least 98%, at least 99%, at least 99.5%, at least 99.6%, at least 99.7%, or least 99.8% identical to SEQ ID NO: 1. Optionally, the modified GCase polypeptide comprises an amino acid sequence that is at least 80%, at least 90%, at least 95%, at least 96%, at least 97%, at least 98%, at least 99%, at least 99.5%, at least 99.6%, or at least 99.7% identical to SEQ ID NO: 2. Preferably, the modified GCase polypeptide comprises an amino acid sequence at least 98% identical to SEQ ID NO: 1. Preferably, the modified GCase polypeptide comprises an amino acid sequence at least 98% identical to SEQ ID NO: 2.

Preferably, the modified GCase polypeptide may comprise an amino acid sequence that is identical to SEQ ID NO: 1 or SEQ ID NO: 2, except that the modified GCase polypeptide comprises the at least one mutation (*i.e.* one or more substitution mutations) as defined herein. In other words, the modified GCase polypeptide may comprise a GCase amino acid sequence that differs from an amino acid sequence set forth in SEQ ID NOs: 1 or 2 only by the at least one mutation. Preferably, the modified GCase polypeptide comprises an amino acid sequence that is identical to SEQ ID NO: 1 or SEQ ID NO: 2, except that the modified GCase polypeptide comprises a mutation at a position corresponding to position 272 of SEQ ID NO: 1. Preferably, the modified GCase polypeptide comprises an amino acid sequence that is identical to SEQ ID NO: 1 or SEQ ID NO: 2, except that the modified GCase polypeptide comprises a substitution with cysteine at a position corresponding to position 351 of SEQ ID NO: 1 and at a position corresponding to position 380 of SEQ ID NO: 1 and, optionally, a mutation at a position corresponding to position 272 of SEQ ID NO: 1.

Optionally, the modified GCase polypeptide comprises a GCase amino acid sequence that differs from the amino acid sequence set forth in SEQ ID NO: 1 or SEQ ID NO: 2 by 20 or fewer, 15 or fewer, 14 or fewer, 13 or fewer, 12 or fewer, 11 or fewer, 10 or fewer, 9 or fewer, 8 or fewer, 7 or fewer, 6 or fewer, 5 or fewer, 4 or fewer, 3 or fewer, 2 or fewer, or 1 substitution mutations. Optionally, the modified GCase polypeptide comprises a GCase amino acid sequence that differs from the amino acid sequence set forth in SEQ ID NO: 1 or SEQ ID NO: 2 by 5 or fewer, 4 or fewer, 3 or fewer, 2 or fewer, or 1 substitution mutations. Optionally, the modified GCase polypeptide comprises a GCase amino acid sequence that differs from the amino acid sequence set forth in SEQ ID NO: 1 or SEQ ID NO: 2 by 3 or fewer substitution mutations. Optionally, the modified GCase polypeptide comprises a GCase amino acid sequence that differs from the amino acid sequence set forth in SEQ ID NO: 1 or SEQ ID NO: 2 by 2 or fewer substitution mutations. Optionally, the modified GCase polypeptide comprises a GCase amino acid sequence that differs from the amino acid sequence set forth in SEQ ID NO: 1 or SEQ ID NO: 2 by at least 1, at least 2, at least 3, at least 4, at least 5, at least 8, at least 10, at least 15 or at least 20 substitution mutations. Optionally, the GCase polypeptide may comprise a GCase amino acid sequence that differs from the amino acid sequence set forth in SEQ ID NO: 1 or SEQ ID NO: 2 by between 1 and 20, between 1 and 15, between 1 and 10, between 1 and 5, between 1 and 3, between 2 and 6, between 3 and 8, between 5 and 10, between 8 and 13, between 10 and 15, between 13 and 18, or between 15 and 20 substitution mutations. Optionally, the substitution mutation is a non-conservative substitution.

Any or all of the modified GCase polypeptides may comprise one or more further modifications, such as substitutions, insertions and/or deletions in addition to the at least one mutation (*i.e.* one or more substitution mutations) defined herein, when the amino acid sequence of the modified GCase polypeptide is compared to a wild-type GCase amino acid sequence. The one or more further modifications may be one or more substitutions. The one or more further modifications may be one or more conservative substitutions. The one or more further modifications may be one or more non-conservative substitutions. The at least one mutation may be located within the one or more further modifications. For example, a contiguous portion (such as up to 50, up to 20, or up to 10 amino acids) of the GCase polypeptide may have been replaced with an alternative portion which comprises the at least one mutation.

### A polynucleotide comprising a glucocerebrosidase (GBA) nucleotide sequence

The invention provides a polynucleotide comprising a modified glucocerebrosidase (GBA) nucleotide sequence, wherein the modified GBA nucleotide sequence encodes the modified GCase polypeptide of the invention. The term *"modified"* means that the nucleotide sequence has at least one difference compared to a wild-type GBA nucleotide sequence, *e.g.* a mutation has been introduced.

The terms *"nucleic acid molecule ", "polynucleotide"* and *"nucleotide sequence "* are intended to refer to a polymeric chain of any length of nucleotides, including deoxyribonucleotides, ribonucleotides, or analogs thereof. For example, the nucleic acid molecule, polynucleotide or nucleotide sequence may comprise DNA (deoxyribonucleotides) or RNA (ribonucleotides). The nucleic acid molecule, polynucleotide or nucleotide sequence may consist of DNA. The nucleic acid molecule, polynucleotide or nucleotide sequence may be mRNA. Since the nucleic acid molecule, polynucleotide or nucleotide sequence may comprise RNA or DNA, all references to T (thymine) nucleotides may be replaced with U (uracil).

In some embodiments, the term *"nucleotide sequence"* can be replaced with the term *"nucleic acid molecule ".*

A GBA nucleotide sequence encodes a GCase polypeptide. The polynucleotide of the invention comprises a modified GBA nucleotide sequence and the modified GBA nucleotide sequence encodes the modified GCase polypeptide of the invention. The term *"sequence that encodes"* or *"sequence encodes"* refers to a nucleotide sequence comprising an open reading frame comprising codons that encode the encoded polypeptide. For example, a nucleotide sequence that encodes a GCase polypeptide comprises codons that encode the amino acid sequence of the GCase polypeptide. An example of a GBA nucleotide sequence that encodes a wild-type GCase is provided in SEQ ID NO: 40.

The codons that encode the polypeptide are also referred to as *"coding nucleotides ".* A GBA nucleotide sequence may be interrupted by non-coding nucleotides (*e.g.* an intron), but only nucleotides that encode the polypeptide (*i.e.* the coding nucleotides) should be considered to be part of the GBA nucleotide sequence. For example, a GBA nucleotide sequence that encodes a GCase polypeptide will comprise any codons (*i.e.* the coding nucleotides) that encode an amino acid forming part of the GCase polypeptide, irrespective of whether those codons are contiguous in sequence or separated by one or more non-coding nucleotides. In other words, a GBA polynucleotide which contains stretches of coding nucleotides interrupted by a stretch of non-coding nucleotides will be considered to comprise a *"GBA nucleotide sequence"* consisting of the non-contiguous coding stretches immediately juxtaposed (*i.e.* minus the stretches of non-coding nucleotides). However, herein, the nucleotides of the stop codon will be considered coding nucleotides.

A GBA nucleotide sequence encoding a GCase polypeptide may also comprise a sequence that encodes a signal peptide. It is well known that some proteins, particularly those which are exported to different tissues, are expressed with a signal peptide. Signal peptides can be at the N-terminus of a protein sequence (and in this case at the 5' end of a coding sequence) and many signal peptides are cleaved following cellular processing. Thus, herein, a mature protein or polypeptide (such as a mature GCase protein or polypeptide) will be considered to be the resulting protein or polypeptide after the signal peptide has been processed and removed/cleaved (and thus no longer forms part of the polypeptide sequence).

The following Table describes codons that encode each amino acid:

**Table 2**

| **Amino Acid** | **Codon** | **Amino Acid** | **Codon** | **Amino Acid** | **Codon** |
|---|---|---|---|---|---|
| Phenylalanine | TTC TTT | Proline | CCT CCC CCA CCG | Asparagine | AAT AAC |
| Leucine | TTA TTG CTT CTC CTA CTG | Threonine | ACT ACC ACA ACG | Lysine | AAA AAG |
| Isoleucine | ATT ATC ATA | Alanine | GCT GCC GCA GCG | Aspartic Acid | GAT GAC |
| Methionine | ATG | Tyrosine | TAT TAC | Glutamic Acid | GAA GAG |
| Valine | GTT GTC GTA GTG | Histidine | CAT CAC | Cysteine | TGT TGC |
| Serine | TCT TCC TCA TCG AGT AGC | Glutamine | CAA CAG | Tryptophan | TGG |
| Arginine | CGT CGC CGA CGG AGA AGG | Glycine | GGT GGC GGA GGG | | |

The corresponding RNA codons will contain Us in place of the Ts in the Table above.

The present invention provides a polynucleotide comprising a modified GBA nucleotide sequence, wherein the modified GBA nucleotide sequence encodes a modified GCase polypeptide of the invention. Optionally, the modified GBA nucleotide sequence comprises a sequence that is at least 80%, at least 85%, at least 90%, at least 95%, at least 96%, at least 97%, at least 98%, at least 99%, at least 99.4%, at least 99.5%, at least 99.6%, at least 99.7%. at least 99.8%, at least 99.9% or 100% identical to a fragment of at least 750, at least 850, at least 950, at least 1000, at least 1200, at least 1300, at least 1400, at least 1494, 1494 or fewer, 1611 or fewer, between 1000 and 1494, between 1000 and 1611, between 1300 and 1494, between 1300 and 1611, or around 1494 nucleotides of any one of SEQ ID NOs: 6 to 29. Optionally, the modified GBA nucleotide sequence comprises a sequence that is at least 80%, at least 90%, at least 95%, at least 98%, at least 99%, at least 99.5%, at least 99.8%, or 100% identical to a fragment of at least 750, at least 850, at least 950, at least 1000, at least 1200, at least 1400, or at least 1494 nucleotides of any one of SEQ ID NOs: 6 to 29. Optionally, the modified GBA nucleotide sequence comprises a sequence that is at least 80%, at least 85%, at least 90%, at least 95%, at least 96%, at least 97%, at least 98%, at least 99%, at least 99.4%, at least 99.5%, at least 99.6%, at least 99.7%. at least 99.8%, at least 99.9% or 100% identical to a nucleotide sequence of any one of SEQ ID NOs: 6 to 29. Optionally, the modified GBA nucleotide sequence comprises a sequence that is at least 80%, at least 90%, at least 95%, at least 98%, at least 99%, at least 99.5%, at least 99.8%, or 100% identical to a nucleotide sequence of any one of SEQ ID NOs: 6 to 29. Optionally, the modified GBA nucleotide sequence comprises a sequence that is at least 80%, at least 85%, at least 90%, at least 95%, at least 96%, at least 97%, at least 98%, at least 99%, at least 99.4%, at least 99.5%, at least 99.6%, at least 99.7%. at least 99.8%, at least 99.9% or 100% identical to a nucleotide sequence of SEQ ID NO: 6 or 10. Optionally, the modified GBA nucleotide sequence comprises a sequence that is at least 80%, at least 90%, at least 95%, at least 98%, at least 99%, at least 99.5%, at least 99.8%, or 100% identical to a nucleotide sequence of SEQ ID NO: 6 or SEQ ID NO: 10. Optionally, the modified GBA nucleotide sequence comprises a nucleotide sequence of SEQ ID NO: 6 or SEQ ID NO: 10. Optionally, the modified GBA nucleotide sequence comprises a sequence that is at least 80%, at least 85%, at least 90%, at least 95%, at least 96%, at least 97%, at least 98%, at least 99%, at least 99.4%, at least 99.5%, at least 99.6%, at least 99.7%. at least 99.8%, at least 99.9% or 100% identical to a nucleotide sequence of SEQ ID NO: 14 or SEQ ID NO: 18. Optionally, the modified GBA nucleotide sequence comprises a sequence that is at least 80%, at least 90%, at least 95%, at least 98%, at least 99%, at least 99.5%, at least 99.8%, or 100% identical to a nucleotide sequence of SEQ ID NO: 14 or SEQ ID NO: 18. Optionally, the modified GBA nucleotide sequence comprises a nucleotide sequence of SEQ ID NO: 14 or SEQ ID NO: 18. Optionally, the modified GBA nucleotide sequence comprises a sequence that is at least 80%, at least 85%, at least 90%, at least 95%, at least 96%, at least 97%, at least 98%, at least 99%, at least 99.4%, at least 99.5%, at least 99.6%, at least 99.7%. at least 99.8%, at least 99.9% or 100% identical to a nucleotide sequence of SEQ ID NO: 22 or SEQ ID NO: 26. Optionally, the modified GBA nucleotide sequence comprises a sequence that is at least 80%, at least 90%, at least 95%, at least 98%, at least 99%, at least 99.5%, at least 99.8%, or 100% identical to a nucleotide sequence of SEQ ID NO: 22 or SEQ ID NO: 26. Optionally, the modified GBA nucleotide sequence comprises a nucleotide sequence of SEQ ID NO: 22 or SEQ ID NO: 26.

The modified GBA nucleotide sequence of the invention may comprise a portion that is codon-optimised. The portion that is codon-optimised can correspond to a sequence encoding part of, or the entirety of, the modified GCase polypeptide. Optionally, the portion of the GBA nucleotide sequence that is codon-optimised is a contiguous portion. The modified GBA nucleotide sequence of the invention may be codon-optimised. The modified GBA nucleotide sequence may be codon-optimised for expression in human liver cells. Optionally, the human liver cell is a Huh-7 cell. As mentioned above, the genetic code is degenerate, and many amino acids may be encoded by more than one alternative codon. However, the genetic code of different organisms, tissues or cells may be biased towards using one particular codon for encoding a particular amino acid. A nucleotide sequence that is *"codon-optimised"* may be optimised for expression in a particular host cell or organism, for example expression in human liver cells. Preferably, a codon-optimised modified GBA nucleotide sequence is modified relative to a non-codon-optimised modified GBA nucleotide sequence whilst the amino acid sequence encoded by the codon-optimised modified GBA nucleotide and the non-codon-optimised modified GBA nucleotide sequence is the same.

The modified GBA nucleotide sequence of the invention may comprise one or more alternative codon(s) in place of the wild-type codon(s), wherein an *"alternative"* codon is a codon which has a different sequence to the wild-type codon but which encodes the same amino acid as the wild-type codon (*i.e.* a degenerate codon). Table 2 describes the codons which encode each amino acid.

A codon-optimised nucleotide sequence may comprise at least one more *"preferred"* codon than a corresponding nucleotide sequence which is not codon-optimised. A codon-optimised nucleotide sequence may comprise a higher percentage of *"preferred"* codons than a corresponding nucleotide sequence which is not codon-optimised. A codon-optimised nucleotide sequence may comprise at least one fewer *"non-preferred"* codon than a corresponding nucleotide sequence which is not codon-optimised. A codon-optimised nucleotide sequence may comprise a lower percentage of *"non-preferred"* codons than a corresponding nucleotide sequence which is not codon-optimised. For example, if a nucleotide sequence is codon-optimised for expression in the human liver, the nucleotide sequence is modified to increase the number of codons that may be preferred (in the sense that such codons correspond to tRNA species which are more abundant than other tRNA species specific for the same amino acid) in the human liver. As a further example, if a nucleotide sequence is codon-optimised for expression in the human liver cells, the nucleotide sequence may be modified to increase the number of codons that may be preferred (in the sense that such codons correspond to tRNA species which are more abundant than other tRNA species specific for the same amino acid) in human liver cells. The skilled person would appreciate that codon-optimising a sequence may not entail changing every codon, not least because a *"preferred codon"* may already be present at some positions.

Such codon-optimisation may be subject to other factors. For example, it can be seen that the presence of CpGs (*i.e.* CG dinucleotides) has an adverse effect on expression and so the user may decide not to use preferred codons at positions where doing so introduces CpGs into the sequence; this will still be considered to be codon-optimisation. In an embodiment, a preferred codon that ends with a C nucleotide will not be included in the portion of the coding sequence that is codon-optimised, where the next codon in the sequence begins with a G. For example, codon CTC encodes leucine. In schemes where CTC is a preferred codon, it should not be used for encoding leucine where the next codon in the sequence begins with a G, such as codon GTT (or alternatively, the next codon - where possible - could be selected to avoid a G at the first position).

By describing a nucleotide sequence as *"codon-optimised",* not all the codons need to be optimised. Thus the portion of the modified GBA nucleotide sequence that is codon-optimised may comprise one or more alternative codon(s) in place of the wild-type codon(s) when compared to the corresponding portion of a wild-type GBA nucleotide sequence. If the modified GBA nucleotide sequence comprises a portion that is codon-optimised, the polypeptide encoded by the GBA nucleotide sequence may be expressed at a higher level than a polypeptide encoded by a wild-type GBA nucleotide sequence. The modified GBA nucleotide sequence or the portion of the modified GBA nucleotide sequence that is codon-optimised may be codon-optimised for expression in human liver cells. Thus the modified GCase polypeptide encoded by the modified GBA nucleotide sequence may be expressed at a higher level than the polypeptide encoded by an equivalent non-codon-optimised modified GBA nucleotide sequence when the sequences are expressed in human liver cells. Optionally, the human liver cell is a Huh-7 cell. An *"equivalent"* non-codon optimised modified GBA nucleotide sequence is identical *(i.e.* encodes the same GCase polypeptide and comprises the same transcription regulatory elements *etc*.) except the codons used to encode the GCase polypeptide will correspond to the corresponding codons of a wild-type GCase sequence such as those of SEQ ID NO: 40. Optionally, the sequence (*e.g.* codon) encoding the at least one mutation of the modified GCase polypeptide of the invention is not codon-optimised. Optionally, the modified GBA nucleotide sequence is codon-optimised except for the sequence encoding the at least one mutation. Typically, the codon-optimised portion of the modified GBA nucleotide sequence does not comprise the stop codon.

A polypeptide encoded by a codon-optimised GBA nucleotide sequence may be expressed in human liver cells at higher levels compared to a non-codon optimised GBA nucleotide sequence. The modified GCase polypeptide of the invention encoded by the codon-optimised modified GBA nucleotide sequence of the invention may be expressed in human liver cells at higher levels compared to a GCase polypeptide encoded by a non-codon optimised GBA nucleotide sequence. The modified GCase polypeptide encoded by the codon-optimised modified GBA nucleotide sequence of the invention may be expressed at a higher level than a GCase polypeptide encoded by a non-codon optimised GBA nucleotide sequence when the sequences are expressed in human liver cells. For example, the modified GCase polypeptide encoded by the codon-optimised modified GBA nucleotide sequence may be expressed in human liver cells at least 1.1 fold, at least 1.2 fold, at least 1.5 fold, at least 1.8 fold, at least 2 fold, at least 5 fold, at least 10 fold, at least 20 fold, at least 30 fold, at least 40 fold, or at least 50 fold higher compared to a reference non-codon optimised GBA nucleotide sequence. The *"reference non-codon optimised GBA nucleotide sequence "* may be any GBA nucleotide sequence that uses wild-type codons to encode a GCase polypeptide, such as the GBA nucleotide sequence of SEQ ID NO: 40. Optionally, the reference non-codon optimised GBA nucleotide sequence is an *"equivalent"* GBA nucleotide sequence *i.e.* the reference non-codon optimised GBA nucleotide sequence encodes the same GCase polypeptide as the GBA nucleotide sequence to which it is being compared. The modified GCase polypeptide of the invention encoded by the codon-optimised modified GBA nucleotide sequence of the invention may be expressed in human liver cells at higher levels compared to a modified GCase polypeptide encoded by a non-codon optimised modified GBA nucleotide sequence. The modified GCase polypeptide encoded by the codon-optimised modified GBA nucleotide sequence of the invention may be expressed at a higher level than a modified GCase polypeptide encoded by a non-codon optimised modified GBA nucleotide sequence when the sequences are expressed in human liver cells. Optionally, the human liver cell is a Huh-7 cell.

In some embodiments, a portion of the modified GBA nucleotide sequence is not codon-optimised, for example a portion of the coding sequence is not codon-optimised for expression in the liver. In some embodiments, the portion that is not codon-optimised is at least 80, at least 90, at least 100, at least 110, 200 or fewer, 170 or fewer, 140 or fewer, or around 117 nucleotides. In some embodiments, the portion that is not codon-optimised in a modified GBA nucleotide sequence is the portion which encodes the signal peptide.

Optionally, the portion of the GBA nucleotide sequence that is codon-optimised encodes a mature modified GCase polypeptide. In some embodiments, the portion of the modified GBA nucleotide sequence that is codon-optimised does not encode all or a portion of a signal peptide. For example, the modified GBA nucleotide sequence may encode an immature GCase polypeptide (*i.e.* including a signal peptide), and the portion of the modified GBA nucleotide sequence that is codon-optimised encodes the mature GCase protein and does not encode all or a portion of the signal peptide. In some embodiments, the portion of the modified GBA nucleotide sequence that is codon-optimised encodes all or a portion of a signal peptide. For example, the modified GBA nucleotide sequence may encode an immature modified GCase polypeptide (*i.e.* including a signal peptide), and the portion of the modified GBA nucleotide sequence that is codon-optimised encodes the mature modified GCase polypeptide and all or a portion of the signal peptide. In some embodiments, the modified GBA nucleotide sequence encoding the entire mature modified GCase polypeptide sequence is codon-optimised.

Optionally, the modified GBA nucleotide sequence comprises the sequence of SEQ ID NO: 59 or 60, except that the modified GCase polypeptide encoded by the modified GBA nucleotide sequence comprises the at least one mutation as described herein. For example, the modified GCase polypeptide encoded by the modified GBA nucleotide sequence may comprise a substitution with cysteine at a position corresponding to position 351 of SEQ ID NO: 1, optionally W351C, and a substitution with cysteine at a position corresponding to position 380 of SEQ ID NO: 1, optionally A380C. As a further example, the modified GCase polypeptide encoded by the modified GBA nucleotide sequence may comprise a mutation at a position corresponding to position 272 of SEQ ID NO: 1, optionally E272Q. As yet a further example, the modified GCase polypeptide encoded by the modified GBA nucleotide sequence may comprise a substitution with cysteine at a position corresponding to position 351 of SEQ ID NO: 1, optionally W351C, and a substitution with cysteine at a position corresponding to position 380 of SEQ ID NO: 1, optionally A380C, and the modified GCase polypeptide encoded by the modified GBA nucleotide sequence may comprise a mutation at a position corresponding to position 272 of SEQ ID NO: 1 optionally E272Q.

Optionally, the modified GBA nucleotide sequence comprises the sequence of SEQ ID NO: 40 or SEQ ID NO: 64, except that the modified GCase polypeptide encoded by the modified GBA nucleotide sequence comprises the at least one mutation as described herein. For example, the modified GCase polypeptide encoded by the modified GBA nucleotide sequence may comprise a substitution with cysteine at a position corresponding to position 351 of SEQ ID NO: 1, optionally W351C, and a substitution with cysteine at a position corresponding to position 380 of SEQ ID NO: 1, optionally A380C. As a further example, the modified GCase polypeptide encoded by the modified GBA nucleotide sequence may comprise a mutation at a position corresponding to position 272 of SEQ ID NO: 1, optionally E272Q. As yet a further example, the modified GCase polypeptide encoded by the modified GBA nucleotide sequence may comprise a substitution with cysteine at a position corresponding to position 351 of SEQ ID NO: 1, optionally W351C, and a substitution with cysteine at a position corresponding to position 380 of SEQ ID NO: 1, optionally A380C, and the modified GCase polypeptide encoded by the modified GBA nucleotide sequence may comprise a mutation at a position corresponding to position 272 of SEQ ID NO: 1 optionally E272Q.

Optionally, the modified GBA nucleotide sequence comprises the sequence of SEQ ID NO: 15 of WO 2019/070893 or WO 2019/070894, except that the modified GCase polypeptide encoded by the modified GBA nucleotide sequence comprises the at least one mutation as described herein. For example, the modified GCase polypeptide encoded by the modified GBA nucleotide may comprise a mutation at a position corresponding to position 272 of SEQ ID NO: 1. As a further example, the modified GCase polypeptide encoded by the modified GBA nucleotide sequence may comprise a substitution with cysteine at a position corresponding to position 351 of SEQ ID NO: 1 and at a position corresponding to position 380 of SEQ ID NO: 1. As yet a further example, the modified GCase polypeptide encoded by the modified GBA nucleotide sequence may comprise a substitution with cysteine at a position corresponding to position 351 of SEQ ID NO: 1 and at a position corresponding to position 380 of SEQ ID NO: 1, and the modified GCase polypeptide encoded by the modified GBA nucleotide sequence may comprise a mutation at a position corresponding to position 272 of SEQ ID NO: 1.

Optionally, the modified GBA nucleotide sequence comprises the sequence of SEQ ID NO: 13, SEQ ID NO: 14, SEQ ID NO: 15 or SEQ ID NO: 16 of WO2020/012149 or WO 2020/012164, except that the modified GCase polypeptide encoded by the modified GBA nucleotide sequence comprises the at least one mutation as described herein. For example, the modified GCase polypeptide encoded by the modified GBA nucleotide sequence may comprise a mutation at a position corresponding to position 272 of SEQ ID NO: 1. As a further example, the modified GCase polypeptide encoded by the modified GBA nucleotide sequence may comprise a substitution with cysteine at a position corresponding to position 351 of SEQ ID NO: 1 and at a position corresponding to position 380 of SEQ ID NO: 1. As yet a further example, the modified GCase polypeptide encoded by the modified GBA nucleotide sequence may comprise a substitution with cysteine at a position corresponding to position 351 of SEQ ID NO: 1 and at a position corresponding to position 380 of SEQ ID NO: 1, and the modified GCase polypeptide encoded by the modified GBA nucleotide sequence may comprise a mutation at a position corresponding to position 272 of SEQ ID NO: 1.

The polynucleotide of the invention may comprise a transcription regulatory element.

Any appropriate transcription regulatory element may be used, such as HLP2, HLP1, LP1, HCR-hAAT, ApoE-hAAT, or LSP, which are all liver-specific transcription regulatory elements. These transcription regulatory elements are described in more detail in the following references: HLP1: McIntosh J. et al., Blood 2013 Apr 25, 121(17):3335-44; LP1: Nathwani et al., Blood. 2006 April 1, 107(7): 2653-2661; HCR-hAAT: Miao et al., Mol Ther. 2000;1: 522-532; ApoE-hAAT: Okuyama et al., Human Gene Therapy, 7, 637-645 (1996); and LSP: Wang et al., Proc Natl Acad Sci U S A. 1999 March 30, 96(7): 3906-3910. The transcription regulatory element may comprise a liver-specific promoter.

Optionally, a transcription regulatory element is *"liver-specific"* if it drives a higher level of expression in liver cells compared to other cells in general. For example, the skilled person can determine whether a transcription regulatory element is a liver-specific transcription regulatory element by comparing expression of the polynucleotide in liver cells (such as Huh 7 cells) with expression of the polynucleotide in cells from other tissues (such as kidney cells, for example HEK293T cells). If the level of expression is higher in the liver cells, compared to the cells from other tissues, the transcription regulatory element is a liver-specific transcription regulatory element. Optionally, a liver-specific transcription regulatory element does not drive an appreciable level of expression in non-liver cells.

The transcription regulatory element may comprise a promoter and/or an enhancer, such as the promoter element and/or enhancer element from HLP2, HLP1, LP1, HCR-hAAT, ApoE-hAAT, or LSP. Each of these transcription regulatory elements comprises a promoter, an enhancer, and optionally other nucleotides.

In some embodiments, the transcription regulatory element comprises a promoter which is a human alpha-1 anti-trypsin promoter (A1AT; Miao et al (2000), Molecular Therapy 1(6):522), or a fragment thereof. In an embodiment, the fragment of an A1AT promoter is at least 100, at least 120, at least 150, at least 180, 255 or fewer, between 100 and 255, between 150 and 225, between 150 and 300, or between 180 and 255 nucleotides in length. Optionally, the fragment of an A1AT promoter is between 150 and 300 nucleotides in length. Optionally, the fragment of an A1AT promoter is between 180 and 255 nucleotides in length. In an embodiment, the fragment of an A1AT promoter is at least 200, at least 250, at least 300, 500 or fewer, between 200 and 500, between 250 and 500, or between 350 and 450 nucleotides in length. Optionally, the fragment of an A1AT promoter is between 350 and 450 nucleotides in length.

Suitable A1AT promoter fragments are described in SEQ ID NOs: 30 and 31. Optionally, the transcription regulatory element comprises a promoter that is at least 100, at least 120, at least 150, at least 180, 255 or fewer, between 100 and 255, between 150 and 300, or between 180 and 255 nucleotides in length and the promoter comprises a polynucleotide sequence that is at least 80%, at least 85%, at least 90%, at least 95%, at least 98%, at least 99%, at least 99.5%, at least 99.8%, or 100% identical to SEQ ID NO: 30. Optionally, the transcription regulatory element comprises a promoter that is between 180 and 255 nucleotides in length and the promoter comprises a polynucleotide sequence that is at least 98%, at least 99%, at least 99.5%, at least 99.8%, or 100% identical to SEQ ID NO: 30.

Optionally, the polynucleotide comprises a promoter that is at least 80%, at least 85%, at least 90%, at least 95%, at least 98%, at least 99%, at least 99.5%, at least 99.8%, or 100% identical to a fragment of at least 100, at least 120, or at least 150 nucleotides of SEQ ID NO: 30. Optionally, the polynucleotide comprises a promoter that is at least 80%, at least 85%, at least 90%, at least 95%, at least 98%, at least 99%, at least 99.5%, at least 99.8%, or 100% identical to SEQ ID NO: 30. Optionally, the polynucleotide comprises a promoter that is at least 98%, at least 99%, at least 99.5%, at least 99.8%, or 100% identical to SEQ ID NO: 30. Optionally, the polynucleotide comprises a promoter of SEQ ID NO: 30. Optionally, the transcription regulatory element comprises a fragment of an A1AT promoter that is equal to or fewer than 418 nucleotides, equal to or fewer than 255 nucleotides, or equal to or fewer than 185 nucleotides in length, and comprises SEQ ID NO: 30.

The transcription regulatory element may comprise a promoter that is at least 200, at least 250, at least 300, 500 or fewer, between 200 and 500, between 250 and 500, between 350 and 450, or around 418 nucleotides in length and the promoter comprises a polynucleotide sequence that is at least 80%, at least 85%, at least 90%, at least 95%, at least 98%, at least 99%, at least 99.5%, at least 99.8%, or 100% identical to SEQ ID NO: 31. Optionally, the transcription regulatory element comprises a promoter that is between 350 and 450 nucleotides in length and the promoter comprises a polynucleotide sequence that is at least 98%, at least 99%, at least 99.5%, at least 99.8%, or 100% identical to SEQ ID NO: 31. Optionally, the polynucleotide comprises a promoter that is at least 80%, at least 85%, at least 90%, at least 95%, at least 98%, at least 99%, at least 99.5%, at least 99.8%, or 100% identical to a fragment of at least 350 nucleotides of SEQ ID NO: 31. Optionally, the polynucleotide comprises a promoter that is at least 80%, at least 85%, at least 90%, at least 95%, at least 98%, at least 99%, at least 99.5%, at least 99.8%, or 100% identical to SEQ ID NO: 31. Optionally, the polynucleotide comprises a promoter that is at least 98%, at least 99%, at least 99.5%, at least 99.8%, or 100% identical to SEQ ID NO: 31. Optionally, the polynucleotide comprises a promoter of SEQ ID NO: 31.

Optionally, the transcription regulatory element may comprise a minimal nephrin promoter NPHS1 or podocin promoter NPHS2.

The transcription regulatory element may comprise an enhancer. In some embodiments, the transcription regulatory element comprises an enhancer which is the human apolipoprotein E (ApoE) hepatic locus control region (HCR; Miao et al (2000), Molecular Therapy 1(6):522), or a fragment thereof. In an embodiment, the transcription regulatory element comprises a fragment of the HCR enhancer which is a fragment of at least 80, at least 90, at least 100, 192 or fewer, between 80 and 192, between 90 and 192, between 100 and 250, or between 117 and 192 nucleotides in length. Optionally, the fragment of the HCR enhancer is between 100 and 250 nucleotides in length. Optionally, the fragment of the HCR enhancer is between 117 and 192 nucleotides in length. In an embodiment, the fragment of an HCR enhancer is a fragment of at least 150, at least 190, at least 230, 400 or fewer, between 150 and 400, between 190 and 370, between 230 and 340, between 250 and 340, or around 321 nucleotides in length. Optionally, the fragment of the HCR enhancer is between 250 and 340 nucleotides in length.

Suitable HCR enhancer element fragments are defined in SEQ ID NOs: 32 and 33. Optionally, the transcription regulatory element comprises an enhancer that is at least 80, at least 90, at least 100, 192 or fewer, between 80 and 192, between 90 and 192, between 100 and 250, or between 117 and 192 nucleotides in length and the enhancer comprises a polynucleotide sequence that is at least 80%, at least 85%, at least 90%, at least 95%, at least 98%, at least 99%, at least 99.5%, at least 99.8%, or 100% identical to SEQ ID NO: 32. Optionally, the transcription regulatory element comprises an enhancer that is between 117 and 192 nucleotides in length and the enhancer comprises a polynucleotide sequence that is at least 98%, at least 99%, at least 99.5%, at least 99.8%, or 100% identical to SEQ ID NO. 32. Optionally, the transcription regulatory element comprises an enhancer that is at least 80%, at least 85%, at least 90%, at least 95%, at least 98%, at least 99%, at least 99.5%, at least 99.8%, or 100% identical to a fragment of at least 90, at least 100, or at least 110 nucleotides of SEQ ID NO: 32. Optionally, the polynucleotide comprises an enhancer that is at least 80%, at least 85%, at least 90%, at least 95% at least 98%, at least 99%, at least 99.5%, at least 99.8%, or 100% identical to SEQ ID NO: 32. Optionally, the polynucleotide comprises an enhancer that is at least 98%, at least 99%, at least 99.5%, at least 99.8%, or 100% identical to SEQ ID NO: 32. Optionally, the polynucleotide comprises an enhancer of SEQ ID NO: 32. Optionally, the transcription regulatory element comprises a fragment of an HCR enhancer that is equal to or fewer than 321 nucleotides, equal to or fewer than 192 nucleotides, or equal to or fewer than 117 nucleotides in length, and comprises SEQ ID NO: 32.

In some embodiments, the transcription regulatory element comprises an enhancer that is at least 150, at least 190, at least 230, fewer than 400, between 150 and 400, between 190 and 370, between 230 and 340, between 250 and 340, or around 321 nucleotides in length and the enhancer comprises a polynucleotide sequence that is at least 80%, at least 85%, at least 90%, at least 95%, at least 98%, at least 99%, at least 99.5%, at least 99.8%, or 100% identical to SEQ ID NO: 33. Optionally, the transcription regulatory element comprises an enhancer that is between 250 and 340 nucleotides in length and the enhancer comprises a polynucleotide sequence that is at least 98%, at least 99%, at least 99.5%, at least 99.8%, or 100% identical to SEQ ID NO: 33. Optionally, the transcription regulatory element comprises an enhancer that is at least 80%, at least 85%, at least 90%, at least 95%, at least 98%, at least 99%, at least 99.5%, at least 99.8%, or 100% identical to a fragment of at least 250 nucleotides of SEQ ID NO: 33. Optionally, the polynucleotide comprises an enhancer that is at least 80%, at least 85%, at least 90%, at least 95%, at least 98%, at least 99%, at least 99.5%, at least 99.8%, or 100% identical to SEQ ID NO: 33. Optionally, the polynucleotide comprises an enhancer that is at least 98%, at least 99%, at least 99.5%, at least 99.8%, or 100% identical to SEQ ID NO: 33. Optionally, the polynucleotide comprises an enhancer of SEQ ID NO: 33.

In an embodiment, the transcription regulatory element is at least 80%, at least 85%, at least 90%, at least 95%, at least 98%, at least 99%, at least 99.5%, at least 99.8%, or 100% identical to SEQ ID NO: 34. In an embodiment, the polynucleotide comprises a transcription regulatory element that is at least 98%, at least 99%, at least 99.5%, at least 99.8%, or 100% identical to SEQ ID NO: 34. Optionally, the polynucleotide comprises a transcription regulatory element that is at least 98% identical to SEQ ID NO: 34. Optionally, the transcription regulatory element comprises a sequence that is 100% identical to SEQ ID NO: 34. Optionally, the polynucleotide comprises a transcription regulatory element that is 100% identical to SEQ ID NO: 34. Optionally, the polynucleotide comprises a transcription regulatory element of SEQ ID NO: 34. Optionally, the polynucleotide comprises a transcription regulatory element consisting of SEQ ID NO: 34.

In an embodiment, the transcription regulatory element is at least 80%, at least 85%, at least 90%, at least 95%, at least 98%, at least 99%, at least 99.5%, at least 99.8%, or 100% identical to SEQ ID NO: 35. In an embodiment, the polynucleotide comprises a transcription regulatory element that is at least 98%, at least 99%, at least 99.5%, at least 99.8%, or 100% identical to SEQ ID NO: 35. Optionally, the polynucleotide comprises a transcription regulatory element that is at least 98% identical to SEQ ID NO: 35. Optionally, the transcription regulatory element comprises a sequence that is 100% identical to SEQ ID NO: 35. Optionally, the polynucleotide comprises a transcription regulatory element that is 100% identical to SEQ ID NO: 35. Optionally, the polynucleotide comprises a transcription regulatory element of SEQ ID NO: 35. Optionally, the polynucleotide comprises a transcription regulatory element consisting of SEQ ID NO: 35.

In an embodiment, the polynucleotide of the invention comprises a woodchuck hepatitis post-transcriptional regulatory element (WPRE) or a variant thereof. For example, the polynucleotide of the invention comprises the mutated WPRE sequence described in Zanta-Boussif et al (2009), Gene Therapy, 16:605-619. Optionally, the woodchuck hepatitis post-transcriptional regulatory element is at least 75%, at least 80%, at least 85%, at least 90%, at least 95%, at least 96%, at least 97%, at least 98%, at least 99%, at least 99.5%, at least 99.8%, or 100% identical to any one of SEQ ID NOs: 61 to 63. Optionally, the polynucleotide comprises a woodchuck hepatitis post-transcriptional regulatory element that is at least 98%, at least 99%, at least 99.5%, at least 99.8%, or 100% identical to any one of SEQ ID NOs 61 to 63. Optionally, the polynucleotide comprises a woodchuck hepatitis post-transcriptional regulatory element that is at least 98% identical to any one of SEQ ID NOs: 61 to 63. Optionally, the polynucleotide comprises a woodchuck hepatitis post-transcriptional regulatory element that has a sequence of any one of SEQ ID NOs: 61 to 63. Optionally, the polynucleotide comprises a woodchuck hepatitis post-transcriptional regulatory element of any one of SEQ ID NOs: 61 to 63. Optionally, the polynucleotide comprises a woodchuck hepatitis post-transcriptional regulatory element consisting of any one of SEQ ID NOs: 61 to 63. In another embodiment, the polynucleotide of the invention does not comprise a woodchuck hepatitis post-transcriptional regulatory element (WPRE).

The polynucleotide of the invention may further comprise one or two ITRs. In one embodiment, the nucleotide sequence of the or each ITR is fewer than 157, fewer than 154, or around 145 nucleotides in length. Optionally, the or each ITR is a wild-type ITR. Optionally, the or each ITR is an AAV2 ITR.

The polynucleotide of the invention may further comprise a poly A sequence. The poly A sequence may be positioned downstream of the modified GBA nucleotide sequence encoding the modified GCase polypeptide of the invention. The poly A sequence may be a bovine growth hormone poly A sequence (bGHpA - SEQ ID NO: 58). The poly A sequence may be between 250 and 270 nucleotides in length.

The polynucleotide of the invention may further comprise an intron sequence, such as a viral intron sequence, optionally an SV40 intron sequence (SEQ ID NO: 57).

In some embodiments, the modified GCase polypeptide comprising at least one mutation may be expressed at a higher level in a host cell than a reference GCase polypeptide as described herein. For example, the modified GCase polypeptide comprising the at least one mutation may be expressed at least 1.1 fold, at least 1.2 fold, at least 1.5 fold, at least 1.8 fold, at least 2 fold, at least 2.5 fold, at least 3 fold, at least 3.5 fold, at least 4 fold, at least 4.5 fold or at least 5 fold higher compared to the reference GCase polypeptide. Optionally, the modified GCase polypeptide comprising the at least one mutation may be expressed between 1.1 fold and 5 fold, between 1.2 fold and 5 fold, between 1.5 fold and 5 fold, between 1.8 fold and 5 fold, between 2 fold and 5 fold, between 2.5 fold and 5 fold, between 3 fold and 5 fold, between 3.5 fold and 5 fold, between 4 fold and 5 fold, or between 4.5 fold and 5 fold higher compared to the reference GCase polypeptide.

The level of expression of a GCase polypeptide (and therefore whether the modified GCase polypeptide is expressed at a higher level in a host cell than the reference GCase polypeptide) may typically be determined by measuring the level of GCase polypeptide in a sample. The level of expression of the modified GCase polypeptide of the invention in a host cell may be compared with the level of expression of the reference GCase polypeptide in a host cell. This may be determined quantitatively. For example, the level of expression of a GCase polypeptide may be determined by an ELISA assay as described above. Alternatively, the level of expression of a GCase polypeptide may be determined semi-quantitatively, for example, by SDS-PAGE electrophoresis or by Western blot.

The level of a GCase polypeptide secreted by a host cell may be used to determine the level of expression of the GCase polypeptide (*i.e.* in contrast to the level of the GCase polypeptide that is retained intracellularly by a host cell). Optionally, the GCase polypeptide of the invention is secreted by a host cell at a higher level than the reference GCase polypeptide. For example, the modified GCase polypeptide comprising the at least one mutation may be secreted at a level that is least 1.1 fold, at least 1.2 fold, at least 1.5 fold, at least 1.8 fold, at least 2 fold, at least 2.5 fold, at least 3 fold, at least 3.5 fold, at least 4 fold, at least 4.5 fold, or at least 5 fold higher compared to the reference GCase polypeptide. Optionally, the modified GCase polypeptide comprising the at least one mutation may be secreted at a level that is between 1.1 fold and 5 fold, between 1.2 fold and 5 fold, between 1.5 fold and 5 fold, between 1.8 fold and 5 fold, between 2 fold and 5 fold, between 2.5 fold and 5 fold, between 3 fold and 5 fold, between 3.5 fold and 5 fold, between 4 fold and 5 fold, or between 4.5 fold and 5 fold higher compared to the reference GCase polypeptide.

The level of a GCase polypeptide secreted by a host cell may be determined, for example, by separating cells (*e.g.* host cells) from a liquid containing the GCase polypeptide (*e.g*. a biological sample, such as blood, serum or plasma, or culture medium such as conditioned medium in which a host cell expressing a GCase polypeptide is cultured) and determining the level of expression of a GCase polypeptide in the liquid. Cells may be separated, for example, by centrifugation or filtration, and/or the liquid may be decanted (*e.g.* by pipetting) from the cells. The level of expression of a GCase polypeptide may be determined in a biological sample, such as blood, serum or plasma, or may be determined in a culture medium, such as conditioned medium in which a host cell expressing a GCase polypeptide is cultured.

The host cell may be any eukaryotic cell such as a mammalian cell or insect cell. The host cell may be any eukaryotic host cell expressing the modified GCase polypeptide of the invention (or the reference GCase polypeptide). Optionally, the host cell may be an insect cell expressing the modified GCase polypeptide of the invention (or the reference GCase polypeptide). Typically, the host cell may be a mammalian host cell expressing the modified GCase polypeptide of the invention (or the reference GCase polypeptide). Mammalian host cells include human (e.g. Expi293F human embryonic kidney cells), dog, pig, mouse, hamster, or guinea pig cells. More particularly, the host cell may be a mammalian liver cell, and more particularly may be a human liver cell. Optionally, the host cell may be an Huh7 cell. Optionally, the host cell may be a host cell within an organism. Optionally, expression may be *in vivo* expression. Optionally, expression may be *in vivo* expression and expression in plasma may be determined.

### A viral particle comprising the polynucleotide

The invention further provides a viral particle comprising a recombinant genome comprising the polynucleotide of the invention. For the purposes of the present invention, the term *"viral particle"* refers to all or part of a virion. For example, the viral particle comprises a recombinant genome and may further comprise a capsid. The viral particle may be a gene therapy vector. Herein, the terms *"viral particle"* and *"vector"* are used interchangeably. For the purpose of the present application, a *"gene therapy"* vector is a viral particle that can be used in gene therapy, *i.e.* a viral particle that comprises all the required functional elements to express a transgene, such as a GBA nucleotide sequence, in a host cell after administration.

Suitable viral particles include a parvovirus, a retrovirus, a lentivirus or a herpes simplex virus. The parvovirus may be an adeno-associated virus (AAV). Optionally, the viral particle is an AAV, adenoviral or lentiviral viral particle. The viral particle is preferably a recombinant AAV vector or a lentiviral vector. More preferably, the viral particle is an AAV viral particle. The terms AAV and rAAV are used interchangeably herein, unless context obviously suggests otherwise.

The genomic organization of all known AAV serotypes is very similar. The genome of AAV is a linear, single-stranded DNA molecule that is fewer than about 5,000 nucleotides in length. Inverted terminal repeats (ITRs) flank the unique coding nucleotide sequences for the non-structural replication (Rep) proteins and the structural (VP) proteins. The VP proteins (VP1, -2 and -3) form the capsid. The terminal ~145 nt (ITRs) are self-complementary and are organized so that an energetically stable intramolecular duplex forming a T-shaped hairpin may be formed. These hairpin structures function as an origin for viral DNA replication, serving as primers for the cellular DNA polymerase complex. Following wild type (wt) AAV infection in mammalian cells the Rep genes (*i.e.* encoding Rep78 and Rep52 proteins) are expressed from the P5 promoter and the P19 promoter, respectively, and both Rep proteins have a function in the replication of the viral genome. A splicing event in the Rep ORF results in the expression of four Rep proteins (*i.e*. Rep78, Rep68, Rep52 and Rep40). However, it has been shown that the unspliced mRNA, encoding Rep78 and Rep52 proteins, in mammalian cells are sufficient for AAV vector production. Also in insect cells the Rep78 and Rep52 proteins suffice for AAV vector production.

The recombinant viral genome of the invention may comprise ITRs. The recombinant genome or viral particle may comprise one or two ITR(s), such as the ITR(s) as described above. It is possible for an AAV vector of the invention to function with only one ITR. Thus, the viral genome typically comprises at least one ITR, but, more typically, two ITRs (generally with one either end of the viral genome, *i.e.* one at the 5' end and one at the 3' end). There may be intervening sequences between the polynucleotide of the invention and one or more of the ITRs. The polynucleotide may be incorporated into a viral particle located between two regular ITRs or located on either side of an ITR engineered with two D regions.

AAV sequences that may be used in the present invention for the production of AAV vectors can be derived from the genome of any AAV serotype. Generally, the AAV serotypes have genomic sequences of significant homology at the amino acid and the nucleic acid levels, provide an identical set of genetic functions, produce virions which are essentially physically and functionally equivalent, and replicate and assemble by practically identical mechanisms. For the genomic sequence of the various AAV serotypes and an overview of the genomic similarities see e.g. GenBank Accession number U89790; GenBank Accession number J01901; GenBank Accession number AF043303; GenBank Accession number AF085716; Chiorini *et al,* 1997; Srivastava *et al,* 1983; Chiorini *et al,* 1999; Rutledge *et al,* 1998; and Wu *et al,* 2000. AAV serotype 1, 2, 3, 3B, 4, 5, 6, 7, 8, 9, 10, 11 or 12 may be used in the present invention. The sequences from the AAV serotypes may be mutated or engineered when being used in the production of gene therapy vectors.

Optionally, an AAV vector comprises ITR sequences which are derived from AAV1, AAV2, AAV4 and/or AAV6. Preferably the ITR sequences are AAV2 ITR sequences. Herein, the term AAVx/y refers to a viral particle that comprises genomic components such as at least ITRs from AAVx (wherein x is a AAV serotype number) and has the capsid from AAVy (wherein y is the number of the same or different serotype). For example, an AAV2/8 vector may comprise a portion of a viral genome, including the ITRs, from an AAV2 strain, and a capsid from an AAV8 strain.

In an embodiment, the viral particle is an AAV viral particle comprising a capsid. AAV capsids are generally formed from three proteins, VP1, VP2 and VP3. The amino acid sequence of VP1 comprises the sequence of VP2. The portion of VP1 which does not form part of VP2 is referred to as VP1unique or VP1U. The amino acid sequence of VP2 comprises the sequence of VP3. The portion of VP2 which does not form part of VP3 is referred to as VP2unique or VP2U. Optionally, the viral particle comprises a liver-tropic or CNS-tropic capsid. Whether a viral particle (capsid) is tropic for a particular tissue can be evaluated for example by administering such a particle expressing a marker gene such as luciferase and imaging *in vivo* at multiple time points (for example as described in Zincarelli et al (2008), Molecular Therapy, 16:1073-1080). A particle driving strong marker expression in liver or CNS tissues, respectively, especially if in contrast to lesser expression in other tissues, would be considered liver- or CNS-tropic.

In some embodiments, a liver-tropic capsid can be an AAV3-, AAV3B-, AAV5, or AAV8-derived capsid. Optionally, the liver-tropic capsid can be an AAV3-, AAV3B-, or AAV8-derived capsid. Optionally, the liver-tropic capsid can be an AAV3-, or AAV3B- derived capsid. Optionally, the liver-tropic capsid comprises a sequence at least 98%, at least 99%, or at least 99.5% identical to a fragment of at least 600, at least 650, at least 700, between 600 and 736, between 650 and 736 or between 700 and 736 amino acids of SEQ ID NO: 36, 37, or 38. Optionally, the liver-tropic capsid comprises a sequence at least 99% identical to SEQ ID NO: 36. Optionally, the liver-tropic capsid comprises a sequence at least 99% identical to SEQ ID NO: 37. Optionally, the liver-tropic capsid comprises a sequence at least 99% identical to SEQ ID NO: 38. Optionally, the liver-tropic capsid comprises a sequence 100% identical to a fragment of at least 600, at least 650, at least 700, between 600 and 736, between 650 and 736 or between 700 and 736 amino acids of SEQ ID NO: 36, 37, or 38. Optionally, the liver-tropic capsid comprises a sequence 100% identical to SEQ ID NO: 36. Optionally, the liver-tropic capsid comprises a sequence 100% identical to SEQ ID NO: 37. Optionally, the liver-tropic capsid comprises a sequence 100% identical to SEQ ID NO: 38. Optionally, the CNS-tropic capsid comprises a sequence at least 98%, at least 99%, or at least 99.5% identical to a fragment of at least 600, at least 650, at least 700, between 600 and 736, between 650 and 736, or between 700 and 736 amino acids of SEQ ID NO: 39. Optionally, the CNS-tropic capsid comprises a sequence 100% identical to a fragment of at least 600, at least 650, at least 700, between 600 and 736, between 650 and 736, or between 700 and 736 amino acids of SEQ ID NO: 39. Optionally, the CNS-tropic capsid comprises a sequence at least 99% identical to SEQ ID NO: 39. Optionally, the CNS-tropic capsid comprises a sequence 100% identical to SEQ ID NO: 39. In some embodiments, a CNS-tropic capsid can be an AAV9 or AAVrh.10-derived capsid. A viral particle of the invention may be a *"hybrid"* particle in which the viral ITRs and viral capsid are from different parvoviruses, such as different AAV serotypes. Preferably, the viral ITRs and capsid are from different serotypes of AAV, in which case such viral particles are known as transcapsidated or pseudotyped. Likewise, the parvovirus may have a *"chimeric"* capsid (*e. g.,* containing sequences from different parvoviruses, preferably different AAV serotypes) or a *"targeted"* capsid (e. g., a directed tropism).

In some embodiments, the recombinant AAV genome comprises intact ITRs, comprising functional terminal resolution sites (TRS). Such an AAV genome may contain one or two resolvable ITRs, *i.e.* ITRs containing a functional TRS at which site-specific nicking can take place to create a free 3' hydroxyl group which can serve as a substrate for DNA polymerase to unwind and copy the ITR.

Preferably, the recombinant genome is single-stranded (*i.e*., it is packaged into the viral particle in a single-stranded form). Optionally, the recombinant genome is not packaged in self-complementary configuration, *i.e.* the genome does not comprise a single covalently-linked polynucleotide strand with substantial self-complementary portions that anneal in the viral particle. Alternatively, the recombinant genome may be packaged in *"monomeric duplex"* form. *"Monomeric duplexes"* are described in WO 2011/122950. The genome may be packaged as two substantially complementary but non-covalently linked polynucleotides which anneal in the viral particle.

As described above, the polynucleotide of the invention may comprise a polyA nucleotide sequence. Thus, the recombinant genome or viral particle may comprise a poly A sequence such as the poly A sequence as described above.

As described above, the polynucleotide of the invention may comprise an intron sequence. Thus, the recombinant genome or viral particle of the invention may comprise an intron sequences such as the intron sequence as described above.

In an embodiment, the viral particle comprises a polynucleotide sequence comprising a transcription regulatory element (comprising *e.g.* a promoter and/or enhancer), the modified GBA nucleotide sequence, and a poly A sequence, such as the bGHpA sequence. In such embodiments, the poly A sequence, such as the bGHpA sequence, may be located downstream of the modified GBA nucleotide sequence. In some embodiments, the viral particle comprises AAV2 ITRs and a poly A sequence (such as the bGHpA sequence).

In an embodiment, the viral particle comprises a polynucleotide sequence comprising a transcription regulatory element (comprising *e.g.* a promoter and/or enhancer), an intron sequence, such as an SV40 intron sequence, the modified GBA nucleotide sequence, and a poly A sequence, such as the bGHpA sequence. In such embodiments, the intron sequence, such as the SV40 intron sequence, may be located between the transcription regulatory element and the modified GBA nucleotide sequence. In such embodiments, the poly A sequence, such as the bGHpA sequence, may be located downstream of the modified GBA nucleotide sequence. In some embodiments, the viral particle comprises AAV2 ITRs, a poly A sequence (such as the bGHpA sequence), and/or an intron (such as an SV40 intron).

In some embodiments, following transduction of the viral particle of the invention into a host cell (*e.g.* a Huh-7 cell), the effective activity of the modified GCase polypeptide is the same or higher compared to the effective activity of a reference GCase polypeptide following transduction into a host cell (*e.g.* a Huh-7 cell) of an otherwise identical viral particle comprising a GBA nucleotide sequence encoding the reference GCase polypeptide. In some embodiments, the reference GCase polypeptide comprises the polypeptide of SEQ ID NO: 41 or 44. Optionally, the reference GCase polypeptide is the polypeptide of SEQ ID NO: 41 or 44. Optionally, the GBA nucleotide sequence encoding the reference GCase polypeptide comprises the nucleotide sequence of SEQ ID NO: 6 or 10. In some embodiments, the reference GCase polypeptide comprises the polypeptide of SEQ ID NO: 43 or 46. Optionally, the reference GCase polypeptide is the polypeptide of SEQ ID NO: 43 or 46. Optionally, the GBA nucleotide sequence encoding the reference GCase polypeptide comprises the nucleotide sequence of SEQ ID NO: 22 or 26. In some embodiments, the reference GCase polypeptide comprises the polypeptide of SEQ ID NO: 42 or 45. Optionally, the reference GCase polypeptide is the polypeptide of SEQ ID NO: 42 or 45. Optionally, the GBA nucleotide sequence encoding the reference GCase polypeptide comprises the nucleotide sequence of SEQ ID NO: 14 or 18. In some embodiments, the reference GCase polypeptide is a wild-type GCase polypeptide. Optionally, the reference GCase polypeptide comprises the polypeptide of SEQ ID NO: 1 or 2. Optionally, the reference GCase polypeptide is the polypeptide of SEQ ID NO: 1 or 2. Optionally, the GBA nucleotide sequence encoding the reference GCase polypeptide comprises the nucleotide sequence of SEQ ID NO: 59 or 60. In some embodiments, the effective activity of the modified GCase polypeptide is higher compared to the effective activity of the reference GCase polypeptide. In some embodiments, the effective activity of the modified GCase polypeptide is at least 2x, at least 3x, at least 4x, at least 5x, at least 7x, at least 10x, at least 20x, at least 30x, at least 40x, at least 50x, at least 60x, or at least 70x higher compared to the effective activity of the reference GCase polypeptide. Optionally, the effective activity of the modified GCase polypeptide is at least 50x, at least 60x, or at least 70x higher compared to the effective activity of the reference GCase polypeptide. Optionally, the effective activity of the modified GCase polypeptide is up to 50x, up to 60x, up to 70x, up to 80x, or up to 100x higher compared to the effective activity of the reference GCase polypeptide. Optionally, the effective activity of the modified GCase polypeptide is between 2x and 100x, between 3x and 100x, between 5x and 100x, between 15x and 100x, between 20x and 100x, between 30x and 100x, between 40x and 100x, between 50x and 100x, or between 70x and 100x higher compared to the effective activity of the reference GCase polypeptide. Optionally, the effective activity of the modified GCase polypeptide is between 2x and 80x, between 5x and 80x, between 20x and 80x, or between 50x and 80x higher compared to the effective activity of the reference GCase polypeptide. Optionally, the effective activity of the modified GCase polypeptide is between 2x and 70 x, between 2x and 10 x, between 3x and 20x, between 5x and 10x, between 15x and 30x, between 20x and 40x, between 30x and 50x, between 40x and 60x, or between 50x and 70x higher compared to the effective activity of the reference GCase polypeptide. In some embodiments, the reference GCase polypeptide is a wild-type GCase polypeptide. Optionally, the reference GCase polypeptide comprises the polypeptide of SEQ ID NO: 1 or 2. Optionally, the reference GCase polypeptide is the polypeptide of SEQ ID NO: 1 or 2. Optionally, the GBA nucleotide sequence encoding the reference GCase polypeptide comprises the nucleotide sequence of SEQ ID NO: 59 or 60. In some embodiments, the reference GCase polypeptide comprises the polypeptide of SEQ ID NO: 41 or 44. Optionally, the reference GCase polypeptide is the polypeptide of SEQ ID NO: 41 or 44. Optionally, the GBA nucleotide sequence encoding the reference GCase polypeptide comprises the nucleotide sequence of SEQ ID NO: 6 or 10. In some embodiments, the reference GCase polypeptide comprises the polypeptide of SEQ ID NO: 43 or 46. Optionally, the reference GCase polypeptide is the polypeptide of SEQ ID NO: 43 or 46. Optionally, the GBA nucleotide sequence encoding the reference GCase polypeptide comprises the nucleotide sequence of SEQ ID NO: 22 or 26. In some embodiments, the reference GCase polypeptide comprises the polypeptide of SEQ ID NO: 42 or 45. Optionally, the reference GCase polypeptide is the polypeptide of SEQ ID NO: 42 or 45. Optionally, the GBA nucleotide sequence encoding the reference GCase polypeptide comprises the nucleotide sequence of SEQ ID NO: 14 or 18. Optionally, the effective activity of the modified GCase polypeptide is at least 50%, at least 60%, at least 70%, at least 80%, at least 90%, at least 95%, or at least 100% of the effective activity of the GCase polypeptide encoded by the sequence of SEQ ID NO: 14 or 18. Optionally, the host cell is a Huh-7 cell. Optionally, the viral particle of the invention and the otherwise identical viral particle comprise the capsid of SEQ ID NO: 37.

Describing the effective activity of the modified GCase polypeptide as being the *"same"* as the effective activity of a reference GCase polypeptide indicates that the effective activity of the modified GCase polypeptide may be identical to the effective activity of the reference GCase polypeptide plus or minus 10%, more particularly plus or minus 5%, or more particularly plus or minus 1%.

The *"otherwise identical"* viral particle comprising the GBA nucleotide sequence encoding a reference GCase polypeptide is a viral particle which is identical (*i.e.* the viral particle comprises the same capsid and the recombinant genome comprises the same transcription regulatory elements *etc*.) to the viral particle of the invention except that the GBA nucleotide sequences are different. For example, the different GBA nucleotide sequences encoding the different GCase polypeptides being compared are operably linked to the same promoter sequence.

In some embodiments, following administration of the viral particle of the present invention, the effective activity of the modified GCase polypeptide is the same or higher in liver, plasma, white blood cells, the spleen, bone marrow, lung tissue, and/or any other Gaucher Disease-affected organ, compared to the effective activity of a reference GCase polypeptide following administration of an otherwise identical viral particle comprising a GBA nucleotide sequence encoding the reference GCase polypeptide. In some embodiments, following administration of the viral particle of the present invention, the effective activity of the modified GCase polypeptide is the same or higher in liver compared to the effective activity of a reference GCase polypeptide following administration of an otherwise identical viral particle comprising a GBA nucleotide sequence encoding the reference GCase polypeptide. In some embodiments, following administration of the viral particle of the present invention, the effective activity of the modified GCase polypeptide is the same or higher in plasma, white blood cells, the spleen, bone marrow, lung tissue, and/or any other Gaucher Disease-affected organ, compared to the effective activity of a reference GCase polypeptide following administration of an otherwise identical viral particle comprising a GBA nucleotide sequence encoding the reference GCase polypeptide. In some embodiments, the effective activity of the modified GCase polypeptide is the same or higher in bone marrow, spleen and/or plasma compared to the effective activity of the reference GCase polypeptide. In some embodiments, the effective activity of the modified GCase polypeptide is the same or higher in spleen, lung and/or bone marrow compared to the effective activity of the reference GCase polypeptide. In some embodiments, the reference GCase polypeptide comprises the polypeptide of SEQ ID NO: 41 or 44. Optionally, the reference GCase polypeptide is the polypeptide of SEQ ID NO: 41 or 44. Optionally, the GBA nucleotide sequence encoding the reference GCase polypeptide comprises the nucleotide sequence of SEQ ID NO: 6 or 10. In some embodiments, the reference GCase polypeptide comprises the polypeptide of SEQ ID NO: 43 or 46. Optionally, the reference GCase polypeptide is the polypeptide of SEQ ID NO: 43 or 46. Optionally, the GBA nucleotide sequence encoding the reference GCase polypeptide comprises the nucleotide sequence of SEQ ID NO: 22 or 26. In some embodiments, the reference GCase polypeptide comprises the polypeptide of SEQ ID NO: 42 or 45. Optionally, the reference GCase polypeptide is the polypeptide of SEQ ID NO: 42 or 45. Optionally, the GBA nucleotide sequence encoding the reference GCase polypeptide comprises the nucleotide sequence of SEQ ID NO: 14 or 18. In some embodiments, the reference GCase polypeptide is a wild-type GCase polypeptide. Optionally, the reference GCase polypeptide comprises the polypeptide of SEQ ID NO: 1 or 2. Optionally, the reference GCase polypeptide is the polypeptide of SEQ ID NO: 1 or 2. Optionally, the GBA nucleotide sequence encoding the reference GCase polypeptide comprises the nucleotide sequence of SEQ ID NO: 59 or 60. In some embodiments, the effective activity of the modified GCase polypeptide is higher compared to the effective activity of the reference GCase polypeptide. In some embodiments, the effective activity of the modified GCase polypeptide is at least 2x, at least 3x, at least 4x, at least 5x, at least 7x, at least 10x, at least 20x, at least 30x, at least 40x, at least 50x, at least 60x, or at least 70x higher compared to the effective activity of the reference GCase polypeptide. In some embodiments, the effective activity of the modified GCase polypeptide is at least 2x, at least 3x, at least 4x, at least 5x, at least 7x, or at least 10x higher compared to the effective activity of the reference GCase polypeptide. In some embodiments, the effective activity of the modified GCase polypeptide is at least 2x, at least 3x, at least 4x, at least 5x, at least 7x, or at least 10x higher in the plasma compared to the effective activity of the reference GCase polypeptide. In some embodiments, the effective activity of the modified GCase polypeptide is at least 15x, at least 20x, at least 25x, at least 30x, at least 35x, or at least 40x higher compared to the effective activity of the reference GCase polypeptide. In some embodiments, the effective activity of the modified GCase polypeptide is at least 15x, at least 20x, at least 25x, at least 30x, at least 35x, or at least 40x higher in plasma compared to the effective activity of the reference GCase polypeptide. In some embodiments, the effective activity of the modified GCase polypeptide is at least 2x, at least 3x, or at least 4x higher compared to the effective activity of the reference GCase polypeptide. In some embodiments, the effective activity of the modified GCase polypeptide is at least 2x, at least 3x, or at least 4x higher in the spleen compared to the effective activity of the reference GCase polypeptide. In some embodiments, the effective activity of the modified GCase polypeptide is at least 2x, at least 3x, or at least 4x greater in the bone marrow compared to the effective activity of the reference GCase polypeptide. In some embodiments, the effective activity of the modified GCase polypeptide is between 2x and 5x, between 2x and 10 x, between 3x and 7x, between 3x and 20x, between 5x and 10x, between 15x and 30x, between 20x and 40x, between 30x and 50x, between 40x and 60x, or between 50x and 70x higher compared to the effective activity of the reference GCase polypeptide. In some embodiments, the effective activity of the modified GCase polypeptide is between 2x and 20x, between 2x and 15x, between 2x and 10x, between 2x and 7x, between 2x and 5x, or between 3x and 5x higher compared to the effective activity of the reference GCase polypeptide. In some embodiments, the reference GCase polypeptide is a wild-type GCase polypeptide. Optionally, the reference GCase polypeptide comprises the polypeptide of SEQ ID NO: 1 or 2. Optionally, the reference GCase polypeptide is the polypeptide of SEQ ID NO: 1 or 2. Optionally, the GBA nucleotide sequence encoding the reference GCase polypeptide comprises the nucleotide sequence of SEQ ID NO: 59 or 60. In some embodiments, the reference GCase polypeptide comprises the polypeptide of SEQ ID NO: 41 or 44. Optionally, the reference GCase polypeptide is the polypeptide of SEQ ID NO: 41 or 44. Optionally, the GBA nucleotide sequence encoding the reference GCase polypeptide comprises the nucleotide sequence of SEQ ID NO: 6 or 10. In some embodiments, the reference GCase polypeptide comprises the polypeptide of SEQ ID NO: 43 or 46. Optionally, the reference GCase polypeptide is the polypeptide of SEQ ID NO: 43 or 46. Optionally, the GBA nucleotide sequence encoding the reference GCase polypeptide comprises the nucleotide sequence of SEQ ID NO: 22 or 26. In some embodiments, the reference GCase polypeptide comprises the polypeptide of SEQ ID NO: 42 or 45. Optionally, the reference GCase polypeptide is the polypeptide of SEQ ID NO: 42 or 45. Optionally, the GBA nucleotide sequence encoding the reference GCase polypeptide comprises the nucleotide sequence of SEQ ID NO: 14 or 18. In some embodiments, the effective activity of the modified GCase polypeptide is at least 50%, at least 60%, at least 70%, at least 80%, at least 90%, at least 95%, or at least 100% of the effective activity of the GCase polypeptide encoded by the sequence of SEQ ID NO: 14 or 18. Optionally, the viral particle of the invention and the otherwise identical viral particle comprises an AAV8 capsid.

In some embodiments, the effective activity is measured after at least 6 weeks, at least 8 weeks, at least 10 weeks or at least 12 weeks from administration of the viral particle of the invention. Preferably, the effective activity is measured at or after 12 weeks. For example, effective activity after at least 6 weeks (e.g. at 6 weeks), at least 8 weeks (e.g. at 8 weeks), at least 10 weeks (e.g. at 10 weeks), or at least 12 weeks (e.g. at 12 weeks) from administration of the viral particle of the invention may be compared to levels after at least 6 weeks (e.g. at 6 weeks), at least 8 weeks (e.g. at 8 weeks), at least 10 weeks (e.g. at 10 weeks) or at least 12 weeks (e.g. at 12 weeks), respectively, from the administration of the otherwise identical viral particle comprising a GBA nucleotide sequence encoding a reference GCase polypeptide. As a particular example, effective activity level may be measured after at least 12 weeks (e.g. at 12 weeks) from administration of the viral particle of the invention and compared to the level measured after at least 12 weeks (e.g. at 12 weeks) from administration of the otherwise identical viral particle comprising a GBA nucleotide sequence encoding a reference GCase polypeptide. Preferably, the effective activity levels are measured in the same assay at the same time point after administration.

In some embodiments, the GCase effective activity is measured using a fluorometric assay such as those described herein, for example using a fluorogenic substrate which is specific for GCase. The fluorogenic substrate may be as described herein.

In some embodiments, the effective activity of the modified GCase polypeptide is the same or higher in plasma, liver, white blood cells, the spleen, bone marrow, lung tissue, and/or any other Gaucher Disease-affected organ, compared to the effective activity of a GCase enzyme replacement therapy. In some embodiments, the effective activity of the modified GCase polypeptide is the same or higher in liver, white blood cells, the spleen, bone marrow, lung tissue, and/or any other Gaucher Disease-affected organ, compared to the effective activity of a GCase enzyme replacement therapy. In one embodiment, the effective activity of the modified GCase polypeptide is the same or higher in bone marrow and/or lung tissue compared to the effective activity of a GCase enzyme replacement therapy.

In one embodiment, the effective activity of the modified GCase polypeptide is higher compared to the effective activity of the GCase enzyme replacement therapy. In one embodiment, the effective activity of the modified GCase polypeptide is at least 1.1 times, at least 1.2 times or at least 1.3 times higher compared to the effective activity of the GCase enzyme replacement therapy. In one embodiment, the effective activity of the modified GCase polypeptide in lung tissue is at least 1.5 times or at least 2 times higher compared to the effective activity of the GCase enzyme replacement therapy.

In one embodiment, the effective activity of the modified GCase polypeptide (e.g. in a subject) is higher compared to the effective activity of the GCase enzyme replacement therapy, preferably when the effective activity is measured after at least 6 weeks, at least 8 weeks, at least 10 weeks or at least 12 weeks after the start of treatment in the subject. Preferably, the effective activity is measured at or after 12 weeks.

*A "GCase enzyme replacement therapy"* may refer to any therapy which comprises the administration of a GCase polypeptide to a subject. The GCase polypeptide may be wild type, such as a GCase polypeptide having the amino acid sequence of SEQ ID NO: 3 and/or velaglucerase alfa. The GCase polypeptide may be administered at any suitable dose, optionally at a dose of between 40 and 100, between 50 and 80, between 60 and 70, or around 60 U/kg BW. The GCase polypeptide may be administered through any appropriate route, optionally administered through intravenous injection or subcutaneous injection. The effective activity of the GCase enzyme replacement therapy is preferably measured 2 hours after administration of a dose (e.g. the previous dose) of the GCase enzyme replacement therapy. By way of example, when the GCase enzyme replacement therapy is administered every two weeks to a subject, measuring the effective activity of the GCase enzyme replacement therapy at 12 weeks would involve obtaining a tissue sample from the subject two hours following the administration of the 12^{th} week (i.e. 7^{th}) dose. Optionally, measuring the effective activity of the GCase enzyme replacement therapy comprises administering doses of the GCase enzyme replacement therapy every two weeks. Optionally, measuring the effective activity of the GCase enzyme replacement therapy comprises administering doses of the GCase enzyme replacement therapy for at least 12 or 12 weeks. Optionally, measuring the effective activity of the GCase enzyme replacement therapy comprises administering doses of the GCase enzyme replacement therapy every two weeks and the effective activity of the GCase enzyme replacement therapy is measured 2 hours after administration of the final dose of the GCase enzyme replacement therapy.

In one embodiment, following administration of the viral particle, the level of hexosylsphingosine and/or hexosylceramide is lower in plasma, liver, the spleen, bone marrow, lung tissue, and/or any other Gaucher Disease-affected organ, compared to the level of hexosylsphingosine and/or hexosylceramide after administration of a GCase enzyme replacement therapy as described above. In a related embodiment, following administration of the viral particle, the level of hexosylsphingosine and/or hexosylceramide is lower in plasma, liver, the spleen, bone marrow, lung tissue, and/or any other Gaucher Disease-affected organ, compared to the level of hexosylsphingosine and/or hexosylceramide in a subject having Gaucher Disease (e.g. in the same or equivalent organ). Optionally, following administration of the viral particle, the level of hexosylsphingosine and/or hexosylceramide is less than 50%, less than 40%, less than 30%, less than 25%, less than 10%, or between 0% and 25% of the level of hexosylsphingosine and/or hexosylceramide after administration of a GCase enzyme replacement therapy. Optionally, following administration of the viral particle, the level of hexosylsphingosine and/or hexosylceramide is less than 50%, less than 40%, less than 30%, less than 25%, less than 10%, or between 0% and 25% of the level of hexosylsphingosine and/or hexosylceramide in a subject having Gaucher Disease (e.g. in the same or equivalent organ). In one embodiment, the level of hexosylsphingosine and/or hexosylceramide is measured by mass spectrometry, for example by the method described in example 13. A subject having Gaucher Disease may be a Gaucher Disease model organism, such as a mouse model of Gaucher Disease (*e.g.* a 9V/null mouse).

In some embodiments, following administration of the viral particle, the level of hexosylsphingosine and/or hexosylceramide is lower in plasma, liver, the spleen, bone marrow, lung tissue, and/or any other Gaucher Disease-affected organ, compared to the level of hexosylsphingosine and/or hexosylceramide following administration of an otherwise identical viral particle comprising a GBA nucleotide sequence encoding a reference GCase polypeptide. Optionally, following administration of the viral particle, the level of hexosylsphingosine and/or hexosylceramide is less than 50%, less than 40%, less than 30%, less than 25%, less than 10%, or between 0% and 25% of the level of hexosylsphingosine and/or hexosylceramide after administration of the otherwise identical viral particle comprising a GBA nucleotide sequence encoding a reference GCase polypeptide. Optionally, the reference GCase polypeptide is a wild-type GCase polypeptide. Optionally, the reference GCase polypeptide comprises the polypeptide of SEQ ID NO: 1 or 2. Optionally, the reference GCase polypeptide is the polypeptide of SEQ ID NO: 1 or 2. Optionally, the GBA nucleotide sequence encoding the reference GCase polypeptide comprises the nucleotide sequence of SEQ ID NO: 59 or 60. Optionally, following administration of the viral particle, the level of hexosylsphingosine and/or hexosylceramide is less than 50%, less than 40%, less than 30%, less than 25%, less than 10%, or between 0% and 25% of the level of hexosylsphingosine and/or hexosylceramide in a subject having Gaucher Disease (e.g. in the same or equivalent organ). In one embodiment, the level of hexosylsphingosine and/or hexosylceramide is measured by mass spectrometry, for example by the method described in example 13. A subject having Gaucher Disease may be a Gaucher Disease model organism, such as a mouse model of Gaucher Disease (*e.g.* a 9V/null mouse).

In one embodiment, following administration of the viral particle, the number of storage cells or level of activated macrophages is lower in the liver or lung tissue, (and/or any other Gaucher Disease-affected organ), compared to the number of storage cells or level of activated macrophages after administration of a GCase enzyme replacement therapy as described above. Optionally, the number of storage cells is less than 50%, less than 40%, less than 30%, less than 25%, less than 15%, or between 0% and 25% of the number of storage cells after administration of the GCase enzyme replacement therapy. Optionally, the level of activated macrophages is less than 50%, less than 40%, less than 30%, less than 25%, less than 15%, or between 0% and 25% of the level of activated macrophages after administration of the GCase enzyme replacement therapy.

In one embodiment, the level of hexosylsphingosine, level of hexosylceramide, number of storage cells and/or level of activated macrophages is measured after at least 6 weeks, at least 8 weeks, at least 10 weeks or at least 12 weeks after the administration of the viral particle. Preferably, the level of hexosylsphingosine, level of hexosylceramide, number of storage cells and/or level of activated macrophages is measured at or after 12 weeks.

In some embodiments, the level of hexosylsphingosine and/or hexosylceramide is measured after at least 6 weeks, at least 8 weeks, at least 10 weeks or at least 12 weeks from administration of the viral particle. Preferably, the level of hexosylsphingosine and/or hexosylceramide is measured at or after 12 weeks. For example, the level of hexosylsphingosine and/or hexosylceramide after at least 6 weeks (e.g. at 6 weeks), at least 8 weeks (e.g. at 8 weeks), at least 10 weeks (e.g. at 10 weeks), or at least 12 weeks (e.g. at 12 weeks) from administration of the viral particle of the invention may be compared to the level after at least 6 weeks (e.g. at 6 weeks), at least 8 weeks (e.g. at 8 weeks), at least 10 weeks (e.g. at 10 weeks), or at least 12 weeks (e.g. at 12 weeks), respectively, from administration of the otherwise identical viral particle comprising a GBA nucleotide sequence encoding a reference GCase polypeptide. As a particular example, the level of hexosylsphingosine and/or hexosylceramide may be measured after at least 12 weeks (e.g. at 12 weeks) from administration of the viral particle of the invention and compared to the level measured after at least 12 weeks (e.g. at 12 weeks) from administration of the otherwise identical viral particle comprising a GBA nucleotide sequence encoding a reference GCase polypeptide. Preferably, the level of hexosylsphingosine and/or hexosylceramide is measured in the same assay at the same time point after administration.

When measuring the level of hexosylsphingosine, level of hexosylceramide, number of storage cells and/or level of activated macrophages following GCase enzyme replacement therapy, the level of hexosylsphingosine, level of hexosylceramide, number of storage cells and/or level of activated macrophages is preferably measured 2 hours after administration of a dose (e.g. the previous dose) of the GCase enzyme replacement therapy. By way of example, when the GCase enzyme replacement therapy is administered every two weeks to a subject, measuring the level of hexosylsphingosine, level of hexosylceramide, number of storage cells and/or level of activated macrophages at 12 weeks would involve obtaining a tissue sample from the subject two hours following the administration of the 12th week (i.e. 7th) dose. Optionally, measuring the level of hexosylsphingosine, level of hexosylceramide, number of storage cells and/or level of activated macrophages after administration of the GCase enzyme replacement therapy comprises administering doses of the GCase enzyme replacement therapy every two weeks. Optionally, measuring the level of hexosylsphingosine, level of hexosylceramide, number of storage cells and/or level of activated macrophages after administration of the GCase enzyme replacement therapy comprises administering doses of the GCase enzyme replacement therapy for at least 12 or 12 weeks. Optionally, measuring the level of hexosylsphingosine, level of hexosylceramide, number of storage cells and/or level of activated macrophages after administration of the GCase enzyme replacement therapy comprises administering doses of the GCase enzyme replacement therapy every two weeks and the level of hexosylsphingosine, level of hexosylceramide, number of storage cells and/or level of activated macrophages after administration of the GCase enzyme replacement therapy is measured 2 hours after administration of the final dose of the GCase enzyme replacement therapy.

In one embodiment, the number of storage cells is measured by microscopy. In one embodiment, the level of activated macrophages is detected by measuring the CD68 density using immunohistochemistry. Identifying storage cells and/or CD68^{positive} cells can be performed by methods known in the art, such as by immunohistochemistry or microscopy, for example the methods described in example 13.

In one example, a reduction in hexosylceramide and/or hexosylsphingosine levels may represent a reduction in glucosylceramide and/or glucosylsphingosine levels, respectively. For example, a reduction in hexosylceramide may represent a reduction in glucosylceramide. As a further example, a reduction in hexosylsphingosine levels may represent a reduction in glucosylsphingosine levels.

### Compositions, methods and uses

In a further aspect of the invention, there is provided a composition comprising the modified GCase polypeptide, polynucleotide, or vector/viral particle of the invention and a pharmaceutically acceptable excipient.

The pharmaceutically acceptable excipient(s) may comprise carriers, diluents and/or other medicinal agents, pharmaceutical agents or adjuvants, etc. Optionally, the pharmaceutically acceptable excipient(s) comprise saline solution. Optionally, the pharmaceutically acceptable excipient(s) comprises human serum albumin.

The invention further provides the modified GCase polypeptide, polynucleotide, vector/viral particle or composition of the invention for use in a method of treatment. Optionally the method of treatment comprises administering an effective amount of the modified GCase polypeptide, polynucleotide, vector/viral particle, or composition of the invention to a patient.

A method of treatment comprising administering an effective amount of the modified GCase polypeptide, polynucleotide, vector/viral particle, or composition described herein to a patient is also described.

Use of the modified GCase polypeptide, polynucleotide, vector/viral particle or composition described herein in the manufacture of a medicament for use in a method of treatment is also described. Optionally the method of treatment comprises administering an effective amount of the modified GCase polypeptide, polynucleotide, vector/viral particle, or composition described herein to a patient.

Optionally, the method of treatment is a gene therapy. A *"gene therapy"* involves administering a vector/viral particle of the invention that is capable of expressing a transgene (such as a GBA nucleotide sequence) in the host (e.g. patient) to which it is administered.

Optionally, the method of treatment is a GCase enzyme replacement therapy. A *"GCase enzyme replacement therapy"* may refer to any therapy which comprises the administration of a GCase polypeptide to a subject. The modified GCase polypeptide of the invention may be administered. The modified GCase polypeptide may be administered at any suitable dose, optionally at a dose of between 40 and 100, between 50 and 80, between 60 and 70, or around 60 U/kg BW. The GCase polypeptide may be administered through any appropriate route, optionally administered through intravenous injection or subcutaneous injection.

Optionally, the method of treatment is a method of treating a disease associated with GCase deficiency. As discussed above, GCase deficiency may lead to accumulation of glucocerebrosides in macrophages that infiltrate many vital organs which can cause a variety of diseases including synucleinopathies (as discussed in WO08/144591) or Parkinson's disease. Optionally, the method of treatment is a method of treating Parkinson's disease or a synucleinopathy. Optionally, the method of treatment is a method of treating Parkinson's disease. Optionally, the method of treatment is a method of treating Lewy body dementia, peripheral neuropathy, multi-system atrophy or pure autonomic failure.

Optionally, the method of treatment is a method of treating a lysosomal storage disorder such as Gaucher disease (GD), for example GD type I, type II or type III. Preferably, the lysosomal storage disorder is characterised by bruising, fatigue, anemia, low blood platelet count and enlargement of the liver and spleen. Optionally, the method of treatment is a method of treating GD. Optionally, the method of treatment is a method of treating GD type I. Optionally, the method of treatment is a method of treating GD type II. Optionally, the method of treatment is a method of treating GD type III. In some embodiments, the patient is a patient suffering from GD. In some embodiments, the patient is a patient suffering from GD type I. In some embodiments, the patient is a patient suffering from GD type II. In some embodiments, the patient is a patient suffering from GD type III. Optionally the patient has antibodies or inhibitors to a recombinant GCase (for example imiglucerase, velaglucerase alfa or taliglucerase alfa) with which the patient has previously been treated as part of an enzyme replacement therapy.

Optionally, the method of treatment is a method of treating Niemann-Pick disease, for example Niemann-Pick type C (NPC).

Optionally, the modified GCase polypeptide, polynucleotide, vector/viral particle and/or composition is administered intravenously. Optionally, the modified GCase polypeptide, polynucleotide, vector/viral particle and/or composition is for administration only once *(i.e.* a single dose) to a patient.

Optionally, the modified GCase polypeptide, polynucleotide, vector/viral particle and/or composition is administered directly to the CNS of the subject, for example by direct injection into the brain and/or spinal cord of the subject. Optionally, the direct injection is intracerebral injection, intraventricular injection, intracisternal injection, intraparenchymal injection, intrathecal injection, or any combination thereof. Optionally, convection enhanced delivery (CED) is used for direct injection to the CNS of a subject. CED is a therapeutic strategy that involves surgical exposure of the brain and placement of a small-diameter catheter directly into a target area of the brain, followed by infusion of a therapeutic agent (e.g. the modified GCase polypeptide, polynucleotide, vector/viral particle and/or composition as described herein) directly to the brain of the subject. CED is described, for example, in Debinski et al. (2009) Expert Rev Neurother. 9(10): 1519-27. Optionally, the modified GCase polypeptide, polynucleotide, vector/viral particle and/or composition is administered peripherally to a subject, for example by peripheral injection. Optionally, peripheral injection is subcutaneous injection, intravenous injection, intra-arterial injection (e.g. injection into the carotid artery of a subject), intraperitoneal injection, or any combination thereof. Optionally, the modified GCase polypeptide, polynucleotide, vector/viral particle and/or composition is administered both peripherally and directly to the CNS of a subject. For example, in some embodiments, a subject is administered a composition by intra-arterial injection (e.g., injection into the carotid artery) and by intraparenchymal injection (e.g., intraparenchymal injection by CED). Optionally, the direct injection to the CNS and the peripheral injection are simultaneous. Optionally, the direct injection occurs prior to (e.g. between 1 minute and 1 week, or more, before) the peripheral injection. Optionally, the direct injection occurs after (e.g. between 1 minute and 1 week, or more, after) the peripheral injection.

Optionally, the modified GCase polypeptide, polynucleotide, vector/viral particle and/or composition is administered by injection into the renal artery. Optionally, the modified GCase polypeptide, polynucleotide, vector/viral particle and/or composition is administered by retrograde administration, e.g. via the ureters using a urinary catheter.

When a disease or disorder (*e.g.* GD, such as GD type I) is *"treated"* as discussed herein (for example in the methods or uses described herein), this means that one or more symptoms of the disease or disorder (*e.g.* GD, such as GD type I) are ameliorated. It does not mean that the symptoms of the disease or disorder (*e.g.* GD, such as GD type I) are completely remedied so that they are no longer present in the patient, although in some methods, this may be the case. Thus, in all instances the term *"treatment"* or *"treating"* can be replaced with the term *"amelioration"* or *"ameliorating",* respectively. The methods or uses described herein (such as the methods of treatment or treating) may result in one or more of the symptoms of the disease or disorder (*e.g.* GD, such as GD type I) being less severe than before treatment. Optionally, relative to the situation pre-administration, the methods or uses described herein (such as the method of treatment or treating) results in an increase in the amount/concentration of circulating GCase in the blood of the patient, and/or an increase in the overall level of GCase activity detectable within a given volume of blood and/or the macrophages of the patient. In an embodiment, relative to the situation pre-administration, the methods or uses described herein (such as the methods of treatment or treating) result in one or more of: an increase in haemoglobin concentration; an increase in platelet count; a decrease in spleen size; a decrease in liver size.

In addition, the methods or uses described herein may *"prevent"* diseases such as Gaucher disease. Gaucher disease is generally associated with an accumulation of glucocerebrosidases in various tissues, and if the methods or uses described herein are carried out on young subjects (such as teenagers, young adults, children or babies) it should be possible to prevent Gaucher disease from establishing. Accordingly, in all instances the term *"treatment"* or *"treating"* may be replaced with the term *"prevention"* or *"preventing",* respectively.

An *"effective amount"* refers to an amount effective, at dosages and for periods of time necessary, to achieve the desired therapeutic result, such as raising the level of functional GCase in a subject (so as to lead to functional GCase production at a level sufficient to ameliorate the symptoms of the disease or disorder such as GD, for example GD type I).

Optionally, the vector/viral particle is administered at a dose of less than 1 x 10¹¹, less than 2 x 10¹¹, less than 1 x 10¹², less than 5 x 10¹², less than 2 x 10¹², less than 1.5 x 10¹², less than 3 x 10¹², less than 1 x 10¹³, less than 2 x 10¹³, or less than 3 x 10¹³ vector genomes per kg of weight of patient (vg/kg). Optionally, the dose of vector/viral particle that is administered is selected such that the subject expresses GCase at a level of 10%-90%, 20%-80%, 30%-70%, 25%-50%, 20%-150%, 30%-140%, 40%-130%, 50%-120%, 60%-110% or 70%-100% of the level of a healthy subject who does not suffer from GD.

The invention further provides the modified GCase polypeptide, polynucleotide, viral particle, or composition of the invention for use in treating a disease, wherein the modified GCase polypeptide or the encoded modified GCase polypeptide may have increased stability at pH 7.4 compared to a wild-type GCase polypeptide, such as the GCase polypeptide encoded by a GBA nucleotide sequence of SEQ ID NO: 40. Optionally, the modified GCase polypeptide or encoded modified GCase polypeptide retains activity which is at least 1.2 times, at least 1.5 times, at least 1.8 times, or at least 2 times higher than the activity retained by the GCase polypeptide encoded by the GBA nucleotide sequence of SEQ ID NO: 40 when measured after 120 minutes of incubation at pH 7.4 and 37 degrees Celsius. Optionally, the modified GCase polypeptide or encoded modified GCase polypeptide retains activity which is at least 3 times, at least 5 times, at least 7 times, at least 10 times, or at least 15 times higher than the activity retained by the GCase polypeptide encoded by a GBA nucleotide sequence of SEQ ID NO: 40 when measured after 120 minutes of incubation at pH 7.4 and 37 degrees Celsius.

A method of treating a disease by administering the modified GCase polypeptide, polynucleotide, viral particle or composition described herein is also described, wherein the modified GCase polypeptide or encoded modified GCase GCase polypeptide has increased stability at pH 7.4 compared to a reference wild-type GCase polypeptide, such as the GCase polypeptide encoded by a GBA nucleotide sequence of SEQ ID NO: 40. Optionally, the modified GCase polypeptide or encoded modified GCase polypeptide retains activity which is at least 1.2 times, at least 1.5 times, at least 1.8 times, or at least 2 times higher than the activity retained by the GCase polypeptide encoded by the GBA nucleotide sequence of SEQ ID NO: 40 when measured after 120 minutes of incubation at pH 7.4 and 37 degrees Celsius. Optionally, the modified GCase polypeptide or encoded modified GCase polypeptide retains activity which is at least 3 times, at least 5 times, at least 7 times, at least 10 times, or at least 15 times higher than the activity retained by the GCase polypeptide encoded by a GBA nucleotide sequence of SEQ ID NO: 40 when measured after 120 minutes of incubation at pH 7.4 and 37 degrees Celsius.

Use of the modified GCase polypeptide, polynucleotide, viral particle or composition described herein in treating a disease is also described, wherein the modified GCase polypeptide or encoded modified GCase polypeptide has increased stability at pH 7.4 compared to a wild-type GCase polypeptide, such as the GCase polypeptide encoded by the GBA nucleotide sequence of SEQ ID NO: 40. Optionally, the modified GCase polypeptide or encoded modified GCase polypeptide retains activity which is at least 1.2 times, at least 1.5 times, at least 1.8 times, or at least 2 times higher than the activity retained by the GCase polypeptide encoded by the GBA nucleotide sequence of SEQ ID NO: 40 when measured after 120 minutes of incubation at pH 7.4 and 37 degrees Celsius. Optionally, the modified GCase polypeptide or encoded polypeptide retains activity which is at least 3 times, at least 5 times, at least 7 times, at least 10 times, or at least 15 times higher than the activity retained by the GCase polypeptide encoded by a GBA nucleotide sequence of SEQ ID NO: 40 when measured after 120 minutes of incubation at pH 7.4 and 37 degrees Celsius.

The invention further provides the modified GCase polypeptide, polynucleotide, viral particle, or composition of the invention for use in treating a disease, which comprises administering a lower dose of the modified GCase polypeptide, polynucleotide, viral particle, or composition compared to the dose required to be administered of an equivalent polypeptide, polynucleotide, viral particle or composition comprising or encoding a GCase polypeptide encoded by a GBA nucleotide sequence of SEQ ID NO: 40.

A method of treating a disease by administering the GCase polypeptide, the polynucleotide, viral particle or composition described herein is also described, wherein treating the disease comprises administering a lower dose of the modified GCase polypeptide, polynucleotide, viral particle or composition described herein compared to the dose required to be administered of an equivalent polypeptide, polynucleotide, viral particle or composition comprising or encoding a GCase polypeptide encoded by a GBA nucleotide sequence of SEQ ID NO: 40.

Use of the modified GCase polypeptide, polynucleotide, viral particle or composition described herein in treating a disease is also described, wherein treating the disease comprises administering a lower dose of the modified GCase polypeptide, polynucleotide, viral particle or composition described herein compared to the dose required to be administered of an equivalent polypeptide, polynucleotide, viral particle or composition comprising or encoding a GCase polypeptide encoded by a GBA nucleotide sequence of SEQ ID NO: 40.

In relation to the phrase *"treating a disease ",* the *"disease "* may be any of one of the diseases or disorders discussed above. As such, treating a disease may be treating a lysosomal storage disorder in a subject or patient such as Gaucher disease (GD), for example GD type I, type II or type III. Preferably, the lysosomal storage disorder is characterised by bruising, fatigue, anemia, low blood platelet count and enlargement of the liver and spleen. Optionally, *"treating a disease"* may refer to treating GD, for example GD type I in a subject or patient. In some embodiments, the subject or patient is a subject or patient suffering from GD, for example GD type I.

In relation to the *"equivalent polypeptide, polynucleotide, viral particle or composition comprising or encoding a GCase polypeptide encoded by a GBA nucleotide sequence of SEQ ID NO: 40",* the *"equivalent"* polypeptide comprises (or is) the GCase polypeptide encoded by the GBA nucleotide sequence of SEQ ID NO: 40. The *"equivalent"* polynucleotide is the same as the polynucleotide of the invention being compared to except that the *"equivalent"* polynucleotide encodes the GCase polypeptide encoded by the GBA nucleotide sequence of SEQ ID NO: 40 rather than the modified GCase polypeptide of the invention. The *"equivalent"* viral particle is the same as the viral particle of the invention being compared to except that the *"equivalent"* viral particle encodes the GCase polypeptide encoded by the GBA nucleotide sequence of SEQ ID NO: 40 rather than the modified GCase polypeptide of the invention. The *"equivalent"* composition is the same as the composition of the invention being compared to except that the *"equivalent"* composition comprises or encodes the GCase polypeptide encoded by the GBA nucleotide sequence of SEQ ID NO: 40 rather than the modified GCase polypeptide of the invention.

A use of the modified GCase polypeptide, the polynucleotide, viral particle or composition described herein in the manufacture of a medicament for:
(i) reducing the level of hexosylsphingosine and/or hexosylceramide;
(ii) reducing the number of storage cells; and/or
(iii) reducing the level of activated macrophages;
in a subject suffering from a disease or condition associated with GCase deficiency, is described.

A method of
(i) reducing the level of hexosylsphingosine and/or hexosylceramide;
(ii) reducing the number of storage cells; and/or
(iii) reducing the level of activated macrophages;
in a subject suffering from a disease or condition associated with GCase deficiency by administering to the subject the modified GCase polypeptide, the polynucleotide, viral particle, or composition described herein is also described. Optionally,
(i) reducing the level of hexosylsphingosine and/or hexosylceramide;
(ii) reducing the number of storage cells; and/or
(iii) reducing the level of activated macrophages;
treats the disease or condition associated with GCase deficiency.

The modified GCase polypeptide, the polynucleotide, viral particle, or composition described herein, for use in a method of
(iv) reducing the level of hexosylsphingosine and/or hexosylceramide;
(v) reducing the number of storage cells; and/or
(vi) reducing the level of activated macrophages;
in a subject suffering from a disease or condition associated with GCase deficiency, is described, optionally wherein
(iv) reducing the level of hexosylsphingosine and/or hexosylceramide;
(v) reducing the number of storage cells; and/or
(vi) reducing the level of activated macrophages;
leads to the treatment of the disease or condition associated with GCase deficiency.

Optionally, the hexosylceramide and/or hexosylsphingosine levels are measured at least 6 weeks, at least 8 weeks, at least 10 weeks, at least 12 weeks or 12 weeks after administration. The hexosylceramide and/or hexosylsphingosine levels may be reduced by 2 times or more, 3 times or more, 4 times or more, 5 times or more, 6 times or more, 2 to 3 times, 2 to 4 times, 2 to 5 times, 2 to 6 times, or 3 to 5 times when compared to the (starting) hexosylceramide and/or hexosylsphingosine levels at the time of administration of the modified GCase polypeptide, polynucleotide, viral particle or composition described herein. For example, after administration of the modified GCase polypeptide, polynucleotide, viral particle, or composition described herein (for example at least 6 weeks, at least 8 weeks, at least 10 weeks, at least 12 weeks or 12 weeks after administration), the hexosylceramide and/or hexosylsphingosine levels in the patient may be 50% or less, 40% or less, 30% or less, 25% or less, 20% or less when compared to the (starting) hexosylceramide and/or hexosylsphingosine levels at the time of administration of the modified GCase polypeptide, polynucleotide, viral particle or composition described herein. Optionally, the patient may have increased hexosylceramide and/or hexosylsphingosine levels when compared to a healthy subject or a subject who does not have a disease or condition associated with GCase deficiency, *i.e.* before administration of the modified GCase polypeptide, polynucleotide, viral particle or composition described herein the patient has increased hexosylceramide and/or hexosylsphingosine levels when compared to a healthy subject or a subject who does not have a disease or condition associated with GCase deficiency. For example, the hexosylceramide and/or hexosylsphingosine levels are measured in the lung, spleen, liver and/or bone marrow of the patient/subject. Optionally, measuring the hexosylceramide and/or hexosylsphingosine levels in the lung, spleen, liver and/or bone marrow comprises estimating the level of hexosylceramine and/or hexosylsphingosine levels in the lung, spleen, liver and/or bone marrow based on data obtained in mouse studies. For example, one could test whether a GCase polypeptide reduces hexosylsphingosine levels in the spleen of a patient, by testing whether it can reduce hexosylsphingosine levels in the spleen of a mouse. The hexosylceramide and/or hexosylsphingosine levels may be measured in the serum and/or white blood cells (e.g. macrophages) of the patient/subject. The hexosylceramide and/or hexosylsphingosine levels may be measured in the plasma of the patient/subject. Methods of measuring hexosylceramide and/or hexosylsphingosine levels are known in the art, and the levels of hexosylceramide and/or hexosylsphingosine are preferably measured using mass spectrometry (LC/MS analysis), for example by the method described in example 13.

Optionally, the reduction of hexosylceramide and/or hexosylsphingosine levels (for example in the lung, serum, white blood cells (e.g. macrophages), spleen, liver and/or bone marrow of the patient/subject; a further example being in the plasma of the patient/subject) are greater than the reduction achieved from GCase enzyme replacement therapy, preferably when the hexosylceramide and/or hexosylsphingosine levels are measured after at least 6 weeks, at least 8 weeks, at least 10 weeks or at least 12 weeks after the start of treatment. For example levels after at least 6 weeks (e.g. at 6 weeks), at least 8 weeks (e.g. at 8 weeks), at least 10 weeks (e.g. at 10 weeks) or at least 12 weeks (e.g. at 12 weeks) from administration of the modified GCase polypeptide, polynucleotide, viral particle or composition described herein may be compared to levels after at least 6 weeks (e.g. at 6 weeks), at least 8 weeks (e.g. at 8 weeks), at least 10 weeks (e.g. at 10 weeks) or at least 12 weeks (e.g. at 12 weeks), respectively, from the first administration of GCase enzyme replacement therapy. As a particular example, hexosylceramide and/or hexosylsphingosine levels may be measured after at least 12 weeks (e.g. at 12 weeks) after administration of the modified GCase polypeptide, polynucleotide, viral particle or composition described herein and compared to the levels measured at least 12 weeks (e.g. at 12 weeks) after first administration of GCase enzyme replacement therapy. Preferably, the levels of hexosylceramide and/or hexosylsphingosine are measured in the same assay at the same time point after administration. Optionally, the GCase enzyme replacement therapy may be administered every two weeks. Optionally, the hexosylsphingosine and/or hexosylceramide level is reduced to less than 50%, less than 40%, less than 30%, less than 25%, less than 10%, or between 5% and 25% of the level of hexosylsphingosine and/or hexosylceramide achieved in a subject undergoing GCase enzyme replacement therapy, preferably when the hexosylceramide and/or hexosylsphingosine levels are measured after at least 6 weeks, at least 8 weeks, at least 10 weeks or at least 12 weeks after the start of treatment. Optionally, the hexosylsphingosine and/or hexosylceramide level is reduced to less than 50%, less than 40%, less than 30%, less than 25%, less than 10%, or between 5% and 25% of the level of hexosylsphingosine and/or hexosylceramide achieved in a subject who has been administered an effective dose of the GCase enzyme replacement therapy, preferably when the hexosylceramide and/or hexosylsphingosine levels are measured after at least 6 weeks, at least 8 weeks, at least 10 weeks or at least 12 weeks after the start of treatment. When measuring the level of hexosylsphingosine and/or hexosylceramide achieved in a subject after administration of the effective dose of the GCase enzyme replacement therapy, the level of hexosylsphingosine and/or hexosylceramide is preferably measured 2 hours after administration of a dose (e.g. the previous dose) of the GCase enzyme replacement therapy. By way of example, when the GCase enzyme replacement therapy is administered every two weeks to a subject, measuring the level of hexosylsphingosine and/or hexosylceramide in e.g. the serum at 12 weeks would involve obtaining a serum sample from the subject two hours following the administration of the 12^{th} week (i.e. 7^{th}) dose.

Optionally, the reduction in the hexosylsphingosine and/or hexosylceramide level (for example in the lung, plasma, serum, white blood cells (e.g. macrophages), spleen, liver and/or bone marrow of the patient/subject) following administration of the viral particle described herein is greater than the reduction achieved in a subject administered an otherwise identical viral particle comprising a GBA nucleotide sequence encoding a reference GCase polypeptide.

Optionally the modified GCase polypeptide described herein or the polynucleotide described herein provides a greater reduction in the hexosylsphingosine and/or hexosylceramide level (for example in the lung, plasma, serum, white blood cells (e.g. macrophages), spleen, liver and/or bone marrow of the patient/subject) compared to a reference GCase polypeptide, if it is provided as part of a viral particle encoding the modified GCase polypeptide described herein or a viral particle comprising the polynucleotide described herein, *i.e.* following administration of a viral particle encoding the modified GCase polypeptide described herein or a viral particle comprising the polynucleotide described herein, the reduction in the hexosylsphingosine and/or hexosylceramide level (for example in the lung, plasma, serum, white blood cells (e.g. macrophages), spleen, liver and/or bone marrow of the patient/subject) is greater than the reduction achieved in a subject administered an otherwise identical viral particle comprising a GBA nucleotide sequence encoding a reference GCase polypeptide.

In such embodiments, the *"otherwise identical"* viral particle comprising a GBA nucleotide sequence encoding a reference GCase polypeptide is a viral particle which is identical to (*i.e.* the viral particle comprises the same capsid and the recombinant genome comprises the same transcription regulatory elements *etc.*)
(i) the viral particle of the invention;
(ii) a viral particle encoding the modified GCase polypeptide of the invention; or
(iii) a viral particle comprising the polynucleotide of the invention;
except that the GBA nucleotide sequences are different. For example, the different GBA nucleotide sequences encoding the different GCase polypeptides are operably linked to the same promoter sequence.

Preferably, the hexosylceramide and/or hexosylsphingosine level is measured after at least 6 weeks, at least 8 weeks, at least 10 weeks, or at least 12 weeks from administration. For example, the level after at least 6 weeks (e.g. at 6 weeks), at least 8 weeks (e.g. at 8 weeks), at least 10 weeks (e.g. at 10 weeks), or at least 12 weeks (e.g. at 12 weeks) from administration of (i) the viral particle described herein, (ii) the viral particle encoding the modified GCase polypeptide described herein, or (iii) the viral particle comprising the polynucleotide described herein may be compared to the level after at least 6 weeks (e.g. at 6 weeks), at least 8 weeks (e.g. at 8 weeks), at least 10 weeks (e.g. at 10 weeks), or at least 12 weeks (e.g. at 12 weeks), respectively, from the administration of the otherwise identical viral particle comprising a GBA nucleotide sequence encoding a reference GCase polypeptide. As a particular example, the hexosylceramide and/or hexosylsphingosine level may be measured after at least 12 weeks (e.g. at 12 weeks) from administration of (i) the viral particle described herein, (ii) the viral particle encoding the modified GCase polypeptide described herein, or (iii) the viral particle comprising the polynucleotide described herein and compared to the level measured at least 12 weeks (e.g. at 12 weeks) after administration of the otherwise identical viral particle comprising a GBA nucleotide sequence encoding a reference GCase polypeptide. Preferably, the level of hexosylceramide and/or hexosylsphingosine is measured in the same assay at the same time point after administration. Optionally, the hexosylsphingosine and/or hexosylceramide level is reduced to less than 50%, less than 40%, less than 30%, less than 25%, less than 10%, or between 5% and 25% of the level of hexosylsphingosine and/or hexosylceramide achieved in the subject administered the otherwise identical viral particle comprising a GBA nucleotide sequence encoding a reference GCase polypeptide, preferably when the hexosylceramide and/or hexosylsphingosine level is measured after at least 6 weeks, at least 8 weeks, at least 10 weeks or at least 12 weeks from administration. In some embodiments, the reference GCase polypeptide is a wild-type GCase polypeptide. Optionally, the reference GCase polypeptide comprises the polypeptide of SEQ ID NO: 1 or 2. Optionally, the reference GCase polypeptide is the polypeptide of SEQ ID NO: 1 or 2. Optionally, the GBA nucleotide sequence encoding the reference GCase polypeptide comprises the nucleotide sequence of SEQ ID NO: 59 or 60.

Optionally, the level of hexosylceramide and/or hexosylsphingosine is measured in the plasma of the subject. Optionally, the level of hexosylceramide and/or hexosylsphingosine is measured in the bone marrow of the subject. Optionally, the level of hexosylceramide and/or hexosylsphingosine is measured in the spleen of the subject. Optionally, the level of hexosylceramide and/or hexosylsphingosine is measured in the liver of the subject. Optionally, the level of hexosylceramide and/or hexosylsphingosine is measured in the lung of the subject. Methods of measuring hexosylceramide and/or hexosylsphingosine levels are known in the art, and the levels of hexosylceramide and/or hexosylsphingosine are preferably measured using mass spectrometry (LC/MS analysis), for example by the method described in example 13.

Optionally, the reduction of hexosylsphingosine and/or hexosylceramide levels in the subject (or patient) after administration of the modified GCase polypeptide, polynucleotide, viral particle or composition described herein are such that the hexosylsphingosine and/or hexosylceramide levels (for example in the bone marrow, lung, serum, white blood cells (e.g. macrophages), liver and/or spleen; a further example being in the plasma of the patient/subject) are no more than 200%, 150%, or 125% of the hexosylsphingosine and/or hexosylceramide levels measured in a healthy subject or a subject not suffering from a disease or condition associated with GCase deficiency. In one example, a reduction in hexosylceramide and/or hexosylsphingosine levels may represent a reduction in glucosylceramide and/or glucosylsphingosine levels, respectively. For example, a reduction in hexosylceramide may represent a reduction in glucosylceramide. As a further example, a reduction in hexosylsphingosine levels may represent a reduction in glucosylsphingosine levels.

In one example, a reduction in hexosylceramide and/or hexosylsphingosine levels is a reduction in glucosylceramide and/or glucosylsphingosine respectively. In other words, a patient (for example, a patient suffering from a disease or condition associated with GCase deficiency) administered the modified GCase polypeptide, polynucleotide, viral particle or composition described herein may have reduced glucosylceramide and/or glucosylsphingosine levels after administration, preferably when the glucosylceramide and/or glucosylsphingosine levels are measured at least 6 weeks, at least 8 weeks, at least 10 weeks, at least 12 weeks, or 12 weeks after administration. The glucosylceramide and/or glucosylsphingosine levels may be reduced by 2 times or more, 3 times or more, 4 times or more, 5 times or more, 6 times or more, 2 to 3 times, 2 to 4 times, 2 to 5 times, 2 to 6 times, or 3 to 5 times when compared to the (starting) glucosylceramide and/or glucosylsphingosine levels at the time of administration of the modified GCase polypeptide, polynucleotide, viral particle or composition described herein. For example, after administration of the polynucleotide viral particle or composition described herein (for example at least 6 weeks, at least 8 weeks, at least 10 weeks, at least 12 weeks or 12 weeks after administration), the glucosylceramide and/or glucosylsphingosine levels in the patient may be 50% or less, 40% or less, 30% or less, 25% or less, 20% or less when compared to the (starting) glucosylceramide and/or glucosylsphingosine levels at the time of administration of modified GCase polypeptide, the polynucleotide, viral particle or composition described herein. Optionally, (prior to administration of the modified GCase polypeptide, polynucleotide, viral particle or composition described herein) the patient may have increased glucosylceramide and/or glucosylsphingosine levels when compared to a healthy subject or a subject who does not have a disease or condition associated with GCase deficiency. For example, the glucosylceramide and/or glucosylsphingosine levels are measured in the lung, spleen, liver and/or bone marrow of the patient/subject. The glucosylceramide and/or glucosylsphingosine levels may be measured in the serum and/or white blood cells (e.g. macrophages) of the patient/subject. The glucosylceramide and/or glucosylsphingosine levels may be measured in the plasma of the patient/subject. Methods of measuring glucosylceramide and/or glucosylsphingosine levels are known in the art, and the levels of glucosylceramide and/or glucosylsphingosine are preferably measured using mass spectrometry (LC/MS analysis), for example by the method described in example 13. Optionally, the reduction of glucosylceramide and/or glucosylsphingosine levels (for example in the serum, white blood cells (e.g. macrophages), spleen, liver and/or bone marrow of the patient/subject; a further example being in the plasma of the patient/subject) are greater than the reduction achieved from GCase enzyme replacement therapy, preferably when the glucosylceramide and/or glucosylsphingosine levels are measured after at least 6 weeks, at least 8 weeks, at least 10 weeks or at least 12 weeks after the start of treatment. For example levels after at least 6 weeks (e.g. at 6 weeks), at least 8 weeks (e.g. at 8 weeks), at least 10 weeks (e.g. at 10 weeks) or at least 12 weeks (e.g. at 12 weeks) from administration of the modified GCase polypeptide, polynucleotide, viral particle or composition described herein may be compared to levels after at least 6 weeks (e.g. at 6 weeks), at least 8 weeks (e.g. at 8 weeks), at least 10 weeks (e.g. at 10 weeks) or at least 12 weeks (e.g. at 12 weeks), respectively, from the first administration of GCase enzyme replacement therapy. As a particular example, glucosylceramide and/or glucosylsphingosine levels may be measured after at least 12 weeks (e.g. at 12 weeks) after administration of the modified GCase polypeptide, polynucleotide, viral particle or composition described herein and compared to the levels measured at least 12 weeks (e.g. at 12 weeks) after first administration of GCase enzyme replacement therapy. Preferably, the levels of glucosylceramide and/or glucosylsphingosine are measured in the same assay at the same time point after administration. Optionally, the GCase enzyme replacement therapy may be administered every two weeks. When measuring the level of glucosylsphingosine and/or glucosylceramide achieved in a subject after administration of the effective dose of the GCase enzyme replacement therapy, the level of glucosylsphingosine and/or glucosylceramide is preferably measured 2 hours after administration of a dose (e.g. the previous dose) of the GCase enzyme replacement therapy. By way of example, when the GCase enzyme replacement therapy is administered every two weeks to a subject, measuring the level of glucosylsphingosine and/or glucosylceramide in e.g. the serum at 12 weeks would involve obtaining a serum sample from the subject two hours following the administration of the 12^{th} week (i.e. 7^{th}) dose.

Optionally, the reduction of the glucosylceramide and/or glucosylsphingosine level (for example in the lung, plasma, serum, white blood cells (e.g. macrophages), spleen, liver and/or bone marrow of the patient/subject) following administration of the viral particle described herein is greater than the reduction achieved in a subject administered an otherwise identical viral particle comprising a GBA nucleotide sequence encoding a reference GCase polypeptide.

Optionally the modified GCase polypeptide described herein or the polynucleotide described herein provides a greater reduction in the glucosylceramide and/or glucosylsphingosine level (for example in the lung, plasma, serum, white blood cells (e.g. macrophages), spleen, liver and/or bone marrow of the patient/subject) compared to a reference GCase polypeptide, if it is provided as part of a viral particle encoding the modified GCase polypeptide described herein or a viral particle comprising the polynucleotide described herein, *i.e.* following administration of a viral particle encoding the modified GCase polypeptide described herein or a viral particle comprising the polynucleotide described herein, the reduction in the glucosylceramide and/or glucosylsphingosine level (for example in the lung, plasma, serum, white blood cells (e.g. macrophages), spleen, liver and/or bone marrow of the patient/subject) is greater than the reduction achieved in a subject administered an otherwise identical viral particle comprising a GBA nucleotide sequence encoding a reference GCase polypeptide.

In such embodiments, the *"otherwise identical"* viral particle comprising a GBA nucleotide sequence encoding a reference GCase polypeptide is a viral particle which is identical to (*i.e.* the viral particle comprises the same capsid and the recombinant genome comprises the same transcription regulatory elements *etc.*)
(i) the viral particle of the invention,
(ii) the viral particle encoding the modified GCase polypeptide of the invention, or
(iii) the viral particle comprising the polynucleotide of the invention,
except that the GBA nucleotide sequences are different. For example, the different GBA nucleotide sequences encoding the different GCase polypeptides are operably linked to the same promoter sequence.

Preferably, the glucosylceramide and/or glucosylsphingosine level is measured after at least 6 weeks, at least 8 weeks, at least 10 weeks or at least 12 weeks from administration. For example, the level after at least 6 weeks (e.g. at 6 weeks), at least 8 weeks (e.g. at 8 weeks), at least 10 weeks (e.g. at 10 weeks), or at least 12 weeks (e.g. at 12 weeks) from administration of (i) the viral particle described herein, (ii) the viral particle encoding the modified GCase polypeptide described herein, or (iii) the viral particle comprising the polynucleotide described herein may be compared to the level after at least 6 weeks (e.g. at 6 weeks), at least 8 weeks (e.g. at 8 weeks), at least 10 weeks (e.g. at 10 weeks), or at least 12 weeks (e.g. at 12 weeks), respectively, from the administration of the otherwise identical viral particle comprising a GBA nucleotide sequence encoding a reference GCase polypeptide. As a particular example, the glucosylceramide and/or glucosylsphingosine level may be measured after at least 12 weeks (e.g. at 12 weeks) from administration of (i) the viral particle described herein, (ii) the viral particle encoding the modified GCase polypeptide described herein or (iii) the viral particle comprising the polynucleotide described herein and compared to the level measured at least 12 weeks (e.g. at 12 weeks) from administration of the otherwise identical viral particle comprising a GBA nucleotide sequence encoding a reference GCase polypeptide. Preferably, the level of glucosylceramide and/or glucosylsphingosine is measured in the same assay at the same time point after administration. Optionally, the glucosylceramide and/or glucosylsphingosine level is reduced to less than 50%, less than 40%, less than 30%, less than 25%, less than 10%, or between 5% and 25% of the level of glucosylceramide and/or glucosylsphingosine achieved in a subject administered an otherwise identical viral particle comprising a GBA nucleotide sequence encoding a reference GCase polypeptide, preferably when the glucosylceramide and/or glucosylsphingosine level is measured after at least 6 weeks, at least 8 weeks, at least 10 weeks or at least 12 weeks from administration. In some embodiments, the reference GCase polypeptide is a wild-type GCase polypeptide. Optionally, the reference GCase polypeptide comprises the polypeptide of SEQ ID NO: 1 or 2. Optionally, the reference GCase polypeptide is the polypeptide of SEQ ID NO: 1 or 2. Optionally, the GBA nucleotide sequence encoding the reference GCase polypeptide comprises the nucleotide sequence of SEQ ID NO: 59 or 60.

Optionally, the reduction of glucosylceramide levels in the subject (or patient) after administration of the modified GCase polypeptide, polynucleotide, viral particle or composition described herein are such that the glucosylceramide levels (for example in the serum, white blood cells (e.g. macrophages), liver and/or spleen) are no more than 200% , 150%, or 125% of the glucosylceramide levels measured in a healthy subject or a subject not suffering from a disease or condition associated with GCase deficiency.

Optionally a patient (for example, a patient suffering from a disease or condition associated with GCase deficiency) administered the modified GCase polypeptide, polynucleotide, viral particle or composition described herein may show a reduced number of storage cells and/or activated macrophages in the liver or lung (e.g. lung tissue) of the subject after administration, preferably when the cells are counted after at least 6 weeks (e.g. at 6 weeks), at least 8 weeks (e.g. at 8 weeks), at least 10 weeks (e.g. at 10 weeks) or at least 12 weeks (e.g. at 12 weeks) after administration. Reduction in the number of storage cells and/or activated macrophages in the liver or lung (e.g. lung tissue) may be an indication of reduced inflammation and thus therapeutic benefit. The number of activated macrophages may be indicated or estimated by measuring the number of CD68^{positive} cells. Identifying storage cells and/or CD68^{positive} cells can be performed by methods known in the art, such as by immunohistochemistry or microscopy, for example the methods described in example 13.

Optionally, measuring the number of activated macrophages and/or the number of storage cells in the liver and/or lung comprises estimating the number of activated macrophages and/or the number of storage cells in the liver and/or lung based on data obtained in mouse studies. For example, one could test whether a GCase polypeptide reduces the number of storage cells in the liver of a patient, by testing whether it can reduce the number of storage cells in the liver of a mouse.

Optionally, the reduction of storage cells and/or activated macrophages in the liver or lung (e.g. lung tissue) of the subject are greater than the reduction achieved from GCase enzyme replacement therapy, preferably when the number of storage cells and/or activated macrophages in the liver or lung (e.g. lung tissue) are measured after at least 6 weeks, at least 8 weeks, at least 10 weeks or at least 12 weeks after the start of treatment. For example levels after at least 6 weeks (e.g. at 6 weeks), at least 8 weeks (e.g. at 8 weeks), at least 10 weeks (e.g. at 10 weeks) or at least 12 weeks (e.g. at 12 weeks) from administration of the modified GCase polypeptide, polynucleotide, viral particle or composition described herein may be compared to levels after at least 6 weeks (e.g. at 6 weeks), at least 8 weeks (e.g. at 8 weeks), at least 10 weeks (e.g. at 10 weeks) or at least 12 weeks (e.g. at 12 weeks), respectively, from the first administration of GCase enzyme replacement therapy. As a particular example, levels (e.g. numbers) of storage cells and/or activated macrophages in the liver or lung (e.g. lung tissue) may be measured after at least 12 weeks (e.g. at 12 weeks) after administration of the modified GCase polypeptide, polynucleotide, viral particle or composition described herein and compared to the levels measured at least 12 weeks (e.g. at 12 weeks) after first administration of GCase enzyme replacement therapy. Preferably, levels (e.g. numbers) of storage cells and/or activated macrophages in the liver or lung (e.g. lung tissue) are measured in the same assay at the same time point after administration. Optionally, the GCase enzyme replacement therapy may be administered every two weeks. Optionally, the levels (e.g. numbers) of storage cells and/or activated macrophages in the liver or lung (e.g. lung tissue) are reduced to less than 60%, less than 50%, less than 40%, less than 30%, less than 25%, less than 10%, between 0% and 25%, or between 5% and 25% of the levels (e.g. numbers) of storage cells and/or activated macrophages in the liver or lung (e.g. lung tissue) achieved in a subject undergoing GCase enzyme replacement therapy, preferably when the levels (e.g. numbers) of storage cells and/or activated macrophages in the liver or lung (e.g. lung tissue) are measured after at least 6 weeks, at least 8 weeks, at least 10 weeks or at least 12 weeks after the start of treatment. Optionally, the levels (e.g. numbers) of storage cells and/or activated macrophages in the liver or lung (e.g. lung tissue) is reduced to less than 50%, less than 40%, less than 30%, less than 25%, less than 10%, between 0% and 25%, or between 5% and 25% of the levels (e.g. numbers) of storage cells and/or activated macrophages in the liver or lung (e.g. lung tissue) achieved in a subject who has been administered an effective dose of the GCase enzyme replacement therapy, preferably when the levels (e.g. numbers) of storage cells and/or activated macrophages in the liver or lung (e.g. lung tissue) are measured after at least 6 weeks, at least 8 weeks, at least 10 weeks, at least 12 weeks or 12 weeks after the start of treatment. When measuring the levels (e.g. numbers) of storage cells and/or activated macrophages in the liver or lung (e.g. lung tissue) achieved in a subject after administration of the effective dose of the GCase enzyme replacement therapy, the levels (e.g. numbers) of storage cells and/or activated macrophages in the liver or lung (e.g. lung tissue) is preferably measured 2 hours after administration of a dose (e.g. the previous dose) of the GCase enzyme replacement therapy. By way of example, when the GCase enzyme replacement therapy is administered every two weeks to a subject, measuring the levels (e.g. numbers) of storage cells and/or activated macrophages in the liver or lung (e.g. lung tissue) at 12 weeks would involve obtaining a tissue sample from the subject two hours following the administration of the 12^{th} week (i.e. 7^{th}) dose.

*A "GCase enzyme replacement therapy"* may refer to any therapy which comprises the administration of a GCase polypeptide to a subject. The GCase polypeptide may be wild type, such as a GCase polypeptide having the amino acid sequence of SEQ ID NO: 3, for example velaglucerase alfa. The GCase polypeptide may be administered at any suitable dose, optionally at a dose of between 40 and 100, between 50 and 80, between 60 and 70, or around 60 U/kg BW. The GCase polypeptide may be administered through any appropriate route, optionally administered through intravenous injection or subcutaneous injection.

The invention will now be described with reference to the following examples, which are merely illustrative and should not in any way be construed as limiting the scope of the present invention.

### EXAMPLES

### Example 1 - GENERAL MATERIALS AND METHODS

### Details of some of the reagents and materials used in the examples:

- Expi293F cells (Thermo Fisher, Cat. no. A14527)
- Expi293 serum-free medium (Thermo Fisher, Cat. no. A1435101)
- Nalgene^{®} Disposable Erlenmeyer Flasks with Vented Closure, PETG, Sterile, Plain Bottom (Thermo Scientific, Cat. no. 4115-0125)
- Trypan Blue stain (Thermo Fisher, Cat No. T10282)
- 50ml Centrifuge Tube CentriStar (Corning, Cat. No. 430829)
- Microplate Deep-well 2.0mL 96 well polypropylene (Fisher Scientific, Cat No. 10447181)
- PureLink^{®} Air Porous Tape (Invitrogen, Cat No. 10544453)
- Custom synthesized genes coding for GCase variants in cloning vectors pCDNA5/FRT (pCDNA5/FRT from Thermo Fisher, Cat. No. V601020; cloning vectors (pCDNA5/FRT) containing the custom synthesised genes *(e.g.* GCase variants or wild type GCase) were produced by GenScript)
- Nuclease-Free Water (not DEPC-Treated) (Invitrogen, Cat. No. AM9937)
- 96 V-bottom shaped well plate (Corning, REF 3894)
- Opti-MEM I reduced serum medium (Gibco, Cat. no. 31985062)
- Transfection reagents: ExpiFectamine^{™} 293 Transfection Kit (Thermo Fisher, Cat. No. A14524)
- Mouse serum (Sigma-Aldrich, Cat. No. M5905-10ML)
- Mouse plasma, EDTA (Rockland, Cat No. D508-04-0050)
- Human serum (Sera Laboratories International Ltd.)
- Human plasma (Sera Laboratories International Ltd.)
- 1x Phosphate-Buffered Saline (PBS) (Gibco, Cat No 14190-094)
- Adhesive Foil for Microplates, Pierceable (VWR, Cat. no. 391-1275)
- EasySeal plate sealer, transparent (Greiner bio-one, Cat No. 676001)
- 4-Methylumbelliferyl-β-D-glucopyranoside (Sigma-Aldrich, Cat. no M3633-500MG)
- Citric acid (Sigma-Aldrich, Cat. no. 251275-100G)
- Sodium taurodeoxycholate hydrate (Sigma-Aldrich, Cat. no. T0557-5g).
- Glycine (Glentham life Sciences, Cat. no. GM2385)
- Sodium citrate (Glentham life Sciences, Cat. no GK0137)
- Sodium hydroxide (Fisher Scientific, Cat. no. BP359-500)
- Velaglucerase alfa (VPRIV), (Shire Human Genetic Therapies)
- DMEM low glucose supplemented with 10% FBS and 1% GlutaMAX (D-10)
- DMEM low glucose supplemented with 1% GlutaMAX (D-0)
- GCase Assay Buffer (AB buffer): 50 mM Sodium Citrate, 25 mM Tauro Cholate, pH 5.75
- Lysis buffer: AB buffer containing 0.25%Triton X, and 1X Halt (protease inhibitor cocktail)
- Amicon Ultra centrifugal filter (Millipore) with a 10 kDa cut-off membrane (10 kD MWCO), (Sigma Aldrich)
- SYPRO^{™} Orange Protein Gel Stain (ThermoFisher, Cat. no. SS6651)
- Disposable PD-10 desalting columns (VWR, Cat. no. 17-0851-01)

### Details of some of the equipment used in the examples:

- Orbital shaker, New Brunswick^{™} S41i 125 rpm, for Expi293F cells. Plate shaker capable of 400 rpm (Eppendorf)
- Microplate rack (Eppendorf, Cat. no. M1289-0700)
- Laboratory centrifuge (Beckman Coulter, Avanti J-15R)
- Incubator in55 (Memmert, Germany)
- Countess^{™} II FL Automated Cell Counter (ThermoFisher, Cat. No. AMQAF 1000)
- The SpectraMax^{®} i3x Multi-Mode Microplate Reader (Molecular Devices)

### METHODS

Unless specified otherwise, the following general methods were followed in the examples described below.

### Expansion of Expi293F cells

Expi293F cells were used to express the GCase variants. The Expi293F cells were grown in Expi293 medium at 37°C, 125 rpm, 8% CO₂ atmosphere with 80% humidity in plastic flasks with ventilated caps. During the maintenance and expansion phase, the cells were split to 0.3 x10⁶ viable cells per ml when they reached a density of 3-5x10⁶ cells/ml. When passaging, cells were counted, and their viability was determined using a hemocytometer and trypan blue exclusion. Cells were spun down at 200 xg for 10 min at room temperature in 50 ml centrifuge tubes and media was removed by aspiration before resuspending the cells in prewarmed fresh Expi293 medium. The cells required splitting every 4th day. The volumes used for splitting were 30 ml Expi293 medium in 125 ml flasks.

### The day before transfection of Expi293F cells

The cells used for transfection displayed a viability of at least 95%. The day before transfection, cells were split and diluted to 2x10⁶ cells/ml in fresh prewarmed Expi293 medium in two 125 ml flasks. The cells were incubated under standard conditions until the day of transfection.

### Transfection of Expi293F cells

On the day of transfection, the cells were counted, and the viability was determined. Cell concentration was adjusted to density of 2.8 × 10⁶ cells/ml for each flask using prewarmed Expi293 medium. For transfection in 96 deep-well plates, 0.5ml of the cells in media were added per well. The plate was sealed with a porous tape and placed in a Microplate rack in the shaker incubator at 400 rpm.

In each well of a 96 deep-well plate, 80µl of Nuclease-Free Water was added to the lyophilized plasmid DNA (4µg/well of pCDNA5/FRT containing custom synthesised genes (*e.g.* a GCase variant or wild type GCase) produced by GenScript). In a well of a separate 96-well plate (V-bottom shaped), 14µl of each reconstituted plasmid DNA was diluted with 21µl Opti-MEM I serum-free medium, while 1.9µl of cationic lipid-based transfection reagent (ExpiFectamine 293 or similar) was diluted with 35µl Opti-MEM I in a well of another plate. DNA-lipid complexes were prepared by mixing the diluted DNA with the diluted transfection reagent. After 20 min of incubation at room temperature, 50µl/well of DNA-lipid complex were added to the Expi293F cells. The plate was sealed, and the cells were cultured at 37 °C, in a 5-8% CO₂ humidified atmosphere with shaking (400 rpm).

The next day, at 16-20 h post-transfection, a cocktail of Enhancer 1 and Enhancer 2 from the ExpiFectamine 293 transfection kit was added (27.5µL/well). The plate was sealed and returned to the humidified 5-8% CO₂ incubator at 37°C shaking at 400 rpm.

### Harvest of transfected Expi293 cells

Four days post-transfection the cells were harvested by centrifugation of the plate at 200 x *g* for 10 min and all the supernatants were collected and transferred to a new 96-Deep Well plate. The GCase (*e*.*g*. wild-type GCase or GCase variants) present in the supernatant could be analysed. For example, the supernatant could be used in the *"Stability assessment"* as set out below.

### Transduction of Huh-7 cells

Huh-7 cells were maintained in DMEM low glucose supplemented with 10% FBS and 1% GlutaMAX (D-10). Cells were counted by Countess^{™} II FL Automated Cell Counter. For transduction, cells were seeded at 2x10⁵ cells/well in a 12-well plate and allowed to adhere for 24 hours prior to transduction. Subsequently, to increase the transduction efficacy *in vitro,* cells were treated with Mitomycin C (10 µg/mL in D-10) for one hour at 37°C and washed with DMEM low glucose supplemented with 1% GlutaMAX (D-0). The transduction mix of D-10 containing AAV viral particles was prepared, added to the cells (500 µL/well) and incubated overnight (for approximately 16 hours) at 37°C, 5% CO₂. Transduction occurred at the required AAV multiplicity of infection (MOI): 5x10³, 1x10⁴ or 5x10⁴ vg/cell. Each transduction was performed in triplicate.

### GBA expression cassettes

GBA expression cassettes were generated which encode a GCase variant or wild-type GCase (see Table 1 below for the nucleotide sequences encoding the polypeptides). The promoter used was "LSP-L", which is a liver-specific transcription regulatory element (SEQ ID NO: 35).

### AAV production

GBA expression cassettes were pseudotyped with either AAV2/8 for *in vivo* experiments, or AAV2/'37' (which uses the capsid of SEQ ID NO: 37 and the ITRs of AAV2) *for in vitro* experiments. AAV viral particles (AAV vectors) were produced by triple plasmid transient transfection of HEK293T cells with plasmids encoding the AAV Rep and Cap functions; adenoviral helper functions; and the recombinant genome containing the GBA expression cassette flanked by AAV2 ITRs. AAV vectors were purified with AVB Sepharose (for AAV2/'37') or POROS^{™} CaptureSelect^{™} (for AAV2/8) affinity chromatography resins. Purified stocks were titred by a qPCR and characterised by alkaline agarose gel electrophoresis.

### Stability assessment

In a 96 deep-well plate, purified proteins (such as GCase variants), purified VPRIV purchased from Shire Human Genetic Therapies (commercial ERT) or supernatants (such as the supernatants obtained from the method above entitled *"Harvest of transfected Expi293F cells"* that contain the GCase variants or wild-type GCase), were mixed with prewarmed AB buffer, PBS, serum or plasma. The samples were diluted such that the initial activity at time point 0 was in the upper range of the dynamic window (less than 1x10⁹ RFU) of the signal (*i.e.* avoiding saturation of the signal, or a signal that is too close to the background) so as to consistently measure a change in activity across different samples. The sealed plate was incubated at 37°C without shaking. Aliquots of 20µL were taken for the GCase activity assay after various lengths of time, such as 0 min, 30 mins, 60 mins, 120 mins, 3 days, 4 days, 5 days, 6 days, and 7 days. Residual activity was calculated for each time point as the percentage of the initial activity. The residual activity (residual enzyme activity) was used to assess stability.

### GCase activity determination

The levels of active GCase were determined fluorometrically with 4-Methylumbelliferyl-β-D-glucopyranoside (4-MUG) as the substrate. Briefly, aliquots (20µL) (such as the 20µL aliquots taken during the above *"Stability assessment",* 20µL of culture media taken from the well following cell transduction and overnight incubation as set out in the section above entitled *"Transduction of Huh-7 cells";* or 20µL of the diluted plasma or lysed WBCs as described in the section below entitled *"Plasma and Tissue Collection"*) were assayed in 100µl of 50mM sodium citrate, 25mM taurocholate, pH 5.75 (assay buffer), 6mM 4-MUG, for 30 mins at 37°C. GCase activity of the tissue collected (as described in the section below entitled *"Plasma and Tissue Collection"*) was measured by lysing the tissue and assaying the tissue protein lysate in 50mM sodium citrate, 25mM taurocholate, pH 5.75, 6mM 4MUG, for 30 mins at 37°C. In all cases above, a solution of 0.3 M sodium hydroxide, 0.5 M glycine at pH 10 was used as the stop solution. Fluorometric measurement of samples and raw data output was performed using a SpectraMax^{®} i3 plate reader using excitation and emission wavelengths of 365 nm and 445 nm, respectively. For the *in vitro* and *in vivo* studies, fluorescence levels were converted to nanomoles/hour/mL based on a 4-Methylumbelliferone (4-MU, Sigma-Aldrich) standard curve. The fluorescence levels in plasma, tissue and WBCs were measured in mU/ml of plasma (for plasma) or mU/mg of protein (for tissue and WBCs), all using a VPRIV standard curve.

### Animals and Treatment Procedures (wild type mice)

Wild type (C57BL/6) male mice (aged 6-8 weeks, n=5) were used in this study. AAV vectors were administered at a dose in the range of from 2x10⁹ vg/kg to 2x10¹² vg/kg via a single tail vein injection. An additional group of animals was left untreated to serve as a control (treatment-naive) for the effects of treatment. Animals were culled either 4 or 6 weeks post treatment depending of the study. At the endpoint, murine blood and tissues were collected for molecular and histological analysis.

### Animals and Treatment Procedures (9V/null mice)

9V/null mice carrying the *Gba1* mutation D409V/D409V (9V/9V) were used. 9V/null mice have a nearly normal lifespan with visceral abnormalities (inflammation and storage cells) and substrate accumulation (Xu et al. Am J Pathol. 2003 Nov; 163(5):2093-101; Xu et al. PLoS One. 2010 May 20;5(5):e10750). 9V/null mice were generated by crossing mice carrying *Gba1* mutation D409V/D409V (9V/9V) and Gba1 null/WT. There are approximately two 9V/null produced in each litter. The strain background of 9V/null and WT mice are C57BL/6, 129SvEvBrd and FVB. 9V/null mice from multiple litters were randomly assigned into each treatment group on a rolling basis. Both male and female mice were enrolled in each group with an attempt to balance gender in the groups. All mice were housed under pathogen-free conditions and were monitored daily and weighed weekly. All AAV treated mice showed normal growth and weight gain.

Aliquots of AAV were stored at -80°C. Before injection, the aliquot was thawed on ice and diluted with X-VIVO 10 (Lonza, pH7.4, 4°C), and gently mixed by vortexing briefly at low speed. The diluted AAV was kept on ice before injection and used within 2 hours.

VPRIV ^{®} was resuspended and aliquoted (25, 50, 100 µl) and stored at -80°C. Before injection, the aliquot was thawed on ice and diluted with acidified X-VIVO 10 (Lonza, pH5.5, 4°C) to the indicated dose, and gently mixed by vortexing briefly at low speed. The diluted enzyme was kept on ice before injection and used within 2 hours.

AAV and vehicle (X-vivo) were given one time to 9V/null mice at 8 weeks of age with indicated doses at 5 µL/g body weight (BW). WT mice were administrated with vehicle at 8 weeks of age. AAV and vehicle administration were via tail vein to the mice while briefly under isoflurane. VPRIV ^{®} was administered by tail vein bolus injection to 9V/null mice anesthetized with mixture of isoflurane and oxygen in bio-bubble room at 60U/kg and 2.5 µL/g BW, starting at 8 weeks of age, biweekly, for 7 injections.

### Animals and treatment procedures (non-human primates)

Rhesus macaques (3 male and 3 female treated) were dosed with AAV2/'37' encoding GCase variant #85 at 2x10¹² vg/kg. The animals are followed for a post-dose observation period of at least 6 months (2 males and 2 females) or up to 60 months (1 male and 1 female).

AAV was thawed at room temperature and administered within 3 hours of removal from the freezer. The AAV was infused (IV, 30 minutes) into three male and three female Rhesus macaques from the Keeling Center for Comparative Medicine and Research (KCCMR) Rhesus Monkey Breeding and Research Resource (RMBRR) colony (MDAndersen, Texas, USA). Animals were prescreened for AAV neutralising antibodies and found negative. Blood samples were taken for analyses weekly for the first month, then monthly intervals and compared with baseline samples.

Measurements include mortality, local tolerance findings, clinical observations, body weights, food consumption, haematology and blood chemistry. The study also includes a full histopathological evaluation of the tissues at termination to establish the effect of chronic supraphysiological plasma exposure to GCase and immunogenicity (anti-GBA).

GCase activity in plasma was monitored weekly during the first month, every two weeks in the second month, and monthly thereafter for the first 6 months. Representative tissue samples are taken at termination to assess GCase uptake and biodistribution.

GCase activity was determined in a fluorometric assay using the fluorescent substrate 4-Methylumbelliferyl-β-D-glucopyranoside (4MUG). GCase assay buffer (AB buffer) composed of 1% Sodium Taurocholate (Sigma, Cat.#: T0557-56) in 50 mM Sodium Citrate (pH 5.75) was prepared fresh before determination of GCase activity in plasma. Plasma samples were diluted to 1:25 in AB buffer prior to the assay. Twenty-five µL of plasma was mixed with 25 µL 6mM 4-Methylumbelliferyl-β-D-glucopyranoside (4-MUG, Sigma Aldrich, Cat.#: M3633 500MG) and incubated for 30 minutes at 37°C. After incubation, 50 µL of stop solution (0.3M Sodium hydroxide, Fisher Scientific Cat# BP359-500; 0.5M Glycine, Glentham Life Science Cat# GM2385) at pH 10 were added per reaction. Enzyme activity was assessed fluorometrically (SpectraMax^{®} i3 plate reader [Molecular Devices]; λex= 365 nm and λem= 445 nm). The GCase-specific activity was expressed as nmol/h/ml based on a 7-point 4-methylumbelliferone (4-MU) standard curve.

### Plasma and Tissue Collection

For wild-type mice, murine plasma was obtained by separation from total blood kept on ice for 10 minutes in the presence of K2-EDTA to avoid clotting. Plasma was separated by centrifugation at 5 xg for 5 minutes in a refrigerated centrifuge. Following separation, plasma was aliquoted, and GCase activity was preserved by diluting plasma with 50mM sodium citrate, 25mM taurocholate, pH 5.75 buffer (AB buffer, dilution 1:25, *i.e.* 1 part of plasma to 24 parts of AB buffer). Tissues (liver, spleen, bone marrow, lung) were harvested and preserved in 10% buffered formalin (histology) or snap frozen (GCase activity).

White blood cells (WBCs) were obtained from total blood using 200 µl of K2-EDTA treated tubes. Upon 10 min incubation on ice, red blood cells were homolysed by adding 1800 µl of water. After mixing for 30 seconds, 10X PBS was added to prevent further cell lysis. WBCs were pelleted by centrifugation at 1000 xg for 5 minutes. Supernatant was discarded, and the cells were resuspended in 200 µl of AB buffer containing 0.25%Triton X, and 1X Halt (Lysis buffer), frozen in dry ice and stored at -80 °C.

### Vector Genome Copy Number

To determine the number of vector genomes in Huh-7 cells post *in vitro* transduction, cells were first washed with PBS-1X and treated with 40µl TrypLE (Thermo Fisher) for five minutes. Upon pelleting, PBS (110µl) was added to the cells, which were frozen and stored at -80°C. After three freeze/thaw cycles to lyse and release DNA, the triplicate wells were pooled and centrifuged (14000xg for five minutes) before an aliquot was taken (15µl) and diluted 1:33 with ddH₂O. AAV copy number estimation was performed using qPCR using primer sets that bind to the promoter, such as primer sets which bind to an LSP-L promoter.

To determine the number of vector genomes per liver cell post-AAV viral particle injection, DNA was isolated from frozen liver samples using QIAGEN DNeasy Blood and Tissue Kit (QIAGEN) following the manufacturer's protocol. Following DNA isolation, AAV copy number estimation was performed using qPCR using primer sets that bind to the promoter, such as primer sets which bind to an LSP-L promoter.

### Immunohistochemistry

Following collection, tissues were preserved in buffered formalin for subsequent paraffin embedding (FFPE). FFPE murine tissues were deparaffinized with xylene and ethanol washes. Antigen retrieval treatment was performed according to Ventana CC1 recommendations. Immunohistochemical staining was performed with a Ventana Discovery XT instrument, using a Ventana DAB Map Detection Kit (760-124). Rabbit anti-human acid β-glucocerebrosidase (Abcam, Ab125065, 1:100-400) was used to visualise GCase in murine tissue. GCase staining was visualised with 3,3'-Diaminobenzidine (DAB). Sections were counterstained with haematoxylin. Images were captured using automated light microscopy (Leica).

### GCase protein purification

The media of transfected Expi293F cells was clarified by centrifugation at 5000 rpm. Proteinase inhibitor (Roche complete tablet) was added to the clarified media at concentration per manufacture's recommendation. The clarified media was then mixed with n-butanol (1:4 v/v, butanol: medium) and underwent delipidation at 4 °C on a rotation-shaker for >1 hour. The mixture was then transferred to a separation funnel and was allowed to set for at least 30 min to separate the lipid layer from the clarified media. The GCase variants were purified with Octyl-Sepharose^{®} CL-4B column using the AKTA pure system. The clarified and delipidated media was loaded at flowrate < 1 ml/min and washed with two column volumes each of 25%, 50% and 75% ethylene glycol in 25 mM citric phosphate buffer at pH 5.75. GCase proteins was eluted with 100% ethylene glycol and buffer exchanged using PD-10 column into 25 mM citric phosphate buffer (pH 5.75) and concentrated using Amicon ultra centrifugal filter (10 kD MWCO membrane) to the desired concentration.

### Thermostability testing

The thermostability of GCase was investigated using a thermal shift assay based on the QuantStudio^{®} 3 and 5 Real-Time PCR Systems and SYPRO^{™} Orange Protein Gel Stain (an environmentally sensitive fluorescent dye that binds to hydrophobic protein regions, increasing fluorescence emission).

GCase in solution at known concentrations was mixed with 5x SYPRO^{™} Orange Protein Gel stain to allow for a fluorescent signal as the protein molecules unfold with increasing temperature. Melt curves were generated in QuantStudio^{®} 3 and 5 Real Time-PCR Systems with 'ROX' selected as the reporter. From a 2-minute hold at 25°C, the temperature was increased by 1°C per minute until 95°C. The melting temperature corresponds to the minimum value of the negative derivative of the melt curve (a plot of fluorescence in RFU against temperature).

### Example 2 - VPRIV Activity at pH 7.4

In order to determine the stability of VPRIV under pH 7.4 (a physiological pH), VPRIV was incubated in PBS (pH 7.4) over 120 minutes in accordance with the section entitled *"Stability assessment"* in Example 1. The enzymatic activity (GCase activity) was measured as described in the section entitled *"GCase activity determination"* in Example 1. The residual enzymatic activity (residual GCase activity) of VPRIV was calculated at each time point (0, 15, 30, 60 and 120 minutes). The residual enzymatic activity for each time point was calculated as a percentage of the initial activity (at time point 0).

The results show that there is little, if any, residual enzymatic activity of VPRIV which remains after incubation for 120 minutes in PBS at pH 7.4 (Figure 1).

### Example 3 - Stability of GCase variants

**Table 1**

| Variant number (or wild type "wt") | Mutation(s) | Following labels also used | Sequence encoding the variant or wt |
|---|---|---|---|
| wt | none | GCase_{wild type} | SEQ ID NO: 60 |
| 19 | GCase_{H262N} | | SEQ ID NO: 47 |
| 21 | GCase_{E272Q} | GCase_{variant #21} | SEQ ID NO: 10 |
| 34 | GCase_{H313N} | | SEQ ID NO: 48 |
| 47 | GCase_{H404K} | | SEQ ID NO: 49 |
| 57 | GCase_{H490K} | | SEQ ID NO: 50 |
| 63 | GCase_{R534N} | | SEQ ID NO: 51 |
| 68 | GCase_{H184L} | GCase_{variant #68} | SEQ ID NO: 52 |
| 69 | GCase_{H184L/K360N} | GCase_{variant #69} | SEQ ID NO: 53 |
| 74 | GCase_{D482C/S503C} | | SEQ ID NO: 54 |
| 79 | GCase_{S494C/R534C} | | SEQ ID NO: 55 |
| 85 | GCase_{W351C/A380C} | GCase_{variant #85} | SEQ ID NO: 18 |
| 89 | GCase_{I407C/D484C} | | SEQ ID NO: 56 |
| 21+85 | GCase_{E272Q/W351C/A380C} | GCase_{variant #21+85} | SEQ ID NO: 26 |

89 GCase variants were generated. Standard procedures can be used to generate GCase variants, such as site-directed mutagenesis. Codon-optimised nucleotide sequences encoding the GCase variants were gene-synthesised and cloned into the commercially available expression vector pcDNA5/FRT (Thermo Fisher) by GenScript.

The 89 GCase variants were tested for residual enzymatic activity. In short, the GCase variants were expressed according to the methods described in the sections entitled *"Expansion of Expi293F cells", "The day before transfection of Expi293F cells", "Transfection of Expi293F cells"* and *"Harvest of transfected Expi293F cells"* in Example 1. The enzymatic activity (GCase activity) was measured according to the methods described in the sections entitled *"Stability assessment"* and *"GCase activity determination"* in Example 1.

In relation to the *"Stability assessment",* the 89 GCase variants and wild type GCase were tested for residual enzymatic activity (residual GCase activity) after 2 hours incubation in PBS buffer at pH 7.4, 37°C. The residual enzymatic activity was calculated as a percentage of activity that remained after 2 hours relative to the initial activity.

12 GCase variants (19, 21, 34, 47, 57, 63, 68, 69, 74, 79, 85 and 89) shown in Table 1 had higher residual activity (at least 10% higher) compared to wild type (data not shown). In addition, GCase variant H262Y (data not shown) had a similar residual activity as variant 19.

Out of the 12 GCase variants, two variants in particular, #21 and #85, showed the highest residual activity and were therefore selected for further characterisation. A GCase variant with all the mutations of variants #21 and #85 was also tested (variant #21+85).

### Example 4 - Rescue of GCase activity at physiological pH achieved by GCase variants with enhanced protein stability

Various GCase variants, wild type GCase and VPRIV were tested for residual enzymatic activity under various conditions.

In short, the GCase variants and wild type GCase were expressed according to the methods described in the sections entitled *"Expansion of Expi293F cells", "The day before transfection of Expi293F cells", "Transfection of Expi293F cells"* and *"Harvest of transfected Expi293F cells"* in Example 1. The enzymatic activity (GCase activity) was measured according to the methods described in the sections entitled *"Stability assessment"* and *"GCase activity determination"* in Example 1. The residual enzymatic activity (residual GCase activity) of the GCase variants, wild type GCase and VPRIV was calculated at various times points (as set out below). The residual enzymatic activity was calculated for each time point as the percentage of the initial activity.

In one experiment, the activities of GCase variants #21 and #85 (the two GCase variants with the highest residual activity from Example 2), variant #21+85, wild type GCase, two references (variants #68 and #69) and VPRIV under different pH conditions were compared. In relation to the *"Stability assessment",* the GCase variants, wild type GCase, reference GCases or VPRIV were incubated for up to 7 days in AB buffer (pH 5.6) or PBS buffer (pH 7.4). The residual enzymatic activity (residual GCase activity) was measured after 0 min, 10 mins, 30 mins, 60 mins, 120 mins, 3 days, 4 days, 5 days, 6 day, and 7 days of incubation. Variants #21, #85 and #21+85 were found to retain higher enzyme activity than wild type GCase, the reference GCases and VPRIV over the course of 7 days under both pH conditions (Figures 2A and B).

In a further experiment, the activities of variants #21, #85 and #21+85 and VRPIV were also compared after incubation in human serum or human plasma for up to 7 days. In relation to the *"Stability assessment"*, the residual enzymatic activity (residual GCase activity) was measured after 0 min, 10 mins, 30 mins, 60 mins, 120 mins, 3 days, 4 days, 5 days, 6 day, and 7 days of incubation in human serum or human plasma. Variants #21 and #85 were found to retain higher enzymatic activity than VPRIV over 2 hours in both human serum and human plasma, and variant #21+85 was found to retain higher enzyme activity than all of the other tested GCase variants and VPRIV over the course of 7 days in both human serum and human plasma (Figured 3A and B).

### Example 5 - Increased half-life of GCase variant #85 in various physiological matrices compared to VPRIV

The half-lives of purified VPRIV (velaglucerase alfa) and purified GCase variant #85 variant were determined.

In short, the GCase variant was expressed according to the methods described in the sections entitled *"Expansion of Expi293F cells", "The day before transfection of Expi293F cells", "Transfection of Expi293F cells"* and *"Harvest of transfected Expi293F cells"* in Example 1. Purified VPRIV (velaglucerase alfa) was purchased from Shire Human Genetic Therapies. GCase variant #85 was purified as described in the section entitled *"GCase protein purification"* in Example 1. The enzymatic activity (GCase activity) was measured according to the methods described in the sections entitled *"Stability assessment"* and *"GCase activity determination"* in Example 1.

In relation to the *"Stability assessment",* the purified proteins (variant #85 and VPRIV) were incubated with AB buffer (pH 5.6), PBS buffer (pH 7.4), mouse serum, mouse plasma or human serum at 37°C for 0, 0.5, 1, 2, 3, 4, 5, 6, 7, 8, 24, 48, 120, and 144 hours (not all time points shown). The residual enzymatic activity (residual GCase activity) of VPRIV and the GCase variant #85 was calculated at each time point. The residual enzymatic activity at each time point was calculated as the percentage of the initial activity.

The half-life was calculated based on the one-phase decay model using GraphPad Prism. GCase variant #85 showed higher residual enzymatic activity compared to VPRIV for each of the matrices tested (Figures 4A, B and C). The half-life was longer for GCase variant #85 than VPRIV in lysosomal pH, physiological pH, mouse serum and human serum (Figure 4C).

### Example 6 - GCase variant #85 displays similar enzymatic efficiency (i.e. ability of the GCase variant to process a substrate) as reported for wild type GCase and ERTs when processing 4-Methylumbelliferyl-β-D-glucopyranosiduronic acid (4-MUG)

The enzymatic efficiency of the GCase variant #85 when processing 4-Methylumbelliferyl-β- D -glucopyranosiduronic acid (4-MUG) was determined. GCase variant #85 protein was purified as described in the section entitled *"GCase protein purification"* in Example 1. Either 1 or 3.5nM of purified protein (GCase variant #85) was incubated with increasing amounts (2.5 x 10⁶, 5 x 10⁶, 7.5 x 10⁶ or 1 x 10⁷ nM) of 4-MUG (a fluorogenic substrate of GCase) in AB buffer. 4-MU formation was monitored over 15 minutes by measuring the resulting fluorescence (excitation wavelength: 365nm, emission wavelength: 445nm). 4-methylumbelliferone (4-MU) formation velocity at each 4-MUG concentration was plotted against 4-MUG concentration and fitted with the Michaelis-Menten model (Figures 5A and B).

Figure 5C shows Km and Kcat of the GCase variant #85 in addition to the Km (mM) values reported by others for wild type GCase and the enzyme replacement therapies (ERTs) Imiglucerase and Velaglucerase alpha.

GCase variant #85 has a similar Km of around 2.0mM as reported by others for wild-type GCase and ERTs which have been used in the treatment of Gaucher disease.

### Example 7 - In silico immunogenicity assessment of GCase variant #85 suggests a lower immunogenicity risk profile than wild type GCase

The potential immunogenicity risk profiles of wild type GCase and GCase variant #85 were assessed and compared using *in silico* calculations and analysis. The assessment was based on the calculation of predicted binding affinities of peptides of certain lengths to MHC receptor molecules. Peptide-MHC binding is a first key obligatory step in immune response. Peptide fragments of nine (9-mers) and fifteen (15-mers) amino acids in length (for calculating binding affinities to HLA-I and HLA-II receptor molecules respectively) from the region spanning 50 amino acids upstream and 50 amino acids downstream of the amino acid substitutions (*i.e.* from a region spanning 50 amino acids upstream of W351C to 50 amino acids downstream of A380C) in the GCase variant were used as inputs for calculations using the Immune Epitope Database and Analysis Resource database (https://www.iedb.org/). The prediction methods used were NetMHCpan EL 4.0 (for HLA-I) and NetMHCIIpan 3.2 (for HLA-II). The calculated IC50 values (given in nanomolar units, nM) were used as a measure of peptide binding affinity and IC50 cut-off values were used to categorise the peptides as "strong" or "intermediate" binders. The lower the IC50 value, the better the binding is predicted to be. For "strong" binders the IC50 cut-off range used was 0 to ≤50nM, and for "intermediate" binders >50 to ≤500nM. Based on the *in silico* analysis and comparison results, GCase variant #85 has an overall lower potential immunogenicity risk profile compared to wild type GCase as shown by its lower number of MHC class I (HLA-I) "strong" binders and lower number of MHC class II (HLA-II) "strong" binders (Figure 6).

### Example 8 - GCase variants show increased GCase effective activity compared to wild type GCase upon AAV2/'37' transduction of Huh-7 cells

*In vitro* studies were carried out on the GCase variants from the previous Examples, namely GCase variant #21, GCase variant #85, GCase variant #21+85, as well as wild type GCase. AAV2/'37' viral particles were produced according to the section entitled *"AAV production"* in Example 1. The AAV2/'37' viral particles expressed sequences encoding one of the GCase variants or wild type GCase. Huh-7 cells were transduced with the AAV2/'37' viral particles according to the section entitled *"Transduction of Huh-7 cells"* in Example 1. The MOI used for transduction were 5x10³, 1x10⁴ and 5x10⁴vg/cell, and an untreated control was included. The GCase effective activity was measured according to the section entitled *"GCase activity determination".* The GCase effective activity was measured in nmol/hour/ml based on a 4-MU standard curve. The results were normalised by vector genome copy number (as determined according to the section entitled *"Vector genome copy number"* in Example 1). The normalisation was across the MOI groups to account for transduction efficiency.

All GCase variants (#21, #85 and #21+85) showed increased effective GCase activity when compared to wild type GCase. In particular, GCase variant #85 (containing the disulphide bond) showed the highest level of GCase activity (Figure 7).

### Example 9 - Upon injection into wild type mice, GCase variants show increased levels of circulating GCase and uptake in key tissues affected by Gaucher disease

*In vivo* studies were carried out on the GCase variants from the previous Examples, namely GCase variant #21, GCase variant #85, GCase variant #21+85, as well as wild type GCase. Circulating GCase levels (Figure 8A) and GCase levels in key tissues affected by Gaucher disease (Figures 8B and 8C) were measured following injection into wild type (C57BL/6) male mice of AAV2/8 viral particles expressing sequences encoding one of the GCase variants (#21, #85 or #21+85) or wild type GCase. AAV2/8 viral particles were produced according to the section entitled *"AAV production"* in Example 1. The AAV2/8 viral particles expressed sequences encoding one of the GCase variants or wild type GCase. The mice and the way the mice were treated are described in the section entitled *"Animals and Treatment Procedures"* in Example 1. The AAV2/8 viral particles were injected into mice at a dose of 6x10¹⁰ vg/kg. The animals were culled 4 weeks post-treatment. The plasma and tissues were collected as set out in the section entitled *"Plasma and Tissue Collection"* in Example 1. The GCase effective activity was measured as described in the section entitled *"GCase activity determination".* The level of effective GCase activity was measured in mU/ml of plasma or mU/mg of protein (spleen and bone marrow) according to a VPRIV standard curve. The results were normalised by vector genome copy number (as determined according to the section entitled *"Vector genome copy number"* in Example 1).

The GCase variants (#21, #85 and #21+85) showed increased effective GCase activity levels when compared to wild type GCase. In particular, GCase variant #85 showed the highest effective GCase activity level for circulating GCase as well as the greatest level of uptake by key organs such as spleen and bone marrow. Variant #85 was selected for further characterisation.

### Example 10 - Greater uptake of GCase is achieved in spleen, lung and bone tissue upon treatment with GCase variant #85

GCase levels in liver, spleen, lung and bone marrow were examined following injection into wild type (C57BL/6) male mice of AAV2/8 viral particles as described in Example 9. As described in Example 9, tissues were collected from the mice. Liver, lung, spleen and bone marrow of the mice in Example 9 were collected and preserved as described in the section entitled *"Plasma and Tissue Collection"* in Example 1. Immunohistochemical staining was performed on the paraffin sections and visualised as described in the section entitled *"Immunohistochemistry"* in Example 1. Figure 9 shows that, in agreement with the activity data in Example 9, tissue uptake of GCase is increased in mice injected with AAV2/8 viral particles expressing sequences encoding GCase variant #85 compared to wild type GCase in Gaucher disease-affected organs such as lung, spleen and bone marrow.

### Example 11 - AAV2/8-GCase variant #85 shows dose dependent increase in circulating GCase in plasma and dose dependent increase in uptake by WBCs, spleen, lung and bone marrow tissue

Circulating GCase levels (Figure 10A), GCase levels in key tissues affected by Gaucher disease (Figures 10B-D) and GCase levels in white blood cells (Figure 10E) were measured following injection into wild type (C57BL/6) male mice of AAV2/8 viral particles expressing sequences encoding the GCase variant #85 as described in Example 9. The methodology is the same as Example 9, except that a dose range of AAV2/8 viral particles of 2x10⁹ vg/kg to 2x10¹² vg/kg was used and the plasma was obtained 14 days post-treatment (by retro-orbital bleeding), 28 days post-treatment (by retro-orbital bleeding) and 42 days post-treatment (following culling). For the tissues and WBCs, animals were culled at 6 weeks post-treatment. The GCase levels were measured as described in the section entitled *"GCase activity determination"* in Example 1. Figure 10A shows a dose dependent increase in active GCase that circulates in plasma over 42 days. A dose dependent uptake of GCase into key Gaucher disease organs such as spleen, bone marrow, and lung was also observed (Figures 10B-D). In addition, a dose dependent uptake of GCase into white blood cells was observed (Figure 10E). A plateau was not reached.

### Example 12 - GCase variant #85 displays greater thermostability than Velaglucerase alfa across a range of concentrations at pH 5. 75 and pH 7.0

The thermostability of GCase variant #85 was investigated and compared to that of Velaglucerase alfa (VPRIV) using a thermal shift assay as described in the section entitled *"Thermostability testing"* in Example 1.

GCase variant #85 protein was expressed according to the methods described in the sections entitled *"Expansion of Expi293F cells", "The day before transfection of Expi293F cells", "Transfection of Expi293F cells"* and *"Harvest of transfected Expi293F cells"* in Example 1. GCase variant #85 was subsequently purified as described in the section entitled *"GCase protein purification"* in Example 1. GCase variant #85 protein was stored in 25 mM sodium citrate buffer pH 5.75, and VPRIV was reconstituted in nuclease-free water. GCase variant #85 and VPRIV were investigated at pH 5.75 and pH 7, with exchange into the respective solutions established using disposable PD-10 desalting columns. 1.5, 3 and 6 µM of GCase variant #85 and VPRIV were mixed with 5x SYPRO^{™} Orange Protein Gel stain and melt curves were generated as described in Example 1.

Respective melting temperatures (i.e. the temperature at which 50% of the protein is unfolded) for GCase variant #85 and VPRIV with increasing protein concentration at pH 5.75 and pH 7 are shown in Figure 12A and Figure 12B, respectively.

### Example 13 - Upon injection into GBA-deficient mice, GCase variant #85 shows increased levels of circulating GCase and uptake in key tissues by comparison with enzyme replacement therapy (ERT) and by comparison with an AAV encoding a wild-type GCase

### Methods

9V/null mice carrying the Gba1 mutation D409V/D409V (9V/9V) were used as a Gaucher disease model in this study, as described in Example 1 above. The objective of this study was to evaluate the therapeutic potential of an AAV vector encoding GCase variant #85 protein in 9V/null mice, which are a well-characterized model of Gaucher disease type 1.

Eight-week-old male and female 9V/null mice received a single intravenous dose of AAV2/8 encoding GCase variant #85 (encoded by SEQ ID NO: 18) at doses of 0 ("untreated" vehicle control, X-vivo 10), 2x10¹⁰, 2x10¹¹ or 2x10¹² vg/kg. An additional group of 9V/null mice was treated with velaglucerase alfa (VPRIV^{®}, a GCase approved as an enzyme replacement therapy [ERT] for Gaucher disease), at the clinical dose of 60 U/kg once every 2-weeks by lateral tail vein injection. An additional group of 9V/null mice was treated with AAV encoding a wild-type GCase (encoded by SEQ ID NO: 60) at doses of 2x10¹⁰, 2x10¹¹ or 2x10¹² vg/kg. The n number for all groups was 9 to 16 per group. The sequence encoding the wild-type GCase was the same codon-optimised sequence used to encode GCase variant #85, except the sequence encoding the wild-type GCase contains different codons at the positions corresponding to the mutations in GCase variant #85 (GCase_{W351C/A-380C}).

AAV treated mice showed comparable body weight gains as those that received either vehicle or VPRIV^{®}; similarly, at necropsy, haematological parameters and weights of liver and spleen were comparable between all groups. Haematology blood sampling: Fresh blood (~100 µL) was collected into 0.5M EDTA (5 µL) tube from tail vein 1 to 3 days before tissue collection at the endpoint and read for Haematological parameters within 24 hours on Hemavet 950FS (Drew Scientific Inc. USA).

Mice tissue collection and processing: Mouse plasma and selected tissues were collected at endpoint corresponding to 12 weeks post-initiation of treatment. Briefly, mice were euthanized by pentobarbital (100 mg/kg). Blood (~700 µL) was collected via portal vein into 0.5 M EDTA (20 µL) tubes. A portion of blood (~400 µL) was processed to collect white blood cells (WBC) for GCase activity assay. The remaining blood was processed to plasma, aliquoted, and then stored at -80°C for subsequent protein and substrate analysis. A fraction of fresh plasma was processed for GCase activity assay within 2 hours after collection.

Prior to tissue harvesting, mice were transcardially perfused with saline. Liver, spleen, lung, brain and spinal cord were dissected out. Whole liver and spleen were weighed prior to further dissection for multiple assays. Liver, lung and spleen were divided into four parts. Three parts were frozen in individual tubes stored at -80° C prior to activity, protein and substrate analysis; one part was fixed in 10% Formalin for histology analysis. The spinal cord was dissected from the cervical region and fixed in 10% Formalin for histology analysis. In addition, bone marrow (BM) cells were collected from femurs and tibias of both legs and frozen in two tubes stored in -80° C freezer for activity and substrate assays.

Each plasma collection and GCase activity assay from the VPRIV ^{®} treatment group was performed within 2 hours after the scheduled enzyme injection.

White blood cells (WBC) isolation: WBCs were isolated from blood by lysis red blood cells (RBC). The blood (~400 µL) collected in 0.5M EDTA tube was suspended in 10X volume of PBS and centrifuged at 3,000 rpm for 20 minutes to separate the cells. The cells were then suspended in 10X volume of RBC lysis buffer (8.3 gm/L NH4Cl in 0.01 M Tris-HCL buffer, pH 7.5) and centrifuged at 3,000 rpm for 20 minutes. The lysis procedure was repeated three times until the pellets were a pale colour. The final WBCs were washed with PBS and collected by centrifuge. Collected WBCs were stored at -80° C prior to GCase activity assay.

GCase activity assay: WBC, BM and tissue samples were homogenized in 1% sodium taurocholate, and 1% Triton X-100 (Tc/Tx) using Precellys^{®} 2 mL Tissue Homogenizing Mixed Beads kit (Bertin Instruments, France) and operated through Precellys Evolution tissue homogenizer with Cryolys thermo control (4° C, Bertin Instruments, France), for two cycles (20 seconds each, 30 seconds interval) at 4° C. Cells (BM and WBC) were homogenized in 1% Tc/Tx with sonication at 4° C. Tissue and cell lysates (2 µL) were diluted (5 X) with reaction buffer in the assay mixture (0.025 M Citric-phosphate buffer, pH 5.6). Diluted lysate (10 µL) in triplicate per sample was loaded to reaction plate. For plasma samples, plasma was diluted in 0.025 M Citric-phosphate buffer, pH 5.6. GCase activity was normalized by plasma volume and presented as nmole/h/mL. GCase activity was determined fluorometrically (Molecular Devise M5 fluorospectrometer, excitation and emission wavelengths of 365 nm and 445 nm, respectively) for relative fluorescence units (RFU), following incubation for 1 hour (h) at 37°C with 4-methylumberlliferyl-β-D-glucopyranoside (4-MU-Glucose, 4 mM) (Biosynth AG, Switzerland) in the presence and absence of 2 mM Conduritol B epoxide (CBE, Millipore. CA, USA), an irreversible GCase inhibitor, to estimate non-acid-β-glucosidase that cleaves 4-MU-Glucose. The 4-MU-Glucose coefficient factor (CF) is 56,000 that was derived from the 4-MU standard curve generated by two measurements of triplicates per measurement. Net RFU/CF = nmole. GCase activity was calculated as nmole/h/ml or nmole/h/mg, for plasma or tissues, respectively.

GCase substrates, glucosylceramide and glucosylsphingosine, were analyzed in plasma, liver, spleen, lung and BM samples by LC/MS.

Frozen liver, spleen and lung were weighed (~50 mg) and homogenized in 3.6 mL of Methanol/Chloroform/H2O (2:1:0.6 v/v/v) for 15 seconds using Omni TipTM Homogenizing kit with 7 mm stainless steel generator probe (OMNI International). An aliquot (500 µL) of lysate was used for LC/MS analysis. The quantitated glucosylceramide and glucosylsphingosine were normalized to tissue weight.

Plasma (~100 µL ) was subjected to LC/MS analysis. Glucosylceramide and glucosylsphingosine levels were normalized by plasma volume used.

BM cells were suspended in 200 µL water, sonicated, and then vortexed to make cell lysate. 160 µL of lysate was used for LC/MS analysis. The remaining lysate was used for protein estimation. Glucosylceramide and glucosylsphingosine levels were normalized to the amount of protein.

LC/MS analysis of hexosylceramide and hexosylsphingosine was performed at Lipidomics Shared Resources, Medical University of South Carolina. The lowest limit of quantitation is 25 pmole/mL for hexosylceramide and hexosylsphingosine. Hexosylceramide is composed of glucosylceramide and galactosylceramide. Hexosylsphingosine includes glucosylsphingosine and galactosylsphignosine. Because galactosylceramide and galactosylsphignosine are undetectable in plasma, liver, spleen, lung and BM, quantitated hexosylceramide and hexosylsphingosine represent glucosylceramide and glucosylsphingosine, respectively.

Visceral pathology in 9V/null mice was determined by counting foamy macrophages as storage cells and quantitating CD68 intensity staining signals, an inflammatory marker in Gaucher disease, on macrophage.

Storage cell count: Tissue sections were stained with hematoxylin and eosin (H&E) by Autostainner (Leica Autostainner XL, CCHMC core facility). The stained tissues were scanned with Aperio AT2 (Leica, 20X). The tissue images were processed with Aperio eSlide ImageScope (V12.4.0.0543). Ten photos at 20X magnitude (500 µm X 800 µm image) from mouse liver and lung were randomly chosen for analysis. Storage cells were manually counted from each image. The average cell counts observed from the ten images analyzed was used and represented in the graphs. Definition of "storage cells" is based on the size of cells (macrophage), e.g. size of storage cells in the liver is >10 µm, in the lung is > 15 µm. Storage cell size was determined from the scale bar in the images processed by Aperio eSlide ImageScope software (V12.4.0.0543). Samples identity were blind to the experimenters.

CD68 staining and quantitation: Tissue sections were stained with rabbit anti-mouse CD68 antibody (1:25. Abcam Ab53444) using Discover Ultra automated IHC/ISH slide staining machine. The tissues were counter-stained with hematoxylin (blue colour) to visualize cell nuclei. Stained tissues were scanned with Aperio AT2 (Leica, 20X), and the images were acquired by Aperio eSlide ImageScope (V12.4.0.0543). The images of liver and lung at 20X magnitude (500 µm X 800 µm) were chosen for quantitative analysis of CD68 signal (brown colour) density. Immunohistochemistry signals from 5 images of liver or lung per mouse were analyzed using Image J (Fiji, v5.1). Sample identity was blind to the experimenter.

Signal density calibration: CD68 staining were performed in two separate experiments:
1) Vehicle-9V/null, WT vehicle and VPRIV;
2) Vehicle-9V/null and AAV-GCase #85.
The density variation (the conversion factor) between two experiments was determined from a set of reference samples (three males and three females of Vehicle-9V/null) from each experiment. The conversion factor was applied to calibrate the CD68 density in the samples from two experiments to the same background level. Average CD68 signals observed in the five images analyzed per mouse was used for data analysis.

### Results

The increase in GCase activity following AAV delivery was measured at 12 weeks post-injection and within 2 hours of last ERT injection, in line with previous data showing that this is within the period where the ERT is at its C-max in the tissues. Injection of the AAV encoding GCase variant #85 increased tissue GCase activity above wild type (WT) level in liver, spleen, lung, white blood cells (WBC) and bone marrow (Figure 13). Furthermore, a significant reduction in glucosylsphingosine, which accumulates with disease progression, was observed in plasma (Figure 14) and all tissues analysed post administration of the AAV encoding GCase variant #85 (Figure 15). A similar reduction was also observed for glucosylceramide.

The tissue GCase activity level following injection of the AAV encoding GCase variant #85 was also higher than the level observed after the injection of the AAV encoding wild-type GCase. See Figure 18, which contains the data from Figure 13 plus the GCase activity levels following injection of the AAV encoding wild-type GCase, the GCase activity levels following injection of the AAV encoding GCase variant #85 at a dose of 2x10¹¹, and data from four additional mice in the untreated group. In addition, the extent of reduction in glucosylsphingosine levels after administration of the AAV encoding GCase variant #85 was also greater than after administration of the AAV encoding wild-type GCase. See Figure 19A, which contains the data from Figure 14 plus the glucosylsphingosine levels following injection of the AAV encoding wild-type GCase, and data from four additional mice in the untreated group. Also, see Figure 19B to E, which contains the data from Figure 15A to D plus the glucosylsphingosine levels following injection of the AAV encoding wild-type GCase, and data from four additional mice in the untreated group.

Lung and bone marrow showed significantly improved substrate clearance upon single injection of AAV encoding GCase variant #85. Glucosylsphingosine, a Gaucher disease biomarker lysoGb1, was reduced to approximately WT levels in both these tissues and plasma with AAV injection. Also, tissue inflammation was significantly reduced in AAV-treated mice, as assessed by anti-CD68 antibody staining and storage cell count in liver and lung. See Figure 16A which shows a comparison in levels of CD68 density in the lung, and Figure 16B which shows a comparison in the number of storage cells in the lung.

At 12 weeks after administration of the AAV encoding GCase variant #85 or the AAV encoding wild-type GCase, a dose-dependent increase in circulating GCase levels in the plasma of the 9V/null mice was observed (Figure 20). In addition, a higher level of GCase activity was observed in the mice administered with AAV encoding GCase variant #85 compared to the mice administered with AAV encoding wild-type GCase (Figure 20). Up to 42-fold higher levels of GCase activity were observed following administration with AAV encoding GCase variant #85 compared to administration with AAV encoding wild-type GCase.

### Example 14 - Long-term investigational study into non-human primates following injection with AAV-GCase#85 shows increased GCase activity relative to pre-treatment levels in all AAV-treated animals at all timepoints

The study goal is to assess the systemic long-term toxic potential, and biodistribution in a 60-month observation period post single AAV IV (infusion) administration in male and female monkeys (Rhesus macaques).

The experiment is carried out as described in Example 1 above. Rhesus macaques (3 male and 3 female treated) were dosed with AAV2/'37' encoding GCase variant #85 as described in Example 1 above. The animals will be followed for a post-dose observation period of at least 6 months (2 males and 2 females) or up to 60 months (1 male and 1 female).

Measurements include mortality, local tolerance findings, clinical observations, body weights, food consumption, haematology and blood chemistry. The study will also include a full histopathological evaluation of the tissues at termination to establish the effect of chronic supraphysiological plasma exposure to GCase and immunogenicity (anti-GBA). GCase activity levels were determined weekly during the first month, every-other-week in the second month, and then monthly thereafter. Representative tissue samples will be taken at termination to assess GCase uptake and biodistribution.

The study is currently ongoing. There have been no deaths during the study or treatment-related effects on clinical condition, post-dose observations, bodyweight, food consumption, haematology, blood chemistry, immunophenotyping of peripheral leukocytes.

Preliminary plasma analysis of samples from Days 8, 15, 22, 29, 43, and 57 detected increased GCase activity relative to pre-treatment levels (i.e. day 0) in all AAV-treated animals at all timepoints (Figure 17A). The GCase levels increased rapidly by Day 8; some fluctuations were noted between Day 22 and 43 and levels plateaued from Days 43 onwards. The GCase levels achieved were consistent with those achieved in Gba-deficient mice and within the range associated with substrate clearance from tissues.

Fig 17B shows the GCase activity levels observed in the same AAV-treated animals at Days 8, 15, 22, 29, 43, 57, 85, 113, 141 and 170 post-AAV administration. Animal 17-020 was sacrificed at day 83 for tissue uptake studies.

## Claims

1. A modified β-Glucocerebrosidase (GCase) polypeptide which comprises at least one mutation, wherein the at least one mutation comprises:
(i) a mutation at a position corresponding to position 351 of SEQ ID NO: 1 that is a substitution with cysteine; and
(ii) a mutation at a position corresponding to position 380 of SEQ ID NO: 1 that is a substitution with cysteine,
wherein the modified GCase polypeptide comprises an amino acid sequence that is at least 90% identical to SEQ ID NO: 2, and
wherein the modified GCase polypeptide has increased stability and has higher effective activity relative to a reference GCase polypeptide, wherein the reference GCase polypeptide is the polypeptide of SEQ ID NO: 3, 4, or 5.

2. The modified GCase polypeptide of claim 1, wherein the effective activity of the modified GCase polypeptide is at least 1.2 fold, at least 1.5 fold, at least 2 fold, at least 2.5 fold, at least 3 fold, at least 3.5 fold, at least 4 fold, at least 4.5 fold, at least 5 fold, at least 5.5. fold, at least 6 fold, at least 6.5 fold, at least 7 fold, at least 7.5 fold, at least 8 fold, at least 10 fold, at least 15 fold, at least 20 fold, at least 35 fold, at least 40 fold, at least 45 fold, or at least 50 fold higher than the effective activity of the reference GCase polypeptide.

3. The modified GCase polypeptide of claim 1 or 2, wherein:
(i) the modified GCase polypeptide is more structurally stable at physiological pH relative to a reference GCase polypeptide, wherein the reference GCase is the polypeptide of SEQ ID NO: 3, 4, or 5, optionally wherein the pH is pH 7.4; or
(ii) the increased stability is increased stability at pH 7.4; or
(iii) the modified GCase polypeptide retains at least 30%, at least 35%, at least 40%, at least 45%, at least 50%, at least 55%, at least 60%, at least 65%, at least 70%, at least 75%, at least 80%, or at least 85% activity compared to the initial activity when measured after at least 10 minutes, at least 30 minutes, at least 60 minutes, at least 120 minutes, at least 1 day, at least 2 days, at least 3 days, at least 4 days, at least 5 days, at least 6 days, or at least 7 days of incubation at pH 7.4 and 37 degrees Celsius; or
(iv) the modified GCase polypeptide retains activity which is at least 1.1 fold, at least 1.2 fold, at least 1.3 fold, at least 1.4 fold, at least 1.5 fold, at least 1.8 fold, at least 2 fold, at least 2.5 fold, at least 2.8 fold, or at least 3 fold higher than the activity of a reference GCase polypeptide when measured after 120 minutes of incubation at pH 7.4 and 37 degrees Celsius, wherein the reference GCase polypeptide is the polypeptide of SEQ ID NO: 3, 4, or 5.

4. The modified GCase polypeptide of any one of the preceding claims, wherein:
(i) the modified GCase polypeptide retains at least 70%, at least 75%, at least 80%, or at least 85% activity compared to the initial activity when measured after 120 mins of incubation at pH 7.4 and 37 degrees Celsius; or
(ii) the modified GCase polypeptide retains at least 40%, at least 45%, at least 50%, at least 55%, or at least 60% activity compared to the initial activity when measured after 72 hours of incubation at pH 7.4 and 37 degrees Celsius, optionally wherein the incubation is in PBS; or
(iii) the modified GCase polypeptide retains at least 15%, or at least 20% activity compared to the initial activity when measured after 7 days of incubation at pH 7.4 and 37 degrees Celsius, optionally wherein the incubation is in serum or plasma; or
(iv) the modified GCase polypeptide retains at least 40% activity compared to the initial activity when measured after 7 days of incubation at pH 7.4 and 37 degrees Celsius, optionally wherein the incubation is in serum or plasma; or
(v) the modified GCase polypeptide retains at least 60% activity compared to the initial activity when measured after 7 days of incubation at pH 7.4 and 37 degrees Celsius; or
(vi) the modified GCase polypeptide retains at least 80% activity compared to the initial activity when measured after 7 days of incubation at pH 7.4 and 37 degrees Celsius; or
(vii) the activity, effective activity, and/or stability is determined using a fluorometric assay.

5. The modified GCase polypeptide of any one of the preceding claims having a longer half-life relative to a reference GCase polypeptide, wherein the reference GCase polypeptide is the polypeptide of SEQ ID NO: 3, 4, or 5, optionally wherein the modified GCase polypeptide has a half-life of at least 1.2 fold, at least 1.5 fold, at least 2 fold, at least 3 fold, at least 4 fold, at least 5 fold, or at least 10 fold longer than the half-life of the reference GCase polypeptide, further optionally wherein:
a. the longer half-life is longer half-life at pH 7.4 or pH 5.6; or
b. the modified GCase polypeptide has a half-life at pH 5.6 of at least 20 fold longer than the half-life of the reference GCase polypeptide; or
c. the longer half-life is longer half-life in serum or plasma; or
d. the half-life is determined using a fluorometric assay.

6. The modified GCase polypeptide of any one of the preceding claims, wherein:
a. the at least one mutation comprises a mutation at a position corresponding to position 272 of SEQ ID NO: 1, optionally wherein the mutation at a position corresponding to position 272 of SEQ ID NO: 1 is a substitution with glutamine, further optionally E272Q; or
b. the at least one mutation comprises a mutation at a position corresponding to position 262 of SEQ ID NO: 1, optionally wherein the mutation at a position corresponding to position 262 of SEQ ID NO: 1 is a substitution with asparagine or tyrosine; or
c. the at least one mutation comprises a mutation at a position corresponding to position 262 of SEQ ID NO: 1, wherein the mutation at a position corresponding to position 262 of SEQ ID NO: 1 is a substitution with asparagine, optionally H262N; or
d. the at least one mutation comprises a mutation at a position corresponding to position 313 of SEQ ID NO: 1, optionally wherein the mutation at a position corresponding to position 313 of SEQ ID NO: 1 is a substitution with asparagine, further optionally H313N; or
e. the at least one mutation comprises a mutation at a position corresponding to position 404 of SEQ ID NO: 1, optionally wherein the mutation at a position corresponding to position 404 of SEQ ID NO: 1 is a substitution with lysine, further optionally H404K; or
f. the at least one mutation comprises a mutation at a position corresponding to position 490 of SEQ ID NO: 1, optionally wherein the mutation at a position corresponding to position 490 of SEQ ID NO: 1 is a substitution with lysine, further optionally H490K; or
g. the at least one mutation comprises a mutation at a position corresponding to position 534 of SEQ ID NO: 1, optionally wherein the mutation at a position corresponding to position 534 of SEQ ID NO: 1 is a substitution with asparagine, further optionally R534N.

7. The modified GCase polypeptide of any one of the preceding claims, wherein:
a. the at least one mutation comprises:
(i) a mutation at a position corresponding to position 482 of SEQ ID NO: 1; and
(ii) a mutation at a position corresponding to position 503 of SEQ ID NO: 1,
optionally wherein
(i) the mutation at a position corresponding to position 482 of SEQ ID NO: 1 is a substitution with cysteine, optionally an aspartic acid to cysteine mutation; and
(ii) the mutation at a position corresponding to position 503 of SEQ ID NO: 1 is a substitution with cysteine, optionally a serine to cysteine mutation; or
b. the at least one mutation comprises:
(i) a mutation at a position corresponding to position 494 of SEQ ID NO: 1; and
(ii) a mutation at a position corresponding to position 534 of SEQ ID NO: 1,
optionally wherein
(i) the mutation at a position corresponding to position 494 of SEQ ID NO: 1 is a substitution with cysteine, optionally a serine to cysteine mutation; and
(ii) the mutation at a position corresponding to position 534 of SEQ ID NO: 1 is a substitution with cysteine, optionally an arginine to cysteine mutation; or
c.
(i) the mutation at a position corresponding to position 351 of SEQ ID NO: 1 is a tryptophan to cysteine mutation; and
(ii) the mutation at a position corresponding to position 380 of SEQ ID NO: 1 is an alanine to cysteine mutation; or
d. the at least one mutation comprises:
(i) a mutation at a position corresponding to position 407 of SEQ ID NO: 1; and
(ii) a mutation at a position corresponding to position 484 of SEQ ID NO: 1,
optionally wherein
(i) the mutation at a position corresponding to position 407 of SEQ ID NO: 1 is a substitution with cysteine, optionally an isoleucine to cysteine mutation; and
(ii) the mutation at a position corresponding to position 484 of SEQ ID NO: 1 is a substitution with cysteine, optionally an aspartic acid to cysteine mutation; or
e. the at least one mutation comprises:
(i) a substitution with glutamine at a position corresponding to position 272 of SEQ ID NO: 1, optionally E272Q; and
(ii) a tryptophan to cysteine mutation at a position corresponding to position 351 of SEQ ID NO: 1; and
(iii) an alanine to cysteine mutation at a position corresponding to position 380 of SEQ ID NO: 1.

8. The modified GCase polypeptide of any one of the preceding claims, wherein:
a. the at least one mutation comprises a substitution with glutamine at a position corresponding to position 272 of SEQ ID NO: 1, and wherein the modified GCase polypeptide has increased stability compared to a reference GCase polypeptide, retaining at least 85% activity when measured after 120 mins of incubation at pH 7.4 and 37 degrees Celsius, wherein the reference GCase polypeptide is the polypeptide of SEQ ID NO: 3; or
b. the at least one mutation comprises:
(i) a tryptophan to cysteine mutation at a position corresponding to position 351 of SEQ ID NO: 1; and
(ii) an alanine to cysteine mutation at a position corresponding to position 380 of SEQ ID NO: 1;
and wherein the modified GCase polypeptide has increased stability compared to a reference GCase polypeptide, retaining at least 85% activity when measured after 120 mins of incubation at pH 7.4 and 37 degrees Celsius, wherein the reference GCase polypeptide is the polypeptide of SEQ ID NO: 3; or
c. the at least one mutation comprises:
(i) a substitution with glutamine at a position corresponding to position 272 of SEQ ID NO: 1, optionally E272Q; and
(ii) a tryptophan to cysteine mutation at a position corresponding to position 351 of SEQ ID NO: 1; and
(iii) an alanine to cysteine mutation at a position corresponding to position 380 of SEQ ID NO: 1;
and wherein the modified GCase polypeptide has increased stability compared to a reference GCase polypeptide, retaining at least 85% activity when measured after 120 mins of incubation at pH 7.4 and 37 degrees Celsius, wherein the reference GCase polypeptide is the polypeptide of SEQ ID NO: 3; or
d. the modified GCase polypeptide has higher effective activity and/or increased stability compared to a reference GCase polypeptide, wherein the reference GCase polypeptide is selected from any one of SEQ ID NOs: 1 to 5.

9. The modified GCase polypeptide of any one of the preceding claims, wherein:
(i) the modified GCase polypeptide comprises an amino acid sequence at least 95%, at least 98%, or at least 99% identical to SEQ ID NO: 1 or SEQ ID NO: 2; or
(ii) the modified GCase polypeptide comprises an amino acid sequence at least 98% identical to a fragment of between 400 and 536 amino acids of SEQ ID NO: 1; or
(iii) the modified GCase polypeptide comprises an amino acid sequence at least 98% identical to a fragment of between 400 and 497 amino acids of SEQ ID NO: 2; or
(iv) the modified GCase polypeptide comprises an amino acid sequence at least 98% identical to SEQ ID NO: 1; or
(v) the modified GCase polypeptide comprises an amino acid sequence at least 98% identical to SEQ ID NO: 2; or
(vi) the modified GCase polypeptide comprises an amino acid sequence that is identical to SEQ ID NO: 1 or SEQ ID NO: 2, except that the modified GCase polypeptide comprises the at least one mutation defined in any one of the preceding claims; or
(vii) the modified GCase polypeptide comprises an amino acid sequence that is identical to SEQ ID NO: 1 or SEQ ID NO: 2, except that the modified GCase polypeptide comprises a mutation at a position corresponding to position 272 of SEQ ID NO: 1; or
(viii) the modified GCase polypeptide comprises an amino acid sequence that is identical to SEQ ID NO: 1 or SEQ ID NO: 2, except that the modified GCase polypeptide comprises a substitution with cysteine at a position corresponding to position 351 of SEQ ID NO: 1 and at a position corresponding to position 380 of SEQ ID NO: 1; or
(ix) the modified GCase polypeptide comprises an amino acid sequence that is identical to SEQ ID NO: 1 or SEQ ID NO: 2, except that the modified GCase polypeptide comprises a substitution with cysteine at a position corresponding to position 351 of SEQ ID NO: 1 and at a position corresponding to position 380 of SEQ ID NO: 1, and the modified GCase polypeptide comprises a mutation at a position corresponding to position 272 of SEQ ID NO: 1.

10. A polynucleotide comprising a modified glucocerebrosidase (GBA) nucleotide sequence, wherein the modified GBA nucleotide sequence encodes the modified GCase polypeptide of any one of the preceding claims.

11. The polynucleotide of claim 10, wherein:
(i) the modified GBA nucleotide sequence comprises a sequence that is at least 80%, at least 90%, at least 95%, at least 98%, at least 99%, at least 99.5%, at least 99.8%, or 100% identical to a fragment of at least 750, at least 850, at least 950, at least 1000, at least 1200, at least 1400, or at least 1494 nucleotides of any one of SEQ ID NOs: 14 to 29; or
(ii) the modified GBA nucleotide sequence comprises a sequence that is at least 80%, at least 90%, at least 95%, at least 98%, at least 99%, at least 99.5%, at least 99.8%, or 100% identical to a nucleotide sequence of SEQ ID NO: 14 or SEQ ID NO: 18; or
(iii) the modified GBA nucleotide sequence comprises a sequence that is at least 80%, at least 90%, at least 95%, at least 98%, at least 99%, at least 99.5%, at least 99.8%, or 100% identical to a nucleotide sequence of SEQ ID NO: 22 or SEQ ID NO: 26.

12. The polynucleotide of claim 10 or 11, wherein the modified GBA nucleotide sequence comprises a sequence that is at least 90%, at least 95%, at least 98%, at least 99%, at least 99.5%, at least 99.8%, or 100% identical to a nucleotide sequence of SEQ ID NO: 14 or SEQ ID NO: 18.

13. A viral particle comprising a recombinant genome comprising the polynucleotide of any one of claims 10 to 12.

14. The viral particle of claim 13:
(i) which is an AAV, adenoviral, or lentiviral viral particle, optionally wherein the viral particle is an AAV viral particle; or
(ii) wherein the viral particle comprises a liver-tropic or CNS-tropic capsid, optionally wherein the liver-tropic capsid comprises a sequence at least 98%, at least 99%, or at least 99.5% identical to a fragment of at least 600, at least 650, at least 700, between 600 and 736, between 650 and 736, or between 700 and 736 amino acids of SEQ ID NO: 36, 37 or 38; or
(iii) wherein the viral particle further comprises:
a) AAV2 ITRs;
b) a polyA sequence; and/or
c) an intron; or
(iv) wherein the recombinant genome is single-stranded; or
(v) wherein the effective activity of the modified GCase polypeptide is at least 50%, at least 60%, at least 70%, at least 80%, at least 90%, at least 95%, or at least 100% of the effective activity of the GCase polypeptide encoded by the sequence of SEQ ID NO: 14 or 18.

15. The viral particle of claim 13 or 14, wherein:
(i) the viral particle is an AAV viral particle, and the viral particle comprises a CNS-tropic capsid;
(ii) the polynucleotide further comprises a transcription regulatory element;
(iii) the modified GBA nucleotide sequence is codon-optimised and/or the modified GBA nucleotide sequence comprises a sequence that is at least 90%, at least 95%, at least 98%, at least 99%, at least 99.5%, at least 99.8%, or 100% identical to a nucleotide sequence of SEQ ID NO: 14 or SEQ ID NO: 18; and
(iv) the modified GCase polypeptide comprises an amino acid sequence that is identical to SEQ ID NO: 1 or SEQ ID NO: 2, except that the modified GCase polypeptide comprises a substitution with cysteine at a position corresponding to position 351 of SEQ ID NO: 1 and at a position corresponding to position 380 of SEQ ID NO: 1.

16. The viral particle of claim 13 or 14, wherein:
(i) the viral particle is an AAV viral particle, and the viral particle comprises a liver-tropic capsid, optionally wherein the liver-tropic capsid comprises a sequence 100% identical to SEQ ID NO: 37;
(ii) the polynucleotide further comprises a transcription regulatory element, wherein (a) the transcription regulatory element comprises a liver-specific promoter and/or (b) the transcription regulatory element comprises a sequence that is 100% identical to SEQ ID NO: 35; and
(iii) the modified GBA nucleotide sequence is codon-optimised and/or the modified GBA nucleotide sequence comprises a sequence that 100% identical to a nucleotide sequence of SEQ ID NO: 14 or SEQ ID NO: 18.

17. A composition comprising the modified GCase polypeptide, polynucleotide, or viral particle of any one of the preceding claims and a pharmaceutically acceptable excipient.

18. The modified GCase polypeptide, polynucleotide, viral particle or composition of any one of the preceding claims for use in a method of treatment, wherein:
(i) the method of treatment is a method of treating a synucleinopathy; or
(ii) the method of treatment is a method of treating Parkinson's disease; or
(iii) the method of treatment is a method of treating Gaucher disease, optionally wherein the Gaucher disease is Gaucher disease type I, II or III; or
(iv) the method of treatment is a method of treating Gaucher disease and wherein the patient has antibodies or inhibitors to a recombinant GCase with which the patient has previously been treated as part of an enzyme replacement therapy, optionally wherein the Gaucher disease is Gaucher disease type I, II or III.

## Patentansprüche

1. Modifiziertes β-Glukozerebrosidase (GCase) Polypeptid, das mindestens eine Mutation umfasst, wobei die mindestens eine Mutation umfasst:
(i) eine Mutation an einer Position, die der Position 351 von SEQ ID NO: 1 entspricht, die eine Substitution mit Cystein ist; und
(ii) eine Mutation an einer Position, die der Position 380 von SEQ ID NO: 1 entspricht, die eine Substitution mit Cystein ist,
wobei das modifizierte GCase-Polypeptid eine Aminosäuresequenz umfasst, die zu mindestens 90% mit SEQ ID NO: 2 identisch ist, und
wobei das modifizierte GCase-Polypeptid eine erhöhte Stabilität und eine höhere effektive Aktivität im Vergleich zu einem Referenz-GCase-Polypeptid aufweist, wobei das Referenz-GCase-Polypeptid das Polypeptid der SEQ ID NO: 3, 4 oder 5 ist.

2. Modifiziertes GCase-Polypeptid nach Anspruch 1, wobei die effektive Aktivität des modifizierten GCase-Polypeptids mindestens 1,2-fach, mindestens 1,5-fach, mindestens 2-fach, mindestens 2,5-fach, mindestens 3-fache, mindestens 3,5-fach, mindestens 4-fach, mindestens 4,5-fach, mindestens 5-fach, mindestens 5,5-fach, mindestens 6-fach, mindestens 6,5-fach, mindestens 7-fach, mindestens 7,5-fach, mindestens 8-fach, mindestens 10-fach, mindestens 15-fach, mindestens 20-fach, mindestens 35-fach, mindestens 40-fach, mindestens 45-fach, oder mindestens 50-fach höher ist als die effektive Aktivität des Referenz-GCase-Polypeptids.

3. Modifiziertes GCase-Polypeptid nach Anspruch 1 oder 2, wobei:
(i) das modifizierte GCase-Polypeptid bei physiologischem pH-Wert im Vergleich zu einem Referenz-GCase-Polypeptid strukturell stabiler ist, wobei die Referenz-GCase das Polypeptid der SEQ ID NO: 3, 4 oder 5 ist, optional wobei der pH-Wert pH 7,4 ist; oder
(ii) die erhöhte Stabilität eine erhöhte Stabilität bei pH 7,4 ist; oder
(iii) das modifizierte GCase-Polypeptid mindestens 30%, mindestens 35%, mindestens 40%, mindestens 45%, mindestens 50%, mindestens 55%, mindestens 60%, mindestens 65%, mindestens 70%, mindestens 75%, mindestens 80%, mindestens 85%, Aktivität bewahrt im Vergleich zur Ausgangsaktivität bei Messung mindestens 10 Minuten, mindestens 30 Minuten, mindestens 60 Minuten, mindestens 120 Minuten, mindestens 1 Tag, mindestens 2 Tage, mindestens 3 Tage, mindestens 4 Tage, mindestens 5 Tage, mindestens 6 Tage oder mindestens 7 Tage nach der Inkubation bei pH 7,4 und 37 Grad Celsius; oder
(iv) das modifizierte GCase-Polypeptid eine Aktivität beibehält, die mindestens mindestens 1,1-fach, mindestens 1,2-fach, mindestens 1,3-fach, mindestens 1,4-fach, mindestens 1,5-fach, mindestens 1,8-fach, mindestens 2-fach, mindestens 2,5-fach, mindestens 2,8-fach, oder mindestens 3-fach höher ist als die Aktivität eines Referenz-GCase-Polypeptids, gemessen nach 120 Minuten Inkubation bei pH 7,4 und 37 Grad Celsius, wobei das Referenz-GCase-Polypeptid das Polypeptid der SEQ ID NO: 3, 4 oder 5 ist.

4. Modifiziertes GCase-Polypeptid nach einem der vorhergehenden Ansprüche, wobei:
(i) das modifizierte GCase-Polypeptid mindestens 70 %, mindestens 75 %, mindestens 80 % oder mindestens 85 % Aktivität im Vergleich zur anfänglichen Aktivität beibehält, wenn es nach 120 Minuten Inkubation bei pH 7,4 und 37 Grad Celsius gemessen wird; oder
(ii) das modifizierte GCase-Polypeptid mindestens 40 %, mindestens 45 %, mindestens 50 %, mindestens 55 % oder mindestens 60 % Aktivität im Vergleich zur anfänglichen Aktivität beibehält, wenn es nach 72 Stunden Inkubation bei pH 7,4 und 37 Grad Celsius gemessen wird, optional wobei die Inkubation in PBS erfolgt; oder
(iii) das modifizierte GCase-Polypeptid mindestens 15 % oder mindestens 20 % Aktivität im Vergleich zur anfänglichen Aktivität beibehält, wenn es nach 7 Tagen Inkubation bei pH 7,4 und 37 Grad Celsius gemessen wird, optional wobei die Inkubation in Serum oder Plasma erfolgt; oder
(iv) das modifizierte GCase-Polypeptid mindestens 40 % Aktivität im Vergleich zur anfänglichen Aktivität beibehält, wenn es nach 7 Tagen Inkubation bei pH 7,4 und 37 Grad Celsius gemessen wird, optional wobei die Inkubation in Serum oder Plasma erfolgt; oder
(v) das modifizierte GCase-Polypeptid mindestens 60 % Aktivität im Vergleich zur anfänglichen Aktivität beibehält, wenn es nach 7 Tagen Inkubation bei pH 7,4 und 37 Grad Celsius gemessen wird, oder
(vi) das modifizierte GCase-Polypeptid mindestens 80 % Aktivität im Vergleich zur anfänglichen Aktivität beibehält, wenn es nach 7 Tagen Inkubation bei pH 7,4 und 37 Grad Celsius gemessen wird, oder
(vii) die Aktivität, die effektive Aktivität und/oder die Stabilität mit Hilfe eines fluorometrischen Assays bestimmt wird.

5. Modifiziertes GCase-Polypeptid nach einem der vorhergehenden Ansprüche mit einer längeren Halbwertszeit im Vergleich zu einem Referenz-GCase-Polypeptid, wobei das Referenz-GCase-Polypeptid das Polypeptid der SEQ ID NO: 3, 4 oder 5 ist, optional wobei das modifizierte GCase-Polypeptid eine mindestens 1,2-fache, mindestens 1,5-fache, mindestens 2-fache, mindestens 3-fache, mindestens 4-fache, mindestens 5-fache oder mindestens 10-fache Halbwertszeit gegenüber der Halbwertszeit des Referenz-GCase-Polypeptids aufweist, wobei ferner optional:
a. Die längere Halbwertszeit eine längere Halbwertszeit bei pH 7,4 oder pH 5,6 ist; oder
b. Das modifizierte GCase-Polypeptid eine Halbwertszeit bei pH 5,6 hat, die mindestens 20-mal länger ist als die Halbwertszeit des Referenz-GCase-Polypeptids; oder
c. Die längere Halbwertszeit eine längere Halbwertszeit im Serum oder Plasma hat; oder
d. Die Halbwertszeit mit einem fluorometrischen Assay bestimmt wird.

6. Modifiziertes GCase-Polypeptid nach einem der vorhergehenden Ansprüche, wobei:
a. Die mindestens eine Mutation eine Mutation an einer Position umfasst, die der Position 272 von SEQ ID NO: 1 entspricht, optional wobei die Mutation an einer Position, die der Position 272 von SEQ ID NO: 1 entspricht, eine Substitution mit Glutamin, ferner optional E272Q ist; oder
b. Die mindestens eine Mutation eine Mutation an einer Position umfasst, die der Position 262 von SEQ ID NO: 1 entspricht, optional wobei die Mutation an einer Position, die der Position 262 von SEQ ID NO: 1 entspricht, eine Substitution mit Asparagin oder Tyrosin ist; oder
c. Die mindestens eine Mutation eine Mutation an einer Position umfasst , die der Position 262 von SEQ ID NO: 1 entspricht, wobei die Mutation an einer Position, die der Position 262 von SEQ ID NO: 1 entspricht, eine Substitution mit Asparagin, optional H262N ist; oder
d. Die mindestens eine Mutation eine Mutation an einer Position umfasst, die der Position 313 von SEQ ID NO: 1 entspricht, optional wobei die Mutation an einer Position, die der Position 313 von SEQ ID NO: 1 entspricht, eine Substitution mit Asparagin, ferner optional H313N ist; oder
e. Die mindestens eine Mutation eine Mutation an einer Position umfasst, die der Position 404 von SEQ ID NO: 1 entspricht, optional wobei die Mutation an einer Position, die der Position 404 von SEQ ID NO: 1 entspricht, eine Substitution mit Lysin, ferner optional H404K ist; oder
f. Die mindestens eine Mutation eine Mutation an einer Position umfasst, die der Position 490 von SEQ ID NO: 1 entspricht, optional wobei die Mutation an einer Position, die der Position 490 von SEQ ID NO: 1 entspricht, eine Substitution mit Lysin, ferner optional H490K ist; oder
g. Die mindestens eine Mutation eine Mutation an einer Position umfasst, die der Position 534 von SEQ ID NO: 1 entspricht, optional wobei die Mutation an einer Position, die der Position 534 der SEQ ID NO: 1 entspricht, eine Substitution mit Asparagin, ferner optional R534N ist.

7. Modifiziertes GCase-Polypeptid nach einem der vorhergehenden Ansprüche, wobei:
a. Die mindestens eine Mutation umfasst:
(i) eine Mutation an einer Position, die der Position 482 von SEQ ID NO: 1 entspricht,
(ii) eine Mutation an einer Position, die der Position 503 von SEQ ID NO: 1 entspricht,
wobei optional
(i) die Mutation an einer Position, die der Position 482 von SEQ ID NO: 1 entspricht, eine Substitution mit Cystein, optional eine Mutation von Asparaginsäurej zu Cystein ist; und
(ii) die Mutation an einer Position, die der Position 503 der SEQ ID NO: 1 entspricht, eine Substitution mit Cystein, optional eine Mutation von Serin zu Cystein ist; oder
b. Die mindestens eine Mutation umfasst:
(i) eine Mutation an einer Position, die der Position 494 von SEQ ID NO: 1 entspricht; und
(ii) eine Mutation an einer Position, die der Position 534 von SEQ ID NO: 1 entspricht, 1.
wobei optional
(i) die Mutation an einer Position, die der Position 494 von SEQ ID NO: 1 entspricht, eine Substitution mit Cystein, optional eine Mutation von Serin zu Cystein ist; und
(ii) die Mutation an einer Position, die der Position 534 von SEQ ID NO: 1 entspricht, eine Substitution mit Cystein, optional eine Mutation von Arginin zu Cystein ist; oder
c.
(i) die Mutation an einer Position, die der Position 351 von SEQ ID NO: 1 entspricht, eine Mutation von Tryptophan zu Cystein ist; und
(ii) die Mutation an einer Position, die der Position 380 der SEQ ID NO: 1 entspricht, eine Mutation von Alanin zu Cystein ist; oder
d. Die mindestens eine Mutation umfasst:
(i) eine Mutation an einer Position, die der Position 407 von SEQ ID NO: 1 entspricht; und
(ii) eine Mutation an einer Position, die der Position 484 von SEQ ID NO: 1 entspricht,
wobei optional
(i) die Mutation an einer Position, die der Position 407 von SEQ ID NO: 1 entspricht, eine Substitution mit Cystein, optional eine Mutation von Isoleucin zu Cystein ist; und
(ii) die Mutation an einer Position, die der Position 484 der SEQ ID NO: 1 entspricht, eine Substitution mit Cystein, optional eine Mutation von Asparaginsäure zu Cystein, ist; oder
e. Die mindestens eine Mutation umfasst:
(i) eine Substitution mit Glutamin an einer Position, die der Position 272 von SEQ ID NO: 1 entspricht, optional E272Q; und
(ii) eine Mutation von Tryptophan zu Cystein an einer Position, die der Position 351 von SEQ ID NO: 1 entspricht; und
(iii) eine Mutation von Alanin zu Cystein an einer Position, die der Position 380 der SEQ ID NO: 1 entspricht.

8. Modifiziertes GCase-Polypeptid nach einem der vorhergehenden Ansprüche,
wobei:
a. Die mindestens eine Mutation eine Substitution mit Glutamin an einer Position umfasst, die der Position 272 von SEQ ID NO: 1 entspricht, und wobei das modifizierte GCase-Polypeptid eine erhöhte Stabilität im Vergleich zu einem Referenz-GCase-Polypeptid aufweist, wobei es mindestens 85% Aktivität beibehält, wenn es nach 120 Minuten Inkubation bei pH 7,4 und 37 Grad Celsius gemessen wird, wobei das Referenz-GCase-Polypeptid das Polypeptid der SEQ ID NO: 3 ist; oder
b. Die mindestens eine Mutation umfasst:
(i) eine Mutation von Tryptophan zu Cystein an einer Position, die der Position 351 von SEQ ID NO: 1 entspricht; und
(ii) eine Mutation von Alanin zu Cystein an einer Position, die der Position 380 der SEQ ID NO: 1 entspricht;
und wobei das modifizierte GCase-Polypeptid eine erhöhte Stabilität im Vergleich zu einem Referenz-GCase-Polypeptid aufweist, wobei es mindestens 85% Aktivität beibehält, wenn es nach 120 Minuten Inkubation bei pH 7,4 und 37 Grad Celsius gemessen wird, wobei das Referenz-GCase-Polypeptid das Polypeptid der SEQ ID NO: 3 ist; oder
c. Die mindestens eine Mutation umfasst:
(i) eine Substitution mit Glutamin an einer Position, die der Position 272 von SEQ ID NO: 1 entspricht, optional E272Q; und
(ii) eine Mutation von Tryptophan zu Cystein an einer Position, die der Position 351 von SEQ ID NO: 1 entspricht; und
(iii) eine Mutation von Alanin zu Cystein an einer Position, die der Position 380 der SEQ ID NO: 1 entspricht;
und wobei das modifizierte GCase-Polypeptid eine erhöhte Stabilität im Vergleich zu einem Referenz-GCase-Polypeptid aufweist, wobei es mindestens 85% Aktivität beibehält, wenn es nach 120 Minuten Inkubation bei pH 7,4 und 37 Grad Celsius gemessen wird, wobei das Referenz-GCase-Polypeptid das Polypeptid der SEQ ID NO: 3 ist; oder
d. Das modifizierte GCase-Polypeptid eine höhere effektive Aktivität und/oder eine erhöhte Stabilität im Vergleich zu einem Referenz-GCase-Polypeptid hat, wobei das Referenz-GCase-Polypeptid aus einer der SEQ ID NOs: 1 bis 5 ausgewählt ist.

9. Modifiziertes GCase-Polypeptid nach einem der vorhergehenden Ansprüche, wobei:
(i) das modifizierte GCase-Polypeptid eine Aminosäuresequenz umfasst, die zu mindestens 95%, mindestens 98% oder mindestens 99% mit SEQ ID NO: 1 oder SEQ ID NO: 2 identisch ist; oder
(ii) das modifizierte GCase-Polypeptid eine Aminosäuresequenz umfasst, die zu mindestens 98 % mit einem Fragment von 400 bis 536 Aminosäuren von SEQ ID NO: 1 identisch ist; oder
(iii) das modifizierte GCase-Polypeptid eine Aminosäuresequenz umfasst, die zu mindestens 98 % mit einem Fragment von 400 bis 497 Aminosäuren der SEQ ID NO: 2 identisch ist; oder
(iv) das modifizierte GCase-Polypeptid eine Aminosäuresequenz umfasst, die zu mindestens 98 % mit SEQ ID NO: 1 identisch ist; oder
(v) das modifizierte GCase-Polypeptid eine Aminosäuresequenz umfasst, die zu mindestens 98 % mit SEQ ID NO: 2 identisch ist; oder
(vi) das modifizierte GCase-Polypeptid eine Aminosäuresequenz umfasst, die mit SEQ ID NO: 1 oder SEQ ID NO: 2 identisch ist, mit der Ausnahme, dass das modifizierte GCase-Polypeptid die mindestens eine in einem der vorhergehenden Ansprüche definierte Mutation aufweist; oder
(vii) das modifizierte GCase-Polypeptid eine Aminosäuresequenz umfasst, die mit SEQ ID NO: 1 oder SEQ ID NO: 2 identisch ist, mit der Ausnahme, dass das modifizierte mit der Ausnahme, dass das modifizierte GCase-Polypeptid eine Mutation an einer Position umfasst, die der Position 272 von SEQ ID NO: 1 entspricht; oder
(viii) das modifizierte GCase-Polypeptid eine Aminosäuresequenz umfasst, die mit SEQ ID NO: 1 oder SEQ ID NO: 2 identisch ist, mit der Ausnahme, dass das modifizierte GCase-Polypeptid eine Substitution mit Cystein an einer Position, die der Position 351 der SEQ ID NO: 1 entspricht, und an einer Position, die der Position 380 der SEQ ID NO: 1 entspricht, umfasst; oder
(ix) das modifizierte GCase-Polypeptid eine Aminosäuresequenz umfasst, die mit SEQ ID NO: 1 oder SEQ ID NO: 2 identisch ist, mit der Ausnahme, dass das modifizierte GCase-Polypeptid eine Substitution mit Cystein an einer Position, die der Position 351 der SEQ ID NO: 1 entspricht, und an einer Position, die der Position 380 der SEQ ID NO: 1 entspricht, umfasst; oder das modifizierte GCase-Polypeptid eine Mutation an einer Position umfasst, die der Position 272 von SEQ ID NO: 1 entspricht.

10. Polynukleotid, das eine modifizierte Glukozerebrosidase- (GBA) Nukleotidsequenz umfasst, wobei die modifizierte GBA-Nukleotidsequenz für das modifizierte GCase-Polypeptid nach einem der vorhergehenden Ansprüche codiert.

11. Polynukleotid nach Anspruch 10, wobei:
(i) die modifizierte GBA-Nukleotidsequenz eine Sequenz umfasst, die mindestens 80%, mindestens 90%, mindestens 95%, mindestens 98%, mindestens 99%, mindestens 99,5%, mindestens 99,8% oder 100% identisch ist mit einem Fragment von mindestens 750, mindestens 850, mindestens 950, mindestens 1000, mindestens 1200, mindestens 1400 oder mindestens 1494 Nukleotiden einer der SEQ ID NOs: 14 bis 29; oder
(ii) die modifizierte GBA-Nukleotidsequenz eine Sequenz umfasst, die zu mindestens 80 %, mindestens 90 %, mindestens 95 %, mindestens 98 %, mindestens 99 %, mindestens 99,5 %, mindestens 99,8 % oder 100 % mit einer Nukleotidsequenz von SEQ ID NO: 14 oder SEQ ID NO: 18 identisch ist; oder
(iii) die modifizierte GBA-Nukleotidsequenz eine Sequenz umfasst, die zu mindestens 80 %, mindestens 90 %, mindestens 95 %, mindestens 98 %, mindestens 99 %, mindestens 99,5 %, mindestens 99,8 % oder 100 % mit einer Nukleotidsequenz von SEQ ID NO: 22 oder SEQ ID NO: 26 identisch ist.

12. Polynukleotid nach Anspruch 10 oder 11, wobei die modifizierte GBA-Nukleotidsequenz eine Sequenz umfasst, die zu mindestens 90 %, mindestens 95 %, mindestens 98 %, mindestens 99 %, mindestens 99,5 %, mindestens 99,8 % oder 100 % mit einer Nukleotidsequenz der SEQ ID NO: 14 oder SEQ ID NO: 18 identisch ist.

13. Virales Partikel, ein rekombinantes Genom umfasst, das das Polynukleotid nach einem der Ansprüche 10 bis 12 umfasst.

14. Virales Partikel nach Anspruch 13:
(i) das ein AAV-, adenovirales oder lentivirales virales Partikel ist, optional wobei das virale Partikel ein AAV-virales Partikel ist; oder
(ii) wobei das virale Partikel ein lebertropes oder ZNS-tropes Kapsid umfasst, optional wobei das lebertrope Kapsid eine Sequenz umfasst, die zu mindestens 98%, mindestens 99% oder mindestens 99,5% identisch ist mit einem Fragment von mindestens 600, mindestens 650, mindestens 700, zwischen 600 und 736, zwischen 650 und 736 oder zwischen 700 und 736 Aminosäuren der SEQ ID NO: 36, 37 oder 38; oder
(iii) wobei das virale Partikel ferner umfasst:
a) AAV2 ITRs;
b) eine polyA-Sequenz; und/oder
c) ein Intron; oder
(iv) wobei das rekombinante Genom einzelsträngig ist; oder
(v) wobei die wirksame Aktivität des modifizierten GCase-Polypeptids mindestens 50 %, mindestens 60 %, mindestens 70 %, mindestens 80 %, mindestens 90 %, mindestens 95 % oder mindestens 100 % der wirksamen Aktivität des GCase-Polypeptids beträgt, das durch die Sequenz von SEQ ID NO: 14 oder 18 codiert wird.

15. Virales Partikel nach Anspruch 13 oder 14, wobei:
(i) das virale Partikel ein AAV-Viruspartikel ist und das virale Partikel ein ZNS-tropisches Capsid umfasst;
(ii) das Polynukleotid ferner ein Transkriptionsregulationselement umfasst;
(iii) die modifizierte GBA-Nukleotidsequenz kodon-optimiert ist und/oder die modifizierte GBA-Nukleotidsequenz eine Sequenz umfasst, die zu mindestens 90 %, mindestens 95 %, mindestens 98 %, mindestens 99 %, mindestens 99,5 %, mindestens 99,8 % oder 100 % mit einer Nukleotidsequenz der SEQ ID NO: 14 oder SEQ ID NO: 18 identisch ist; und
(iv) das modifizierte GCase-Polypeptid eine Aminosäuresequenz umfasst, die mit SEQ ID NO: 1 oder SEQ ID NO: 2 identisch ist, mit der Ausnahme, dass das modifizierte GCase-Polypeptid eine Substitution mit Cystein an einer Position, die der Position 351 der SEQ ID NO: 1 entspricht, und an einer Position, die der Position 380 der SEQ ID NO: 1 entspricht, umfasst.

16. Virales Partikel nach Anspruch 13 oder 14, wobei:
(i) das virale Partikel ein AAV-Viruspartikel ist und das virale Partikel ein lebertropes Kapsid umfasst, optional wobei das lebertrope Kapsid eine Sequenz umfasst, die zu 100% mit SEQ ID NO: 37 identisch ist;
(ii) das Polynukleotid ferner ein transkriptionsregulatorisches Element umfasst, wobei (a) das transkriptionsregulatorische Element einen leberspezifischen Promotor umfasst und/oder (b) das transkriptionsregulatorische Element eine Sequenz umfasst, die zu 100% identisch mit SEQ ID NO: 35 ist; und
(iii) die modifizierte GBA-Nukleotidsequenz kodon-optimiert ist und/oder die modifizierte GBA-Nukleotidsequenz eine Sequenz umfasst, die zu 100% mit einer Nukleotidsequenz der SEQ ID NO: 14 oder SEQ ID NO: 18 identisch ist.

17. Zusammensetzung, die das modifizierte GCase-Polypeptid, Polynukleotid oder virale Partikel nach einem der vorhergehenden Ansprüche und einen pharmazeutisch akzeptablen Hilfsstoff enthält.

18. Modifiziertes GCase-Polypeptid, Polynukleotid, virales Partikel oder Zusammensetzung nach einem der vorhergehenden Ansprüche zur Verwendung in einem Behandlungsverfahren, wobei:
(i) das Behandlungsverfahren ein Verfahren zur Behandlung einer Synucleinopathie ist; oder
(ii) das Behandlungsverfahren ein Verfahren zur Behandlung der Parkinson-Krankheit ist; oder
(iii) das Behandlungsverfahren ein Verfahren zur Behandlung der Gaucher-Krankheit ist, optional wobei die Gaucher-Krankheit Gaucher-Krankheit Typ I, II oder III ist; oder
(iv) das Behandlungsverfahren ein Verfahren zur Behandlung der Gaucher-Krankheit ist und wobei der Patient Antikörper oder Inhibitoren gegen eine rekombinante GCase hat, mit der der Patient zuvor als Teil einer Enzymersatztherapie behandelt worden ist, optional wobei die Gaucher-Krankheit die Gaucher-Krankheit Typ I, II oder III ist.

## Revendications

1. Polypeptide de β-Glucocérébrosidase (GCase) modifiée qui comprend au moins une mutation, dans lequel l'au moins une mutation comprend :
(i) une mutation à une position correspondant à la position 351 de SEQ ID n° : 1 qui est une substitution avec cystéine ; et
(ii) une mutation à une position correspondant à la position 380 de SEQ ID n° : 1 qui est une substitution avec cystéine,
dans lequel le polypeptide de GCase modifiée comprend une séquence d'acides aminés qui est au moins 90 % identique à SEQ ID n° : 2, et
dans lequel le polypeptide de GCase modifiée a une stabilité accrue et a une activité efficace plus élevée relativement à un polypeptide de GCase de référence, dans lequel le polypeptide de GCase de référence est le polypeptide de SEQ ID n° : 3, 4, ou 5.

2. Polypeptide de GCase modifiée de la revendication 1, dans lequel l'activité efficace du polypeptide de GCase modifiée est au moins 1,2 fois, au moins 1,5 fois, au moins 2 fois, au moins 2,5 fois, au moins 3 fois, au moins 3,5 fois, au moins 4 fois, au moins 4,5 fois, au moins 5 fois, au moins 5,5, fois, au moins 6 fois, au moins 6,5 fois, au moins 7 fois, au moins 7,5 fois, au moins 8 fois, au moins 10 fois, au moins 15 fois, au moins 20 fois, au moins 35 fois, au moins 40 fois, au moins 45 fois, ou au moins 50 fois plus élevée que l'activité efficace du polypeptide de GCase de référence.

3. Polypeptide de GCase modifiée de la revendication 1 ou 2, dans lequel :
(i) le polypeptide de GCase modifiée est plus structuralement stable à un pH physiologique relativement à un polypeptide de GCase de référence, dans lequel le polypeptide de GCase de référence est le polypeptide de SEQ ID n° : 3, 4, ou 5, facultativement dans lequel le pH est pH 7,4 ; ou
(ii) la stabilité accrue est une stabilité accrue à pH 7,4 ; ou
(iii) le polypeptide de GCase modifiée maintient au moins 30 %, au moins 35 %, au moins 40 %, au moins 45 %, au moins 50 %, au moins 55 %, au moins 60 %, au moins 65 %, au moins 70 %, au moins 75 %, au moins 80 %, ou au moins 85 % d'activité par rapport à l'activité initiale lorsqu'elle est mesurée après au moins 10 minutes, au moins 30 minutes, au moins 60 minutes, au moins 120 minutes, au moins 1 jour, au moins 2 jours, au moins 3 jours, au moins 4 jours, au moins 5 jours, au moins 6 jours, ou au moins 7 jours d'incubation à un pH 7,4 et à 37 degrés Celsius ; ou
(iv) le polypeptide de GCase modifiée maintient une activité qui est au moins 1,1 fois, au moins 1,2 fois, au moins 1,3 fois, au moins 1,4 fois, au moins 1,5 fois, au moins 1,8 fois, au moins 2 fois, au moins 2,5 fois, au moins 2,8 fois, ou au moins 3 fois plus élevée que l'activité d'un polypeptide de GCase de référence lorsqu'elle est mesurée après 120 minutes d'incubation à un pH 7,4 et à 37 degrés Celsius, dans lequel le polypeptide de GCase de référence est le polypeptide de SEQ ID n° : 3, 4, ou 5.

4. Polypeptide de GCase modifiée de l'une quelconque des revendications précédentes, dans lequel :
(i) le polypeptide de GCase modifiée maintient au moins 70 %, au moins 75 %, au moins 80 %, ou au moins 85 % d'activité par rapport à l'activité initiale lorsqu'elle est mesurée après 120 mins d'incubation à un pH 7,4 et à 37 degrés Celsius ; ou
(ii) le polypeptide de GCase modifiée maintient au moins 40 %, au moins 45 %, au moins 50 %, au moins 55 %, ou au moins 60 % d'activité par rapport à l'activité initiale lorsqu'elle est mesurée après 72 heures d'incubation à un pH 7,4 et à 37 degrés Celsius, facultativement dans lequel l'incubation est dans PBS ; ou
(iii) le polypeptide de GCase modifiée maintient au moins 15 %, ou au moins 20 % d'activité par rapport à l'activité initiale lorsqu'elle est mesurée après 7 jours d'incubation à un pH 7,4 et à 37 degrés Celsius, facultativement dans lequel l'incubation est dans du sérum ou du plasma ; ou
(iv) le polypeptide de GCase modifiée maintient au moins 40 % d'activité par rapport à l'activité initiale lorsqu'elle est mesurée après 7 jours d'incubation à un pH 7,4 et à 37 degrés Celsius, facultativement dans lequel l'incubation est dans du sérum ou du plasma ; ou
(v) le polypeptide de GCase modifiée maintient au moins 60 % d'activité par rapport à l'activité initiale lorsqu'elle est mesurée après 7 jours d'incubation à un pH 7,4 et à 37 degrés Celsius ; ou
(vi) le polypeptide de GCase modifiée maintient au moins 80 % d'activité par rapport à l'activité initiale lorsqu'elle est mesurée après 7 jours d'incubation à un pH 7,4 et à 37 degrés Celsius ; ou
(vii) l'activité, l'activité efficace, et/ou la stabilité est déterminée en utilisant un dosage fluorimétrique.

5. Polypeptide de GCase modifiée de l'une quelconque des revendications précédentes ayant une demi-vie plus longue relativement à un polypeptide de GCase de référence, dans lequel le polypeptide de GCase de référence est le polypeptide de SEQ ID n° : 3, 4, ou 5, facultativement dans lequel le polypeptide de GCase modifiée a une demi-vie d'au moins 1,2 fois, d'au moins 1,5 fois, d'au moins 2 fois, d'au moins 3 fois, d'au moins 4 fois, d'au moins 5 fois, ou d'au moins 10 fois plus longue que la demi-vie du polypeptide de GCase de référence, en outre facultativement dans lequel :
a. la demi-vie plus longue est une demi-vie plus longue à un pH 7,4 ou un pH 5,6 ; ou
b. le polypeptide de GCase modifiée a une demi-vie à un pH 5,6 d'au moins 20 fois plus longue que la demi-vie du polypeptide de GCase de référence ; ou
c. la demi-vie plus longue est une demi-vie plus longue dans du sérum ou du plasma ; ou
d. la demi-vie est déterminée en utilisant un dosage fluorimétrique.

6. Polypeptide de GCase modifiée de l'une quelconque des revendications précédentes, dans lequel :
a. l'au moins une mutation comprend une mutation à une position correspondant à la position 272 de SEQ ID n° : 1, facultativement dans lequel la mutation à une position correspondant à la position 272 de SEQ ID n° : 1 est une substitution avec glutamine, en outre facultativement E272Q ; ou
b. l'au moins une mutation comprend une mutation à une position correspondant à la position 262 de SEQ ID n° : 1, facultativement dans lequel la mutation à une position correspondant à la position 262 de SEQ ID n° : 1 est une substitution avec asparagine ou tyrosine ; ou
c. l'au moins une mutation comprend une mutation à une position correspondant à la position 262 de SEQ ID n° : 1, dans lequel la mutation à une position correspondant à la position 262 de SEQ ID n° : 1 est une substitution avec asparagine, facultativement H262N ; ou
d. l'au moins une mutation comprend une mutation à une position correspondant à la position 313 de SEQ ID n° : 1, facultativement dans lequel la mutation à une position correspondant à la position 313 de SEQ ID n° : 1 est une substitution avec asparagine, en outre facultativement H313N ; ou
e. l'au moins une mutation comprend une mutation à une position correspondant à la position 404 de SEQ ID n° : 1, facultativement dans lequel la mutation à une position correspondant à la position 404 de SEQ ID n° : 1 est une substitution avec lysine, en outre facultativement H404K ; ou
f. l'au moins une mutation comprend une mutation à une position correspondant à la position 490 de SEQ ID n° : 1, facultativement dans lequel la mutation à une position correspondant à la position 490 de SEQ ID n° : 1 est une substitution avec lysine, en outre facultativement H490K ; ou
g. l'au moins une mutation comprend une mutation à une position correspondant à la position 534 de SEQ ID n° : 1, facultativement dans lequel la mutation à une position correspondant à la position 534 de SEQ ID n° : 1 est une substitution avec asparagine, en outre facultativement R534N.

7. Polypeptide de GCase modifiée de l'une quelconque des revendications précédentes, dans lequel :
a. l'au moins une mutation comprend :
(i) une mutation à une position correspondant à la position 482 de SEQ ID n° : 1 ; et
(ii) une mutation à une position correspondant à la position 503 de SEQ ID n° : 1,
facultativement dans lequel
(i) la mutation à une position correspondant à la position 482 de SEQ ID n° : 1 est une substitution avec cystéine, facultativement une mutation acide aspartique-cystéine ; et
(ii) la mutation à une position correspondant à la position 503 de SEQ ID n° : 1 est une substitution avec cystéine, facultativement une mutation sérine-cystéine ; ou
b. l'au moins une mutation comprend :
(i) une mutation à une position correspondant à la position 494 de SEQ ID n° : 1 ; et
(ii) une mutation à une position correspondant à la position 534 de SEQ ID n° : 1,
facultativement dans lequel
(i) la mutation à une position correspondant à la position 494 de SEQ ID n° : 1 est une substitution avec cystéine, facultativement une mutation sérine-cystéine ; et
(ii) la mutation à une position correspondant à la position 534 de SEQ ID n° : 1 est une substitution avec cystéine, facultativement une mutation arginine-cystéine ; ou
c.
(i) la mutation à une position correspondant à la position 351 de SEQ ID n° : 1 est une mutation tryptophane-cystéine ; et
(ii) la mutation à une position correspondant à la position 380 de SEQ ID n° : 1 est une mutation alanine-cystéine ; ou
d. l'au moins une mutation comprend :
(i) une mutation à une position correspondant à la position 407 de SEQ ID n° : 1 ; et
(ii) une mutation à une position correspondant à la position 484 de SEQ ID n° : 1,
facultativement dans lequel
(i) la mutation à une position correspondant à la position 407 de SEQ ID n° : 1 est une substitution avec cystéine, facultativement an isoleucine to cystéine mutation ; et
(ii) la mutation à une position correspondant à la position 484 de SEQ ID n° : 1 est une substitution avec cystéine, facultativement une mutation acide aspartique-cystéine ; ou
e. l'au moins une mutation comprend :
(i) une substitution avec glutamine à une position correspondant à la position 272 de SEQ ID n° : 1, facultativement E272Q ; et
(ii) une mutation tryptophane-cystéine à une position correspondant à la position 351 de SEQ ID n° : 1 ; et
(iii) une mutation alanine-cystéine à une position correspondant à la position 380 de SEQ ID n° : 1.

8. Polypeptide de GCase modifiée de l'une quelconque des revendications précédentes, dans lequel :
a. l'au moins une mutation comprend une substitution avec glutamine à une position correspondant à la position 272 de SEQ ID n° : 1, et dans lequel le polypeptide de GCase modifiée a une stabilité accrue par rapport à un polypeptide de GCase de référence, maintenant au moins 85 % d'activité lorsqu'elle est mesurée après 120 mins d'incubation à un pH 7,4 et à 37 degrés Celsius, dans lequel le polypeptide de GCase de référence est le polypeptide de SEQ ID n° : 3 ; ou
b. l'au moins une mutation comprend :
(i) une mutation tryptophane-cystéine à une position correspondant à la position 351 de SEQ ID n° : 1 ; et
(ii) une mutation alanine-cystéine à une position correspondant à la position 380 de SEQ ID n° :1 ;
et dans lequel le polypeptide de GCase modifiée a une stabilité accrue par rapport à un polypeptide de GCase de référence, maintenant au moins 85 % d'activité lorsqu'elle est mesurée après 120 mins d'incubation à un pH 7,4 et à 37 degrés Celsius,
dans lequel le polypeptide de GCase de référence est le polypeptide de SEQ ID n° : 3 ; ou
c. l'au moins une mutation comprend :
(i) une substitution avec glutamine à une position correspondant à la position 272 de SEQ ID n° : 1, facultativement E272Q ; et
(ii) une mutation tryptophane-cystéine à une position correspondant à la position 351 de SEQ ID n° : 1 ; et
(iii) une mutation alanine-cystéine à une position correspondant à la position 380 de SEQ ID n° :1 ;
et dans lequel le polypeptide de GCase modifiée a une stabilité accrue par rapport à un polypeptide de GCase de référence, maintenant au moins 85 % d'activité lorsqu'elle est mesurée après 120 mins d'incubation à un pH 7,4 et à 37 degrés Celsius, dans lequel le polypeptide de GCase de référence est le polypeptide de SEQ ID n° : 3 ; ou
d. le polypeptide de GCase modifiée a une activité efficace plus élevée et/ou une stabilité accrue par rapport à un polypeptide de GCase de référence, dans lequel le polypeptide de GCase de référence est sélectionné parmi l'un quelconque de SEQ ID n° : 1 à 5.

9. Polypeptide de GCase modifiée de l'une quelconque des revendications précédentes, dans lequel :
(i) le polypeptide de GCase modifiée comprend une séquence d'acides aminés au moins 95 %, au moins 98 %, ou au moins 99 % identique à SEQ ID n° : 1 ou à SEQ ID n° : 2 ; ou
(ii) le polypeptide de GCase modifiée comprend une séquence d'acides aminés au moins 98 % identique à un fragment d'entre 400 et 536 acides aminés de SEQ ID n° : 1 ; ou
(iii) le polypeptide de GCase modifiée comprend une séquence d'acides aminés au moins 98 % identique à un fragment d'entre 400 et 497 acides aminés de SEQ ID n° : 2 ; ou
(iv) le polypeptide de GCase modifiée comprend une séquence d'acides aminés au moins 98 % identique à SEQ ID n° : 1 ; ou
(v) le polypeptide de GCase modifiée comprend une séquence d'acides aminés au moins 98 % identique à SEQ ID n° : 2 ; ou
(vi) le polypeptide de GCase modifiée comprend une séquence d'acides aminés qui est identique à SEQ ID n° : 1 ou SEQ ID n° : 2, à l'exception que le polypeptide de GCase modifiée comprend l'au moins une mutation définie dans l'une quelconque des revendications précédentes ; ou
(vii) le polypeptide de GCase modifiée comprend une séquence d'acides aminés qui est identique à SEQ ID n° : 1 ou à SEQ ID n° : 2, à l'exception que le polypeptide de GCase modifiée comprend une mutation à une position correspondant à la position 272 de SEQ ID n° : 1 ; ou
(viii) le polypeptide de GCase modifiée comprend une séquence d'acides aminés qui est identique à SEQ ID n° : 1 ou à SEQ ID n° : 2, à l'exception que le polypeptide de GCase modifiée comprend une substitution avec cystéine à une position correspondant à la position 351 de SEQ ID n° : 1 et à une position correspondant à la position 380 de SEQ ID n° : 1 ; ou
(ix) le polypeptide de GCase modifiée comprend une séquence d'acides aminés qui est identique à SEQ ID n° : 1 ou à SEQ ID n° : 2, à l'exception que le polypeptide de GCase modifiée comprend une substitution avec cystéine à une position correspondant à la position 351 de SEQ ID n° : 1 et à une position correspondant à la position 380 de SEQ ID n° : 1, et le polypeptide de GCase modifiée comprend une mutation à une position correspondant à la position 272 de SEQ ID n° : 1.

10. Polynucléotide comprenant une séquence nucléotidique de glucocérébrosidase (GBA) modifiée, dans lequel la séquence nucléotidique de GBA modifiée encode le polypeptide de GCase modifiée de l'une quelconque des revendications précédentes.

11. Polynucléotide de la revendication 10, dans lequel :
(i) la séquence nucléotidique de GBA modifiée comprend une séquence qui est au moins 80 %, au moins 90 %, au moins 95 %, au moins 98 %, au moins 99 %, au moins 99,5 %, au moins 99,8 %, ou 100 % identique à un fragment d'au moins 750, au moins 850, d'au moins 950, d'au moins 1 000, d'au moins 1 200, d'au moins 1 400, ou d'au moins 1 494 nucléotides d'un quelconque de SEQ ID n° : 14 à 29 ; ou
(ii) la séquence nucléotidique de GBA modifiée comprend une séquence qui est au moins 80 %, au moins 90 %, au moins 95 %, au moins 98 %, au moins 99 %, au moins 99,5 %, au moins 99,8 %, ou 100 % identique à une séquence nucléotidique de SEQ ID n° : 14 ou de SEQ ID n° : 18 ; ou
(iii) la séquence nucléotidique de GBA modifiée comprend une séquence qui est au moins 80 %, au moins 90 %, au moins 95 %, au moins 98 %, au moins 99 %, au moins 99,5 %, au moins 99,8 %, ou 100 % identique à une séquence nucléotidique de SEQ ID n° : 22 ou de SEQ ID n° : 26.

12. Polynucléotide de la revendication 10 ou 11, dans lequel la séquence nucléotidique de GBA modifiée comprend une séquence qui est au moins 90 %, au moins 95 %, au moins 98 %, au moins 99 %, au moins 99,5 %, au moins 99,8 %, ou 100 % identique à une séquence nucléotidique de SEQ ID n° : 14 ou de SEQ ID n° : 18.

13. Particule virale, comprenant un génome recombinant comprenant le polynucléotide de l'une quelconque des revendications 10 à 12.

14. Particule virale de la revendication 13 :
(i) qui est une particule virale AAV, adénovirale, ou lentivirale, facultativement dans laquelle la particule virale est une particule virale AAV ; ou
(ii) dans laquelle la particule virale comprend un capside hépatotrope ou CNS-trope, facultativement dans laquelle le capside hépatotrope comprend une séquence au moins 98 %, au moins 99 %, ou au moins 99.5 % identique à un fragment d'au moins 600, d'au moins 650, d'au moins 700, d'entre 600 et 736, d'entre 650 et 736, ou d'entre 700 et 736 acides aminés de SEQ ID n° : 36, 37 ou 38 ; ou
(iii) dans laquelle la particule virale comprend en outre :
a) des ITRs AAV2 ;
b) une séquence polyA ; et/ou
c) un intron ; ou
(iv) dans laquelle le génome recombinant est monobrin ; ou
(v) dans laquelle l'activité efficace du polypeptide de GCase modifiée est au moins 50 %, au moins 60 %, au moins 70 %, au moins 80 %, au moins 90 %, au moins 95 %, ou au moins 100 % de l'activité efficace du polypeptide de GCase encodé par la séquence de SEQ ID n° : 14 ou 18.

15. Particule virale de la revendication 13 ou 14, dans laquelle :
(i) la particule virale est une particule virale AAV, et la particule virale comprend un capside CNS-trope ;
(ii) le polynucléotide comprend en outre un élément régulateur de transcription ;
(iii) la séquence nucléotidique de GBA modifiée est optimisée par codon et/ou la séquence nucléotidique de GBA modifiée comprend une séquence qui est au moins 90 %, au moins 95 %, au moins 98 %, au moins 99 %, au moins 99,5 %, au moins 99,8 %, ou 100 % identique à une séquence nucléotidique de SEQ ID n° : 14 ou SEQ ID n° : 18 ; et
(iv) le polypeptide de GCase modifiée comprend une séquence d'acides aminés qui est identique à SEQ ID n° : 1 ou à SEQ ID n° : 2, à l'exception que le polypeptide de GCase modifiée comprend une substitution avec cystéine à une position correspondant à la position 351 de SEQ ID n° : 1 et à une position correspondant à la position 380 de SEQ ID n° : 1.

16. Particule virale de la revendication 13 ou 14, dans laquelle :
(i) la particule virale est une particule virale AAV, et la particule virale comprend un capside hépatotrope, facultativement dans laquelle le capside hépatotrope comprend une séquence 100 % identique à SEQ ID n° : 37 ;
(ii) le polynucléotide comprend en outre un élément régulateur de transcription,
dans laquelle (a) l'élément régulateur de transcription comprend un promoteur hépatospécifique et/ou (b) l'élément régulateur de transcription comprend une séquence qui est 100 % identique à SEQ ID n° : 35 ; et
(iii) la séquence nucléotidique de GBA modifiée est optimisée par codon et/ou la séquence nucléotidique de GBA modifiée comprend une séquence qui est 100 % identique à une séquence nucléotidique de SEQ ID n° : 14 ou SEQ ID n° : 18.

17. Composition comprenant le polypeptide de GCase modifiée, polynucléotide, ou particule virale de l'une quelconque des revendications précédentes et un excipient pharmaceutiquement acceptable.

18. Polypeptide de GCase modifiée, polynucléotide, particule virale ou composition de l'une quelconque des revendications précédentes pour utilisation dans une méthode de traitement, dans lequel ou laquelle :
(i) la méthode de traitement est une méthode de traitement d'une synucléinopathie ; ou
(ii) la méthode de traitement est une méthode de traitement de maladie de Parkinson ; ou
(iii) la méthode de traitement est une méthode de traitement de maladie de Gaucher, facultativement dans lequel ou laquelle la maladie de Gaucher est une maladie de Gaucher de type I, II ou III ; ou
(iv) la méthode de traitement est une méthode de traitement de maladie de Gaucher et dans lequel ou laquelle le patient a des anticorps ou inhibiteurs de GCase recombinante avec lesquels le patient a auparavant été traité en tant que partie d'une thérapie de remplacement enzymatique, facultativement dans lequel ou laquelle la maladie de Gaucher est une maladie de Gaucher de type I, II ou III.
